# EUROPEAN PATENT APPLICATION

(11) **EP 4 174 175 A1**
(43) Date of publication of application: **03.05.2023**
(21) Application number: 21306488.4
(22) Date of filing: 26.10.2021
(51) Int. Cl.: C12N 9/04, C12N 9/06, C12N 9/10, C12N 9/12, C12N 9/24, C12N 9/90, C12N 9/00, C12P 19/26

(54) **HYALURONIC ACID-PRODUCING RECOMBINANT CELLS**

(71) Applicant: Givaudan SA, 1214 Vernier (CH)
(72) Inventor: The designation of the inventor has not yet been filed
(74) Representative: Cabinet Nony

(57) **Abstract**

The present invention relates to the field of bio-production of hyaluronic acid.

There is a need in the art for hyaluronic acid production methods allowing its highly efficient synthesis and secretion.

The solution proposed in the present invention is the use of a genetically modified cell comprising many modifications as described in the present text.

The present invention further proposes methods allowing the bio-production of hyaluronic acid having a controlled molecular weight using the genetically modified cells of the invention.

## Description

### FIELD OF THE INVENTION

The present invention relates to the field of bio-production of hyaluronic acid.

### BACKGROUND OF THE INVENTION

Hyaluronic acid, also known as hyaluronan or HA, is a naturally ocurring high molecular weight polysaccharide composed of D-glucuronic acid and N-acetyl-D-glucosamine, linked via alternating β-(1→4) and β-(1→3) glycosidic bonds and having the chemical formula (C₁₄H₂₁NO₁₁)n. Hyaluronic acid can be 25,000 disaccharide repeats in length. Polymers of hyaluronic acid can range in size from 5,000 to 20,000,000 Da *in vivo.*

HA has many applications in the medical and cosmetic fields including scaffolding for tissue engineering, dermatological fillers, and viscosupplementation for osteoarthritis treatment. In particular, a reduction in HA mass or molecular weight via degradation or slowing of synthesis affects physical and chemical properties such as tissue volume, viscosity, and elasticity. There is therefore a constant need for HA production.

Today's main known sources of HA are human ombilical cords, rooster combs and fermentation of certain microorganisms.

Fermentation methods for preparing HA from microorganisms are particularly of interest since they facilitate the production of a large quantity of HA through possible scaleup, at a reduced cost. Contrary to isolating HA from animal sources which provide hyaluronic acid of very high molecular weights, microbial fermentation allows to control, to a certain extent, the size of the starting molecular weight. This avoids the need for further fractionation steps by mechanical, physical or chemical means.

In this aim, bacterial cultures such as group A and C hemolytic streptococci have been shown to represent good sources of HA (US5316926, US4801539, JP2009011315). However, such bacteria, and in particular *Streptococcus zooepidemicus* which is mainly used in the art, is not generally recognized as safe. Recombinant *Bacillus* host cells have also shown the ability to produce HA in the range of 20 to 800 KDa (US2008038780). It has also been demonstrated in US2006168690 that transforming plant cells to include a DNA encoding hyaluronic acid synthase successfully allowed for the production of HA. Finally, production of small molecules of HA has also been demonstrated in yeasts such as in a recombinant *Pichia pastoris* in CN104263666.

Contrary to other microorganisms that are commonly used for the production of biological molecules, yeasts are generally recognised as safe. They can grow rapidly and be cultivated at higher density, compared to bacteria, and do not require an aseptic environment. Furthermore, yeast cells can be more easily separated from the culture medium than bacteria, greatly simplifying the process for product extraction and purification. Finally, yeasts present the advantage of being more resistant to pH changes in the culture medium, and as such represent stronger fermentation systems.

However, among yeasts, distinguishing features between species may also present certain challenges. This is mainly due to the difference in metabolisms between the species. For example, genome integrations in *P. pastoris* are stable but often a high variability of clones from one transformation is encountered, displaying various productivity characteristics or changes in their physiology. This requires a time-consuming screening process so as to find the clone with the optimal features for the desired application. This clonal variability is an important drawback for the further development of *P. pastoris* as a platform for producing value-added chemicals. Another drawback of using *P. pastoris* is that it is a methanotrophic organism.

On the contrary, *Saccharomyces cerevisiae* is particularly useful as a tool for the production of molecules of interest since it has a long safe history when it comes to being used by humans (such as in wine, beer, or bread) and as such is a well-established model whose genetic information is well known in the art. *S. cerevisiae* further presents the advantage of being generally recognised as safe for humans and animals. What's more, the acidification of the medium which occurs when cultivating this yeast reduces the possibility of contamination of the bio fermenters, and therefore eliminates the need to add antibiotics to the medium. Finally, many genetic tools have been developed allowing for stable modifications of its genome (integration within the chromosome).

Accordingly, there is still a need in the art for further hyaluronic acid production methods allowing its highly efficient synthesis and secretion. In particular, there is still a need to provide production methods that are cost-efficient and supply hyaluronic acid which is safe for human application.

In a particular case, there is still a need in the art for hyaluronic acid methods allowing to obtain large amounts of hyaluronic acid of a particular and controlled size.

### SUMMARY OF THE INVENTION

The present invention accordingly relates to the following items:
**Item 1:** a recombinant yeast cell producing hyaluronic acid (HA) wherein the recombinant cell comprises:
(a) one or more recombinant nucleic acids encoding a polypeptide having hyaluronan synthase activity;
(b) one or more recombinant nucleic acids encoding a polypeptide having UDP-Glucose dehydrogenase (UDP-GlcDH or HASB) activity;
(c) one or more recombinant nucleic acids encoding a polypeptide having hyaluronidase activity wherein the polypeptide having hyaluronidase activity comprises a secretion signal so that hyaluronic acid, in particular of a desired molecular weight (HAMW) is produced by the recombinant yeast cell, and
(d)
   (i) one or more recombinant nucleic acids encoding a polypeptide having a glutamine synthetase (GLN1) activity; and/or
   (ii) one or more disrupted endogeneous nucleic acids encoding a glutamate synthase (GLT1);
wherein said recombinant yeast cell belongs to the *Saccharomyces* genus, or to the *Candida* genus, or to the *Kluyveromyces* genus, or to the *Ogataea* genus, or to the *Yarrowia* genus, or to the *Debaryomyces* genus, or to the *Ashbya* genus.

As illustrated in the examples, the recombinant yeasts of the invention allow for the production of hyaluronic acid in a yeast cell which is not naturally able to produce hyaluronic acid. It is further demonstrated in the examples that the size of the hylaruonic acid produced by the recombinant yeast may be controlled.

Said advantageous properties can be further increased by recombining the yeast with additional modifications described here-after.

**Item** 2: A recombinant host cell producing hyaluronic acid (HA) wherein the recombinant host cell comprises:
(a) one or more recombinant nucleic acids encoding a polypeptide having hyaluronan synthase activity;
(b) one or more recombinant nucleic acids encoding a polypeptide having UDP-Glucose dehydrogenase (UDP-GlcDH or HASB) activity;
(c) one or more recombinant nucleic acids encoding a polypeptide having hyaluronidase activity wherein the polypeptide having hyaluronidase activity comprises a secretion signal and an anchoring signal so that hyaluronic acid, in particular of a desired molecular weight (HAMW) is produced by the host cell; and
(d)
   (i) one or more recombinant nucleic acids encoding a polypeptide having a glutamine synthetase (GLN1) activity; and/or
   (ii) one or more disrupted endogeneous nucleic acids encoding a glutamate synthase (GLT1).

**Item 3:** The recombinant cell according to item 1 or 2, wherein the molecular weight of the HA is in the range of less than 50kDa, preferably in the range of about 20kDa to about 50kDa.

**Item 4:** The recombinant cell according to item 1 or 2, wherein the molecular weight of the HA is in the range of greater than 50kDa, preferably in the range of about 50kDa to about 250kDa.

**Item 5:** The recombinant cell according to item 1 or 2, wherein the molecular weight of the HA is in the range of greater than 100kDa, preferably in the range of about 100kDa to about 1500kDa.

**Item 6:** The recombinant cell according to any one of items 1 to 5, wherein the nucleic acid encoding a polypeptide having a glutamine synthetase activity is obtained or derived from *Saccharomyces cerevisiae.*

**Item 7:** The recombinant cell according to any one of items 1 to 6, wherein the nucleic acid encoding a polypeptide having hyaluronidase activity is obtained or derived from at least one of *Cupiennius salei, Loxosceles intermedia, Hirudo nipponia, Bothrops atrox* or *Tityus serrulatus.*

**Item 8**: The recombinant cell according to any one of items 1 to 7 wherein the nucleic acid encoding a polypeptide having hyaluronan synthase activity is obtained or derived from at least one of *Streptococcus zooepidemicus, Chlorella virus PBCV1, Chlorella virus CviKl, Chlorella virus IL-5-2s1, Chlorella virus CZ-2, Chlorella virus CVG-1, Xenopus laevis* or *Pasteurella multocida,* and is in particular obtained or derived from at least one of *Streptococcus zooepidemicus, Chlorella virus PBCV1, Chlorella virus CviKl, Chlorella virus IL-5-2s1, Chlorella virus CZ-2, Chlorella virus CVG-1,* or *Xenopus laevis.*

**Item 9**: The recombinant cell according to any one of items 1 to 8, wherein the nucleic acid encoding a polypeptide having UDP-Glucose dehydrogenase activity is obtained or derived from at least one of *Arabidopsis thaliana, Chlorella virus PPCV1* or *Streptococcus zooepidemicus,* and in particular from *Arabidopsis thaliana* or *Chlorella virus PBCV1.*

**Item 10**: The recombinant cell according to any one of items 1 to 9, wherein the recombinant cell comprises a recombinant nucleic acid encoding one or more of:
(i) a polypeptide having glutamine-fructose-6-phosphate amidotransferase (GFA1) activity; and/or
(ii) a polypeptide having UDP-N-acetylglucosamine pyrophosphorylase (QRI1) activity.

**Item 11:** The recombinant cell according to any one of items 1 to 10, wherein the recombinant cell comprises at least one recombinant nucleic acid encoding one or more of:
(i) a polypeptide having Phosphoglucomutase-1 (PGM1) activity; and/or
(ii) a polypeptide having UTP-glucose-1-phosphate uridylyltransferase (UGP1) activity; and/or
(iii) a polypeptide having Glucosamine-6-phosphate N-acetyltransferase (GNA1) activity; and/or
(iv) a polypeptide having phosphoacetylglucosamine mutase (PCM1) activity.

**Item 12**: The recombinant yeast cell according to any one of items 1 and 3 to 11, wherein the recombinant yeast cell is selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens, Ogataea polymorpha, Yarrowia lypolytica, Debaryomyces hansenii,* and *Ashbya gossypii,* and is preferably *Saccharomyces cerevisiae.*

**Item 13**: The recombinant host cell according to any one of items 2 to 11 wherein the recombinant host cell belongs to the *Saccharomycetales* order, in particular to the *Saccharomycetaceae* family, and is in particular selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens, Ogataea polymorpha, Yarrowia lypolytica, Debaryomyces hansenii,* and *Ashbya gossypii,* and is preferably *Saccharomyces cerevisiae.*

**Item 14**: A method of producing hyaluronic acid (HA) of a desired molecular weight (HAMW) comprising:
(a) cultivating a recombinant cell as defined in any one of items 1 to 13 in a cultivation medium for a time sufficient to produce hyaluronic acid (HA) of the desired molecular weight; and
(b) optionally isolating or recovering the hyaluronic acid (HA) from the recombinant cell and/or from the cultivation medium.

**Item 15**: The method according to item 14, wherein the HA has a molecular weight of from about 20kDa to about 50kDA, and preferably from about 20 kDa to about 30 kDa.

**Item 16**: The method according to item 15, wherein the HA has a molecular weight of from about 30 kDa to about 50 kDa.

**Item 17**: The method according to item 14, wherein the molecular weight of the HA is of from about 50 kDa to about 150 kDa.

**Item 18**: The method according to item 14, wherein the molecular weight of the HA is of from about 150 kDa to about 1500 kDa.

**Item 19**: The method according to any one of items 14 to 18 wherein the recombinant cell comprises at least one recombinant nucleic acid encoding one or more of:
(i) a polypeptide having glutamine-fructose-6-phosphate amidotransferase (GFA1) activity; and/or
(ii) a polypeptide having UDP-N-acetylglucosamine pyrophosphorylase (QRI1) activity.

**Item 20**: The method according to any one of items 14 to 19, wherein the recombinant cell comprises at least one recombinant nucleic acid encoding one or more of:
(i) a polypeptide having Phosphoglucomutase-1 (PGM1) activity;
(ii) a polypeptide having UTP-glucose-1-phosphate uridylyltransferase (UGP1) activity;
(iii) a polypeptide having Glucosamine-6-phosphate N-acetyltransferase (GNA1) activity; and/or
(iv) a polypeptide having phosphoacetylglucosamine mutase (PCM1) activity.

**Item 21:** The method according to any one of items 14 to 20, wherein the recombinant cell is a member of the genus *Saccharomyces,* and in particular is *Saccharomyces cerevisiae.*

**Item 22**: The method according according to any one of items 14 to 21, wherein the time sufficient to produce hyaluronic acid (HA) of the desired molecular weight is a period of from about 35 hours to about 50 hours, preferably from about 40 hours to about 50 hours, preferably about 48 hours.

**Item 23**: The method according according to any one of items 14 to 22, wherein the molecular weight of the hyaluronic acid is controlled by regulating the pH of the cultivation medium.

**Item 24**: The method according to any one of items 14 to 23, wherein the method is carried out on an industrial scale, preferably where the cultivation medium is at least about 100L, more preferably in the range of about 1000L to about 3000L, even more preferably about 10,000L, even more preferably about 100,000L, or even about 250,000L.

**Item 25**: Hyaluronic acid (HA) obtainable from a recombinant cell of any one of items 1 to 10 or from the method according to any one of items 14 to 24.

**Item 26**: A cultivation medium comprising the HA according to item 25.

**Item 27**: A composition comprising the Hyaluronic acid (HA) according to item 24.

**Item 28**: An industrial product or a consumer product or a consumable comprising (i) the HA according to item 25, (ii) the cultivation medium of item 26 or (iii) the composition of item 27.

**Item 29**: The industrial product or the consumer product or the consumable of item 27, wherein the industrial product or the consumer product or the consumable is a cosmetic product, a flavour product, a fragrance product, a food product, a food, a beverage, a texturant, a pharmaceutical composition, a dietary supplement, a nutraceutical, a cleaning product and/or a dental and/or an oral hygiene composition.

**Item 30**: Use of a recombinant cell as defined in any one of items 1 to 13 for the production of hyaluronic acid (HA) having a molecular weight in the range of from about 20kDa to about 50kDa or from about 50kDa to about 1000 kDa.

**Item 31:** A method for producing hyaluronic acid comprising the steps of:
(a) culturing a recombinant yeast as defined in any one of items 1 and 3 to 12 in a culture medium; and
(b) recovering the hyaluronic acid from said culture medium,
   wherein the hyaluronic acid recovered in step (b) has a molecular weight controlled through the selection of:
   - the nature and origin of the nucleic acid encoding the hyaluronidase of the recombinant yeast,
   - the nature and origin of the promoter controlling the expression of the nucleic acid encoding the hyaluronidase(s) of the recombinant yeast,
   - the presence of an anchoring and/or of a secretion signal associated to the encoded hyaluronidase(s) of the recombinant yeast,
   - the pH of the culture medium during the step of culturing the recombinant yeast, and/or
   - the duration of the culturing of the recombinant yeast.

Certain embodiments of the present invention may provide one or more of the following advantages:
- a process for producing Hyaluronic acid of controlled molecular weight;
- a process for producing Hyaluronic acid of controlled molecular weight in a *Saccharomyces* yeast cell; and
- a process for producing Hyaluronic acid of controlled molecular weight by varying genetic parameters (eg regulatory sequences) and/or process parameters (pH or fermentation time).

The details, examples and preferences provided in relation to any particular one or more of the stated aspects of the present invention will be further described herein and apply equally to all aspects of the present invention. Any combination of the embodiments, examples and preferences described herein in all possible variations thereof is encompassed by the present invention unless otherwise indicated herein, or otherwise clearly contradicted by context.

### BRIEF DESCRIPTION OF THE FIGURES

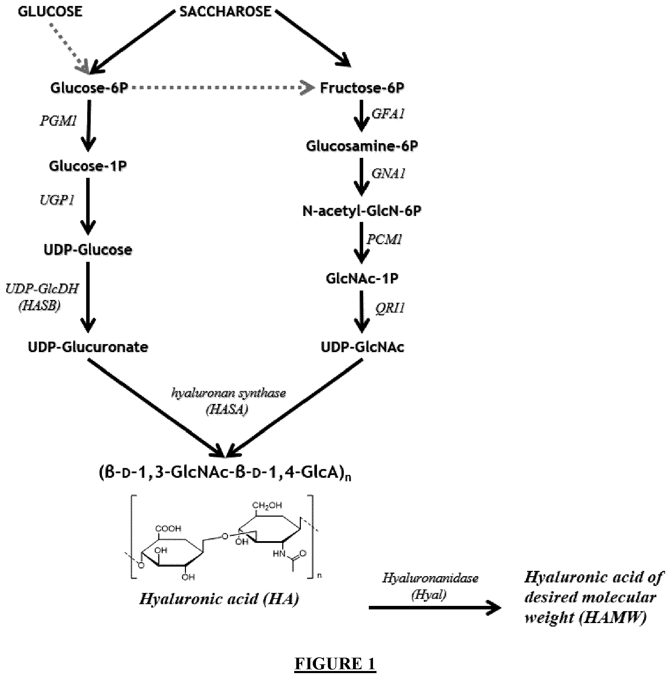

Figure 1 shows a schematic pathway for the production of hyaluronic acid
Figures 2A and 2B show agarose gels used to determine the molecular weight of the hyaluronic acid produced by strains according to the invention, (for example strain YA5235-1 (Figure 2A) and strain YA5359-10 (Figure 2B)). The agarose gels are obtained after running an aliquot of the supernatant of the considered strain and staining it with "stains all" (CAS Number 7423-31-6). HA molecular weight standards are present in each gel for reference.

### SUMMARY OF THE SEQUENCES

**SEQ ID NO: 1** is a reencoded nucleic acid sequence of hyaluronan synthase (HASA) originating from *Chlorella virus PBCV-1*
**SEQ ID NO: 2** is a reencoded nucleic acid sequence of hyaluronan synthase (HASA) originating from *Chlorella virus PBCV-1*
**SEQ ID NO: 3** is a reencoded nucleic acid sequence of hyaluronan synthase (HASA) originating from *Pasteurella multocida*
**SEQ ID NO: 4** is a reencoded nucleic acid sequence of hyaluronan synthase (HASA) originating from *Pasteurella multocida*
**SEQ ID NO: 5** is a reencoded nucleic acid sequence of hyaluronan synthase (HASA) originating from *Pasteurella multocida*
**SEQ ID NO: 6** is a reencoded nucleic acid sequence of hyaluronan synthase (HASA) originating from *Xenopus laevis*
**SEQ ID NO: 7** is a reencoded nucleic acid sequence of hyaluronan synthase (HASA) originating from *Streptococcus zooepidemicus*
**SEQ ID NO: 8** is an amino acid sequence of hyaluronan synthase (HASA) originating from *Chlorella virus PBCV-1*
**SEQ ID NO: 9** is an amino acid sequence of hyaluronan synthase (HASA) originating from *Pasteurella multocida*
**SEQ ID NO: 10** is an amino acid sequence of hyaluronan synthase (HASA) originating from *Xenopus laevis*
**SEQ ID NO: 11** is an amino acid sequence of hyaluronan synthase (HASA) originating from *Streptococcus zooepidemicus*
**SEQ ID NO: 12** is a nucleic acid sequence of UDP-Glucose dehydrogenase (HASB) originating from *Arabidopsis thaliana*
**SEQ ID NO: 13** is a reencoded nucleic acid sequence of UDP-Glucose dehydrogenase (HASB) originating from *Chlorella virus PBCV-1*
**SEQ ID NO: 14** is a reencoded nucleic acid sequence of UDP-Glucose dehydrogenase (HASB) originating from *Chlorella virus PBCV-1*
**SEQ ID NO: 15** is a reencoded nucleic acid sequence of UDP-Glucose dehydrogenase (HASB) originating from *Streptococcus zooepidemicus*
**SEQ ID NO: 16** is an amino acid sequence of UDP-Glucose dehydrogenase (HASB) originating from *Arabidopsis thaliana*
**SEQ ID NO: 17** is an amino acid sequence of UDP-Glucose dehydrogenase (HASB) originating from *Chlorella virus PBCV1*
**SEQ ID NO: 18** is an amino acid sequence of UDP-Glucose dehydrogenase (HASB) originating from *Streptococcus zooepidemicus*
**SEQ ID NO: 19** is a reencoded nucleic acid sequence of hyaluronidase (HYAL) originating from *Bothrops atrox* with a N-terminal secretion signal
**SEQ ID NO: 20** is a reencoded nucleic acid sequence of hyaluronidase (HYAL) originating from *Bothrops atrox* with a N-terminal secretion signal and a C-terminal anchoring signal
**SEQ ID NO: 21** is a reencoded nucleic acid sequence of hyaluronidase (HYAL) originating from *Cupiennius salei* with a N-terminal secretion signal
**SEQ ID NO: 22** is a reencoded nucleic acid sequence of hyaluronidase (HYAL) originating from *Cupiennius salei* with a N-terminal secretion signal and a C-terminal anchoring signal
**SEQ ID NO: 23** is a reencoded nucleic acid sequence of hyaluronidase (HYAL) originating from *Hirudo Nipponia* with a N-terminal secretion signal
**SEQ ID NO: 24** is a reencoded nucleic acid sequence of hyaluronidase (HYAL) originating from *Hirudo Nipponia* with a N-terminal secretion signal and a C-terminal anchoring signal
**SEQ ID NO: 25** is a reencoded nucleic acid sequence of hyaluronidase (HYAL) originating from *Loxosceles intermedia* with a N-terminal secretion signal
**SEQ ID NO: 26** is a reencoded nucleic acid sequence of hyaluronidase (HYAL) originating from *Loxosceles intermedia* with a N-terminal secretion signal and a C-terminal anchoring signal
**SEQ ID NO: 27** is a reencoded nucleic acid sequence of hyaluronidase (HYAL) originating from *Tityus serrulatus* with a N-terminal secretion signal
**SEQ ID NO: 28** is a reencoded nucleic acid sequence of hyaluronidase (HYAL) originating from *Tityus serrulatus* with a N-terminal secretion signal and a C-terminal anchoring signal
**SEQ ID NO: 29** is a reencoded nucleic acid sequence of hyaluronidase (HYAL) originating from *Vespa magnifica* with a N-terminal secretion signal
**SEQ ID NO: 30** is a reencoded nucleic acid sequence of hyaluronidase (HYAL) originating from *Vespa magnifica* with a N-terminal secretion signal and a C-terminal anchoring signal
**SEQ ID NO: 31** is an amino acid sequence of hyaluronidase (HYAL) originating from *Bothrops atrox* with a N-terminal secretion signal
**SEQ ID NO: 32** is an amino acid sequence of hyaluronidase (HYAL) originating from *Bothrops atrox* with a N-terminal secretion signal and a C-terminal anchoring signal
**SEQ ID NO: 33** is an amino acid sequence of hyaluronidase (HYAL) originating from *Cupiennius salei* with a N-terminal secretion signal
**SEQ ID NO: 34** is an amino acid sequence of hyaluronidase (HYAL) originating from *Cupiennius salei* with a N-terminal secretion signal and a C-terminal anchoring signal
**SEQ ID NO: 35** is an amino acid sequence of hyaluronidase (HYAL) originating from *Hirudo Nipponia* with a N-terminal secretion signal
**SEQ ID NO: 36** is an amino acid sequence of hyaluronidase (HYAL) originating from *Hirudo Nipponia* with a N-terminal secretion signal and a C-terminal anchoring signal
**SEQ ID NO: 37** is an amino acid sequence of hyaluronidase (HYAL) originating from *Loxosceles intermedia* with a N-terminal secretion signal
**SEQ ID NO: 38** is an amino acid sequence of hyaluronidase (HYAL) originating from *Loxosceles intermedia* with a N-terminal secretion signal and a C-terminal anchoring signal
**SEQ** ID NO: 39 is an amino acid sequence of hyaluronidase (HYAL) originating from *Tityus serrulatus* with a N-terminal secretion signal
**SEQ ID NO: 40** is an amino acid sequence of hyaluronidase (HYAL) originating from *Tityus serrulatus* with a N-terminal secretion signal and a C-terminal anchoring signal
**SEQ ID NO: 41** is an amino acid sequence of hyaluronidase (HYAL) originating from *Vespa magnifica* with a N-terminal secretion signal
**SEQ ID NO: 42** is an amino acid sequence of hyaluronidase (HYAL) originating from *Vespa magnifica* with a N-terminal secretion signal and a C-terminal anchoring signal
**SEQ ID NO: 43** is a nucleic acid sequence of the secretion sequence added in 5'
**SEQ ID NO: 44** is an amino acid sequence of the secretion sequence added in N-ter
**SEQ ID NO: 45** is a nucleic acid sequence of the anchoring sequence added in 3'
**SEQ ID NO: 46** is an amino acid sequence of the anchoring sequence added in C-ter
**SEQ ID NO: 47** is a nucleic acid sequence of glutamine-fructose-6-phosphate amidotransferase (GFA1) originating from *Saccharomyces cerevisiae*
**SEQ ID NO: 48** is a reencoded nucleic acid sequence of glutamine-fructose-6-phosphate amidotransferase (GFA1) originating from *Chlorella virus 1* (PBCV-1)
**SEQ ID NO: 49** is a reencoded nucleic acid sequence of glutamine-fructose-6-phosphate amidotransferase (GFA1) originating from *Chlorella virus 1* (PBCV-1)
**SEQ ID NO: 50** is an amino acid sequence of glutamine-fructose-6-phosphate amidotransferase (GFA1) originating from *Saccharomyces cerevisiae*
**SEQ ID NO: 51** is an amino acid sequence of glutamine-fructose-6-phosphate amidotransferase (GFA1) originating from *Chlorella virus 1* (PBCV-1)
**SEQ ID NO: 52** is a nucleic acid sequence of UDP-N-acetylglucosamine pyrophosphorylase (QRI1) originating from *Saccharomyces cerevisiae*
**SEQ ID NO: 53** is an amino acid sequence of UDP-N-acetylglucosamine pyrophosphorylase (QRI1) originating from *Saccharomyces cerevisiae*
**SEQ ID NO: 54** is a nucleic acid sequence of Phosphoglucomutase-1 (PGM1) originating from *Saccharomyces cerevisiae*
**SEQ ID NO: 55** is an amino acid sequence Phosphoglucomutase-1 (PGM1) originating from *Saccharomyces cerevisiae*
**SEQ ID NO: 56** is a nucleic acid sequence of UTP-glucose-1-phosphate uridylyltransferase (UGP1) originating from *Saccharomyces cerevisiae*
**SEQ ID NO: 57** is an amino acid sequence of UTP-glucose-1-phosphate uridylyltransferase (UGP1) originating from *Saccharomyces cerevisiae*
**SEQ ID NO: 58** is a nucleic acid sequence of Glucosamine 6-phosphate N-acetyltransferase (GNA1) originating from *Saccharomyces cerevisiae*
**SEQ ID NO: 59** is an amino acid sequence of Glucosamine 6-phosphate N-acetyltransferase (GNA1) originating from *Saccharomyces cerevisiae*
**SEQ ID NO: 60** is a nucleic acid sequence of phosphoacetylglucosamine mutase (PCM1) originating from *Saccharomyces cerevisiae*
**SEQ ID NO: 61** is an amino acid sequence of phosphoacetylglucosamine mutase (PCM1) originating from *Saccharomyces cerevisiae*
**SEQ ID NO: 62** is the nucleic acid sequence of promotor pTDH3
**SEQ ID NO:63** is the nucleic acid sequence of promotor pTDH3.Sk
**SEQ ID NO: 64** is the nucleic acid sequence of promotor pTDH3-1.sba
**SEQ ID NO: 65** is the nucleic acid sequence of promotor pTDH3.Sar
**SEQ ID NO: 66** is the nucleic acid sequence of promotor pENO2
**SEQ ID NO: 67** is the nucleic acid sequence of promotor pTEF3
**SEQ ID NO: 68** is the nucleic acid sequence of promotor pTEF1
**SEQ ID NO: 69** is the nucleic acid sequence of promotor pTEF1.ago
**SEQ ID NO: 70** is the nucleic acid sequence of promotor pTEF1.Sba
**SEQ ID NO: 71** is the nucleic acid sequence of promotor pPDC1
**SEQ ID NO: 72** is the nucleic acid sequence of promotor pCCW12
**SEQ ID NO: 73** is the nucleic acid sequence of promotor pCCW12.Sm
**SEQ ID NO: 74** is the nucleic acid sequence of promotor pCCW12.Sk
**SEQ ID NO: 75** is the nucleic acid sequence of promotor pCCW12.Sba
**SEQ ID NO: 76** is the nucleic acid sequence of promotor pCCW12.Sar
**SEQ ID NO: 77** is the nucleic acid sequence of promotor pNUP57
**SEQ ID NO: 78** is the nucleic acid sequence of promotor pCCW10.ago
**SEQ ID NO: 79** is the nucleic acid sequence of promotor pCWP2
**SEQ ID NO: 80** is the nucleic acid sequence of promotor pRPLA1
**SEQ ID NO: 81** is the nucleic acid sequence of promotor pCUP1
**SEQ ID NO: 82** is the nucleic acid sequence of promotor pMET6
**SEQ ID NO: 83** is the nucleic acid sequence of promotor pMET25
**SEQ ID NO: 84** is the nucleic acid sequence of promotor pSAM1
**SEQ ID NO: 85** is the nucleic acid sequence of terminator tTPI1
**SEQ ID NO: 86** is the nucleic acid sequence of terminator tMET25
**SEQ ID NO: 87** is the nucleic acid sequence of terminator tDIT1
**SEQ ID NO: 88** is the nucleic acid sequence of terminator tRPL3
**SEQ ID NO: 89** is the nucleic acid sequence of terminator tRPL3.sm
**SEQ ID NO: 90** is the nucleic acid sequence of terminator tRPL3.sba
**SEQ ID NO: 91** is the nucleic acid sequence of terminator tRPL41B
**SEQ ID NO: 92** is the nucleic acid sequence of terminator tRPL15A
**SEQ ID NO: 93** is the nucleic acid sequence of terminator tRPL15A.sba
**SEQ ID NO: 94** is the nucleic acid sequence of terminator tIDP1
**SEQ ID NO: 95** is the nucleic acid sequence of terminator tTEF1.sba
**SEQ ID NO: 96** is a nucleic acid sequence of Glutamine synthetase (GLN1) originating from *Saccharomyces cerevisiae*
**SEQ ID NO: 97** is an amino acid sequence of Glutamine synthetase (GLN1) originating from *Saccharomyces cerevisiae*
**SEQ ID NO: 98** is a nucleic acid sequence of Glutamate synthase (GLT1) originating from *Saccharomyces cerevisiae*
**SEQ ID NO: 99** is an amino acid sequence of Glutamate synthase (GLT1) originating from *Saccharomyces cerevisiae*
**SEQ ID NO: 100** is the nucleic acid sequence of terminator tTDH3.
**SEQ ID NO: 101** is a reencoded nucleic acid sequence of hyaluronan synthase (HASA) originating from *Chlorella virus CviKl*
**SEQ ID NO: 102** is a reencoded nucleic acid sequence of hyaluronan synthase (HASA) originating from *Chlorella virus IL-5-2s1*
**SEQ ID NO: 103** is a reencoded nucleic acid sequence of hyaluronan synthase (HASA) originating from *Chlorella virus CZ-2*
**SEQ ID NO: 104** is a reencoded nucleic acid sequence of hyaluronan synthase (HASA) originating from *Chlorella virus CVG-1*
**SEQ ID NO: 105** is an amino acid sequence of hyaluronan synthase (HASA) originating from *Chlorella virus CviKl*
**SEQ ID NO: 106** is an amino acid sequence of hyaluronan synthase (HASA) originating from *Chlorella virus IL-5-2s1*
**SEQ ID NO: 107** is an amino acid sequence of hyaluronan synthase (HASA) originating from *Chlorella virus CZ-2*
**SEQ ID NO: 108** is an amino acid sequence of hyaluronan synthase (HASA) originating from *Chlorella virus CVG-1*

### DETAILED DESCRIPTION OF THE INVENTION

The inventors have conceived genetically modified cells, and especially genetically modified yeasts, having the ability to produce hyaluronic acid, as compared to the parent cells, and especially as compared to the parent yeasts which are not naturally capable of doing so.

These genetically modified cells are described throughout the present specification.

### Definitions

The term "Hyaluronic acid" also known as hyaluronan or HA, is polysaccharide composed of D-glucuronic acid and N-acetyl-D-glucosamine, linked via alternating β-(1→4) and β-(1→3) glycosidic bonds and having the chemical formula (C₁₄H₂₁NO₁₁)n.

Hyalyuronic acid may be produced in a recombinant cell.

As used herein, the term "recombinant", when used in reference to a cell, indicates that the cell has been modified by the introduction of an endogenous and/or heterologous nucleic acid or protein into the cell or the alteration of a native cell or that the cell is derived from a cell so modified. Thus, for example, recombinant cells express genes or nucleic acid that are not found within the native (non-recombinant) form of the cell or express native (eg endogenous) genes at a different level than their native level or express additional or supplementary copies of native (eg endogenous) at a different level than their native level.

As used herein, the term "recombinant", when used in reference to a nucleic acid or vector, are sequences formed/obtained by technics of genetic engineering well known to the man skilled in the art. The guidelines of the US National Institute for Health (NIH) accordingly indicate that:
"*recombinant [...] nucleic acids are defined as:*
*i. molecules that (a) are constructed by joining nucleic acid molecules and (b) that can replicate in a living cell, i.e., recombinant nucleic acids;*",
which reflects the conventional use of the word "recombinant" attached to nucleic acid sequences to mean recombined following the insertion of, or joining together with, another nucleic acid.

Proteins that result from the expression of recombinant DNA or recombinant vector within living cells are also termed recombinant proteins.

The term "recombinant" is accordingly synonymous with the term "genetically modified". The term "gene" is synonymous with the term "nucleic acid" or "nucleotide sequence".

A recombinant nucleic acid sequence for use in a recombinant cell, and in particular a recombinant yeast, of the invention may be provided in the form of a nucleic acid construct. The term "nucleic acid construct" refers to as a nucleic acid molecule, either singleor double-stranded, which is isolated or derived from a native (eg endogenous) naturally occurring gene or is a heterologous nucleic acid or which has been modified to contain segments of nucleic acid which are combined and juxtaposed in a manner which would not otherwise exist in nature. The term "nucleic acid construct" is synonymous with the term "expression cassette" or "heterologous nucleic acid expression cassette" when the nucleic acid construct contains one or more regulatory elements required for expression of a coding sequence, wherein said control sequences are operably linked to said coding sequence. Nonlimiting examples of regulatory elements include promoters, enhancers, silencers, terminators, and poly-A signals.

A recombinant nucleic acid sequence for use in a recombinant cell of the invention may be provided in the form of an expression vector, wherein the polynucleotide sequence is operably linked to at least one control sequence for the expression of the polynucleotide sequence in a recombinant cell.

The terms "obtained from" or "originate from" or "originating from" a microorganism or animal generally means that a substance (e.g., a nucleic acid molecule or polypeptide) originating from the microorganism or animal is native to that microorganism or animal.

The terms "derived from" a microorganism or animal generally means that a substance (e.g., a nucleic acid molecule or polypeptide) derived from the microorganism or animal is the result of modifications brought to the substance native to (i.e. present as such) in that microorganism or animal. For example, regarding a nucleic acid sequence derived from a microorganism or animal, said nucleic acid sequence can correspond to a reencoded and/or truncated version of the native nucleic acid sequence from this microorganism or animal. Other modifications well known to the man skilled in the art can be brought to the native substance of a microorganism or animal leading to the substance used being "derived from" said microorganism or animal.

As used herein, the term "polypeptide" refers to a molecule comprising amino acid residues linked by peptide bonds and containing more than five amino acid residues. The amino acids are identified by either the single-letter or three-letter designations. The term "protein" as used herein is synonymous with the term "polypeptide" and may also refer to two or more polypeptides. Thus, the terms "protein", "peptide" and "polypeptide" can be used interchangeably. Polypeptides may optionally be modified (e.g., glycosylated, phosphorylated, acylated, farnesylated, prenylated, sulfonated, and the like) to add functionality. Polypeptides exhibiting activity may be referred to as enzymes. It will be understood that, as a result of the degeneracy of the genetic code, a multitude of nucleotide sequences encoding a given polypeptide may be produced.

A polypeptide encoded by a recombinant nucleic acid for use in a recombinant cell, and in particular in a recombinant yeast, of the invention may comprise a signal peptide and/or a propeptide sequence. In the event that a polypeptide expressed by a recombinant cell, and in particular a recombinant yeast, of the invention comprises a signal peptide and/or a propeptide, sequence identity may be calculated over the mature polypeptide sequence.

The term "operably linked" as used herein refers to two or more nucleic acid sequence elements that are physically linked and are in a functional relationship with each other. For instance, a promoter is operably linked to a coding sequence if the promoter is able to initiate or regulate the transcription or expression of the coding sequence, in which case the coding sequence should be understood as being "under the control of" the promoter. Generally, when two nucleic acid sequences are operably linked, they will be in the same orientation and usually also in the same reading frame. They usually will be essentially contiguous, although this may not be required.

The term "native" or "endogenous" as used herein with reference to molecules, and in particular enzymes and nucleic acids, indicates molecules that are expressed in the organism in which they originated or are found in nature.

The term "endogenous gene" means that the gene was present in the cell before any genetic modification, in the wild-type strain. Endogenous genes may be overexpressed by introducing heterologous sequences in addition to, or to replace endogenous regulatory elements, or by introducing one or more additional or supplementary copies of the gene into the chromosome or a plasmid (said additional or supplementary copies being designated "exogenous or heterologous genes" or "heterologous nucleotide sequences" or "heterologous nucleic acids" as defined herein). Endogenous genes may also be modified to modulate their expression and/or activity. For example, mutations may be introduced into the coding sequence to modify the gene product or heterologous sequences may be introduced in addition to or to replace endogenous regulatory elements. Modulation of an endogenous gene may result in the up-regulation and/or enhancement of the activity of the gene product, or alternatively, in the down-regulation and/or attenuation of the activity of the endogenous gene product. Another way to enhance expression of endogenous genes is to introduce one or more additional or supplementary copies of the gene onto the chromosome or a plasmid (said supplementary copies being designated "exogenous or heterologous genes" or "heterologous nucleotide sequences" or heterologous nucleic acids" as defined herein).

By "one or more additional or supplementary copies of a gene" according to the invention, it is for example understood in the present invention from 1 to 50 copies, in particular from 1 to 30 copies, more particularly from 1 to 20 copies, and preferably from 1 to 10 copies. Said copies may be inserted into the same locus or into different loci of a recombinant cell of the invention.

The term "exogenous gene" means that the gene was introduced into a cell, by means well known to the man skilled in the art, whereas this gene is or is not naturally occurring in the wild-type cell. Cells can express exogenous genes if these genes are introduced into the cell with all the elements allowing their expression in the cell. Transforming cells with exogenous DNA is a routine task for the man skilled in the art. Exogenous genes may be integrated into the host chromosome, or be expressed extrachromosomally from plasmids or vectors. A variety of plasmids, which differ with respect to their origin of replication and their copy number in the cell, are all known in the art. The sequence of exogenous genes may be adapted for its expression in the cell. Indeed, the man skilled in the art knows the notion of codon usage bias and how to adapt nucleic sequences for a particular codon usage bias without modifying the deduced protein. In particular embodiments, codon optimized genes express native enzymes.

The term "heterologous gene" or heterologous nucleic acid sequence" refers to a gene or nucleic acid sequence not normally found in a given cell in nature. As such, a heterologous nucleic acid sequence may be: (a) foreign to its host cell (i.e. is"exogenous" to the cell); (b) naturally found in the host cell (i.e. "endogenous") but present at an unnatural quantity in the cell (i.e., greater or lesser quantity than naturally found in the host cell); or (c) be naturally found in the host cell but positioned outside of its natural locus.

In the present application, all genes are referenced with their common names and with references to their nucleotide sequences and, the case arising, to their amino acid sequences. Using the references given in accession number for known genes, those skilled in the art are able to determine the equivalent genes in other organisms, bacterial strains, yeast, fungi, mammals, plants, etc. This routine work is advantageously done using consensus sequences that can be determined by carrying out sequence alignments with genes derived from other cells and designing degenerated probes to clone the corresponding gene in another organism.

The man skilled in the art knows different means to modulate, and in particular up-regulate or down-regulate, the expression of endogenous genes. For example, a way to enhance expression of, or over express, endogenous genes is to introduce one or more additional or supplementary copies of the gene onto the chromosome or a plasmid.

Another way is to replace the endogenous promoter of a gene with a stronger promoter. These promoters may be homologous or heterologous. Promoters particularly interesting in the present invention are described in more detail elsewhere in the present specification.

The nucleic acid expression construct may further comprise 5' and/or 3' recognition sequences and/or selection markers.

The term "inducible promoter" is used to qualify a promoter whose activity is induced, i.e. increased:
- in the presence of one or more particular metabolite(s). The higher the metabolite concentration in the medium, the stronger the promoter activity; or
- in the presence of a low concentration, or in the absence, of one or more metabolite(s). These metabolites are different from those whose increasing presence induces the activity of the promoter. The lower the metabolite concentration in the medium, the stronger the promoter activity.

The term "repressible promoter" is used to qualify a promoter whose activity is repressed, i.e. reduced:
- in the presence of one or more particular metabolite(s). The higher the metabolite concentration in the medium, the weaker the promoter activity; or
- in the presence of a low concentration, or in the absence, of one or more metabolite(s). These metabolites are different from those whose increasing presence represses the activity of the promoter. The lower the metabolite concentration in the medium, the weaker the promoter activity.

As used herein, the term "anchoring signal" when used in conjunction with a protein or polypeptide such as an enzyme (such as, for example hyaluronidase) means for example a first nucleic acid encoding a protein that is operably linked to a second nucleic acid encoding a protein or a polypeptide, or a first protein or polypeptide that is operably linked to a second protein or polypeptide, such as an enzyme (such as, for example hyaluronidase to form, for example a fusion protein), and that enables the cellular transport machinery of a cell, in particular of a *S. cerevisiae* cell, to correctly anchor and/or position in the membrane of the cell the second protein operably linked to the first protein.

As used herein, the term "secretion signal" when used in conjunction with a protein or polypeptide such as an enzyme (such as, for example hyaluronidase) means for example a first nucleic acid encoding a peptide or protein that is operably linked to a second nucleic acid encoding a protein, or a first protein that is linked to a second protein, such as an enzyme (such as, for example hyaluronidase to form, for example a fusion protein), and that enables the cellular transport machinery of a cell, in particular of a *S. cerevisiae* cell, to locate at least the second protein to the membrane of the cell and to secrete the second protein outside of the cell, after the first protein has, for example, been cleaved from the second protein.

As used herein the terms "secretion signal" and "anchoring signal" when used in conjunction with a protein or polypeptide such as an enzyme (such as, for example, hyaluronidase), mean for example a first nucleic acid encoding a peptide or protein that is operably linked to a second nucleic acid encoding a protein, or a first protein that is operably linked to a second protein, such as an enzyme (such as, for example hyaluronidase), and that enables the cellular transport machinery of a cell, in particular of a *S. cerevisiae* cell, to locate at least the second protein to the membrane of the cell wherein if the second protein is also operably linked to an "anchoring signal" the second protein is not secreted but remains attached to the membrane of the cell. In some cases, a secretion-anchoring signal may provide a dual secretion signal and anchoring signal function.

Sequences of secretion and anchoring signals, methods for the expression, anchoring and/or secretion of heterologous proteins, such as enzymes (such as, for example, hyaluronidase) on the surface of a cell (such, as for example, a yeast cell) are well known in the art (see for example, Ast et al (2013) Cell 152: 1134-1145, Ast and Schuldiner (2013) Crit Rev Biochem Mol Biol 48(3) 273-288, Van der Vaart et al (1997) Applied Environmental Microbiology 63(2) 615-620 and the entire contents of each of these publications are incorporated herein by reference).

The "activity" of an enzyme is used interchangeably with the term "function" and designates, in the context of the invention, the capacity of an enzyme to catalyze a desired reaction. The amount of an enzyme in a host cell may be altered by modifying the transcription of the gene that encodes the enzyme. This can be achieved for example by modifying the copy number of the nucleotide sequence encoding the enzyme (e.g., by using a higher or lower copy number expression vector comprising the nucleotide sequence, or by introducing additional copies of the nucleotide sequence into the genome of the host cell or by deleting or disrupting the nucleotide sequence in the genome of the host cell), by changing the order of coding sequences on a polycistronic mRNA of an operon or breaking up an operon into individual genes each with its own control elements, or by increasing the strength of the promoter or operator to which the nucleotide sequence is operably linked.

Alternatively or in addition, the copy number of an enzyme in a host cell may be altered by modifying the level of translation of an mRNA that encodes the enzyme. This can be achieved for example by modifying the stability of the mRNA, modifying the sequence of the ribosome binding site, modifying the distance or sequence between the ribosome binding site and the start codon of the enzyme coding sequence, modifying the entire intercistronic region located "upstream of' or adjacent to the 5' side of the start codon of the enzyme coding region, stabilizing the 3'-end of the mRNA transcript using hairpins and specialized sequences, modifying the codon usage of enzyme, altering expression of rare codon tRNAs used in the biosynthesis of the enzyme, and/or increasing the stability of the enzyme, as, for example, via mutation of its coding sequence.

The activity of an enzyme in a host cell can be altered in a number of ways, including, but not limited to, expressing a modified form of the enzyme that exhibits increased or decreased solubility in the host cell, expressing an altered form of the enzyme that lacks a domain through which the activity of the enzyme is inhibited, expressing a modified form of the enzyme that has a higher or lower Kcat or a lower or higher Km for the substrate, or expressing an altered form of the enzyme that is more or less affected by feed back or feed-forward regulation by another molecule in the pathway.

The terms "encoding" or "coding for" refer to the process by which a polynucleotide, through the mechanisms of transcription and translation, produces an amino-acid sequence.

The gene(s) encoding the enzyme(s) considered in the present invention can be exogenous or endogenous.

The methods implemented in the present invention preferably require the use of one or more chromosomal integration constructs for the stable introduction of a heterologous nucleotide sequence into a specific location on a chromosome or for the functional disruption of one or more target genes in a genetically modified cell. In some embodiments, disruption of a target gene prevents the expression of the related functional protein. In some embodiments, disruption of a target gene results in the expression of a non-functional protein from the disrupted gene.

Accordingly, a "disrupted endogeneous nucleic acid" in the present invention relates to an endogeneous nucleic acid, or gene, unable to encode the functional, or fully functional, protein or polypeptide it codes for before it was disrupted. The nucleic acid can for example be disrupted by the introduction of an integration construct into the nucleic acid, as illustrated in the examples. Said integration can for example prevent the expression of the related functional protein or polypeptide or result in the expression of a non-functional, or non-fully functional protein or polypeptide from the disrupted gene.

Parameters of chromosomal integration constructs that may be varied in the practice of the present invention include, but are not limited to, the lengths of the homologous sequences; the nucleotide sequence of the homologous sequences; the length of the integrating sequence; the nucleotide sequence of the integrating sequence; and the nucleotide sequence of the target locus. In some embodiments, an effective range for the length of each homologous sequence is 20 to 5,000 base pairs, preferentially 50 to 100 base pairs. In particular embodiments, the length of each homologous sequence is about 50 base pairs. For more information on the length of homology required for gene targeting, see D. Burke et al., Methods in yeast Genetics - A cold spring harbor laboratory course Manual (2000).

In some embodiments, (a) disrupted gene(s) in which the above-mentioned DNA construct(s) is/are intended to be inserted may advantageously comprises one or more selectable markers useful for the selection of transformed cells. Preferably, said selectable marker(s) are comprised in the DNA construct(s) according to the present invention.

In some embodiments, the selectable marker is an antibiotic resistance marker. Illustrative examples of antibiotic resistance markers include, but are not limited to the, NAT1, AUR1-C, HPH, DSDA, KAN<R>, and SH BLE gene products. The NAT 1 gene product from *S. noursei* confers resistance to nourseothricin; the AUR1-C gene product from *Saccharomyces cerevisiae* confers resistance to Auerobasidin A (AbA); the HPH gene product of *Klebsiella pneumoniae* confers resistance to Hygromycin B; the DSDA gene product of *E*. *coli* allows cells to grow on plates with D-serine as the sole nitrogen source; the KAN<R> gene of the Tn903 transposon confers resistance to G418; and the SH BLE gene product from *Streptoalloteichus hindustanus* confers resistance to Zeocin (bleomycin).

In some embodiments, the antibiotic resistance marker is deleted after the genetically modified cell of the invention is isolated. The man skilled in the art is able to choose suitable marker in specific genetic context.

In a particular embodiment, a recombinant cell according to the invention is devoid of any antibiotic resistance marker. This advantageously prevents the necessity to add antibiotics in the selection medium.

In some embodiments, the selectable marker rescues an auxotrophy (e.g., a nutritional auxotrophy) in the genetically modified cell. In such embodiments, a parent cell, and in particular a parent yeast, comprises a functional disruption in one or more gene products that function in an amino acid or nucleotide biosynthetic pathway, such as, for example, the HIS3, LEU2, LYS1, LYS2, MET15, TRP1, ADE2, and URA3 gene products in yeast, which renders the parent cell incapable of growing in media without supplementation with one or more nutrients (auxotrophic phenotype). The auxotrophic phenotype can then be rescued by transforming the parent cell with a chromosomal integration encoding a functional copy of the disrupted gene product (in some embodiments the functional copy of the gene may originate from close species, such as *Kluveromyces, Candida* etc.), and the genetically modified cell generated can be selected based on the loss of the auxotrophic phenotype of the parent microbial cell.

For each of the nucleic acid sequences comprising a promoter sequence, a coding sequence (e.g. an enzyme coding sequence), or a terminator sequence, reference sequences are described herein. The present description also encompasses nucleic acid sequences having specific percentages of nucleic acid identity with a reference nucleic acid sequence.

For each or the amino acid sequences of interest, reference sequences are described herein. The present description also encompasses amino acid sequences (e.g. enzyme amino acid sequences), having specific percentages of amino acid identity with a reference amino acid sequence.

For obvious reasons, in all the present description, a specific nucleic acid sequence or a specific amino acid sequence which complies with, respectively, the considered nucleotide or amino acid identity, should further lead to obtaining a protein (or enzyme) which displays the desired biological activity. As used herein, the "percentage of identity" between two nucleic acid sequences or between two amino acid sequences is determined by comparing both optimally aligned sequences through a comparison window.

The portion of the nucleotide or amino-acid sequence in the comparison window may thus include additions or deletions (for example "gaps") as compared to the reference sequence (which does not include these additions or these deletions) so as to obtain an optimal alignment between both sequences.

The terms "sequence homology" or "sequence identity" or "homology" or "identity" are used interchangeably herein. For the purpose of the invention, it is defined here that in order to determine the percentage of sequence homology or sequence identity of two amino acid sequences or of two nucleic acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more nucleic acids/based or amino acids. The sequence identity is the percentage of identical matches between the two sequences over the reported aligned region.

A comparison of sequences and determination of percentage of sequence identity between two sequences can be accomplished using a mathematical algorithm. The skilled person will be aware of the fact that several different computer programs are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley).

The percent sequence identity between two amino acid sequences or between two nucleotide sequences may be determined using the Needleman and Wunsch algorithm for the alignment of two sequences. (Needleman, S. B. and Wunsch, C. D. (1970) J. Mol. Biol. 48, 443-453). Both amino acid sequences and nucleotide sequences can be aligned by the algorithm. The Needleman-Wunsch algorithm has been implemented in the computer program NEEDLE.

For the purpose of the invention, the NEEDLE program from the EMBOSS package was used (version 2.8.0 or higher, EMBOSS: The European Molecular Biology Open Software Suite (2000) Rice, P. LongdenJ. and Bleasby,A. Trends in Genetics 16, (6) pp276-277, http://emboss.bioinformatics.nl/). For protein sequences EBLOSUM62 is used for the substitution matrix. For nucleotide sequence, EDNAFULL is used. The optional parameters used are a gap opening penalty of 10 and a gap extension penalty of 0.5. No end gap penalty is added. In the Output section, Yes has been indicated in response to the question "Brief identity and similarity" and "SRS pairwise" indicated as Output alignment format.

After alignment by the program NEEDLE as described above the percentage of sequence identity between a query sequence and a sequence of the invention is calculated as follows: Number of corresponding positions in the alignment showing an identical amino acid or identical nucleotide in both sequences divided by the total length of the alignment after subtraction of the total number of gaps in the alignment. The identity defined as herein can be obtained from NEEDLE by using the NOBRIEF option and is labeled in the output of the program as "longest-identity".

The similarity of nucleotide and amino acid sequences, i.e. the percentage of sequence identity, can be determined via sequence alignments using several other art-known algorithms, preferably with the mathematical algorithm of Karlin and Altschul (Karlin & Altschul (1993) Proc. Natl. Acad. Sci. USA 90: 5873-5877), with hmmalign (HMMER package, http://hmmer.wustl.edu/) or with the CLUSTAL algorithm (Thompson, J. D., Higgins, D. G. & Gibson, T. J. (1994) Nucleic Acids Res. 22, 4673-80) available e.g. on https://www.ebi.ac.uk/Tools/msa/clustalo/ or the GAP program (mathematical algorithm of the University of Iowa) or the mathematical algorithm of Myers and Miller (1989 - Cabios 4: 11-17) or Clone Manager 9. Preferred parameters used are the default parameters as they are set on https://www.ebi.ac.uk/Tools/msa/clustalo/.

The grade of sequence identity (sequence matching) may be calculated using e.g. BLAST, BLAT or BlastZ (or BlastX). A similar algorithm is incorporated into the BLASTN and BLASTP programs of Altschul et al (1990) J. Mol. Biol. 215, 403-410. BLAST polynucleotide searches are performed with the BLASTN program, score = 100, word length = 12, to obtain polynucleotide sequences that are homologous to those nucleic acids which encode the relevant protein.

BLAST protein searches are performed with the BLASTP program, score = 50, word length = 3, to obtain amino acid sequences homologous to the SHC polypeptide. To obtain gapped alignments for comparative purposes, Gapped BLAST is utilized as described in Altschul et al (1997) Nucleic Acids Res. 25, 3389-3402. When utilizing BLAST and Gapped BLAST programs, the default parameters of the respective programs are used. Sequence matching analysis may be supplemented by established homology mapping techniques like Shuffle-LAGAN (Brudno M., Bioinformatics 2003b, 19 Suppl 1: 154-162) or Markov random fields. When percentages of sequence identity are referred to in the present application, these percentages are calculated in relation to the full length of the longer sequence, if not specifically indicated otherwise.

In particular embodiments, % identity between two sequences is determined using CLUSTAL O (version 1.2.4).

The "fermentation" or "culture" is generally conducted in fermenters with an appropriate culture medium adapted to the cell being cultivated, containing at least one simple carbon source, and if necessary co-substrates.

The term "fermentation composition" refers to a composition which comprises genetically modified host cells and products or metabolites produced by the genetically modified host cells. An example of a fermentation composition is a whole cell broth, which can be the entire contents of a vessel (e.g., a flasks, plate, or fermentor), including cells, aqueous phase, and compounds produced from the genetically modified host cells.

The term "medium" refers to a culture medium or cultivation medium or fermentation medium.

For maximal production of hyaluronic acid, the recombinant cells used as production hosts preferably have a high rate of carbohydrate utilization. These characteristics may be conferred by mutagenesis and selection, genetic engineering, or may be natural. Fermentation media, or "culture medium" or "cultivation medium", for the present cells may contain at least about 10 g/L of glucose and/or sucrose. Additional carbon substrates may include but are not limited to monosaccharides such as fructose, mannose, xylose and arabinose; oligosaccharides such as lactose, maltose, galactose, or sucrose; polysaccharides such as starch or cellulose or mixtures thereof and unpurified mixtures from renewable feedstocks such as cheese whey permeate cornsteep liquor, sugar beet molasses, and barley malt. Other carbon substrates may include glycerol, acetate and/or ethanol.

Hence, it is contemplated that the source of carbon utilized in the present invention may encompass a wide variety of carbon containing substrates and will only be limited by the choice of cell, and in particular of yeast.

Although it is contemplated that all of the above-mentioned carbon substrates and mixtures thereof are suitable in the present invention, preferred carbon substrates are glucose, fructose, and sucrose, or mixtures of these with C5 sugars such as xylose and/or arabinose for cells, and in particular yeasts, modified to use C5 sugars, and more particularly glucose.

Preferred carbon substrates are glucose or sucrose.

In addition to an appropriate carbon source, fermentation media may contain suitable minerals, salts, cofactors, buffers and other components, known to those skilled in the art, suitable for the growth of the cultures and promotion of the enzymatic pathway necessary for the production of the desired product.

Besides, additional genetic modifications suitable for the growth of recombinant cells according to the invention may be considered.

The terms "Aerobic conditions" refers to concentrations of oxygen in the culture medium that are sufficient for an aerobic or facultative anaerobic cell, and in particular yeast, to use di-oxygene as a terminal electron acceptor.

"Microaerobic condition" refers to a culture medium in which the concentration of oxygen is less than that in air, i.e. oxygen concentration up to 6% O₂.

An "appropriate culture medium" designates a medium (e.g. a sterile, liquid medium) comprising nutrients essential or beneficial to the maintenance and/or growth of the cell such as carbon sources or carbon substrate, nitrogen sources, for example, peptone, yeast extracts, meat extracts, malt extracts, urea, ammonium sulfate, ammonium chloride, ammonium nitrate and ammonium phosphate; phosphorus sources, for example, monopotassium phosphate or dipotassium phosphate; trace elements (e.g., metal salts), for example magnesium salts, cobalt salts and/or manganese salts; as well as growth factors such as amino acids, vitamins, growth promoters, and the like. The term "carbon source" or "carbon substrate" or "source of carbon" according to the present invention denotes any source of carbon that can be used by those skilled in the art to support the normal growth of a cell, including hexoses (such as glucose, galactose or lactose), pentoses, monosaccharides, oligosaccharides, disaccharides (such as sucrose, cellobiose or maltose), molasses, starch or its derivatives, cellulose, hemicelluloses and combinations thereof.

Culture mediums that are particularly appropriate to produce recombinant cells of the invention, and in particular recombinant yeasts of the invention, are described in greater details further below in the text.

As used herein, the term "about" refers to a reasonable range about a value as determined by the practitioner of skill. In certain embodiments, the term about refers to ± one, two, or three standard deviations. In certain embodiments, the term about refers to ± 5%, 10%, 20%, or 25%. In certain embodiments, the term about refers to ± 0.1, 0.2, or 0.3 logarithmic units, e.g. pH units.

### General features of genetic modifications introduced according to the invention

- All the genome modifications are inserted in recombinant cells, and in particular recombinant yeasts, according to known genetic engineering techniques:
- The successive nucleic acid sequences included in a gene construct that is introduced in the recombinant cell genome according to the invention are of the following structure:
Prom₁-ORF₁-term₁-ORF₂-gene₂-term₂- .../... -Promₙ-ORFₙ-termₙ, wherein:
- Prom1 is a sequence regulating the expression of the coding sequence ORF1,
- ORF1 is a nucleic acid sequence encoding a desired protein PROT1, and especially a desired enzyme PROT1,
- Term1 is a transcription terminator sequence that mediates transcriptional termination by providing signals in the newly synthesized mRNA that trigger processes which release the mRNA from the transcriptional complex, and
- "1", "2", .../... "n" may or may not describe the same ORF (Open Reading Frame), promoter or terminator. The order of the nucleic acid sequences does not matter. "n" is an integer usually ranging from 5 and 20. These constructs are inserted in one of the recombinant cell chromosome at a controlled location. In some embodiments, the insertion site is neither essential for the functionality of the inserted construct, nor for the viability of the resulting genetically modified cell.

As will be understood by those of skill in the art, it can be advantageous to modify a coding sequence to enhance its expression in a particular host. The genetic code is redundant with 64 possible codons, but most organisms typically use a subset of these codons. The codons that are utilized most often in a species are called optimal codons, and those not utilized very often are classified as rare or low-usage codons. Codons can be substituted to reflect the preferred codon usage of the host, in a process sometimes called "codon optimization" or"controlling for species codon bias." Codon optimization for other host cells can be readily determined using codon usage tables or can be performed using commercially available software, such as CodonOp (www.idtdna.com/CodonOptfrom) from Integrated DNA Technologies. Optimized coding sequences containing codons preferred by a particular prokaryotic or eukaryotic host (Murray et al, 1989 , Nucl Acids Res. 17: 477-508) can be prepared, for example, to increase the rate of translation or to produce recombinant RNA transcripts having desirable properties, such as a longer half-life, as compared with transcripts produced from a non-optimized sequence. Translation stop codons can also be modified to reflect host preference. For example, typical stop codons for S. cerevisiae and mammals are UAA and UGA, respectively. The typical stop codon for monocotyledonous plants is UGA, whereas insects and E. coli commonly use UAA as the stop codon (Dalphin et al, 1996, Nucl Acids Res. 24: 216-8).
- When the recombinant cell is a yeast cell, and in particular is *Saccharomyces cerevisiae* yeast cell, nucleic acid sequences introduced in the yeast genome and originating from other organisms than *Saccharomyces cerevisiae* are generally "transcoded" (generally "codon-optimized"), meaning that these nucleic acid sequences are synthesized with an optimal codon usage for expression in *S. cerevisiae.* The nucleotide sequence (and not the protein sequence) of some nucleic acid sequences from *S. cerevisiae* has also been modified ("transcoded") to minimize recombination with an endogenous copy of the said gene.
- Genes may be deleted through standard procedures used in cell genetic engineering. In some embodiments, the genes targeted for deletion may be interrupted by insertion of one of the above-described gene constructs, or alternatively the genes targeted for deletion are replaced by a short stretch of nucleotide.
- A nucleic acid sequences may be rendered "inducible or repressible" by deleting an endogenous copy of the nucleic acid sequences (if necessary) and placing a new copy of the ORF under the control of an inducible or repressible promoter. An inducible or repressible promoter is a promoter which activity is modulated or controlled, i.e. either increased or decreased, upon a change in the environmental conditions or external stimuli. Induction or repression may be artificially controlled, which encompasses induction or repression by abiotic factors such as chemical compounds not found naturally in the cell, and in particular yeast, of interest, light, oxygen levels, heat or cold. A list and sequences of inducible or repressible promoters are described elsewhere in the present specification.

### Recombinant cells according to the invention

The inventors have conceived recombinant cells, in particular recombinant yeasts, having an ability of producing hyaluronic acid.

The present invention relates to recombinant cells, and in particular recombinant yeasts, having the ability to produce hyaluronic acid, and wherein this ability to produce hyaluronic acid is obtained through a plurality of alterations that have been introduced in the genome thereof, by genetic engineering methods.

The present invention pertains to a recombinant yeast cell producing hyaluronic acid (HA), wherein the recombinant cell comprises:
(a) one or more recombinant nucleic acids encoding a polypeptide having hyaluronan synthase activity;
(b) one or more recombinant nucleic acids encoding a polypeptide having UDP-Glucose dehydrogenase (UDP-GlcDH or HASB) activity;
(c) one or more recombinant nucleic acids encoding a polypeptide having hyaluronidase activity wherein the polypeptide having hyaluronidase activity comprises a secretion signal so that hyaluronic acid, in particular of a desired molecular weight (HAMW), is produced by the recombinant yeast cell, and
(d)
   (i) one or more recombinant nucleic acids encoding a polypeptide having a glutamine synthetase (GLN1) activity; and/or
   (ii) one or more disrupted endogeneous nucleic acids encoding a glutamate synthase (GLT1);
wherein said recombinant yeast cell belongs to the *Saccharomyces* genus, or to the *Candida* genus, or to the *Kluyveromyces* genus, or to the *Ogataea* genus, or to the *Yarrowia* genus, or to the *Debaryomyces* genus, or to the *Ashbya* genus, and is in particular selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens, Ogataea polymorpha, Yarrowia lypolytica, Debaryomyces hansenii,* and *Ashbya gossypii,* and is preferably *Saccharomyces cerevisiae.*

The present invention further relates to a recombinant host cell producing hyaluronic acid (HA) wherein the recombinant host cell comprises:
(a) one or more recombinant nucleic acids encoding a polypeptide having hyaluronan synthase activity;
(b) one or more recombinant nucleic acids encoding a polypeptide having UDP-Glucose dehydrogenase (UDP-GlcDH or HASB) activity;
(c) one or more recombinant nucleic acids encoding a polypeptide having hyaluronidase activity wherein the polypeptide having hyaluronidase activity comprises a secretion signal and an anchoring signal so that hyaluronic acid, in particular of a desired molecular weight (HAMW) is produced by the host cell; and
(d)
   (i) one or more recombinant nucleic acids encoding a polypeptide having a glutamine synthetase (GLN1) activity; and/or
   (ii) one or more disrupted endogeneous nucleic acids encoding a glutamate synthase (GLT1).

The inventors have found that an ability to produce hyaluronic acid by cells, and in particular yeast cells, may be reached by introducing in the genome of these cells a plurality of genetic alterations.

The production of hyaluronic acid by cells of the invention, and in particular by yeast cells of the invention, has been achieved by optimizing the endogenous metabolism of UDP-Glucose, and optionally UDP-N-Acetyl-glucosamine, and direct the subsequent artificially modified metabolic pathway mainly towards hyaluronic acid production while in the same time maintaining an optimal viability of the resulting genetically modified cells.

It has been determined that a hyaluronic acid production by recombinant cells according to the invention may be increased by increasing the conversion of glucose-6-phosphate into the successive intermediate metabolites (i) glucose-1-phosphate, UDP-glucose, UDP-glucuronate and hyaluronic acid and (ii) fructose-6-phosphate, glucosamine-6-phosphate, N-acetyl-glucosamine-6-phosphate, N-acetyl-glucosamine-1-phosphate, UDP-N-acetyl-glucosamine and hyaluronic acid, while maintaining a metabolic balance allowing a good viability of the resulting recombinant cells.

Indeed, in order to obtain a viable recombinant cell of the invention, many different constructs were tested in order to obtain a viable and efficient recombinant cell, and in particular a viable recombinant yeast. In particular such recombinant yeast were difficult to obtain because the temporary accumulation of some intermediates appeared to be toxic for yeasts.

Unexpected technical difficulties were encountered in establishing the conditions suitable for the preparation of a recombinant cell able to produce hyaluronic acid, and in particular to produce hyaluronic acid with a controlled molecular weight.

By "controlled" molecular weight of a hyaluronic acid of the invention is intended to mean that at least 80%, in particular at least 85%, of the hyaluronic acid produced by a method of the invention has a molecular weight comprised within a certain molecular weight's range through the regulation of at least one parameter of the method and/or of the recombinant cell of the invention, such as for example:
- the nature and origin of the nucleic acid encoding the hyaluronidase of the recombinant cell,
- the nature and origin of the promoter controlling the expression of the nucleic acid encoding the hyaluronidase(s) of the recombinant cell,
- the presence of an anchoring and/or of a secretion signal associated to the encoded hyaluronidase(s) of the recombinant cell,
- the pH of the culture medium during the step of culturing the recombinant cell, and/or
- the duration of the culturing of the recombinant cell.

Indeed, after much research and experimental trial, the inventors discovered that it was possible to cultivate a recombinant cell, and in particular a recombinant yeast cell, more particularly a recombinant *Saccharomyces cerevisiae* yeast cell, able to produce hyaluronic acid having a controlled molecular weight controlled using the following parameters:
- the selection of the nature and origin of the nucleic acid sequences encoding the polypeptide having hyaluronidase activity of the recombinant cell, in particular the recombinant yeast, of the invention; and/or
- the nature and origin of the promoter controlling the expression of the nucleic acid sequences encoding the polypeptide having hyaluronidase activity of the recombinant cell, in particular the recombinant yeast, of the invention; and/or
- the optional presence of an anchoring signal, in addition to a secretion signal, associated to the encoded polypeptide having hyaluronidase activity of the recombinant cell, in particular the recombinant yeast, according to the invention; and/or
- the pH of the culture medium during the step of culturing the recombinant cell, in particular the recombinant yeastc cell, according to the invention; and/or
- the duration of the culturing of the recombinant cell, in particular the recombinant yeast cell, according to the invention.

To the inventors' knowledge, this has never been achieved before.

The molecular weight of the hyaluronic acid (HA) can be in the range of less than 50kDa, preferably in the range of about 20kDa to about 50kDa.

Alternatively, the molecular weight of the hyaluronic acid (HA) can be in the range of greater than 50kDa, preferably in the range of about 50kDa to about 250kD.

In another alternative, the molecular weight of the HA can be in the range of greater than 100kDa, preferably in the range of about 100kDa to about 1500kDa.

The nucleic acid encoding a polypeptide having a glutamine synthetase activity may be obtained or derived from *Saccharomyces cerevisiae.*

The nucleic acid encoding a polypeptide having hyaluronidase activity in a recombinant cell of the invention may be obtained or derived from at least one of *Cupiennius salei, Loxosceles intermedia, Hirudo nipponia, Bothrops atrox* or *Tityus serrulatus.*

The nucleic acid encoding a polypeptide having hyaluronan synthase activity in a recombinant cell of the invention may be obtained or derived from at least one of *Streptococcus zooepidemicus, Chlorella virus PBCV1, Chlorella virus CviKl, Chlorella virus IL-5-2s1, Chlorella virus CZ-2, Chlorella virus CVG-1, Xenopus laevis* or *Pasteurella multocida,* and is in particular obtained or derived from *Streptococcus zooepidemicus, Chlorella virus PBCV1, Chlorella virus CviKl, Chlorella virus IL-5-2s1, Chlorella virus CZ-2, Chlorella virus CVG-1* or *Xenopus laevis.*

The nucleic acid encoding a polypeptide having UDP-Glucose dehydrogenase activity in a recombinant cell of the invention may be obtained or derived from at least one of *Arabidopsis thaliana, Chlorella virus PBCV1* or *Streptococcus zooepidemicus,* and in particular from *Arabidopsis thaliana* or *Chlorella virus PBCV1.*

The recombinant cell according to the invention can comprise a recombinant nucleic acid encoding one or more of:
(i) a polypeptide having glutamine-fructose-6-phosphate amidotransferase (GFA1) activity; and/or
(ii) a polypeptide having UDP-N-acetylglucosamine pyrophosphorylase (QRI1) activity.

The recombinant cell according to the invention can comprise at least one recombinant nucleic acid encoding one or more of:
(i) a polypeptide having Phosphoglucomutase-1 (PGM1) activity; and/or
(ii) a polypeptide having UTP-glucose-1-phosphate uridylyltransferase (UGP1) activity; and/or
(iii) a polypeptide having Glucosamine-6-phosphate N-acetyltransferase (GNA1) activity; and/or
(iv) a polypeptide having phosphoacetylglucosamine mutase (PCM1) activity.

The recombinant yeast cell according to the invention can be in particular selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens, Ogataea polymorpha, Yarrowia lypolytica, Debaryomyces hansenii,* and *Ashbya gossypii,* and is preferably *Saccharomyces cerevisiae.*

The recombinant host cell according to the invention can belong to the *Saccharomycetales* order and is in particular selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens, Ogataea polymorpha, Yarrowia lypolytica, Debaryomyces hansenii,* and *Ashbya gossypii,* and is preferably *Saccharomyces cerevisiae.*

Another object of the invention relates to a method of producing hyaluronic acid (HA) of a desired molecular weight (HAMW) comprising:
(a) cultivating a recombinant cell as defined above, i.e. a recombinant yeast cell or a recombinant host cell of the invention, in a cultivation medium for a time sufficient to produce hyaluronic acid (HA) of the desired molecular weight; and
(b) optionally isolating or recovering the hyaluronic acid (HA) from the recombinant cell and/or from the cultivation medium.

In the method of the invention, the HA may have a molecular weight of from about 20 kDa to about 50 kDA, and preferably from 20 kDa to 30 kDa.

In the method of the invention, the HA may alternatively have a molecular weight of from 30 kDa to 50 kDa.

In the method of the invention, the HA may alternatively have a molecular weight of from about 50 kDa to about 150 kDa.

In the method of the invention, the HA may alternatively have a molecular weight of from about 150 kDa to about 1500 kDa.

In a method of the invention, the nucleic acid encoding a polypeptide having hyaluronidase activity may be obtained or derived from *Cupiennius salei, Loxosceles intermedia, Hirudo nipponia, Bothrops atrox* or *Tityus serrulatus.*

In a method of the invention, the nucleic acid encoding a polypeptide having hyaluronan synthase activity may be obtained or derived from at least one of *Streptococcus zooepidemicus, Chlorella virus PBCV1, Chlorella virus CviKl, Chlorella virus IL-5-2s1, Chlorella virus CZ-2, Chlorella virus CVG-1, Xenopus laevis* or *Pasteurella multocida,* and is in particular obtained or derived from at least one of *Streptococcus zooepidemicus, Chlorella virus PBCV1, Chlorella virus CviKl, Chlorella virus IL-5-2s1, Chlorella virus CZ-2, Chlorella virus CVG-1,* or *Xenopus laevis.*

In a method of the invention, the nucleic acid encoding a polypeptide having UDP-Glucose dehydrogenase activity may be obtained or derived from at least one of *Arabidopsis thaliana, Chlorella virus PBCV1* or *Streptococcus zooepidemicus,* and in particular obtained or derived from *Arabidopsis thaliana* or *Chlorella virus PBCV1.*

In the method of the invention, the recombinant cell may comprise at least one recombinant nucleic acid encoding one or more of:
(i) a polypeptide having glutamine-fructose-6-phosphate amidotransferase (GFA1) activity; and/or
(ii) a polypeptide having UDP-N-acetylglucosamine pyrophosphorylase (QRI1) activity.

In the method of the invention, the recombinant cell may comprise at least one recombinant nucleic acid encoding one or more of:
(i) a polypeptide having Phosphoglucomutase-1 (PGM1) activity;
(ii) a polypeptide having UTP-glucose-1-phosphate uridylyltransferase (UGP1) activity;
(iii) a polypeptide having Glucosamine-6-phosphate N-acetyltransferase (GNA1) activity; and/or
(iv) a polypeptide having phosphoacetylglucosamine mutase (PCM1) activity.

In the method of the invention, the recombinant cell may be a member of the genus *Saccharomyces,* and in particular is *Saccharomyces cerevisiae.*

In the method of the invention, the molecular weight of the hyaluronic acid may be controlled by the fermentation time.

In the method of the invention, the time sufficient to produce hyaluronic acid (HA) of a desired molecular weight may be a period of from about 35 hours to about 50 hours, preferably from about 40 hours to about 50 hours, preferably about 48 hours.

In the method of the invention, the molecular weight of the hyaluronic acid may be controlled by the pH of the cultivation medium.

In the method of the invention, the molecular weight of the hyaluronic acid may be controlled by regulating the pH of the cultivation medium during the cultivation step (a) of a method of the invention.

In the method of the invention, the molecular weight of the hyaluronic acid may be controlled by removing the biomass from the cultivation medium.

The method of the invention can be carried out on an industrial scale, preferably where the cultivation medium is at least about 100L, more preferably in the range of about 1000L to about 3000L, even more preferably about 10,000L or even more preferably about 100,000L, or even about 250,000L.

Another object of the invention relates to hyaluronic acid (HA) obtained or obtainable from a recombinant cell of the invention or from the method of the invention.

A further object of the invention relates to a cultivation medium comprising the hyaluronic acid (HA) according to the invention.

The present invention further relates to a composition comprising the hyaluronic acid (HA) according to the invention.

The present invention moreover relates to an industrial product or a consumer product or a consumable comprising (i) the HA having a molecular weight in the range of greater than 100kDa, preferably in the range of about 100kDa to about 1500kDa, (ii) the cultivation medium of the invention or (iii) the composition of the invention.

The present invention also relates to use of a recombinant cell according to the invention for the production of hyaluronic acid (HA) having a molecular weight in the range of from about 20kDa to about 50kDa or from about 50kDa to about 1000 kDa.

Another object of the present invention relates to a method for producing hyaluronic acid comprising the steps of:
(a) culturing a recombinant yeast according to the invention in a culture medium; and
(b) recovering the hyaluronic acid from said culture medium,
   wherein the hyaluronic acid recovered in step (b) has a molecular weight controlled through the selection of:
   - the nature and origin of the nucleic acid encoding the hyaluronidase of the recombinant yeast,
   - the nature and origin of the promoter controlling the expression of the nucleic acid encoding the hyaluronidase(s) of the recombinant yeast,
   - the presence of an anchoring and/or of a secretion signal associated to the encoded hyaluronidase(s) of the recombinant yeast,
   - the pH of the culture medium during the step of culturing the recombinant yeast, and/or
   - the duration of the culturing of the recombinant yeast.

More particularly, the invention pertains to a hyaluronic acid-producing recombinant yeast, wherein the recombinant yeast comprises:
(A) one or more recombinant nucleic acids encoding a polypeptide having glutamine-fructose-6-phosphate amidotransferase (GFA1) activity;
(B) one or more recombinant nucleic acids encoding a polypeptide having a UDP-N-acetylglucosamine pyrophosphorylase (QRI1) activity;
(C) one or more recombinant nucleic acids encoding a polypeptide having a UDP-Glucose dehydrogenase (UDP-GlcDH or HASB) activity;
(D) one or more recombinant nucleic acids encoding a polypeptide having a hyaluronan synthase (HASA) activity; and
(E) one or more recombinant nucleic acids encoding a polypeptide having a hyaluronidase activity wherein the polypeptide having hyaluronidase activity comprises a secretion signal so that hyaluronic acid, in particular of a desired molecular weight (HAMW), is produced by the recombinant yeast cell,
said recombinant yeast being *Saccharomyces cerevisiae.*

In a particular embodiment, the recombinant cell according to the invention, and in particular the recombinant yeast according to the invention, comprises one recombinant nucleic acid encoding a polypeptide having glutamine synthetase (GLN1) activity.

In a particular embodiment, the recombinant cell according to the invention, and in particular the recombinant yeast according to the invention, is such that at least one, and in particular all, its endogeneous nucleic acids encoding a glutamate synthase (GLT1) are disrupted.

In a particular embodiment, the recombinant cell according to the invention, and in particular the recombinant yeast according to the invention:
- comprises one recombinant nucleic acid encoding a polypeptide having glutamine synthetase (GLN1) activity, and in particular a polypeptide having glutamine synthetase (GLN1) activity obtained or derived from *Saccharomyces cerevisiae;* and
- is such that at least one, and in particular all, its endogeneous nucleic acids encoding a glutamate synthase (GLT1) are disrupted.

In a particular embodiment, the recombinant cell according to the invention, and in particular the recombinant yeast according to the invention comprises only one recombinant nucleic acid encoding a polypeptide having glutamine-fructose-6-phosphate amidotransferase (GFA1) activity.

In a particular embodiment, the recombinant cell according to the invention, and in particular the recombinant yeast comprises between 5 and 10 recombinant nucleic acids encoding a polypeptide having UDP-N-acetylglucosamine pyrophosphorylase (QRI1) activity.

In a particular embodiment, the recombinant cell according to the invention, and in particular the recombinant yeast comprises between 3 and 7 recombinant nucleic acids encoding a polypeptide having UDP-Glucose dehydrogenase (UDP-GlcDH or HASB) activity.

In another embodiment, the one or more nucleic acids encoding a polypeptide having a UDP-Glucose dehydrogenase (UDP-GlcDH or HASB) activity is obtained or derived from at least one of *Arabidopsis thaliana, Chlorella virus PPCV1* or *Streptococcus zooepidemicus,* and in particular obtained or derived from *Arabidopsis thaliana* or *Chlorella virus PBCV1.*

In a particular embodiment, the recombinant cell according to the invention, and in particular the recombinant yeast comprises between 4 and 8 recombinant nucleic acids encoding a polypeptide having hyaluronan synthase (HASA) activity.

In another embodiment, the one or more recombinant nucleic acids encoding a polypeptide having a hyaluronan synthase (HASA) activity is obtained or derived from at least one of *Streptococcus zooepidemicus, Chlorella virus PBCV1, Chlorella virus CviKl, Chlorella virus IL-5-2s1, Chlorella virus CZ-2, Chlorella virus CVG-1, Xenopus laevis* or *Pasteurella multocida,* and is in particular obtained or derived from *Streptococcus zooepidemicus, Chlorella virus PBCV1, Chlorella virus CviKl, Chlorella virus IL-5-2s1, Chlorella virus CZ-2, Chlorella virus CVG-1* or *Xenopus laevis.*

In a particular embodiment, the recombinant cell according to the invention, and in particular the recombinant yeast of the invention only comprises one recombinant nucleic acid encoding a polypeptide having hyaluronidase activity.

In another embodiment, the one or more recombinant nucleic acids encoding a polypeptide having a hyaluronidase activity is obtained or derived from *Cupiennius salei, Loxosceles intermedia, Hirudo nipponia, Bothrops atrox* or *Tityus serrulatus.*

In another embodiment, the recombinant cell according to the invention, and in particular the recombinant yeast cell may comprise at least one recombinant nucleic acid encoding one or more of:
(A) a polypeptide having Phosphoglucomutase-1 (PGM1) activity; and/or
(B) a polypeptide having UTP-glucose-1-phosphate uridylyltransferase (UGP1) activity; and/or
(C) a polypeptide having Glucosamine-6-phosphate N-acetyltransferase (GNA1) activity; and/or
(D) a polypeptide having phosphoacetylglucosamine mutase (PCM1) activity.

In particular, the recombinant cell according to the invention, and in particular the recombinant yeast cell of the invention comprises at least two, in particular at least three, and more particularly all of the modifications indicated above.

In a particular embodiment, the nucleic acid encoding a polypeptide having Phosphoglucomutase-1 (PGM1) activity, the nucleic acid encoding a polypeptide having UTP-glucose-1-phosphate uridylyltransferase (UGP1) activity, the nucleic acid encoding a polypeptide having glutamine-fructose-6-phosphate amidotransferase (GFA1) activity, the nucleic acid encoding a polypeptide having Glucosamine-6-phosphate N-acetyltransferase (GNA1) activity, the nucleic acid encoding a polypeptide having phosphoacetylglucosamine mutase (PCM1) activity and the nucleic acid encoding a polypeptide having UDP-N-acetylglucosamine pyrophosphorylase (QRI1) activity are nucleic acids originating or derivating from a yeast, preferably from *Saccharomyces cerevisiae.*

In a particular embodiment, the one or more recombinant nucleic acids encoding a polypeptide defined above and comprised in a recombinant cell according to the invention, and in particular comprised in the recombinant yeast of the invention are under the control of a promoter selected from the group consisting of pPDC1, pTDH3, pCCW12, pCCW12.Sm, pCCW12.sk, pCCW12.sba, pCCW12.sar, pTEF1, pENO2, pRPLA1, pNUP57 and pTEF3.

In a particular embodiment, the inducible or repressible promoters mentioned in the present specification are selected from the group consisting of promoters inducible or repressible with copper or promoters inducible or repressible with methionine, in particular selected from the group consisting of pMET6, pMET25 and pSAM1.

The invention further relates to a method for producing hyaluronic acid as previously described, and comprising the steps of:
(a) culturing a recombinant cell according to the invention, and in particular a recombinant yeast as defined herein in a culture medium; and
(b) recovering the hyaluronic acid from said culture medium,
   wherein the hyaluronic acid recovered in step (b) has a molecular weight controlled through the selection of:
   - the nature and origin of the nucleic acid encoding the hyaluronidase of the recombinant cell according to the invention, and in particular the recombinant yeast according to the invention, and/or
   - the nature and origin of the promoter controlling the expression of the nucleic acid encoding the hyaluronidase(s) of the recombinant cell according to the invention, and in particular the recombinant yeast, and/or
   - the presence of an anchoring and/or of a secretion signal associated to the encoded hyaluronidase(s) of the recombinant cell according to the invention, and in particular the recombinant yeast, and/or
   - the pH of the culture medium during the step of culturing the recombinant cell according to the invention, and in particular the recombinant yeast; and/or
   - the duration of the culturing of the recombinant cell according to the invention, and in particular the recombinant yeast.

### Recombinant nucleic acids encoding a polypeptide having hyaluronan synthase activity

A recombinant cell according to the invention, and in particular a recombinant yeast of the invention, comprises one or more recombinant nucleic acids encoding a polypeptide having hyaluronan synthase activity.

A polypeptide having hyaluronan synthase activity according to the invention means a polypeptide that converts the intermediate metabolites UDP-Glucoronate and UDP-N-acetylglucosamine (UDP-GlcNAc) into hyaluronic acid ((β-D-1,3-GlcNAc-β-D-1,4-GlcA)n).

In an embodiment, one or more of the recombinant nucleic acids encoding a polypeptide having hyaluronan synthase activity are under the control of an inducible or repressible promoter that is functional in the recombinant cells of the invention, such as for example the inducible or repressible promoters pCUP1or pMET25.

One or more of the recombinant nucleic acids encoding a polypeptide having hyaluronan synthase activity may be under the control of a promoter selected from the group consisting of pCCW12, pCCW12.Sm, pTDH3-1.Sba and pTDH3.Sar.

The said one or more recombinant nucleic acids encoding a polypeptide having hyaluronan synthase activity may originate or be derived from at least one of *Streptococcus zooepidemicus (sz), Chlorella virus PBCV1 (Vir), Chlorella virus CviKl (Vir), Chlorella virus IL-5-2s1 (Vir), Chlorella virus CZ-2 (Vir), Chlorella virus CVG-1 (Vir), Xenopus laevis (xl)* or *Pasteurella multocida (pm),* and may originate or derive in particular from *Streptococcus zooepidemicus, Chlorella virus PBCV1, Chlorella virus CviKl, Chlorella virus IL-5-2s1, Chlorella virus CZ-2, Chlorella virus CVG-1* or *Xenopus laevis,* as shown in the examples herein.

A recombinant cell according to the invention, and in particular a recombinant yeast according to the invention, may comprise between 2 and 8, in particular between 4 and 8 recombinant nucleic acids encoding a polypeptide having hyaluronan synthase (HASA) activity. A recombinant cell according to the invention, and in particular a recombinant yeast according to the invention, may for example comprise 2 or 6, and in particular 6, recombinant nucleic acids encoding a polypeptide having hyaluronan synthase (HASA) activity.

Illustratively, the one or more recombinant nucleic acids encoding a polypeptide having hyaluronan synthase activity may be inserted within the JLP1 gene and/or within the SAM3 gene and/or within the TRP1 gene and/or within the LYP1 gene of the recombinant cell, and in particular of the recombinant yeast, as it is shown in the examples herein.

In an embodiment of the invention, a recombinant cell, and in particular a recombinant yeast, of the invention comprises:
- between 2 and 8, in particular between 4 and 8 recombinant nucleic acids encoding a polypeptide having hyaluronan synthase (HASA) activity;
- said one or more recombinant nucleic acids encoding a polypeptide having hyaluronan synthase activity originating or being derived from at least one of *Streptococcus zooepidemicus (sz), Chlorella virus PBCV1 (Vir), Xenopus laevis (xl)* or *Pasteurella multocida (pm),* and originating or being derived in particular from at least one of *Streptococcus zooepidemicus, Chlorella virus PBCV1* or *Xenopus laevis; and*
- said one or more recombinant nucleic acids encoding a polypeptide having hyaluronan synthase activity being under the control of an inducible or repressible promoter that is functional in the recombinant cells of the invention, such as for example the inducible or repressible promoters pCUP or pMET25 and/or under the control of a promoter selected from the group consisting of pCCW12, pCCW12.Sm, pTDH3-1.Sba and pTDH3.Sar.

### Recombinant nucleic acids encoding a polypeptide having UDP-Glucose dehydrogenase activity

A recombinant cell according to the invention, and in particular a recombinant yeast according to the invention, comprises one or more recombinant nucleic acids encoding a polypeptide having UDP-Glucose dehydrogenase (UDP-GlcDH or HASB) activity.

A polypeptide having UDP-Glucose dehydrogenase (UDP-GlcDH or HASB) activity according to the invention means a polypeptide that converts the intermediate metabolite Uridine-Diphosphate-Glucose (UDP-Glucose) into UDP-Glucuronate.

In an embodiment, one or more of the recombinant nucleic acids encoding a polypeptide having UDP-Glucose dehydrogenase (UDP-GlcDH or HASB) activity are under the control of an inducible or repressible promoter that is functional in recombiant cells of the invention, such as for example the inducible or repressible promoters pMET25 or pMET6.

One or more of the recombinant nucleic acids encoding a polypeptide having UDP-Glucose dehydrogenase activity may be under the control of a promoter selected from the group consisting of pCCW12, in particular the pCCW12.sk and the pCCW12.sba promoters; pTEF1.Sba and pTDH3.Sk.

The one or more recombinant nucleic acids encoding a polypeptide having UDP-Glucose dehydrogenase activity may originate or be derived from at least one of *Arabidopsis thaliana, Chlorella virus PPCV1* or *Streptococcus zooepidemicus*, and in particular may originate or derive from *Arabidopsis thaliana* or *Chlorella virus PBCV1.*

A recombinant cell according to the invention, and in particular a recombinant yeast according to the invention, may comprise between 2 and 7, in particular between 3 and 7 recombinant nucleic acids encoding a polypeptide having UDP-Glucose dehydrogenase activity. A recombinant cell according to the invention, and in particular a recombinant yeast according to the invention, may for example comprise 2 or 5 recombinant nucleic acids encoding a polypeptide having UDP-Glucose dehydrogenase activity.

Illustratively, the one or more recombinant nucleic acids encoding a polypeptide having UDP-Glucose dehydrogenase activity may be inserted within the JLP1 gene and/or within the TRP1 gene, and/or within the LYP1 gene of the recombinant cell, and in particular of the recombinant yeast, as it is shown in the examples herein.

In an embodiment of the invention, a recombinant cell, and in particular a recombinant yeast, of the invention comprises:
- between 2 and 7, and in particular between 3 and 7 recombinant nucleic acids encoding a polypeptide having UDP-Glucose dehydrogenase activity;
- said one or more recombinant nucleic acids encoding a polypeptide having UDP-Glucose dehydrogenase activity originating or being derived from at least one of *Arabidopsis thaliana, Chlorella virus PPCV1* or *Streptococcus zooepidemicus,* and originating or being derived in particular from *Arabidopsis thaliana* or *Chlorella virus PBCV1*; and
- said one or more recombinant nucleic acids encoding a polypeptide having UDP-Glucose dehydrogenase activity being under the control of an inducible or repressible promoter that is functional in the recombinant cells of the invention, such as for example the inducible or repressible promoters pMET25 or pMET6 and/or under the control of a promoter selected from the group consisting of pCCW12, in particular the pCCW12.sk and the pCCW12.sba promoters; pTEF1.Sba and pTDH3.Sk.

### Recombinant nucleic acids encoding a polypeptide having Hyaluronidase activity

A recombinant cell according to the invention, and in particular a recombinant yeast according to the invention, comprises one or more recombinant nucleic acids encoding a polypeptide having hyaluronidase activity.

A polypeptide having hyaluronidase activity according to the invention means a polypeptide that degrades hyaluronic acid, i.e. that converts a hyaluronic acid of a given molecular weight into a hyaluronic acid of a lower molecular weight.

As previously indicated, polypeptides having hyaluronidase activity of the present invention comprise a secretion signal.

In an embodiment, polypeptide having hyaluronidase activity comprise both a secretion signal and an anchoring signal.

In an embodiment, the said one or more recombinant nucleic acids encoding a polypeptide having hyaluronidase activity are under the control of an inducible or repressible promoter that is functional in the recombinant cells of the invention.

One or more of the recombinant nucleic acids encoding a polypeptide having hyaluronidase activity may be under the control of a promoter selected from the group consisting of pTEF1, pCCW12, pCCW12.sba, pCCW12.Sar, pPDC1, pTEF3, pTDH3, pNUP57, pCWP2 and pCCW10.ago.

The said one or more recombinant nucleic acids encoding a polypeptide having hyaluronidase activity may originate or be derived from at least one of *Cupiennius salei (Csa), Loxosceles intermedia (Li), Hirudo nipponia (Hn), Bothrops atrox (Ba)* or *Tityus serrulatus (Ts)* as shown in the examples herein.

A recombinant cell according to the invention, and in particular a recombinant yeast according to the invention, may comprise only one recombinant nucleic acid encoding a polypeptide having hyaluronidase activity.

Illustratively, the one or more recombinant nucleic acids encoding a polypeptide having hyaluronidase activity may be inserted within the JLP1 gene and/or within the LYP1 gene, as it is shown in the examples herein.

In an embodiment of the invention, a recombinant cell, and in particular a recombinant yeast, of the invention comprises:
- only one recombinant nucleic acid encoding a polypeptide having hyaluronidase activity;
- said recombinant nucleic acid encoding a polypeptide having hyaluronidase activity originating or being derived from *Cupiennius salei (Csa), Loxosceles intermedia (Li), Hirudo nipponia (Hn), Bothrops atrox (Ba)* or *Tityus serrulatus (Ts);*
- said recombinant nucleic acid encoding a polypeptide having hyaluronidase activity comprising either (i) a secretion signal and no anchoring signal or (ii) a secretion signal and an anchoring signal; and
- said recombinant nucleic acid encoding a polypeptide having hyaluronidase activity being under the control of a promoter selected from the group consisting of pTEF1, pCCW12, pCCW12.sba, pCCW12.Sar, pPDC1, pTEF3, pTDH3, pNUP57, pCWP2 and pCCW10.ago.

### Recombinant nucleic acids encoding a polypeptide having glutamine synthetase activity

A recombinant cell according to the invention, and in particular a recombinant yeast cell according to the invention, may comprise one or more recombinant nucleic acids encoding a polypeptide having glutamine synthetase activity.

A polypeptide having glutamine synthetase activity according to the invention means a polypeptide that converts glutamate into glutamine while consuming one ATP and one NH₄⁺.

In an embodiment, one or more of the recombinant nucleic acids encoding a polypeptide having glutamine synthetase activity are under the control of an inducible or repressible promoter that is functional in the recombinant cells of the invention.

One or more of the recombinant nucleic acids encoding a polypeptide having glutamine synthetase activity may be under the control of a promoter selected from the group consisting of pTEF1 and pTEF1.Ago.

The said one or more recombinant nucleic acids encoding a polypeptide having glutamine synthetase activity can originate or be derived from *Saccharomyces cerevisiae,* as shown in the examples herein.

A recombinant cell according to the invention, and in particular a recombinant yeast cell according to the invention, may comprise only one recombinant nucleic acid encoding a polypeptide having glutamine synthetase activity.

Illustratively, the one or more recombinant nucleic acids encoding a polypeptide having glutamine synthetase activity may be inserted within the LYP1 or GLT1 gene of the recombinant cell, and in particular of the recombinant yeast cell, as it is shown in the examples herein.

In an embodiment of the invention, a recombinant cell, and in particular a recombinant yeast cell, of the invention comprises:
- only one recombinant nucleic acid encoding a polypeptide having glutamine synthetase activity;
- said recombinant nucleic acid encoding a polypeptide having glutamine synthetase activity originating or being derived from *Saccharomyces cerevisiae;* and
- said recombinant nucleic acid encoding a polypeptide having glutamine synthetase activity being under the control of a promoter selected from the group consisting of pTEF1 and pTEF1.Ago.

In a further embodiment of the invention, a recombinant cell, and in particular a recombinant yeast cell, of the invention comprises:
- only one recombinant nucleic acid encoding a polypeptide having glutamine synthase activity;
- said recombinant nucleic acid encoding a polypeptide having glutamine synthetase activity originating or being derived from *Saccharomyces cerevisiae;*
- said recombinant nucleic acid encoding a polypeptide having glutamine synthetase activity being under the control of a promoter selected from the group consisting of pTEF1 and pTEF1.Ago; and
- said recombinant nucleic acid encoding a polypeptide having glutamine synthetase activity being inserted within the LYP1 or GLT1 gene of the recombinant cell, and in particular of the recombinant yeast cell.

### Recombinant nucleic acids encoding a polypeptide having Glutamine-fructose-6-phosphate amidotransferase activity

A recombinant cell according to the invention, and in particular a recombinant yeast cell according to the invention, may comprise one or more recombinant nucleic acids encoding a polypeptide having glutamine-fructose-6-phosphate amidotransferase activity.

A polypeptide having glutamine-fructose-6-phosphate amidotransferase activity according to the invention means a polypeptide that converts fructose-6-phosphate into glucosamine-6-phosphate.

In an embodiment, one or more of the recombinant nucleic acids encoding a polypeptide having glutamine-fructose-6-phosphate amidotransferase activity are under the control of an inducible or repressible promoter that is functional in the recombinant cells of the invention.

One or more of the recombinant nucleic acids encoding a polypeptide having glutamine-fructose-6-phosphate amidotransferase activity may be under the control of a promoter selected from the group consisting of pTEF1 and pTEF1.Ago.

The said one or more recombinant nucleic acids encoding a polypeptide having glutamine-fructose-6-phosphate amidotransferase activity can originate or be derived from *Saccharomyces cerevisiae,* as shown in the examples herein.

A recombinant cell according to the invention, and in particular a recombinant yeast cell according to the invention, may comprise only one recombinant nucleic acid encoding a polypeptide having glutamine-fructose-6-phosphate amidotransferase activity.

Illustratively, the one or more recombinant nucleic acids encoding a polypeptide having glutamine-fructose-6-phosphate amidotransferase activity may be inserted within the SAM3 gene of the recombinant cell, and in particular of the recombinant yeast cell, as it is shown in the examples herein.

In an embodiment of the invention, a recombinant cell, and in particular a recombinant yeast cell, of the invention comprises:
- only one recombinant nucleic acid encoding a polypeptide having glutaminefructose-6-phosphate amidotransferase activity;
- said recombinant nucleic acid encoding a polypeptide having glutaminefructose-6-phosphate amidotransferase activity originating or being derived from *Saccharomyces cerevisiae;* and
- said recombinant nucleic acid encoding a polypeptide having glutaminefructose-6-phosphate amidotransferase activity being under the control of a promoter selected from the group consisting of pTEF1 and pTEF1.Ago.

### Recombinant nucleic acids encoding a polypeptide having UDP-N-acetylglucosamine pyrophosphorylase activity

A recombinant cell according to the invention, and in particular a recombinant yeast cell according to the invention, can comprise one or more recombinant nucleic acids encoding a polypeptide having UDP-N-acetylglucosamine pyrophosphorylase activity.

A polypeptide having UDP-N-acetylglucosamine pyrophosphorylase activity according to the invention means a polypeptide that can convert N-acetyl-glucosamine into UDP-N-acetyl-glucosamine.

In an embodiment, one or more of the recombinant nucleic acids encoding a polypeptide having UDP-N-acetylglucosamine pyrophosphorylase activity are under the control of an inducible or repressible promoter that is functional in the recombinant cells of the invention, such as for example the inducible or repressible promoters pMET6 or pCUP1.

One or more of the recombinant nucleic acids encoding a polypeptide having UDP-N-acetylglucosamine pyrophosphorylase activity may be under the control of the promoter pTDH3.

The said one or more recombinant nucleic acids encoding a polypeptide having UDP-N-acetylglucosamine pyrophosphorylase activity can originate or be derived from *Saccharomyces cerevisiae,* as shown in the examples herein.

A recombinant cell according to the invention, and in particular a recombinant yeast according to the invention, may comprise between 5 and 10 recombinant nucleic acids encoding a polypeptide having UDP-N-acetylglucosamine pyrophosphorylase activity. A recombinant cell according to the invention, and in particular a recombinant yeast cell according to the invention, may for example comprise 5, 7 or 8 recombinant nucleic acids encoding a polypeptide having UDP-N-acetylglucosamine pyrophosphorylase activity.

Illustratively, the one or more recombinant nucleic acids encoding a polypeptide having UDP-N-acetylglucosamine pyrophosphorylase activity may be inserted within the HIS3 gene and/or within the JLP1 gene and/or within the SAM3 gene of the recombinant cell, and in particular of the recombinant yeast, as it is shown in the examples herein.

In an embodiment of the invention, a recombinant cell, and in particular a recombinant yeast cell, of the invention comprises:
- between 5 and 10 recombinant nucleic acids encoding a polypeptide having UDP-N-acetylglucosamine pyrophosphorylase activity;
- said one or more recombinant nucleic acids encoding a polypeptide having UDP-N-acetylglucosamine pyrophosphorylase activity originating or being derived from *Saccharomyces cerevisiae;*
- said one or more recombinant nucleic acids encoding a polypeptide having UDP-N-acetylglucosamine pyrophosphorylase activity being under the control of an inducible or repressible promoter that is functional in the recombinant cells of the invention, such as for example the inducible or repressible promoters pMET6 or pCUP1and/or under the control of the promoter pTDH3.

### Recombinant nucleic acids encoding a polypeptide having Phosphoglucomutase-1 activity

A recombinant cell according to the invention, and in particular a recombinant yeast cell according to the invention, may comprise one or more recombinant nucleic acids encoding a polypeptide having Phosphoglucomutase-1 (PGM1) activity.

A polypeptide having Phosphoglucomutase-1 (PGM1) activity according to the invention means a polypeptide that converts Glucose-6-phosphate into the intermediate metabolite Glucose-1-phosphate.

In an embodiment, one or more of the recombinant nucleic acids encoding a polypeptide having Phosphoglucomutase-1 (PGM1) activity are under the control of an inducible or repressible promoter that is functional in recombiant cells of the invention, such as for example the inducible or repressible promoters.

One or more of the recombinant nucleic acids encoding a polypeptide having Phosphoglucomutase-1 (PGM1) activity may be under the control of the promoter pPDC1.

The one or more recombinant nucleic acids encoding a polypeptide having Phosphoglucomutase-1 (PGM1) activity may originate or be derived from *Saccharomyces cerevisiae,* as shown in the examples herein.

A recombinant cell according to the invention, and in particular a recombinant yeast cell according to the invention, may comprise only one recombinant nucleic acid encoding a polypeptide having Phosphoglucomutase-1 (PGM1) activity.

Illustratively, the one or more recombinant nucleic acids encoding a polypeptide having Phosphoglucomutase-1 activity may be inserted within the SAM3 gene of the recombinant cell, and in particular of the recombinant yeast, as it is shown in the examples herein.

In an embodiment of the invention, a recombinant cell, and in particular a recombinant yeast, of the invention comprises:
- only one recombinant nucleic acid encoding a polypeptide having Phosphoglucomutase-1 activity;
- said recombinant nucleic acid encoding a polypeptide having Phosphoglucomutase-1 activity originating or being derived from *Saccharomyces cerevisiae;* and
- said recombinant nucleic acid encoding a polypeptide having Phosphoglucomutase-1 activity being under the control of the promoter pPDC1.

### Recombinant nucleic acids encoding a polypeptide having UTP-glucose-1-phosphate uridylyltransferase activity

A recombinant cell according to the invention, and in particular a recombinant yeast cell according to the invention, comprises one or more recombinant nucleic acids encoding a polypeptide having UTP-glucose-1-phosphate uridylyltransferase (UGP1) activity.

A polypeptide having UTP-glucose-1-phosphate uridylyltransferase (UGP1) activity according to the invention means a polypeptide that converts the intermediate metabolite Glucose-1-Phosphate into the intermediate metabolite UDP-glucose.

In an embodiment, one or more of the recombinant nucleic acids encoding a polypeptide having UTP-glucose-1-phosphate uridylyltransferase activity are under the control of an inducible or repressible promoter that is functional in recombiant cells of the invention, such as for example the inducible or repressible promoters pSAM1 or pCUP1.

One or more of the recombinant nucleic acids encoding a polypeptide having UTP-glucose-1-phosphate uridylyltransferase activity may be under the control of a promoter selected from the group consisting of pPDC1and pENO2.

The one or more recombinant nucleic acids encoding a polypeptide having UTP-glucose-1-phosphate uridylyltransferase activity can originate or be derived from *Saccharomyces cerevisiae,* as shown in the examples herein.

A recombinant cell according to the invention, and in particular a recombinant yeast cell according to the invention, can comprise between 5 and 10 recombinant nucleic acids encoding a polypeptide having UTP-glucose-1-phosphate uridylyltransferase activity. A recombinant cell according to the invention, and in particular a recombinant yeast cell according to the invention, may for example comprise 5, 7 or 8 recombinant nucleic acids encoding a polypeptide having UTP-glucose-1-phosphate uridylyltransferase activity.

Illustratively, the one or more recombinant nucleic acids encoding a polypeptide having UTP-glucose-1-phosphate uridylyltransferase activity gene may be inserted within the HIS3 gene and/or within the JLP1 gene and/or within the SAM3 gene of the recombinant cell, and in particular of the recombinant yeast, as it is shown in the examples herein.

In an embodiment of the invention, a recombinant cell, and in particular a recombinant yeast cell, of the invention comprises:
- between 5 and 10 recombinant nucleic acids encoding a polypeptide having UTP-glucose-1-phosphate uridylyltransferase activity;
- said one or more recombinant nucleic acids encoding a polypeptide having UTP-glucose-1-phosphate uridylyltransferase activity originating or being derived from *Saccharomyces cerevisiae*; and
- said one or more recombinant nucleic acids encoding a polypeptide having being under the control of an inducible or repressible promoter that is functional in the recombinant cells of the invention, such as for example the inducible or repressible promoters pSAM1 or pCUP1 and/or under the control of a promoter selected from the group consisting of pPDC1 and pENO2.

### Recombinant nucleic acids encoding a polypeptide having Glucosamine-6-phosphate N-acetyltransferase activity

A recombinant cell according to the invention, and in particular a recombinant yeast cell according to the invention, can comprise one or more recombinant nucleic acids encoding a polypeptide having Glucosamine-6-phosphate N-acetyltransferase (GNA1) activity.

A polypeptide having Glucosamine-6-phosphate N-acetyltransferase (GNA1) activity according to the invention means a polypeptide that can convert Glucosamine-6-phosphate into N-acetyl-glucosamine-6-phosphate.

In an embodiment, one or more of the recombinant nucleic acids encoding a polypeptide having Glucosamine-6-phosphate N-acetyltransferase activity are under the control of an inducible or repressible promoter that is functional in the recombinant cells of the invention.

One or more of the recombinant nucleic acids encoding a polypeptide having Glucosamine-6-phosphate N-acetyltransferase activity may be under the control of the promoter pCWP2.

The said one or more recombinant nucleic acids encoding a polypeptide having Glucosamine-6-phosphate N-acetyltransferase activity may originate or be derived from *Saccharomyces cerevisiae,* as shown in the examples herein.

A recombinant cell according to the invention, and in particular a recombinant yeast cell according to the invention, can comprise only one recombinant nucleic acid encoding a polypeptide having Glucosamine-6-phosphate N-acetyltransferase activity.

Illustratively, the one or more recombinant nucleic acids encoding a polypeptide having Glucosamine-6-phosphate N-acetyltransferase activity may be inserted within the SAM3 gene of the recombinant cell, and in particular of the recombinant yeast, as it is shown in the examples herein.

In an embodiment of the invention, a recombinant cell, and in particular a recombinant yeast, of the invention comprises:
- only one recombinant nucleic acid encoding a polypeptide having Glucosamine-6-phosphate N-acetyltransferase activity;
- said recombinant nucleic acid encoding a polypeptide having Glucosamine-6-phosphate N-acetyltransferase activity originating or being derived from *Saccharomyces cerevisiae;* and
- said recombinant nucleic acid encoding a polypeptide having Glucosamine-6-phosphate N-acetyltransferase activity being under the control of the promoter pCWP2.

### Recombinant nucleic acids encoding a polypeptide having Phosphoacetylglucosamine mutase activity

A recombinant cell according to the invention, and in particular a recombinant yeast cell according to the invention, can comprise one or more recombinant nucleic acids encoding a polypeptide having phosphoacetylglucosamine mutase (PCM1) activity.

A polypeptide having phosphoacetylglucosamine mutase (PCM1) activity according to the invention means a polypeptide that can convert N-acetyl-glucosamine-6-phosphate N-acetyl-glucosamine-1-phosphate.

In an embodiment, one or more of the recombinant nucleic acids encoding a polypeptide having phosphoacetylglucosamine mutase activity are under the control of an inducible or repressible promoter that is functional in the recombinant cells of the invention.

One or more of the recombinant nucleic acids encoding a polypeptide having phosphoacetylglucosamine mutase activity may be under the control a promoter selected from the group consisting of pTEF3 and pTEF1.

The said one or more recombinant nucleic acids encoding a polypeptide having phosphoacetylglucosamine mutase activity can originate or be derived from *Saccharomyces cerevisiae,* as shown in the examples herein.

A recombinant cell according to the invention, and in particular a recombinant yeast cell according to the invention, may comprise only one recombinant nucleic acid encoding a polypeptide having phosphoacetylglucosamine mutase activity.

Illustratively, the one or more recombinant nucleic acids encoding a polypeptide having phosphoacetylglucosamine mutase activity may be inserted within the SAM3 gene of the recombinant cell, and in particular of the recombinant yeast, as it is shown in the examples herein.

In an embodiment of the invention, a recombinant cell, and in particular a recombinant yeast cell, of the invention comprises:
- only one recombinant nucleic acid encoding a polypeptide having phosphoacetylglucosamine mutase activity;
- said recombinant nucleic acid encoding a polypeptide having phosphoacetylglucosamine mutase activity originating or being derived from *Saccharomyces cerevisiae;* and
- said recombinant nucleic acid encoding a polypeptide having phosphoacetylglucosamine mutase activity being under the control of the promoter a promoter selected from the group consisting of pTEF3 and pTEF1.

### HYALURONAN SYNTHASE (HASA)

The hyaluronan synthase enzyme is a protein which is described in the art for catalyzing the conversion of UDP-glucuronate or UDP-N-acetyl-glucose into hyaluronic acid. The hyaluronan synthase originating from *Streptococcus zooepidemicus, Chlorella virus PBCV1, Xenopus laevis* or *Pasteurella multocida* may be termed HASA.

A method implemented to measure the activity level of a polypeptide having hyaluronan synthase activity belongs to the general knowledge of the one skilled in the art.

In this regard, the one skilled in the art may advantageously refer to the method of colorimetric determination after treatment with concentrated sulfuric acid and carbazole described by Bitter and Muir (Analytical Biochemistry, 4, 330-334, 1962).

Preferred polypeptide having hyaluronan synthase activity in the present invention is an enzyme having an EC number of n° 2.4.1.212.

According to a preferred embodiment, the one or more recombinant nucleic acids encoding a polypeptide having hyaluronan synthase activity may originate or be derived from organisms preferably selected in a group consisting of prokaryotic organisms and eukaryotic organisms. In some embodiments, the one or more recombinant nucleic acids encoding a polypeptide having hyaluronan synthase activity may originate or be derived from archaebacteria. In some embodiments, the one or more recombinant nucleic acids encoding a polypeptide having hyaluronan synthase activity may originate or be derived from bacteria, and especially from *Streptococcus zooepidemicus (Sz)* or from *Pasteurella multocida (Pm), from Chlorella virus PBCV1 (Vir), Chlorella virus CviKl* (Vir), *Chlorella virus IL-5-2s1* (Vir), *Chlorella virus CZ-2* (Vir), *Chlorella virus CVG-1* (Vir) or from *Xenopus laevis* (Xl).

According to a yet preferred embodiment, the one or more recombinant nucleic acids encoding a polypeptide having hyaluronan synthase activity may be selected from the group consisting of nucleic acid sequences having (i) at least 65%, advantageously at least 70%, preferably at least 80%, nucleic acid identity with a nucleic acid sequence as set forth as sequence SEQ ID NO: 1 (Vir), SEQ ID NO: 2 (Vir), SEQ ID NO: 3 (Pm), SEQ ID NO: 4 (Pm), SEQ ID NO: 5 (Pm), SEQ ID NO: 6 (Xl), SEQ ID NO: 7 (sz), SEQ ID NO: 101 (Vir), SEQ ID NO: 102 (Vir), SEQ ID NO: 103 (Vir) or SEQ ID NO: 104 (Vir) and (ii) a biological activity of the same nature as the nucleic acid sequence having, respectively, the nucleic acid sequence as set forth as sequence SEQ ID NO: 1 (Vir), SEQ ID NO: 2 (Vir), SEQ ID NO: 3 (Pm), SEQ ID NO: 4 (Pm), SEQ ID NO: 5 (Pm), SEQ ID NO: 6 (Xl), SEQ ID NO: 7 (sz), SEQ ID NO: 101 (Vir), SEQ ID NO: 102 (Vir), SEQ ID NO: 103 (Vir) or SEQ ID NO: 104 (Vir).

A biological activity of the same nature regarding this sequence is, as previously explained, the capacity to code for a polypeptide that converts UDP-glucuronate or UDP-N-acetyl-glucose into hyaluronic acid.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having hyaluronan synthase activity originating from *Streptococcus zooepidemicus, Chlorella virus PBCV1, Xenopus laevis* or *Pasteurella multocida,* the one skilled in the art may refer, respectively, to the accession numbers B4U0D4, Q84419, P13563, Q7BLV3, M1GZS8, M1H5V3, M1H2Q1 and M1HN86 in the UniProt database, or to SEQ ID NO: 8 (Vir), SEQ ID NO: 9 (Pm) SEQ ID NO: 10 (Xl), SEQ ID NO:11 (Sz), SEQ ID NO: 105 (Vir), SEQ ID NO: 106 (Vir), SEQ ID NO: 107 (Vir) or SEQ ID NO: 108 (Vir) described herein, in particular to to SEQ ID NO: 8 (Vir), SEQ ID NO: 9 (Pm) SEQ ID NO: 10 (Xl), SEQ ID NO:11 (Sz), SEQ ID NO: 105 (Vir), SEQ ID NO: 106 (Vir) or SEQ ID NO: 108 (Vir), and more particularly to SEQ ID NO: 8 (Vir), SEQ ID NO: 9 (Pm) SEQ ID NO: 10 (Xl), SEQ ID NO:11 (Sz).

According to another particular embodiment, the one or more recombinant nucleic acids encoding a polypeptide having hyaluronan synthase activity may be nucleic acid(s) encoding polypeptide having an amino acid sequence selected from the group consisting of sequences having at least 50%, advantageously at least 65%, preferably at least 80%, amino acid identity with the amino acid sequence set forth as SEQ ID NO: 8 (Vir), SEQ ID NO: 9 (Pm) SEQ ID NO: 10 (Xl), SEQ ID NO:11 (Sz), SEQ ID NO: 105 (Vir), SEQ ID NO: 106 (Vir), SEQ ID NO: 107 (Vir) or SEQ ID NO: 108 (Vir), and also a biological activity of the same nature as the amino acid sequence set forth as SEQ ID NO: 8 (Vir), SEQ ID NO: 9 (Pm) SEQ ID NO: 10 (Xl), SEQ ID NO:11 (Sz), SEQ ID NO: 105 (Vir), SEQ ID NO: 106 (Vir), SEQ ID NO: 107 (Vir) or SEQ ID NO: 108 (Vir).

A biological activity of the same nature regarding this sequence is as described previously, i.e. the capacity to catalyze the conversion of UDP-glucuronate or UDP-N-acetyl-glucose into hyaluronic acid.

As described herein, an amino acid sequence having at least 50% amino acid identity with a reference amino acid sequence encompasses amino acid sequences having at least 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having hyaluronan synthase activity in the present invention is regulated by at least one promoter and at least one terminator, such as herein after defined more in details, which are present in 5' and 3' position respectively of the one or more recombinant nucleic acids encoding a polypeptide having hyaluronan synthase activity.

### UDP-GLUCOSE DEHYDROGENASE (UDP-GlcDH OR HASB)

The UDP-Glucose dehydrogenase is a protein which is known in the art to catalyze the conversion of UDP-glucose into UDP-glucuronate. The UDP-Glucose dehydrogenase originating from the genome of *Arabidopsis thaliana, Chlorella virus PBCV1* or *Streptococcus zooepidemicus* may be termed HASB.

A method implemented to measure the activity level of a polypeptide having UDP-Glucose dehydrogenase activity belongs to the general knowledge of the one skilled in the art.

In this regard, the one skilled in the art may advantageously refer to the method described by Oka and Jigami (FEBS Journal 273, 2645-2657, 2006).

Preferred polypeptide having UDP-Glucose dehydrogenase activity in the present specification is an enzyme having an EC number 1.1.1.22.

According to a preferred embodiment, the one or more recombinant nucleic acids encoding a polypeptide having UDP-Glucose dehydrogenase activity may originate or be derived from organisms preferably selected in a group comprising prokaryotic organisms and eukaryotic organisms. In some embodiments, the one or more recombinant nucleic acids encoding a polypeptide having UDP-Glucose dehydrogenase activity may originate or be derived from archaebacteria. In some preferred embodiments, the one or more recombinant nucleic acids encoding a polypeptide having UDP-Glucose dehydrogenase activity may originate or be derived from yeast, and especially from *Arabidopsis thaliana, Chlorella virus PBCV1* or *Streptococcus zooepidemicus haromyces.*

According to a yet preferred embodiment, the one or more recombinant nucleic acids encoding a polypeptide having UDP-Glucose dehydrogenase activity may be selected from the group consisting of nucleic acid sequences having (i) at least 65%, advantageously at least 70%, preferably at least 80%, nucleic acid identity with a nucleic acid as set forth as sequence SEQ ID NO: 12 (At), SEQ ID NO. 13 (Vir), SEQ ID NO. 14 (Vir) or SEQ ID NO: 15 (Sz), and (ii) a biological activity of the same nature as the nucleic acid as set forth as sequence SEQ ID NO: 12 (At), SEQ ID NO. 13 (Vir), SEQ ID NO. 14 (Vir) or SEQ ID NO: 15 (Sz). The nucleic acids set forth as sequences SEQ ID NO: 12 (At), SEQ ID NO: 13 (Vir), SEQ ID NO. 14 (Vir) and SEQ ID NO: 15 (Sz) encode a polypeptide having UDP-Glucose dehydrogenase activity originating, respectively, from *Arabidopsis thaliana (At), Chlorella virus PBCV1 (Vir)* or *Streptococcus zooepidemicus (Sz),* that may herein also be collectively termed HASB.

A biological activity of the same nature regarding this sequence is, as previously explained, the capacity to code for a polypeptide that converts UDP-glucose into UDP-glucuronate.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequences, and also a biological activity of the same nature as the said reference nucleic acid sequences.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequences.

For the amino acid sequence of the polypeptide having UDP-Glucose dehydrogenase activity from *Arabidopsis thaliana, Chlorella virus PBCV1* or *Streptococcus zooepidemicus,* the one skilled in the art may refer to the accession numbers NP_173979.1, NP_048965 or KIS19289, respectively, in the UniProt database, or to the sequences as set forth in SEQ ID NO: 16 (At), SEQ ID NO. 17 (Vir) and SEQ ID NO: 18 (Sz) described herein.

According to another particular embodiment, the one or more recombinant nucleic acids encoding a polypeptide having UDP-Glucose dehydrogenase activity may be nucleic acid(s) encoding a polypeptide having an amino acid sequence selected from the group consisting of sequences having at least 55%, advantageously at least 65%, preferably at least 80%, amino acid identity with the amino acid sequence of SEQ ID NO: 16 (At), SEQ ID NO. 17 (Vir) and SEQ ID NO: 18 (Sz), and also a biological activity of the same nature as the amino acid sequence of SEQ ID NO: 16 (At), SEQ ID NO. 17 (Vir) and SEQ ID NO: 18 (Sz).

A biological activity of the same nature regarding this sequence is as described previously, i.e. the capacity to catalyze the conversion of UDP-glucose into UDP-glucuronate.

As described herein, an amino acid sequence having at least 55% amino acid identity with a reference amino acid sequence encompasses amino acid sequences having at least 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having UDP-Glucose dehydrogenase activity in the present invention is regulated by at least one promoter and at least one terminator, such as herein after defined more in details, which are present in 5' and 3' position respectively of the one or more recombinant nucleic acids encoding a polypeptide having UDP-Glucose dehydrogenase activity.

### HYALURONIDASE (HYAL)

The hyaluronidase enzyme is a protein which is described in the art for catalyzing the degradation of hyaluronic acid molecules into smaller hyaluronic acid molecules. The hyaluronidase originating from *Cupiennius salei, Loxosceles intermedia, Hirudo nipponia, Bothrops atrox, Tityus serrulatus* or *Vespa magnifica* may be termed HYAL.

The polypeptide having hyaluronidase activity of the invention may possess both a secretion signal and an anchoring signal or a secretion signal and no anchoring signal or a secretion-anchor signal with a dual secretion and anchoring function. When the encoded polypeptide having hyaluronidase activity possesses both a secretion signal and an anchoring signal, it may be termed HYAL-31 as represented in the examples. When the encoded polypeptide having hyaluronidase activity possesses a secretion signal and no anchoring signal, it may be termed HYAL-3 as represented in the examples.

A method implemented to measure the activity level of a polypeptide having hyaluronidase activity belongs to the general knowledge of the one skilled in the art.

In this regard, the one skilled in the art may advantageously monitor the molecular weight of the obtained hyaluronic acid on an agarose gel.

Preferred polypeptide having hyaluronidase activity in the present specification is an enzyme having an EC number of n° EC 3.2.1.35.

According to a preferred embodiment, the one or more recombinant nucleic acids encoding a polypeptide having hyaluronidase activity may originate or be derived from organisms preferably selected in a group comprising prokaryotic organisms and eukaryotic organisms. In some embodiments, the one or more recombinant nucleic acids encoding a polypeptide having hyaluronidase activity may originate or be derived from archaebacteria. In some embodiments, the one or more recombinant nucleic acids encoding a polypeptide having hyaluronidase activity may originate or be derived from organisms preferably selected from yeasts. In some other preferred embodiments, the one or more recombinant nucleic acids encoding a polypeptide having hyaluronidase activity may originate or be derived from *Cupiennius salei (Csa), Loxosceles intermedia (Li), Hirudo nipponia (Hn), Bothrops atrox (Ba), Tityus serrulatus (Ts) or Vespa magnifica (Vm), and in particular from Cupiennius salei (Csa), Loxosceles intermedia (Li), Hirudo nipponia (Hn), Bothrops atrox (Ba) and Tityus serrulatus (Ts).*

According to a yet preferred embodiment, the one or more recombinant nucleic acids encoding a polypeptide having hyaluronidase activity may be selected from the group consisting of nucleic acid sequences having at least 65%, advantageously at least 70%, preferably at least 80%, nucleic acid identity with a nucleic acid of SEQ ID NO: 19 (Ba), SEQ ID NO: 25 (Li), SEQ ID NO: 21 (Csa), SEQ ID NO: 27 (Ts), SEQ ID NO: 23 (Hn), or SEQ ID NO: 29 (Vm) and also a biological activity of the same nature as the nucleic acid of SEQ ID NO: 19 (Ba), SEQ ID NO: 25 (Li), SEQ ID NO: 21 (Csa), SEQ ID NO: 27 (Ts), SEQ ID NO: 23 (Hn), or SEQ ID NO: 29 (Vm). The nucleic acid of SEQ ID NO: 19 (Ba), SEQ ID NO: 25 (Li), SEQ ID NO: 21 (Csa), SEQ ID NO: 27 (Ts), SEQ ID NO: 23 (Hn) or SEQ ID NO: 29 (Vm) encode a polypeptide having hyluronidase activity, comprise a secretion signal and no anchoring signal, and originate or derive from *Bothrops atrox, Loxosceles intermedia, Cupiennius salei, Tityus serrulatus, Hirudo nipponia* or *Vespa magnifica,* respectively.

According to another preferred embodiment, the one or more recombinant nucleic acids encoding a polypeptide having hyaluronidase activity may be selected from the group consisting of nucleic acid sequences having at least 65%, advantageously at least 70%, preferably at least 80%, nucleic acid identity with a nucleic acid of SEQ ID NO: 22 (Csa), SEQ ID NO: 26 (Li), SEQ ID NO: 24 (Hn), SEQ ID NO: 20 (Ba), SEQ ID NO: 28 (Ts) or SEQ ID NO: 30 (Vm), and also a biological activity of the same nature as the nucleic acid of SEQ ID NO: 22 (Csa), SEQ ID NO: 26 (Li), SEQ ID NO: 24 (Hn), SEQ ID NO: 20 (Ba), SEQ ID NO: 28 (Ts) or SEQ ID NO: 30 (Vm). The nucleic acid of SEQ ID NO: 22 (Csa), SEQ ID NO: 26 (Li), SEQ ID NO: 24 (Hn), SEQ ID NO: 20 (Ba) SEQ ID NO: 28 (Ts) or SEQ ID NO: 30 (Vm) encode a polypeptide having hyluronidase activity, comprise a secretion signal and an anchoring signal, and originate or derive from *Cupiennius salei, Loxosceles intermedia, Hirudo nipponia, Bothrops atrox, Tityus serrulatus* or *Vespa magnifica,* respectively.

In a particular embodiment, the one or more recombinant nucleic acids encoding a polypeptide having hyaluronidase activity may be selected from the group consisting of nucleic acid sequences (i) having at least 65%, advantageously at least 70%, preferably at least 80%, nucleic acid identity with a nucleic acid of SEQ ID NO: 19 (Ba), SEQ ID NO: 25 (Li), SEQ ID NO: 21 (Csa), SEQ ID NO: 27 (Ts), or SEQ ID NO: 23 (Hn) and (ii) having a biological activity of the same nature as the nucleic acid of SEQ ID NO: 19 (Ba), SEQ ID NO: 25 (Li), SEQ ID NO: 21 (Csa), SEQ ID NO: 27 (Ts), or SEQ ID NO: 23 (Hn).

In a particular embodiment, the one or more recombinant nucleic acids encoding a polypeptide having hyaluronidase activity may be selected from the group consisting of nucleic acid sequences having (i) at least 65%, advantageously at least 70%, preferably at least 80%, nucleic acid identity with a nucleic acid of SEQ ID NO: 22 (Csa), SEQ ID NO: 26 (Li), SEQ ID NO: 24 (Hn), SEQ ID NO: 20 (Ba), or SEQ ID NO: 28 (Ts) and (ii) having a biological activity of the same nature as the nucleic acid of SEQ ID NO: 22 (Csa), SEQ ID NO: 26 (Li), SEQ ID NO: 24 (Hn), SEQ ID NO: 20 (Ba), or SEQ ID NO: 28 (Ts).

A biological activity of the same nature regarding this sequence is, as previously explained, the capacity to code for a polypeptide that catalyzes the degradation of hyaluronic acid molecules into smaller hyaluronic acid molecules.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the one or more recombinant nucleic acids encoding a polypeptide having hyaluronidase activity from *Cupiennius salei, Loxosceles intermedia, Hirudo nipponia, Bothrops atrox* or *Tityus serrulatus,* the one skilled in the art may refer to the accession numbers A0A0S4JYH2, R4J7Z9, X4Y2L4, A0A2H4Z8F4 or P85841, respectively in the UniProt database, or to SEQ ID NO: 33 (Csa), SEQ ID NO: 37 (Li), SEQ ID NO: 35 (Hn), SEQ ID NO: 31 (Ba) SEQ ID NO: 39 (Ts) or SEQ ID NO: 41 (Vm), described herein.

According to another particular embodiment, the one or more recombinant nucleic acids encoding a polypeptide having hyaluronidase activity may be nucleic acid(s) encoding polypeptide having an amino acid sequence selected from the group consisting of sequences having at least 50%, advantageously at least 65%, preferably at least 80%, amino acid identity with the amino acid sequence of SEQ ID NO: 33 (Csa), SEQ ID NO: 37 (Li), SEQ ID NO: 35 (Hn), SEQ ID NO: 31 (Ba), SEQ ID NO: 39 (Ts) or SEQ ID NO: 41 (Vm) which comprise a secretion signal and no anchoring signal, and also a biological activity of the same nature as the amino acid sequence SEQ ID NO: 33 (Csa), SEQ ID NO: 37 (Li), SEQ ID NO: 35 (Hn), SEQ ID NO: 31 (Ba), SEQ ID NO: 39 (Ts) or SEQ ID NO: 41 (Vm).

According to another particular embodiment, the one or more recombinant nucleic acids encoding a polypeptide having hyaluronidase activity may be nucleic acid(s) encoding polypeptide having an amino acid sequence selected from the group consisting of sequences having at least 55%, advantageously at least 65%, preferably at least 80%, amino acid identity with the amino acid sequence of SEQ ID NO: 34 (Csa), SEQ ID NO: 38 (Li), SEQ ID NO: 36 (Hn), SEQ ID NO: 32 (Ba), SEQ ID NO: 40 (Ts) or SEQ ID NO: 42 (Vm) which comprise a secretion signal and an anchoring signal, and also a biological activity of the same nature as the amino acid sequence of SEQ ID NO: 34 (Csa), SEQ ID NO: 38 (Li), SEQ ID NO: 36 (Hn), SEQ ID NO: 32 (Ba), SEQ ID NO: 40 (Ts) or SEQ ID NO: 42 (Vm).

In a particular embodiment, the one or more recombinant nucleic acids encoding a polypeptide having hyaluronidase activity may be nucleic acid(s) encoding a polypeptide having an amino acid sequence selected from the group consisting of sequences having at least 55%, advantageously at least 65%, preferably at least 80%, amino acid identity with the amino acid sequence of SEQ ID NO: 33 (Csa), SEQ ID NO: 37 (Li), SEQ ID NO: 35 (Hn), SEQ ID NO: 31 (Ba), or SEQ ID NO: 39 (Ts), and also a biological activity of the same nature as the amino acid sequence of SEQ ID NO: 33 (Csa), SEQ ID NO: 37 (Li), SEQ ID NO: 35 (Hn), SEQ ID NO: 31 (Ba), or SEQ ID NO: 39 (Ts).

In another particular embodiment, the one or more recombinant nucleic acids encoding a polypeptide having hyaluronidase activity may be nucleic acid(s) encoding a polypeptide having an amino acid sequence selected from the group consisting of sequences having at least 55%, advantageously at least 65%, preferably at least 80%, amino acid identity with the amino acid sequence of SEQ ID NO: 34 (Csa), SEQ ID NO: 38 (Li), SEQ ID NO: 36 (Hn), SEQ ID NO: 32 (Ba) or SEQ ID NO: 40 (Ts), and also a biological activity of the same nature as the amino acid sequence of SEQ ID NO: 34 (Csa), SEQ ID NO: 38 (Li), SEQ ID NO: 36 (Hn), SEQ ID NO: 32 (Ba) or SEQ ID NO: 40 (Ts).

A biological activity of the same nature regarding this sequence is as described previously, i.e. the capacity to catalyze the degradation of hyaluronic acid molecules into smaller hyaluronic acid molecules.

As described herein, an amino acid sequence having at least 55% amino acid identity with a reference amino acid sequence encompasses amino acid sequences having at least 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having hyaluronidase activity in the present invention is regulated by at least one promoter and at least one terminator, such as herein after defined more in details, which are present in 5' and 3' position respectively of the one or more recombinant nucleic acids encoding a polypeptide having hyaluronidase activity.

### GLUTAMINE SYNTHETASE (GLN1)

The glutamine synthetase enzyme is a protein which is described in the art for catalyzing the conversion of glutamate into glutamine. The glutamine synthetase originating from *Saccharomyces cerevisiae* may be termed GLN1.

A method implemented to measure the activity level of a polypeptide having glutamine synthetase activity belongs to the general knowledge of the one skilled in the art.

In this regard, the one skilled in the art may advantageously refer to the method described by Legrain et al. (1982) European Journal of Biochemistry 123, 611-616.

Preferred polypeptide having glutamine synthetase activity in the present invention is an enzyme having an EC number of n° EC 6.3.1.2.

According to a preferred embodiment, the one or more recombinant nucleic acids encoding a polypeptide having glutamine synthetase activity may originate or be derived from organisms preferably selected in a group comprising prokaryotic organisms and eukaryotic organisms. In some embodiments, the one or more recombinant nucleic acids encoding a polypeptide having glutamine synthetase activity may originate or be derived from archaebacteria. In some embodiments, the one or more recombinant nucleic acids encoding a polypeptide having glutamine synthetase activity may originate or be derived from a yeast, and especially from *Saccharomyces cerevisiae.*

According to a yet preferred embodiment, the one or more recombinant nucleic acids encoding a polypeptide having glutamine synthetase activity may be selected from the group consisting of nucleic acid sequences having (i) at least 65%, advantageously at least 70%, preferably at least 80%, nucleic acid identity with the nucleic acid sequence as set forth as sequence SEQ ID NO: 96 (Sc), and (ii) a biological activity of the same nature as the nucleic acid sequence as set forth as sequence SEQ ID NO: 96 (Sc). The nucleic acid as set forth as sequence SEQ ID NO: 96 encodes a polypeptide having glutamine synthetase activity originating from *Saccharomyces cerevisiae,* that may also be termed GLN1.

A biological activity of the same nature regarding this sequence is, as previously explained, the capacity to code for a polypeptide that converts glutamate into glutamine, in particular through the consumption of one ATP and one NH₄⁺.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having glutamine synthetase activity originating from *Saccharomyces cerevisiae,* the one skilled in the art may refer to the accession number P32288 in the UniProt database, or to the sequence SEQ ID NO: 97 described herein.

According to another particular embodiment, the one or more recombinant nucleic acids encoding a polypeptide having glutamine synthetase activity may be nucleic acid(s) encoding polypeptide having an amino acid sequence selected from the group consisting of sequences having at least 35%, advantageously at least 65%, preferably at least 80%, amino acid identity with the amino acid sequence of SEQ ID NO: 97, and also a biological activity of the same nature as the amino acid sequence of SEQ ID NO: 97.

A biological activity of the same nature regarding this sequence is as described previously, i.e. the capacity to catalyze the conversion of glutamate into glutamine.

As described herein, an amino acid sequence having at least 35% amino acid identity with a reference amino acid sequence encompasses amino acid sequences having at least 36%, 37%, 38%, 39%, 40% 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the polypeptide having glutamine synthetase activity in the present invention is regulated by at least one promoter and at least one terminator, such as herein after defined more in details, which are present in 5' and 3' position respectively of one or more recombinant nucleic acids encoding a polypeptide having glutamine synthetase activity.

### GLUTAMATE SYNTHASE (GLT1)

The glutamate synthase enzyme is a protein which is described in the art for catalyzing the conversion of glutamine into glutamate. The glutamate synthase originating from *Saccharomyces cerevisiae* may be termed GLT1.

A method implemented to measure the activity level of a polypeptide having glutamate synthase activity belongs to the general knowledge of the one skilled in the art.

In this regard, the one skilled in the art may advantageously refer to the method described by Roon et al. (1974) Journal of bacteriology 118,89-95.

Preferred polypeptide having glutamate synthase activity in the present invention is an enzyme having an EC number of n° EC 1.4.1.14 (SEQ ID NO: 98).

For the amino acid sequence of the polypeptide having glutamate synthase activity from *Saccharomyces cerevisiae,* the one skilled in the art may refer to the accession number Q12680 in the UniProt database, or to the sequence SEQ ID NO: 99 described herein.

As above-mentioned, the expression level of a polypeptide having glutamate synthase activity may be reduced in a recombinant cell, and in particular in a recombinant yeast according to the invention as compared to the said cell, and in particular yeast, in its non-recombined form, i.e. at least one endogeneous gene of the recombinant cell, and in particular of the recombinant yeast, is disrupted.

### GLUTAMINE-FRUCTOSE-6-PHOSPHATE AMIDOTRANSFERASE (GFA1)

The glutamine-fructose-6-phosphate amidotransferase enzyme is a protein which is described in the art for catalyzing the conversion of fructose-6-phosphate into glucosamine-6-phosphate. The glutamine-fructose-6-phosphate amidotransferase originating from *Saccharomyces cerevisiae* may be termed GFA1.

A method implemented to measure the activity level of a polypeptide having glutamine-fructose-6-phosphate amidotransferase activity belongs to the general knowledge of the one skilled in the art.

In this regard, the one skilled in the art may advantageously refer to the method described by Shiga Shibatan and Hiroaki Kitazawa (Plant Biotechnology 26, 149-152, 2009).

Preferred polypeptide having glutamine-fructose-6-phosphate amidotransferase activity in the present invention is an enzyme having an EC number of n° EC 2.6.1.16.

According to a preferred embodiment, the one or more recombinant nucleic acids encoding a polypeptide having glutamine-fructose-6-phosphate amidotransferase activity may originate or be derived from organisms preferably selected in a group comprising prokaryotic organisms and eukaryotic organisms. In some embodiments, the one or more recombinant nucleic acids encoding a polypeptide having glutamine-fructose-6-phosphate amidotransferase activity may originate or be derived from archaebacteria. In some embodiments, the one or more recombinant nucleic acids encoding a polypeptide having glutamine-fructose-6-phosphate amidotransferase activity may originate or be derived from organisms preferably selected from *Bacillus subtilis,* and yeasts. In some other preferred embodiments, the one or more recombinant nucleic acids encoding a polypeptide having glutamine-fructose-6-phosphate amidotransferase activity may originate or be derived from a yeast, and especially from *Saccharomyces cerevisiae.*

According to a yet preferred embodiment, the one or more recombinant nucleic acids encoding a polypeptide having glutamine-fructose-6-phosphate amidotransferase activity may be selected from the group consisting of nucleic acid sequences having (i) at least 65%, advantageously at least 70%, preferably at least 80%, nucleic acid identity with the nucleic acid sequence as set forth as sequence SEQ ID NO: 47 (Sc), and (ii) a biological activity of the same nature as the nucleic acid sequence as set forth as sequence SEQ ID NO: 47 (Sc). The nucleic acid as set forth as sequence SEQ ID NO: 47 encodes a polypeptide having glutamine-fructose-6-phosphate amidotransferase activity originating from *Saccharomyces cerevisiae,* that may also be termed GFA1.

According to yet another embodiment, the one or more recombinant nucleic acids encoding a polypeptide having glutamine-fructose-6-phosphate amidotransferase activity may be selected from the group consisting of nucleic acid sequences having (i) at least 65%, advantageously at least 70%, preferably at least 80%, nucleic acid identity with the nucleic acid sequence as set forth as sequence SEQ ID NO: 48 or SEQ ID NO: 49, and (ii) a biological activity of the same nature as the nucleic acid sequence as set forth as sequence SEQ ID NO: 48 or SEQ ID NO: 49. The nucleic acid sequence as set forth as sequence SEQ ID NO: 48 or SEQ ID NO: 49 encodes a polypeptide having glutamine-fructose-6-phosphate amidotransferase activity originating from *Chlorella virus PBCV1.*

A biological activity of the same nature regarding this sequence is, as previously explained, the capacity to code for a polypeptide that converts fructose-6-phosphate into glucosamine-6-phosphate.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having glutamine-fructose-6-phosphate amidotransferase activity originating from *Saccharomyces cerevisiae,* the one skilled in the art may refer to the accession number NP8012818 in the UniProt database, or to the sequence SEQ ID NO: 50 described herein.

For the amino acid sequence of the polypeptide having glutamine-fructose-6-phosphate amidotransferase activity originating from *Chlorella virus PBCV1,* the one skilled in the art may also refer to the accession number NP_048448 in the UniProt database, or to the sequence SEQ ID NO: 51 described herein.

According to another particular embodiment, the one or more recombinant nucleic acids encoding a polypeptide having glutamine-fructose-6-phosphate amidotransferase activity may be nucleic acid(s) encoding polypeptide having an amino acid sequence selected from the group consisting of sequences having at least 35%, advantageously at least 65%, preferably at least 80%, amino acid identity with the amino acid sequence of SEQ ID NO: 50 or with SEQ ID NO: 51, and also a biological activity of the same nature as the amino acid sequence of SEQ ID NO: 50 or with SEQ ID NO: 51.

A biological activity of the same nature regarding this sequence is as described previously, i.e. the capacity to catalyze the conversion of of fructose-6-phosphate into glucosamine-6-phosphate.

As described herein, an amino acid sequence having at least 35% amino acid identity with a reference amino acid sequence encompasses amino acid sequences having at least 36%, 37%, 38%, 39%, 40% 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 65% amino acid identity with a reference amino acid sequence encompasses amino acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the polypeptide having glutamine-fructose-6-phosphate amidotransferase activity in the present invention is regulated by at least one promoter and at least one terminator, such as herein after defined more in details, which are present in 5' and 3' position respectively of one or more recombinant nucleic acids encoding a polypeptide having glutamine-fructose-6-phosphate amidotransferase activity.

### UDP-N-ACETYLGLUCOSAMINE PYROPHOSPHORYLASE (QRI1)

The UDP-N-acetylglucosamine pyrophosphorylase enzyme is a protein which is described in the art for catalyzing the conversion of N-acetyl-glucosmine-6-phosphate into UDP-N-acetyl-glucose. The UDP-N-acetylglucosamine pyrophosphorylase originating from *Saccharomyces cerevisiae* may be termed QRI1.

A method implemented to measure the activity level of a polypeptide having UDP-N-acetylglucosamine pyrophosphorylase activity belongs to the general knowledge of the one skilled in the art.

In this regard, the one skilled in the art may advantageously refer to the method described by Mio et al. (The Journal of Biological Chemistry, Col. 273, No 23, June 5, 1998, 14392-14397) except that UDP-N-acetyl-glucosamine is detected by LC MS/MS using a Synergi RP Fusion column.

Preferred polypeptide having UDP-N-acetylglucosamine pyrophosphorylase activity in the present invention is an enzyme having an EC number of n° EC 2.7.7.23

According to a preferred embodiment, the one or more recombinant nucleic acids encoding a polypeptide having UDP-N-acetylglucosamine pyrophosphorylase activity may originate or be derived from organisms preferably selected in a group consisting of prokaryotic organisms and eukaryotic organisms. In some embodiments, the one or more recombinant nucleic acids encoding a polypeptide having UDP-N-acetylglucosamine pyrophosphorylase activity may originate or be derived from archaebacteria. In some embodiments, the one or more recombinant nucleic acids encoding a polypeptide having UDP-N-acetylglucosamine pyrophosphorylase activity may originate or be derived from organisms preferably selected from *Bacillus subtilis,* and yeasts. In some other preferred embodiments, the one or more recombinant nucleic acids encoding a polypeptide having UDP-N-acetylglucosamine pyrophosphorylase activity may originate or be derived from yeasts, and especially from *Saccharomyces cerevisiae.*

According to a yet preferred embodiment, the one or more recombinant nucleic acids encoding a polypeptide having UDP-N-acetylglucosamine pyrophosphorylase activity may be selected from the group consisting of nucleic acid sequences having (i) at least 65%, advantageously at least 70%, preferably at least 80%, nucleic acid identity with a nucleic acid sequence as set forth as SEQ ID NO: 52, and (ii) a biological activity of the same nature as the nucleic acid sequence as set forth as SEQ ID NO: 52. The nucleic acid sequence set forth as SEQ ID NO: 52 encodes a polypeptide having UDP-N-acetylglucosamine pyrophosphorylase activity originating from *Saccharomyces cerevisiae,* that may also be termed QRI1.

A biological activity of the same nature regarding this sequence is, as previously explained, the capacity to code for a polypeptide that converts N-acetyl-glucosmine-6-phosphate into UDP-N-acetyl-glucose.

As described herein, a nucleic acid sequence having at least 65% nucleotide identity with a reference nucleic acid sequence encompasses nucleic acid sequences having at least 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 70% nucleotide identity with a reference nucleic acid sequence encompasses nucleic acid sequences having at least 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having UDP-N-acetylglucosamine pyrophosphorylase activity originating from *Saccharomyces cerevisiae,* the one skilled in the art may refer to the accession number NP_010180 in the UniProt database, or to SEQ ID NO. 53 described herein.

According to another particular embodiment, the one or more recombinant nucleic acids encoding a polypeptide having UDP-N-acetylglucosamine pyrophosphorylase activity may be nucleic acid(s) encoding polypeptide having an amino acid sequence selected from the group consisting of sequences having at least 35%, advantageously at least 45%, preferably at least 80%, amino acid identity with the amino acid sequence set forth as sequence SEQ ID NO. 53, and also a biological activity of the same nature as the amino acid sequence set forth as sequence SEQ ID NO. 53.

A biological activity of the same nature regarding this sequence is as described previously, i.e. the capacity to catalyze the conversion of of N-acetyl-glucosmine-6-phosphate into UDP-N-acetyl-glucose.

As described herein, an amino acid sequence having at least 35% amino acid identity with a reference amino acid sequence encompasses amino acid sequences having at least 36%, 37%, 38%, 39%, 40% 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 45% amino acid identity with a reference amino acid sequence encompasses amino acid sequences having at least 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having UDP-N-acetylglucosamine pyrophosphorylase activity in the present invention is regulated by at least one promoter and at least one terminator, such as herein after defined more in details, which are present in 5' and 3' position respectively of the one or more recombinant nucleic acids encoding a polypeptide having UDP-N-acetylglucosamine pyrophosphorylase activity.

### PHOSPHOGLUCOMUTASE-1 (PGM1)

The Phosphoglucomutase-1 enzyme is a protein which is described in the art for catalyzing the conversion of Glucose-6-phosphate into Glucose-1-phosphate. The Phosphoglucomutase-1 originating from *Saccharomyces cerevisiae* may be termed PGM1.

A method implemented to measure the activity level of a polypeptide facing Phosphoglucomutase-1 activity belongs to the general knowledge of the one skilled in the art.

In this regard, the one skilled in the art may advantageously refer to the method described by Tiwari and Bhat (Biochemical and Biophysical Research Communications 366, 340-345, 2008).

Preferred polypeptide having Phosphoglucomutase-1 activity in the present invention is an enzyme having an EC number of n° 5.4.2.2.

According to a preferred embodiment, the one or more recombinant nucleic acids encoding a polypeptide having Phosphoglucomutase-1 activity may originate from organisms preferably selected in a group consisting of prokaryotic organisms and eukaryotic organisms. In some embodiments, the one or more recombinant nucleic acids encoding a polypeptide having Phosphoglucomutase-1 activity may originate or be derived from archaebacteria. In some embodiments, the one or more recombinant nucleic acids encoding a polypeptide having Phosphoglucomutase-1 activity may originate or be derived from organisms preferably selected from bacteria. In a preferred embodiment, the one or more recombinant nucleic acids encoding a polypeptide having Phosphoglucomutase-1 activity may orginate or be derived from *Saccharomyces cerevisiae.*

According to an embodiment, the one or more recombinant nucleic acids encoding a polypeptide having Phosphoglucomutase-1 activity may be selected from the group consisting of nucleic acid sequences having (i) at least 80% nucleic acid identity with the nucleic acid sequence set forth as sequence SEQ ID NO: 54, which originates from *Saccharomyces cerevisiae,* and (ii) a biological activity of the same nature as the nucleic acid sequence set forth as sequence SEQ ID NO: 54.

A biological activity of the same nature regarding this sequence is, as previously explained, the capacity to code for a polypeptide that converts Glucose-6-phosphate into Glucose-1-phosphate.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the peptide having Phosphoglucomutase-1 activity from *Saccharomyces cerevisiae,* the one skilled in the art may refer to the accession number NP33401 in the UniProt database, or to SEQ ID NO: 55 described herein.

According to another particular embodiment, the one or more recombinant nucleic acids encoding a polypeptide having Phosphoglucomutase-1 activity may be nucleic acid(s) encoding polypeptide having an amino acid sequence selected from the group consisting of sequences having at least least 80% amino acid identity with the amino acid sequence set forth as sequence SEQ ID NO: 55, and also a biological activity of the same nature as the amino acid sequence set forth as sequence SEQ ID NO: 55.

A biological activity of the same nature regarding this sequence is as described previously, i.e. the capacity to catalyze the conversion of Glucose-6-phosphate into Glucose-1-phosphate.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having Phosphoglucomutase-1 activity in the present invention is regulated by at least one promoter and at least one terminator, such as herein after defined more in details, which are present in 5' and 3' position respectively of the one or more recombinant nucleic acids encoding a polypeptide having Phosphoglucomutase-1 activity.

### UTP-GLUCOSE-1-PHOSPHATE URIDYLYLTRANSFERASE (UGP1)

The UTP-glucose-1-phosphate uridylyltransferase enzyme is a protein which is described in the art for catalyzing the conversion of Glucose-1-Phosphate into UDP-glucose. The UTP-glucose-1-phosphate uridylyltransferase originating from *Saccharomyces cerevisiae* may be termed UGP1.

A method implemented to measure the activity level of a polypeptide having UTP-glucose-1-phosphate uridylyltransferase activity belongs to the general knowledge of the one skilled in the art.

In this regard, the one skilled in the art may advantageously refer to the method described by Roeben (J. Mol. Biol 364, 551-560, 2006).

Preferred polypeptide having UTP-glucose-1-phosphate uridylyltransferase activity in the present invention is an enzyme having an EC number of n° 2.7.7.9.

According to a preferred embodiment, the one or more recombinant nucleic acids encoding a polypeptide having UTP-glucose-1-phosphate uridylyltransferase activity mayoriginate or be derived from organisms preferably selected in a group consisting of prokaryotic organisms and eukaryotic organisms. In some embodiments, the one or more recombinant nucleic acids encoding a polypeptide having UTP-glucose-1-phosphate uridylyltransferase activity may originate or be derived from archaebacteria. In some embodiments, the one or more recombinant nucleic acids encoding a polypeptide having UTP-glucose-1-phosphate uridylyltransferase activity may originate or be derived from organisms preferably selected from bacteria. In a preferred embodiment, the one or more recombinant nucleic acids encoding a polypeptide having UTP-glucose-1-phosphate uridylyltransferase activity may originate or be derived from *Saccharomyces cerevisiae.*

According to a particular embodiment, the one or more recombinant nucleic acids encoding a polypeptide having UTP-glucose-1-phosphate uridylyltransferase activity may be selected from the group consisting of nucleic acid sequences having (i) at least 80% nucleic acid identity with a nucleic acid as set forth as sequence SEQ ID NO: 56, originating from *Saccharomyces cerevisiae,* and (ii) a biological activity of the same nature as the nucleic acid as set forth as sequence SEQ ID NO: 56.

A biological activity of the same nature regarding this sequence is, as previously explained, the capacity to code for a polypeptide that converts Glucose-1-phosphate into UDP-glucose.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having UTP-glucose-1-phosphate uridylyltransferase activity originating from *Saccharomyces cerevisiae,* the one skilled in the art may refer to the accession number NP_32861 in the UniProt database, or to the sequence set forth as sequence SEQ ID NO: 57 described herein.

According to another particular embodiment, the one or more recombinant nucleic acids encoding a polypeptide having UTP-glucose-1-phosphate uridylyltransferase activity may be nucleic acid(s) encoding polypeptide having an amino acid sequence selected from the group consisting of sequences having at least 80% amino acid identity with the amino acid sequence set forth as SEQ ID NO: 57, and also a biological activity of the same nature as the amino acid sequence set forth as SEQ ID NO: 57.

A biological activity of the same nature regarding this sequence is as described previously, i.e. the capacity to catalyze the conversion of Glucose-1-phosphate into UDP-glucose.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having UTP-glucose-1-phosphate uridylyltransferase activity in the present invention is regulated by at least one promoter and at least one terminator, such as herein after defined more in details, which are present in 5' and 3' position respectively of the one or more recombinant nucleic acids encoding a polypeptide having UTP-glucose-1-phosphate uridylyltransferase activity.

### GLUCOSAMINE-6-PHOSPHATE N-ACETYLTRANSFERASE (GNA1)

The Glucosamine-6-phosphate N-acetyltransferase enzyme is a protein which is described in the art for catalyzing the conversion of Glucosamine-6-phosphate into N-acetyl-glucosamine-6-phosphate. The Glucosamine-6-phosphate N-acetyltransferase originating from *Saccharomyces cerevisiae* may be termed GNA1.

A method implemented to measure the activity level of a polypeptide having Glucosamine-6-phosphate N-acetyltransferase activity belongs to the general knowledge of the one skilled in the art.

In this regard, the one skilled in the art may advantageously refer to the method described by Li et al. (Anal. Biochem. 370, 142-146, 2007).

Preferred polypeptide having Glucosamine-6-phosphate N-acetyltransferase activity in the present invention is an enzyme having an EC number of n° 2.3.1.4.

According to a preferred embodiment, the one or more recombinant nucleic acids encoding a polypeptide having Glucosamine-6-phosphate N-acetyltransferase activity may originate or be derived from organisms preferably selected in a group consisting of prokaryotic organisms and eukaryotic organisms. In some preferred embodiments, the one or more recombinant nucleic acids encoding a polypeptide having Glucosamine-6-phosphate N-acetyltransferase activity may originate or be derived from a yeast, and especially from *Saccharomyces cerevisiae.*

According to a particular embodiment, the one or more recombinant nucleic acids encoding a polypeptide having Glucosamine-6-phosphate N-acetyltransferase activity may be selected from the group consisting of nucleic acid sequences having (i) at least 80% nucleic acid identity with a nucleic acid sequence as set forth as sequence SEQ ID NO: 58, and (ii) a biological activity of the same nature as the nucleic acid sequence as set forth as sequence SEQ ID NO: 58. The nucleic acid sequence as set forth as sequence SEQ ID NO: 58 encodes a polypeptide having Glucosamine-6-phosphate N-acetyltransferase activity originating from *Saccharomyces cerevisiae,* that may also be termed GNA1.

A biological activity of the same nature regarding this sequence is, as previously explained, the capacity to code for a polypeptide that converts Glucosamine-6-phosphate into N-acetyl-glucosamine-6-phosphate.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having Glucosamine-6-phosphate N-acetyltransferase activity originating from *Saccharomyces cerevisiae,* the one skilled in the art may refer to the accession number NP_116637 in the UniProt database, or to SEQ ID NO. 59 described herein.

According to another particular embodiment, the one or more recombinant nucleic acids encoding a polypeptide having Glucosamine-6-phosphate N-acetyltransferase activity may be nucleic acid(s) encoding polypeptide having an amino acid sequence selected from the group consisting of sequences having at least 80% amino acid identity with the amino acid sequence set forth as SEQ ID NO. 59, and also a biological activity of the same nature as the amino acid sequence set forth as SEQ ID NO. 59.

A biological activity of the same nature regarding this sequence is as described previously, i.e. the capacity to catalyze the conversion of Glucosamine-6-phosphate into N-acetyl-glucosamine-6-phosphate.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having Glucosamine-6-phosphate N-acetyltransferase activity in the present invention is regulated by at least one promoter and at least one terminator, such as herein after defined more in details, which are present in 5' and 3' position respectively of the one or more recombinant nucleic acids encoding a polypeptide having Glucosamine-6-phosphate N-acetyltransferase activity.

### PHOSPHOACETYLGLUCOSAMINE MUTASE (PCM1)

The phosphoacetylglucosamine mutase enzyme is a protein which is described in the art for catalyzing the conversion of N-acetyl-glucosamine-6-phosphate into N-acetyl-glucosamine-1-phosphate. The phosphoacetylglucosamine mutase originating from *Saccharomyces cerevisiae* may be termed PCM1.

A method implemented to measure the activity level of a polypeptide having phosphoacetylglucosamine mutase activity belongs to the general knowledge of the one skilled in the art.

In this regard, the one skilled in the art may advantageously refer to the method described by Bandini et al. (Molecular Microbiology 85(3), 513-534, 2012).

Preferred polypeptide having phosphoacetylglucosamine mutase activity in the present invention is an enzyme having an EC number of n° 5.4.2.3.

According to a preferred embodiment, the one or more recombinant nucleic acids encoding a polypeptide having phosphoacetylglucosamine mutase activity mayoriginate or be derived from organisms preferably selected in a group consisting of prokaryotic organisms and eukaryotic organisms. In some preferred embodiments, the one or more recombinant nucleic acids encoding a polypeptide having phosphoacetylglucosamine mutase activity may originate or be derived from a yeast, and especially from *Saccharomyces cerevisiae.*

According to a particular embodiment, the one or more recombinant nucleic acids encoding a polypeptide having phosphoacetylglucosamine mutase activity may be selected from the group consisting of nucleic acid sequences having (i) at least 80% nucleic acid identity with a nucleic acid sequence set forth as SEQ ID NO: 60, and (ii) a biological activity of the same nature as the nucleic acid sequence set forth as SEQ ID NO: 60. The nucleic acid set forth as SEQ ID NO: 60 encodes a polypeptide having phosphoacetylglucosamine mutase activity originating from *Saccharomyces,* that may also be termed PCM1.

A biological activity of the same nature regarding this sequence is, as previously explained, the capacity to code for a polypeptide that converts N-acetyl-glucosamine-6-phosphate into N-acetyl-glucosamine-1-phosphate.

As described herein, a nucleic acid sequence having at least 80% nucleotide identity with a reference nucleic acid sequence encompasses nucleic acid sequences having at least 81%, 82%, 83%, 84%, 85%, 86%, 87%, 88%, 89%, 90%, 91%, 92%, 93%, 94%, 95%, 96%, 97%, 98% and 99% nucleotide identity with the said reference nucleic acid sequence, and also a biological activity of the same nature as the said reference nucleic acid sequence.

For the amino acid sequence of the polypeptide having phosphoacetylglucosamine mutase activity originating from *Saccharomyces cerevisiae,* the one skilled in the art may refer to the accession number NP_010856 in the UniProt database, or to SEQ ID NO. 61 described herein.

According to another particular embodiment, the one or more recombinant nucleic acids encoding a polypeptide having phosphoacetylglucosamine mutase activity may be nucleic acid(s) encoding polypeptide having an amino acid sequence selected from the group consisting of sequences having at least 80% amino acid identity with the amino acid sequence set forth as SEQ ID NO. 61, and also a biological activity of the same nature as the amino acid sequence set forth as SEQ ID NO. 61.

A biological activity of the same nature regarding this sequence is as described previously, i.e. the capacity to catalyze the conversion of N-acetyl-glucosamine-6-phosphate into N-acetyl-glucosamine-1-phosphate.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

As above-mentioned, the expression level of the one or more recombinant nucleic acids encoding a polypeptide having phosphoacetylglucosamine mutase activity in the present invention is regulated by at least one promoter and at least one terminator, such as herein after defined more in detail, which are present in 5' and 3' position respectively of the one or more recombinant nucleic acids encoding a polypeptide having phosphoacetylglucosamine mutase activity.

### PROMOTERS

As disclosed herein, the expression of the genes of interest that have been genetically engineered for obtaining a recombinant cell according to the invention comprise appropriate regulatory sequences that are functional in the recombinant cells of the invention, and in particular in recombinant yeast cells of the invention, including in particular *Saccharomyces cerevisiae.*

Various promoters may be used for the desired expression of the coding sequences of interest.

Promoters according to the invention can be selected from the group consisting of the following promoters:
- pTDH3 (SEQ ID NO: 62),
- pTDH3.sk (SEQ ID NO: 63),
- pTDH3-1.Sba (SEQ ID NO: 64),
- pTDH3.Sar (SEQ ID NO: 65),
- pEN02 (SEQ ID NO: 66),
- pTEF3 (SEQ ID NO: 67),
- pTEF1 (SEQ ID NO: 68),
- pTEF1.ago (SEQ ID NO: 69),
- p TEF1.sba (SEQ ID NO: 70),
- pPDC1 (SEQ ID NO: 71),
- pCCW12 (SEQ ID NO: 72),
- pCCW12.Sm (SEQ ID NO: 73),
- pCCW12.sk (SEQ ID NO: 74),
- pCCW12.sba (SEQ ID NO: 75),
- pCCW12.sar (SEQ ID NO: 76),
- pNUP57 (SEQ ID NO: 77),
- pCCW10.ago (SEQ ID NO: 78),
- pCWP2 (SEQ ID NO: 79), and
- pRPLA1 (SEQ ID NO: 80).

Promoters more particularly interesting in the present invention may be selected from the group consisting of:
- pTDH3 (SEQ ID NO: 62),
- pTDH3.sk (SEQ ID NO: 63),
- pTDH3-1.Sba (SEQ ID NO: 64),
- pTDH3.Sar (SEQ ID NO: 65),
- pEN02 (SEQ ID NO: 66),
- pTEF3 (SEQ ID NO: 67),
- pTEF1 (SEQ ID NO: 68),
- pTEF1.ago (SEQ ID NO: 69),
- pTEF1.sba (SEQ ID NO: 70),
- pPDC1 (SEQ ID NO: 71),
- pCCW12 (SEQ ID NO: 72),
- pCCW12.Sm (SEQ ID NO: 73),
- pCCW12.sk (SEQ ID NO: 74),
- pCCW12.sba (SEQ ID NO: 75), and
- pCCW12.sar (SEQ ID NO: 76),

Said promoters can in particular be selected from the group consisting of pTDH3, pTDH3-1.Sba, pTDH3.Sar, pENO2, pTEF3, pTEF1, pPDC1, pCCW12, pCCW12.Sm, pCCW12.sk, pCCW12.sba, and pCCW12.sar.

Alternatively, promoters interesting in the present invention may be selected from the group consisting of:
- pNUP57 (SEQ ID NO: 77), and
- pCCW10.ago (SEQ ID NO: 78).

Other promoters of interest in the present invention may be:
- pCWP2 (SEQ ID NO: 79); and
- pRPLA1 (SEQ ID NO: 80).

As previously mentioned, inducible or repressible promoters are promoters whose activity is controlled by the presence or absence of biotic or abiotic factors and also by the quantity of said factor. Accordingly, for some promoters, their activity will in particular be induced and thus increased when the quantity of a given factor increases or is increased, and, accordingly, the activity of these same promoters can be repressed and thus reduced when the quantity of said factor diminishes or is reduced. The quantity of said factor(s) in the culture medium of a recombinant yeast cell of the invention comprising inducible or repressible promoters can be decided and thus controlled by the man skilled in the art.

For example, increasing the quantity of copper in a culture medium of a recombinant yeast cell according to the invention comprising a pCUP-1 promoter will induce and thus increase transcription of the gene under the control of this promoter. On the contrary, reducing the quantity of copper in said culture medium will lead to a repression, and thus a reduced, transcription of the gene under the control of this promoter.

In another example, increasing the quantity of methionine in a culture medium of a recombinant yeast cell according to the invention comprising a pMET6 promoter will repress and thus decrease transcription of the gene under the control of this promoter. On the contrary, reducing the quantity of methionine in said culture medium will lead to an induced, and thus an increased, transcription of the gene under the control of this promoter.

For this reason, the following promoters are referred to in the present text as being inducible or repressible promoters.

According to a first embodiment, inducible or repressible promoters according to the invention may be selected from the group comprising promoters inducible or repressible with copper, promoters inducible or repressible with methionine and promoters inducible or repressible with threonine, and are in particular is CUP1 - copper inducible or repressible (SEQ ID NO: 81).

According to this embodiment, the inducible or repressible promoter according to the invention can in particular be pCUP1.

The activity of these promoters is thus induced by the increasing presence of methionine, copper or threonine as indicated above, and their activity diminishes, i.e. is repressed, when the quantity of methionine, copper or threonine is reduced.

According to a second embodiment, inducible or repressible promoters according to the invention may be selected from the group comprising promoters inducible or repressible with copper, promoters inducible or repressible with lysine and promoters inducible or repressible with methionine, and in particular selected from the group consisting of:
- pMET6 - methionine inducible or repressible (SEQ ID NO: 82),
- pMET25 - methionine inducible or repressible (SEQ ID NO: 83), and
- pSAM1 - methionine inducible or repressible (SEQ ID NO: 84),

According to this particular embodiment, the inducible or repressible promoter according ot the invention may, be selected from the group consisting of pMET6, pMET25 and pSAM1.

The activity of these promoters is thus repressed by the increasing presence of methionine, copper, lysine or glucose as indicated above, and their activity increases, i.e. is induced, when the quantity of methionine, copper, lysine or glucose is reduced.

In a particular embodiment, inducible or repressible promoters according to the invention may be selected from the group comprising promoters inducible or repressible with copper, promoters inducible or repressible with glucose, promoters inducible or repressible with lysine, promoters inducible or repressible with methionine and promoters inducible or repressible with threonine.

In a more particular embodiment, the inducible or repressible promoter according to the invention may be selected from the group consisting of pCUP1, pMET6, pSAM1, and pMET25.

Synthetic promoters as described in Blazeck & Alper (2013) Biotechnol. J. 8 46-58 can also be used.

The promoters of the invention can originate from any organism from the Saccharomycetes class and can in particular originate from an organism selected from the group consisting of at least one of *Saccharomyces cerevisiae, Saccharomyces boulardii, Saccharomyces castelii, Saccharomyces bayanus, Saccharomyces arboricola, Saccharomyces kudriavzevii, Saccharomyces mikatae, Ashbya gossypii, Kluveromyces lactis, Pichia pastoris, Candida glabrata, Candida tropicalis, Debaryomyces castelii, Yarrowia lipolitica,* and *Cyberlindnera jadinii, .*

The promoters of the invention can preferably originate from an organism selected from the group consisting of *Saccharomyces cerevisiae (sc), Saccharomyces mikatae (Sm), Saccharomyces kudriavzevii (sk), Saccharomyces bayanus (sba), and Saccharomyces arboricola (Sar).*

### TERMINATORS

As it is disclosed herein, the expression of the genes of interest that have been genetically engineered for obtaining a recombinant cell according to the invention, and in particular a recombinant yeast according to the invention, comprise appropriate transcription terminator sequences that are functional in recombinant cells of the invention, and in particular in recombinant yeast cells of the invention, in particular in *Saccharomyces cerevisiae.*

Said transcription terminators, identical or different, may be found in literature Yamanishi et al., (2013) ACS synthetic biology 2, 337-347.

Terminators more particularly interesting in the present invention may be selected from the group comprising:
- tTPI1 from the gene encoding for the Triose Phosphate Isomerase (SEQ ID NO: 85),
- tMET25 from the gene encoding for the O-acetyl homoserine-O-acetyl serine sulfhydrylase (SEQ ID NO: 86),
- tDIT1 (SEQ ID NO: 87),
- tRPL3 (SEQ ID NO: 88),
- tRPL3.sm (SEQ ID NO: 89),
- tRPL3.sba (SEQ ID NO: 90),
- tRPL41B (SEQ ID NO: 91),
- tRPL15A (SEQ ID NO: 92),
- tRPL15A.sba (SEQ ID NO: 93),
- tIDP1 (SEQ ID NO: 94),
- tTEF1.sba (SEQ ID NO: 95), and
tTDH3 (SEQ ID NO: 100).

In particular, said terminator may be selected from the group consisting of tTPI1, tMET25, tDIT1, tRPL3, tRPL3.sm, tRPL3.sba, tRPL41B, tRPL15A, tRPL15A.sba, tIDP1, tTEF1.sba and tTDH3.

The terminators of the invention can originate from any organism from the Saccharomycetes class and can in particular originate from an organism selected from the group consisting of *Saccharomyces cerevisiae* and *Saccharomyces Bayanus.*

### RECOMBINANT CELLS

Recombinant cells of the invention can be selected from the group consisting of yeasts and bacteria.

Recombinant cells of the invention, such as a recombinant host cell of the invention, are preferably recombinant yeast cells.

Generally, yeast can grow rapidly and can be cultivated at higher density as compared with bacteria, and does not require an aseptic environment in the industrial setting. Furthermore, yeast cells can be more easily separated from the culture medium compared to bacterial cells, greatly simplifying the process for product extraction and purification.

A recombinant cell of the invention, and in particular a recombinant yeast cell of the invention is preferably a *Saccharomycetales* cell.

A recombinant cell of the invention, and in particular a recombinant yeast of the invention can in particular belong to the *Saccharomyces* genus, or to the *Candida* genus, or to the *Kluyveromyces* genus, or to the *Ogataea* genus, or to the *Yarrowia* genus, or to the *Debaryomyces* genus, or to the *Ashbya* genus.

A recombinant cell of the invention belonging to the *Saccharomyces* genus can be selected from the group consisting of *Saccharomyces cerevisiae, Saccharomyces boulardii, Saccharomyces bayanus, Saccharomyces paradoxus, Saccharomyces mikatae, Saccharomyces castelli, Saccharomyces cariocanus, Saccharomyces kudriavzevii, Saccharomyces arboricolus, Saccharomyces pastorianus, Saccharomyces uvarum* and *Saccharomyces delbrueckii.*

A recombinant cell of the invention belonging to the *Candida* genus can be selected from the group consisting of *Candida albicans, Candida glabrata, Candida tropicalis, Candida dubliniensis, Candida parapsilosis, Candida lusitaniae* and *Candida guilliermondii.*

A recombinant cell of the invention belonging to the *Kluyveromyces* genus can be selected from the group consisting of *Kluyveromyces lactis, Kluyveromyces marxianus, Kluyveromyces polysporus, Kluyveromyces thermotoleren, Kluyveromyces dobzhanskii* and *Kluyveromyces wickerhamii.*

A recombinant cell of the invention belonging to the *Ogataea* genus can be selected from the group consisting of *Ogataea polymorpha, Ogataea histrianica, Ogataea deakii, Ogataea kolombanensis, Ogataea philodendra, Ogataea siamensis, Ogataea angusta, Ogataea parapolymorpha, Ogataea minuta, Ogataea nonfermentans* and *Ogataea kodamae.*

A recombinant cell of the invention belonging to the *Yarrowia* genus can be selected from the group consisting of *Yarrowia lypolytica, Yarrowia parophonii, Yarrowia galli, Yarrowia oslonensis, Yarrowia alimentaria, Yarrowia hollandica* and *Yarrowia yakushimensis.*

A recombinant cell of the invention belonging to the *Debaryomyces* genus can be selected from the group consisting of *Debaryomyces hansenii, Debaryomyces carsonii, Debaryomyces castellii, Debaryomyces marama, Debaryomyces occidentalis, Debaryomyces oviformis, Debaryomyces nepalensis, Debaryomyces coudertii, Debaryomyces udenii, Debaryomyces psychrosporus* and *Debaryomyces yamadae.*

A recombinant cell of the invention belonging to the *Ashbya* genus can be selected from the group consisting of *Ashbya gossypii* and *Ashbya aceri.*

A recombinant cell of the invention, and in particular a recombinant yeast cell of the invention, can in particular be selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens, Ogataea polymorpha, Yarrowia lypolytica, Debaryomyces hansenii,* and *Ashbya gossypii,* and is preferably *Saccharomyces cerevisiae.*

In a particular embodiment, the recombinant yeast cell according to the invention is of the genus *Saccharomyces, Kluyveromyces,* or *Eremothecium,* more particularly is of the species selected from the group consisting of *Saccharomyces cerevisiae, Kluyveromyces Marxianus, Ogataea polymorpha* and *Ashbya gossypii.*

In an embodiment, a recombinant host cell of the invention is a yeast selected belonging to the *Saccharomycetales* order, in particular to the *Saccharomycetaceae* family, and is in particular selected from the group consisting of *Yarrowia lipolitica, Kluyveromyces Marxianus, Ogataea polymorpha, Ashbya gossypii and Saccharomyces cerevisiae.*

A recombinant cell of the invention can most preferably be a *Saccharomyces cerevisiae* cell.

As above-mentioned, a recombinant cell according to the invention has the ability to produce hyaluronic acid by insertion of the one or more recombinant nucleic acids according to the present invention. In certain embodiments, a recombinant yeast according to the invention has the ability to produce hyaluronic acid having a controlled size (controlled molecular weight) by insertion of the one or more recombinant nucleic acids according to the present invention.

Methods implemented to insert a specific DNA construct within a gene belong to the general knowledge of a man skilled in the art. A related method is described in more details in the herein after examples.

However, unexpected technical difficulties were encountered because the consequences of the insertion of DNA constructs within the genome of a cell, and in particular within the genome of a yeast, such as for example in the genome of *Saccharomyces cerevisiae,* are unpredictable. In particular, the survival rate of the cells, and in particular of the yeasts, and their ability to grow and produce the desired hyaluronic acid is also unpredictable.

In order to obtain a recombinant cell, and in particular a recombinant yeast, of the invention, many different constructs were tested by the inventors in order to obtain a viable and efficient recombinant cell, and in particular yeast.

### CULTURE CONDITIONS

The present invention also relates to the use of a recombinant cell of the invention, for the production of hyaluronic acid, in particular of hyaluronic acid of controlled molecular weight.

The present invention further relates to a method of producing hyaluronic acid (HA) of a desired molecular weight (HAMW) comprising:
(a) cultivating a recombinant cell of the present invention in a cultivation medium for a time sufficient to produce hyaluronic acid (HA) of the desired molecular weight; and
(b) optionally isolating or recovering the hyaluronic acid (HA) from the recombinant cell and/or from the cultivation medium.

Typically, cells of the invention, and in particular yeasts of the invention, are grown at a temperature in the range of about 20°C to about 37°C, preferably at a temperature ranging from 27 to 34°C, in an appropriate culture medium.

Suitable growth media for cells of the invention, and in particular for yeasts of the invention, are common commercially prepared media such as broth that includes yeast nitrogen base, ammonium sulfate, and dextrose as the carbon/energy source or YPD Medium, a blend of peptone, yeast extract, and dextrose in optimal proportions for growing most. Other defined or synthetic growth media may also be used and the appropriate medium for growth of the particular cell, and in particular yeast, will be known by one skilled in the art of microbiology or fermentation science.

A particular medium that is suitable herein is the SY medium which comprises the following elements:
KH2PO4: 100 mM; MgSO4 7H2O: 2,8 mM; K2SO4: 11,5 mM; Na2SO4: 1,1 mM; NaCl: 2,6 mM; CaCl2 2H2O: 0,7 mM; CuSO4 5H2O: 15 µM; KI: 6 µM; FeCl3: 30 µM; ZnSO4 7H2O: 61 µM; MnSO4 H2O: 25 µM; H2SO4: 110 µM; Panthotenic Acids hemicalcium salt: 42 µM; Thiamin hydrochloride: 59 µM; Pyridoxin hydrochloride: 49 µM ; Myo-Inositol (C6H12O6): 555 µM; Nicotinic acid (C6H5NO2): 29 µM; D-Biotine: 0,82 µM; Ammonium citrate tribasic: 33 mM; and glucose or sucrose 2-30%.

Carbon sources that may be used in the culture medium include fructose, mannose, xylose and arabinose; oligosaccharides such as lactose, maltose, galactose, or sucrose; polysaccharides such as starch or cellulose or mixtures thereof; and unpurified mixtures from renewable feedstocks such as cheese whey permeate cornsteep liquor, sugar beet molasses, and barley malt.

Nitrogen sources that may be included in the culture medium include peptone, yeast extracts, meat extracts, malt extracts, urea, ammonium sulfate, ammonium chloride, ammonium nitrate, ammonium phosphate, ammonium citrate and combinations thereof.

The culture medium may further comprise trace elements (e.g., metal salts), for example magnesium salts, cobalt salts and/or manganese salts; as well as growth factors such as amino acids, vitamins, growth promoters, and the like.

Examples of vitamins that may be included are Panthotenic Acids hemicalcium, Thiamin hydrochloride; Pyridoxin hydrochloride; Myo-Inositol; Nicotinic acid; D-Biotine, folic acid, p-aminobenzoïque acid, riboflavin.

A culture medium of the invention may further include rare elements, such as CuSO₄· 5H₂O, KI, FeCl₃, ZnSO₄ ·7H₂O, MnSO₄ ·H₂O, or H₂SO₄, MgCl2, CaCl2, NaCl, K2HPO4, KH2PO4, ZnCl, H3BO3, MnSO4, Na2MoO4.

The term "appropriate culture medium" is above-defined.

Examples of known culture media for a recombinant cell according to the present invention are known to the person skilled in the art, and are presented in the following publication D. Burke et al., Methods in yeast Genetics - A cold spring harbor laboratory course Manual (2000).

Suitable pH ranges for the fermentation may be between pH 3.0 to pH 7.5, where pH 4 to pH 6 is preferred as the initial condition.

As mentioned elsewhere in this specification, the pH value of the culture medium can be regulated during the cultivation step of a method of the invention in order to modulate the activity of the polypeptide facing hyaluronidase activity, which will impact the molecular weight of the hyaluronic acid molecules produced by the recombinant cell of the invention, and in particular by the recombinant yeast of the invention.

In particular, the pH of the culture medium can be modified according to the hyaluronic acid that is meant to be produced by the recombinant yeast. For example, the pH of the culture medium may remain at a pH of 4, of 5,5 or of 6 during the culture time.

In a particular embodiment, the pH of the culture medium may change or be changed during the time length of the culture of the recombinant cell of the invention, in particular of the recombinant yeast of the invention. As previously mentioned, *Saccharomyces cerevisiae* acidifies the medium in which it is cultivated and thus reduces the pH of its culture medium. For example, the pH of the culture medium may begin at 6, may be taken down to 4 and then brought back up to 6. In another example, the pH of the culture medium may begin at 6, remain or be maintained at 6, and then may be lowered to 4.

In a particular embodiment, the pH of the culture medium may be modulated during the cultivating step (a) of a method of the invention so that at the end of the cultivating step of a method of the invention, the pH of the cultivation medium is the same as the pH at the beginning of said cultivation step (a).

In another embodiment, the pH of the culture medium may remain the same during the time length of the culture of the recombinant cell of the invention, and in particular the recombinant yeast cell of the invention.

Said time length of the culture of a recombinant cell according to the invention, and in particular of the recombinant yeast of the invention, can vary depending on the molecular weight of the hyaluronic acid of interest. The longer said time length, the lower the molecular weight of hyaluronic acid in a given culture medium of a recombinant cell of the invention, and in particular a recombinant yeast cell of the invention.

The time length of the culture time of a recombinant cell of the invention, and in particular a recombinant yeast cell in the present invention can be a period of from about 35 hours to about 50 hours, preferably from about 40 hours to about 50 hours, and is in particular about 48 hours.

Fermentations may be performed under aerobic conditions or micro-aerobic conditions.

The amount of hyaluronic acid product in the fermentation medium can be determined using a number of methods known in the art, for example, high performance liquid chromatography (HPLC) or gas chromatography (GC).

The present process may employ a batch method of fermentation. A classical batch fermentation is a closed system where the composition of the medium is set at the beginning of the fermentation and not subject to artificial alterations during the fermentation. Thus, at the beginning of the fermentation, the medium is inoculated with the desired organism or organisms, and fermentation is permitted to occur without adding anything to the system. Typically, however, a "batch" fermentation method or system is batch with respect to the addition of carbon source and attempts are often made at controlling factors such as temperature, pH and oxygen concentration. In batch systems, the metabolite and biomass compositions of the system change constantly up to the time when the fermentation is stopped. Within batch cultures cells progress through a static lag phase to a high growth log phase and finally to a stationary phase where growth rate is diminished or halted. If untreated, cells in the stationary phase will eventually die. Cells in log phase generally are responsible for the bulk of production of end product or intermediate.

A Fed-Batch system may also be used in the present invention. A Fed-Batch system is similar to a typical batch system with the exception that the carbon source substrate is added in increments as the fermentation progresses. Fed-Batch systems are useful when catabolite repression (e.g. glucose repression) is apt to inhibit the metabolism of the cells and where it is desirable to have limited amounts of substrate in the media. Measurement of the actual substrate concentration in Fed-Batch systems is difficult and is therefore estimated on the basis of the changes of measurable factors such as pH, dissolved oxygen and the partial pressure of waste gases such as CO₂.

Batch and Fed-Batch culturing methods are common and well known in the art and examples may be found in Biotechnology: A Textbook of Industrial Microbiology, Crueger, Crueger, and Brock, Second Edition (1989) Sinauer Associates, Inc., Sunderland, MA, or Deshpande, Mukund V., Appl. Biochem. Biotechnol., 36, 227, (1992). Although the present invention is performed in batch mode it is contemplated that the method would be adaptable to continuous fermentation.

Continuous fermentation is an open system where a defined fermentation medium is added continuously to a bioreactor and an equal amount of conditioned media is removed simultaneously for processing. Continuous fermentation generally maintains the cultures at a constant high density where cells are primarily in log phase growth.

Continuous fermentation allows for the modulation of one factor or any number of factors that affect cell growth or end product concentration. For example, one method will maintain a limiting nutrient such as the carbon source or nitrogen level at a fixed rate and allow all other parameters to vary. In other systems a number of factors affecting growth can be altered continuously while the cell concentration, measured by media turbidity, is kept constant. Continuous systems strive to maintain steady state growth conditions and thus the cell loss due to the medium being drawn off must be balanced against the cell growth rate in the fermentation. Methods of modulating nutrients and growth factors for continuous fermentation processes as well as techniques for maximizing the rate of product formation are well known in the art of industrial microbiology.

It is contemplated that the present invention may be practiced using either batch, fed- batch or continuous processes and that any known mode of fermentation would be suitable. Additionally, it is contemplated that cells may be immobilized on a substrate as whole cell catalysts and subjected to fermentation conditions for production.

In order to still improve the hyaluronic acid production, a particular embodiment may consist of culturing the recombinant cells of the invention, in particular the recombinant yeast cells of the invention, in an appropriate culture medium, such as above-mentioned, wherein the said culture medium comprises an optimal amount of carbon source, especially glucose or sucrose.

In preferred embodiments, the carbon source comprised in said optimal culture medium consists of glucose and/or sucrose. In preferred embodiments, the said optimal culture medium comprises 1% w/w or more glucose and/or sucrose, in particular comprises 5% w/w or more glucose and/or sucrose, in particular comprises 10% w/w or more glucose and/or sucrose, in particular comprises 15% w/w or more glucose and/or sucrose. In preferred embodiments, the said optimal culture medium comprises at most 40% w/w glucose, which includes at most 35% w/w glucose.

In a preferred embodiment, the method of the invention is carried out on an industrial scale.

More particularly, the cultivation medium of the method according to the invention can be at least about 100L, more preferably in the range of about 1000L to about 3000L, even more preferably about 10,000L, even more preferably 100,000L, or even about 250,000L.

The invention further relates to a method for producing hyaluronic acid as previously described, and comprising the steps of:
(a) culturing a recombinant cell of the invention in a culture medium; and
(b) recovering the hyaluronic acid from said culture medium,
   wherein the hyaluronic acid recovered in step (b) has a molecular weight controlled through the selection of:
   - the nature and origin of the one or more recombinant nucleic acids encoding a polypeptide having hyaluronidase activity of the recombinant cell of the invention, and in particular of the recombinant yeast of the invention,
   - the nature and origin of the promoter controlling the expression of the one or more recombinant nucleic acids encoding a polypeptide having hyaluronidase activity of the recombinant cell of the invention, and in particular of the recombinant yeast of the invention,
   - the presence or absence of an anchoring signal associated to the one or more recombinant nucleic acids encoding a polypeptide having hyaluronidase activity of the recombinant cell of the invention, and in particular of the recombinant yeast of the invention,
   - the pH of the culture medium during the step of culturing the recombinant cell of the invention, and in particular of the recombinant yeast of the invention, and/or
   - the duration of the culturing of the recombinant cell of the invention, and in particular of the recombinant yeast of the invention.

The molecular weight of the hyaluronic acid can be a particular molecular weight or more preferably a specific range of molecular weights such as, for example, less than 50 kDa, in the range of about 20 kDa to about 50 kDa, greater than or equal to 50 kDa, in the range of about 50 kDa to about 150kDa, in the range of about 50 kDa to about 250kDa, greater than or equal to 100 kDa, in the range of about 100kDa to about 1500 kDa, in the range of about 150kDa to about 1500 kDa, greater than 1000 kDa or greater than 1500kDa.

The present invention also relates to the use of a recombinant cell according to the invention, and in particular a recombinant yeast cell of the invention, for the production of hyaluronic acid (HA) having a molecular weight in the range of from about 20kDa to about 50kDa or from about 50kDa to about 1000 kDa.

The one or more recombinant nucleic acids encoding a polypeptide having hyaluronidase activity of a recombinant cell of the invention can for example be selected from those originating or being derived from at least one of *Cupiennius salei (Csa), Loxosceles intermedia (Li), Hirudo nipponia (Hn), Bothrops atrox (Ba), Tityus serrulatus (Ts) or Vespa magnifica (Vm), in particular from at least one of Cupiennius salei (Csa), Loxosceles intermedia (Li), Hirudo nipponia (Hn), Bothrops atrox (Ba)* or *Tityus serrulatus (Ts),* more particularly from those of sequences set forth as sequences SEQ ID NO: 19 (Ba), SEQ ID NO: 25 (Li), SEQ ID NO: 21 (Csa), SEQ ID NO: 27 (Ts) or SEQ ID NO: 23 (Hn) (in the presence of a secretion signal and in the absence of an anchoring signal).

The one or more recombinant nucleic acids encoding a polypeptide having hyaluronidase activity of a recombinant cell of the invention can for example be selected from those originating or being derived from at least one of *Cupiennius salei (Csa), Loxosceles intermedia (Li), Hirudo nipponia (Hn), Bothrops atrox (Ba), Tityus serrulatus (Ts) or Vespa magnifica (Vm), in particular from Cupiennius salei (Csa), Loxosceles intermedia (Li), Hirudo nipponia (Hn), Bothrops atrox (Ba)* or *Tityus serrulatus (Ts),* in particular from those of sequences set forth as sequences SEQ ID NO: 22 (Csa), SEQ ID NO: 26 (Li), SEQ ID NO: 24 (Hn), SEQ ID NO: 20 (Ba) or SEQ ID NO: 28 (Ts) (in the presence of both a secretion signal and an anchoring signal).

The one or more recombinant nucleic acids encoding a polypeptide having hyaluronidase activity of a recombinant cell of the invention can for example be placed under the control of a promoter selected from the group consisting of pTEF1, pCCW12, pCCW12.sba, pCCW12.Sar, pPDC1, pTEF3, pTDH3, pNUP57 and pCCW10.ago.

In particular, the promoters of the one or more recombinant nucleic acids encoding a polypeptide having hyaluronidase activity of a recombinant cell of the invention, and in particular of a recombinant yeast of the invention, may originate or be derived from *Saccharomyces Bayanus, Saccharomyces kudriavzevii, Saccharomyces mikatae, Saccharomyces arboricola* or other saccharomycetales, or *Abishia gossypii.*

The secretion signal of the present invention may for example have:
- the nucleic acid sequence set forth as SEQ ID NO: 43; and/or
- the amino acid sequence set forth as SEQ ID NO: 44.

The anchoring signal of the present invention may for example have:
- the nucleic acid sequence set forth as SEQ ID NO: 45; and/or
- the amino acid sequence set forth SEQ ID NO: 46.

The secretion signal may be fused to the polypeptide having hyluronidase activity by creating a chimeric nucleic acid starting by a nucleic acid sequence encoding the signal peptide followed by a recombinant nucleic acid encoding a polypeptide having hyaluronidase activity as previously defined.

The secretion signal and anchoring signal may be fused to the polypeptide having hyluronidase activity by creating a chimeric nucleic acid starting by a nucleic acid sequence encoding the signal peptide followed by a recombinant nucleic acid encoding a polypeptide having hyaluronidase activity as previously defined, followed by a nucleic acid sequence encoding for the anchoring signal.

Such chimeric nucleic acid sequences may be obtained by techniques known by the man skilled in the art such as chemical synthesis of nucleic acids or any recombination techniques such as cloning or PCR.

The invention further relates to a method as previously described, for producing hyaluronic acid having a molecular weight less than or equal to about 50 kDa, and in particular less than 50 kDa and greater than or equal to about 20 kDa, wherein:
(a) the pH of the medium is superior to 4, and is in particular in the range of from about 5 to about 7;
(b) the one or more recombinant nucleic acids encoding a polypeptide having hyaluronidase activity integrated in the genome of the recombinant cell of the invention originates or is derived:
   (i) from *Tityus serrulatus,* possesses a secretion signal but is devoid of an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar;
   (ii) from *Cupiennius salei,* possesses a secretion signal but is devoid of an anchoring signal, and is under the control of a promoter selected from the group consisting of pNUP57 and pCCW10.ago;
   (iii) from *Cupiennius salei,* possesses a secretion signal and an anchoring signal, and is under the control of the promoter pCWP2;
   (iv) from *Loxosceles intermedia* possesses a secretion signal but is devoid of an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar; or
   (v) from *Hirudo nipponia,* possesses a secretion signal but is devoid of an anchoring signal, and is under the control of a promoter selected from the group consisting of pNUP57 and pCCW10.ago; or
   (vi) from *Hirudo nipponia,* possesses both a secretion signal and an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar; and
(c) the step of recovering the hyaluronic acid from the culture medium is performed about 48 hours after the beginning of the culturing of the recombinant cell of the invention, in particular the recombinant yeast cell of the invention;
the recombinant cell being in particular a recombinant yeast cell, and more particularly a *Saccharomyces cerevisiae* cell.

The invention further relates to a method as previously described, for producing hyaluronic acid having a molecular weight greater than or equal to about 50 kDa and less than or equal to about 1000 kDa, wherein:
(a) the pH of the medium is between about 4 and about 6 and is optionally varied from about 6 to about 4 during the culturing step of the recombinant cell, in particular the recombinant yeast cell, of the invention;
(b) the one or more recombinant nucleic acids encoding a polypeptide having hyaluronidase activity integrated in the genome of the recombinant cell of the invention originates or is derived:
   (i) from *Tityus serrulatus,* possesses a secretion signal but is devoid of an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar;
   (ii) from *Tityus serrulatus,* possesses both a secretion signal and an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar;
   (iii) from *Loxosceles intermedia,* possesses both a secretion signal and an anchoring signal, and is under the control of a promoter selected from the group consisting of pNUP57 and pCCW10.ago;
   (iv) from *Loxosceles intermedia,* possesses a secretion signal but is devoid of an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar,
   (v) from *Cupiennius salei,* possesses both a secretion signal and an anchoring signal, and is under the control of a promoter selected from the group consisting of pNUP57 and pCCW10.ago,
   (vi) from *Cupiennius salei,* possesses a secretion signal but is devoid of an anchoring signal, and is under the control of a promoter selected from the group consisting of pNUP57 and pCCW10.ago, or
   (vii) from *Hirudo nipponia,* possesses both a secretion signal and an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar; and
(c) the step of recovering the hyaluronic acid from the culture medium is performed about 48 hours after the beginning of the culturing of the recombinant cell of the invention, in particular the recombinant yeast cell of the invention;
the recombinant cell being in particular a recombinant yeast cell, and more particularly a *Saccharomyces cerevisiae* cell.

The invention further relates to a method as previously described, for producing hyaluronic acid having a molecular weight greater than about 1000 kDa, and in particular having a molecular weight from about 1000 kDa to about 1.5 Million Da, wherein:
(a) the pH of the medium is from about 4 to about6 and is optionally varied from about6 to about 4 during the culturing step of the recombinant cell, in particular recombinant yeast, of the invention;
(b) the one or more recombinant nucleic acids encoding a polypeptide having hyaluronidase activity integrated in the genome of the recombinant cell of the invention originates or is derived:
   (i) from *Tityus serrulatus,* possesses a secretion signal but is devoid of an anchoring signal, and is under the control of a promoter selected from the group consisting of pNUP57 and pCCW10.ago;
   (ii) from *Loxosceles intermedia,* possesses a secretion signal but is devoid of an anchoring signal, and is under the control of a promoter selected from the group consisting of pNUP57 and pCCW10.ago;
   (iii) from *Bothrops atrox,* possesses a secretion signal but is devoid of an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar; or
   (iv) from *Bothrops atrox,* possesses both a secretion signal and an anchoring signal, and is under the control of a promoter selected from the group consisting of pTDH3, pENO2, pTEF3, pTEF1, pPDC1, pCCW12, pCCW12.Sm, pCCW12.sk, pCCW12.sba, pCCW12.sar, pTDH3-1.Sba and pTDH3.Sar; and
(c) the step of recovering the hyaluronic acid from the culture medium is performed about 48 hours after the beginning of the culturing of the recombinant cell of the invention, in particular the recombinant yeast cell of the invention;
the recombinant cell being in particular a recombinant yeast, and more particularly a *Saccharomyces cerevisiae* cell.

Another aspect of the present invention relates to hyaluronic acid (HA) obtained or obtainable from a recombinant cell of the invention or from a method according to the invention.

A further aspect of the invention is a cultivation medium comprising the hyaluronic acid of the invention.

The invention further relates to a composition comprising the hyaluronic acid according to the invention.

The invention also relates to an industrial product or a consumer product or a consumable comprising (i) the hyaluronic acid of the invention, (ii) the cultivation medium comprising hyaluronic acid of the invention or (iii) the composition comprising hyaluronic acid of the invention.

In particular, said industrial product or consumer product or consumable according to the invention can be a cosmetic product, a flavour product, a fragrance product, a food product, a food, a beverage, a texturant, a pharmaceutical composition, a dietary supplement, a nutraceutical, a cleaning product and/or a dental and/or an oral hygiene composition.

### PURIFICATION OF HYALURONIC ACID

According to a specific aspect of the invention, the fermentative production of hyaluronic acid preferably comprises a step of isolation of the hyaluronic acid produced from the culture medium. Recovering the hyaluronic acid from the culture medium is a routine task for a man skilled in the art. It may be achieved by a number of techniques well known in the art including but not limiting to pervaporation, selective precipitation, filtration, centrifugation, spray drying, lyophylisation or liquid extraction. The expert in the field knows how to adapt parameters of each technique dependant on the characteristics of the material to be separated.

The yeast as model of a cell in the present invention is preferred in that the synthesized hyaluronic acid is/are entirely exported outside the cells, thus simplifying the purification process.

Gas stripping is achieved with a stripping gas chosen among helium, argon, carbon dioxide, hydrogen, nitrogen or mixture thereof.

Liquid extraction is achieved with organic solvent as the hydrophobic phase such as pentane, hexane, heptane or dodecane. Renewal solvents may also be used.

Those of skill in the art will recognize that, due to the degenerate nature of the genetic code, a variety of DNA molecules differing in their nucleotide sequences can be used to encode a given enzyme of the disclosure. The native DNA sequence encoding the biosynthetic enzymes described above are referenced herein merely to illustrate an embodiment of the disclosure, and the disclosure includes DNA molecules of any sequence that encode the amino acid sequences of the polypeptides and proteins of the enzymes utilized in the methods of the disclosure. In similar fashion, a polypeptide can typically tolerate one or more amino acid substitutions, deletions, and insertions in its amino acid sequence without loss or significant loss of a desired activity. The disclosure includes such polypeptides with different amino acid sequences than the specific proteins described herein so long as the modified or variant polypeptides have the enzymatic anabolic or catabolic activity of the reference polypeptide. Furthermore, the amino acid sequences encoded by the DNA sequences shown herein merely illustrate embodiments of the disclosure.

Described herein are specific genes and proteins useful in the methods, compositions and organisms of the disclosure; however it will be recognized that absolute identity to such genes is not necessary. For example, changes in a particular gene or polynucleotide comprising a sequence encoding a polypeptide or enzyme can be performed and screened for activity. Typically such changes comprise conservative mutations and silent mutations. Such modified or mutated polynucleotides and polypeptides can be screened for expression of a functional enzyme using methods known in the art.

Due to the inherent degeneracy of the genetic code, other polynucleotides which encode substantially the same or functionally equivalent polypeptides can also be used to clone and express the polynucleotides encoding such enzymes

Techniques known to those skilled in the art may be suitable to identify additional homologous genes and homologous enzymes. Generally, analogous genes and/or analogous enzymes can be identified by functional analysis and will have functional similarities.

Techniques known to those skilled in the art may be suitable to identify analogous genes and analogous enzymes or any biosynthetic pathway genes, proteins, or enzymes, techniques may include, but are not limited to, cloning a gene by PCR using primers based on a published sequence of a gene/enzyme of interest, or by degenerate PCR using degenerate primers designed to amplify a conserved region among a gene of interest. Further, one skilled in the art can use techniques to identify homologous or analogous genes, proteins, or enzymes with functional homology or similarity. Techniques include examining a cell or cell culture for the catalytic activity of an enzyme through *in vitro* enzyme assays for said activity (e.g. as described herein or in Kiritani, K., Branched-Chain Amino Acids Methods Enzymology, 1970), then isolating the enzyme with said activity through purification, determining the protein sequence of the enzyme through techniques such as Edman degradation, design of PCR primers to the likely nucleic acid sequence, amplification of said DNA sequence through PCR, and cloning of said nucleic acid sequence. To identify homologous or similar genes and/or homologous or similar enzymes, analogous genes and/or analogous enzymes or proteins, techniques also include comparison of data concerning a candidate gene or enzyme with databases such as BRENDA, KEGG, or MetaCYC. The candidate gene or enzyme may be identified within the above mentioned databases in accordance with the teachings herein.

### DERIVATIVES

The term "Hyaluronic acid (HA)", as used herein, is also intended to cover derivatives of Hyaluronic acid, such as but not limited to acetylated or sulfated Hyaluronic acid useful for applications in a cosmetic product, flavor product a fragrance product, a food product, a food, a beverage, a texturant, a pharmaceutical composition, a dietary supplement, a nutraceutical, a cleaning product and/or a dental and/or an oral hygiene composition or combinations thereof

Hyaluronic acid may be prepared as a composition.

### FORMULATIONS AND PRODUCTS

A composition of the invention may be incorporated into a formulation/product, such as a nutraceutical, pharmaceutical, veterinary, oenological or cosmetic formulation/product.

Thus, the present invention provides a formulation comprising a composition of the invention. For example, the present invention may provide a cosmetic formulation comprising the composition of the invention.

The present invention also provides a product comprising a composition of the invention. For example, the present invention may provide a cosmetic product comprising the composition of the invention.

A "cosmetic product" is intended to mean any substance or mixture intended to be placed in contact with the external parts of the human body (epidermis, hair system, nails, lips and external genital organs) or with the teeth and the mucous membranes of the oral cavity with a view exclusively or mainly to cleaning them, perfuming them, changing their appearance, protecting them, keeping them in good condition, correcting body odours and/or combinations thereof.

A "substance" is intended to mean a chemical element and its compounds in the natural state or obtained by any manufacturing process, including any additive necessary to preserve its stability and any impurity deriving from the process used but excluding any solvent which may be separated without affecting the stability of the substance or changing its composition.

A "mixture" is intended to mean a mixture or solution composed of two or more substances.

The present invention also provides the use of a composition of the invention in a nutraceutical, pharmaceutical, veterinary, oenological or cosmetic formulation/product.

Such formulations or products are hereinafter referred to as the "formulations or products of the invention".

The nutraceutical, pharmaceutical, veterinary, oenological or cosmetic formulation/product may optionally further comprise pharmaceutically/veterinary/cosmetic (including cosmetic active) ingredients, such as excipients, carriers and mixtures thereof as appropriate.

"Cosmetic" or "Cosmetic Active Ingredients" means any and all natural, naturally occurring, nature identical, synthetic synthetically produced, biosynthetically produced, sustainable, renewable and/or biodegradable compounds, ingredients, intermediates, molecules, substances, raw materials or products individually or as part of a mixture of compounds, ingredients, intermediates, molecules, substances, raw materials or products, blends, compositions, formulations (including but not limited to skin moisturizers, creams, balms, serums, oils, eye, facial makeup, wash off hair products, leave-on hair products, hair colorants (including but not limited to natural hair colorants) and/or combinations thereof), finished products and related technologies including, but not limited to components, incorporated, for example, into a cosmetic formulation (such as but not limited to natural colorants, preservatives, emulsifiers, anti-oxidants and the like which do not, for example, have an activity on the skin, hair, scalp and the like but play a role in the formulation of the finished product), delivery systems, marketing aids (such as, for example, coloured unispheres applied to translucent formulations) and methods of making anything related thereto useful in/used for/intended for:
- application by rubbing, pouring, sprinkling, spraying or otherwise directly on or to a human or animal body and/or by placing in contact with the various external and/or on surface parts of a human or animal body (including but not limited to the skin, hair, body hair, the hair system, scalp, nails, lips, external genitalia, teeth, oral and/or nasal mucosa and the like); and/or application indirectly on or to a human or animal body such as for example, application as part of a textile or application to a textile as part of a delivery device (such as a capsule) or a delivery system (such as blends or formulations) applied to a textile; and/or
- cleansing, caring, cooling, beautifying, conditioning, treating, soothing, texturizing, promoting attractiveness, protecting, maintaining, improving, enhancing, altering and/or changing an external part and/or surface of a human or animal body (such as, but not limited to the scalp) or the aesthetic appearance of a human or animal body; and/or with a view to mainly cleaning or perfuming, or protecting or maintaining in good condition or combating body odour or changing the appearance of or correcting or repairing a state of imbalance in skin, oral mucosa, scalp or hair by providing a calming, healing, repairing, or revitalization, hydration of the skin or in order to provide relief to, lubricate, moisten, tone, heal, sterilize, relieve, correct and/or remedy states of dryness, irritation, injury or fatigue, and/or with a view to correcting pigmentation disorders or providing a non-pharmaceutical prevention and/or treatment of dandruff, acne, irritation and/or inflammation and the like and/or rebalancing the bacterial flora (such as, for example, the microbiome) on the surface of the skin (such as, for example, by promoting the level of beneficial bacterial flora on the skin surface) and/or for the purposes of keeping a human or animal body in good condition for health and/or wellbeing purposes and/or for improving the appearance of a human or animal body by, for example, improving the appearance of a product applied to a human or body; and/or
- providing a cosmetic and/or dermatological function and/or benefit with a biological activity benefit (but without affecting a body's structure or function. For the avoidance of doubt, a Cosmetic or Cosmetic Active Ingredient or any part thereof may also qualify as a Functional Ingredient and/or Nutraceutical.

"Functional Ingredient" means a food ingredient or part of a food that provides medicinal or health benefits, including any of the following: a carotenoid, dietary fiber, fatty acid, saponin, antioxidant, flavonoid, isothiocyanate, phenol, polyphenol (such as resveratrol), plant sterol or stanol (phytosterols and phytostanols), a polyol, a prebiotic, a phytoestrogen, soy protein, sulfides/thiol, a vitamin, glucosamine, preservatives, hydration agents, edible gelling ingredients, edible gel mixes and gel compositions, long chain primary aliphatic saturated alcohols, colour agents, texturizing agents, emulsifiers and combinations thereof.

"Nutraceutical" means any and all natural, naturally occurring, sustainable, synthetically-produced and bio-synthetically-produced compounds, mixtures of compounds, Functional Ingredients, molecules, compositions, raw materials, and intermediates (including components and delivery devices (such as capsules) related thereto, delivery systems thereof (such as blends or formulations) and methods of making the foregoing) that are associated with health and/or cosmetic benefits, as well as improving or maintaining the appearance of the human body. For the avoidance of doubt, Nutraceuticals includes compounds that can be used as supplements to food or beverage, whether a solid formulation, capsule, tablet, liquid formulation, solution or suspension.

Alternatively, the nutraceutical, pharmaceutical, veterinary, oenological or cosmetic formulation/product may consist or consist essentially of the composition of the invention.

The cosmetic formulation/product may be an anti-aging formulation.

As used herein, references to pharmaceutically, veterinary or cosmetically acceptable excipients may refer to pharmaceutically, veterinary or cosmetically acceptable adjuvants, diluents and/or carriers as known to those skilled in the art.

By "pharmaceutically/veterinary/cosmetically acceptable" we mean that the additional components of the composition are generally safe, non-toxic, and neither biologically nor otherwise undesirable. For example, the additional components may be generally sterile and pyrogen free. Such components must be "acceptable" in the sense of being compatible with the composition of the invention and not deleterious to the recipients thereof. Thus, "pharmaceutically acceptable excipients" includes any compound(s) used in forming a part of the formulation that is intended to act merely as an excipient, i.e. not intended to have biological activity itself.

The nutraceutical, pharmaceutical, veterinary, oenological or cosmetic formulation/product may be in the form of a liquid or a solid.

Liquid dosage formulations/products for oral administration include solutions, emulsions, aqueous or oily suspensions, syrups and elixirs.

Formulations and products (e.g. pharmaceutical, veterinary or cosmetic formulations/products) described herein, such as those intended for oral administration, may be prepared according to methods known to those skilled in the art, such as by mixing the components of the formulation/product together.

The formulation or product (e.g. pharmaceutical, veterinary or cosmetic formulation/product) may contain one or more additional ingredients, such as pharmaceutical ingredients and excipients, such as sweetening agents, flavouring agents, colouring agents and preserving agents.

The formulation or product (e.g. pharmaceutical, veterinary or cosmetic formulation/product) may also contain one or more additional active ingredients, such as cosmetic or pharmaceutical active ingredients, such as hyaluronic acid, centella asiatica extract, peptides such as Matrixyl^{®} and Argireline^{®}, and mixtures thereof.

The formulation or product of the invention may contain the active ingredient(s) in admixture with non-toxic pharmaceutically acceptable excipients (or ingredients). These excipients (or ingredients) may, for example, be: inert diluents, such as calcium carbonate, sodium carbonate, lactose, calcium phosphate or sodium phosphate; granulating and disintegrating agents, for example, corn starch, maltodextrin or alginic acid; binding agents, for example, starch, gelatine or acacia; or lubricating agents, for example magnesium stearate, stearic acid, talc and mixtures thereof.

Liquid formulations or products (e.g. pharmaceutical, veterinary or cosmetic formulations/products) may be contained within a capsule, which may be uncoated or coated as defined above.

Suitable pharmaceutical or veterinary carriers include inert solid diluents or fillers, sterile aqueous solutions and various organic solvents. Examples of liquid carriers are syrup, peanut oil, olive oil, phospholipids, fatty acids, fatty acid amines, polyoxyethylene and water.

Moreover, the carrier or diluent may include any sustained release material known in the art, such as glyceryl monostearate or glyceryl distearate, alone or mixed with a wax.

Suitable pharmaceutical carriers include inert sterile aqueous solutions and various organic solvents. Examples of liquid carriers are syrup, vegetables oils, phospholipids, fatty acids, fatty acid amines, polyoxyethylene and water. Moreover, the carrier or diluent may include any sustained release material known in the art, such as glyceryl monostearate or glyceryl distearate, alone or mixed with a wax.

Suitable cosmetic carriers are typically those that are suitable for topical administration to the outer surface of the human body, such as the skin and/or hair and/or scalp.

Typically, such carriers are dermatologically acceptable.

The phrase "dermatologically acceptable carrier" means that the carrier is suitable for topical application to the keratinous tissue, has good aesthetic properties, is compatible with the actives in the composition, and will not cause any unreasonable safety or toxicity concerns.

The carrier can be in a wide variety of forms. In some instances, the solubility or dispersibility of the components (e.g. extracts, sunscreen active, additional components) may dictate the form and character of the carrier. Non-limiting examples include simple solutions (e.g. aqueous or anhydrous), dispersions, emulsions, and solid forms (e.g. gels, sticks, flowable solids, or amorphous materials).

The dermatologically acceptable carrier may be in the form of an emulsion. An emulsion may be generally classified as having a continuous aqueous phase (e.g. oil-in-water and water-in-oil-in-water) or a continuous oil phase (e.g. water-in-oil or oil-in-water). The oil phase of the present invention may comprise silicone oils, non-silicone oils such as hydrocarbon oils, esters, ethers, and the like, and mixtures thereof. The aqueous phase typically comprises water and water-soluble ingredients (e.g. water-soluble moisturizing agents, conditioning agents, anti-microbials, humectants and/or other skin care actives). However, in some instances, the aqueous phase may comprise components other than water, including but not limited to watersoluble moisturizing agents, conditioning agents, antimicrobials, humectants and/or other watersoluble skin care actives. In some instances, the non-water component of the composition comprises a humectant, such as glycerin and/or other polyol(s). Emulsions may also contain an emulsifier. Emulsifiers may be non-ionic, anionic or cationic.

The carrier may contain one or more dermatologically acceptable, hydrophilic diluents. As used herein, "diluent" includes materials in which the composition of the invention can be dispersed, dissolved, or otherwise incorporated. Hydrophilic diluents include water, organic hydrophilic diluents, such as lower monovalent alcohols (e.g., C1-C4), and low molecular weight glycols and polyols, including propylene glycol, polyethylene glycol, polypropylene glycol, glycerol, butylene glycol, 1,2,4-butanetriol, sorbitol esters, 1,2,6-hexanetriol, ethanol, isopropanol, sorbitol esters, butanediol, ether propanol, ethoxylated ethers, propoxylated ethers and combinations thereof.

The cosmetic formulation/product may optionally include one or more additional ingredients commonly used in cosmetic compositions (e.g., colorants, skin tone agents, skin anti-aging agents, anti-inflammatory agents, sunscreen agents, combinations of these and the like), provided that the additional ingredients do not undesirably alter the anti-glycation benefits provided by the composition.

In some instances, it may be desirable to select skin tone agents that function via different biological pathways so that the actives do not interfere with one another, which could reduce the efficacy of both agents. The additional ingredients, when incorporated into the composition, should be suitable for use in contact with human skin tissue without undue toxicity, incompatibility, instability, allergic response, and the like.

The term "carrier" as used herein, may also refer to a natural product or a product originating from nature that has been transformed or modified so that it is distinct from the natural product from which it originated, such as maltodextrin.

The amount of the composition of the invention present in nutraceutical, pharmaceutical, veterinary, oenological or cosmetic formulations or products will vary depending on the application.

Typically, the amount of composition of the invention that may be present in nutraceutical, pharmaceutical, veterinary, oenological or cosmetic formulations or products will be from about 0.001 to about 50% by weight, such as from about 0.01% to about 30% or from about 1% to about 20% of the nutraceutical, pharmaceutical, veterinary, oenological or cosmetic formulations or products, such as from about 0.01 to about 20%, or from about 0.1 to 10% or from about 1 to about 5% by weight of the formulation or product.

Those of skill in the art will recognize that, due to the degenerate nature of the genetic code, a variety of DNA molecules differing in their nucleotide sequences can be used to encode a given enzyme of the disclosure. The native DNA sequence encoding the biosynthetic enzymes described above are referenced herein merely to illustrate an embodiment of the disclosure, and the disclosure includes DNA molecules of any sequence that encode the amino acid sequences of the polypeptides and proteins of the enzymes utilized in the methods of the disclosure. In similar fashion, a polypeptide can typically tolerate one or more amino acid substitutions, deletions, and insertions in its amino acid sequence without loss or significant loss of a desired activity. The disclosure includes such polypeptides with different amino acid sequences than the specific proteins described herein so long as the modified or variant polypeptides have the enzymatic anabolic or catabolic activity of the reference polypeptide. Furthermore, the amino acid sequences encoded by the DNA sequences shown herein merely illustrate embodiments of the disclosure.

Described herein are specific genes and proteins useful in the methods, compositions and organisms of the disclosure; however it will be recognized that absolute identity to such genes is not necessary. For example, changes in a particular gene or polynucleotide comprising a sequence encoding a polypeptide or enzyme can be performed and screened for activity. Typically such changes comprise conservative mutations and silent mutations. Such modified or mutated polynucleotides and polypeptides can be screened for expression of a functional enzyme using methods known in the art.

Due to the inherent degeneracy of the genetic code, other polynucleotides which encode substantially the same or functionally equivalent polypeptides can also be used to clone and express the polynucleotides encoding such enzymes.

Techniques known to those skilled in the art may be suitable to identify additional homologous genes and homologous enzymes. Generally, analogous genes and/or analogous enzymes can be identified by functional analysis and will have functional similarities.

Techniques known to those skilled in the art may be suitable to identify analogous genes and analogous enzymes or any biosynthetic pathway genes, proteins, or enzymes, techniques may include, but are not limited to, cloning a gene by PCR using primers based on a published sequence of a gene/enzyme of interest, or by degenerate PCR using degenerate primers designed to amplify a conserved region among a gene of interest. Further, one skilled in the art can use techniques to identify homologous or analogous genes, proteins, or enzymes with functional homology or similarity. Techniques include examining a cell or cell culture for the catalytic activity of an enzyme through *in vitro* enzyme assays for said activity (e.g. as described herein or in Kiritani, K., Branched-Chain Amino Acids Methods Enzymology, 1970), then isolating the enzyme with said activity through purification, determining the protein sequence of the enzyme through techniques such as Edman degradation, design of PCR primers to the likely nucleic acid sequence, amplification of said DNA sequence through PCR, and cloning of said nucleic acid sequence. To identify homologous or similar genes and/or homologous or similar enzymes, analogous genes and/or analogous enzymes or proteins, techniques also include comparison of data concerning a candidate gene or enzyme with databases such as BRENDA, KEGG, or MetaCYC. The candidate gene or enzyme may be identified within the above-mentioned databases in accordance with the teachings herein.

The terms "between... and..." and "ranging from... to..." should be understood as being inclusive of the limits, unless otherwise specified. Throughout this specification and the claims which follow, unless the context requires otherwise, the word "comprise", and variations such as "comprises", "comprising" and "possesses", will be understood to imply the inclusion of a stated integer or step or group of integers or steps but not the exclusion of any other integer or step or group of integer or step. The term "comprising" also means "including" as well as "consisting" e.g. a composition "comprising" X may consist exclusively of X or may include something additional e.g. X + Y. It must be noted also that, as used in this specification and the appended claims, the singular forms "a", "an" and "the" include plural referents unless the content clearly dictates otherwise. By way of example, a reference to "a gene" or "an enzyme" is a reference to "one or more genes" or "one or more enzymes".

It is to be understood that this disclosure is not limited to the particular methodology, protocols and reagents described herein as these may vary. It is also to be understood that the terminology used herein is for the purpose of describing particular embodiments only, and is not intended to limit the scope of the present disclosure which will be limited only by the appended claims. Unless defined otherwise, all technical and scientific terms used herein have the same meanings as commonly understood by the person skilled in the art. In accordance with the present disclosure there may be conventional molecular biology, microbiology, and recombinant DNA techniques employed which are within the skill of the art.

This disclosure is not limited in its application to the details of construction and the arrangement of components set forth in the following description or illustrated in the drawings. The disclosure is capable of other embodiments and of being practiced or of being carried out in various ways. Also, the phraseology and terminology used herein is for the purpose of description and should not be regarded as limiting. Preferably, the terms used herein are defined as described in "A multilingual glossary of biotechnological terms: (IUPAC Recommendations)", Leuenberger, H.G.W, Nagel, B. and Kolbl, H. eds. (1995), Helvetica Chimica Acta, CH-4010 Basel, Switzerland).

Several documents are cited throughout the text of this specification. Each of the documents cited herein (including all patents, patent applications, scientific publications, manufacturer's specifications, instructions, GenBank Accession Number sequence submissions etc.), whether supra or infra, is hereby incorporated by reference in its entirety.

The examples and figures which follow are presented by way of illustration and without implied limitation of the invention.

### EXAMPLES

### Exemple 1: Protocol for making a recombinant Saccharomyces cerevisiae strain according to the invention

All the hereinafter implemented recombinant *Saccharomyces cerevisiae* strains were constructed from standard strains using standard yeast molecular genetics procedure (Methods in yeast Genetics - A cold spring harbor laboratory course Manual (2000) by D. Burke, D. Dawson, T. Stearns CSHL Press).

Cluster of the following-mentioned genes were integrated in recombinant yeast at once using the ability of yeast to efficiently recombine free DNA ends which have sequence homology.

In addition, for a better comprehension of following genotypes:
- jlp1, lyp1, sam3, his3, leu2, trp1 and ura3 are insertion sites.
- Lowercase letters mean that the considered gene is inactive, uppercase letters reflect an active gene.
- "::": following a gene name means that the gene is interrupted by what follows (if more than one gene are inserted, they are noted in brackets []). The interruption of the gene is concomitant with an entire deletion of the coding sequence but preserves the promoter. In consequence the gene followed by "::" is inactive and is noted in lowercase. If not specified the transcription of the gene inserted is controlled by the promoter of the disrupted gene.
- "gene.Kl" means that the gene originates from *Kluyveromyces lactis.*

More particularly, the coding sequences to be cloned were artificially synthetized. For heterologous sequences (non-yeast), the nucleic sequences were modified in order to obtain a synonymous coding sequence using the yeast codon usage. Using restriction enzyme and classical cloning technology, each synthetic sequence was cloned in between a transcription promoter and a transcription terminator. Each promoter sequence is preceded by a 50 to 200 nucleotide sequence homologous to the sequence of the terminator of the upstream gene. Similarly, the terminator of each gene (a gene comprising the promoter-coding sequence-terminator) is followed by sequences homologous to the gene immediately following. So that each of the unit to be integrated have a 50-200 nucleotide overlap with both the unit upstream and the unit downstream. For the first unit, the promoter is preceded by 50-200 nucleotides homologous to the yeast chromosome nucleotide for the locus in which it will be integrated. Similarly, for the last unit, the terminator is followed by 50-200 nucleotides homologous to the yeast chromosome nucleotide for the locus in which it will be integrated.

Each unit is then PCR amplified from the plasmids constructs, yielding X unit of linear DNA having overlapping sequences. At least one of this gene is an auxotrophic marker, in order to select for recombination event. All the linear fragments are transformed in the yeast at once, and a recombinant yeast cell is selected for the auxotrophy related to the marker used. The integrity of the sequence is then verified by PCR and sequencing.

### Example 2: Comparative examples for the production of Hyaluronic acid

### 1. Production of Hyaluronic acid of less than 50 kDa

**A.** Firstly, three recombinant strains are obtained: YA5234-1, YA5235-1 and YA5302-2.

Accordingly, these three strains are as follows:
**YA5234-1** : MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tTPI1, pTDH3-QRI1-tMET25, pCCW12-HASB.At-tRPL15A], leu2, lyp1::[pCCW12.sba-HYAL-3.Ts-tRPL15A, pCCW12.Sk-HASB-A.Vir-tTEF1.Sba, pCCW12-HASA-1.Vir-tDIT1, pCCW12. Sba-HASB.vir-tRPL3, pCCW12. Sm-HASA-A.Vir-tRPL15A. Sba], sam3::[LEU2.K1, pPDC1-PGM1-tIDP1, pTEF1-GFA1-tR-PL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25 -HASA-1.Vir-tIDP1- TRP1]x2
**YA5235-1:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tTPI1, pTDH3-QRI1-tMET25, pCCW12-HASB.At-tRPL15A], leu2, lyp1::[pNUP57-HYAL-3.Csa-tRPL15A, pCCW12.Sk-HASB-A.Vir-tTEF1.Sba, pCCW12-HASA-1.Vir-tDIT1, pCCW12. Sba-HASB.vir-tRPL3, pCCW12. Sm-HASA-A.Vir-tRPL15A. Sba], sam3::[LEU2.K1, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5302-2** : MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2, lypl::[pCCW12.Sba-HYAL-3.Li-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.K1, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.Xl-tRPL3, pTDH3-QRI1-tIDP1], trp1::[pMET6-HASB.Vir-tRPL3, pMET25-HASA-1.Vir-tIDP1- TRP1]x2

HYAL-3 represents a nucleic acid sequence encoding a polypeptide having hyaluronidase activity associated with a secretion signal but no anchoring signal.

HASA, HASA-1, HASA-A, HASA2 and XHASA2 all represent a nucleic acid encoding a polypepyide having hyaluronan synthase activity. They differ from one another in that they are different reencoded versions of a nucleic acid sequence encoding the hyaluronan synthase enzymes. HASA-1 has the sequence SEQ ID NO: 1, HASA-A has the sequence SEQ ID NO: 2, HASA2 has the sequence SEQ ID NO: 7 and XHASA2 has the sequence SEQ ID NO: 6.

HASB and HASB-A represent a nucleic acid sequence encoding a polypeptide having UDP-glucose 6-dehydrogenase activity. They differ from one another in that they are different reencoded versions of a nucleic acid sequence encoding the enzyme. HASB.At has the sequence SEQ ID NO: 12, HASB.Vir has the sequence SEQ ID NO: 13 and HASB-A.Vir has the sequence SEQ ID NO: 14.

All these strains were incubated in 25 ml of SY medium buffered with MES 0,1 M ph 5.5 in a baffled erlenmeyer for 48 hours at 28°C under vigourous agitation.

SY medium comprises the following elements:
KH2PO4: 100 mM; MgSO4 7H2O: 2,8 mM; K2SO4: 11,5 mM; Na2SO4: 1,1 mM; NaCl: 2,6 mM; CaCl2 2H2O: 0,7 mM; CuSO4 5H2O: 15 µM; KI: 6 µM; FeCl3: 30 µM; ZnSO4 7H2O: 61 µM; MnSO4 H2O: 25 µM; H2SO4: 110 µM; Panthotenic Acids hemicalcium salt: 42 µM; Thiamin hydrochloride: 59 µM; Pyridoxin hydrochloride: 49 µM ; Myo-Inositol (C6H12O6): 555 µM; Nicotinic acid (C6H5NO2): 29 µM; D-Biotine: 0,82 µM; Ammonium citrate tribasic: 33 mM; and glucose or sucrose 2-30%.

Growth medium was recovered at 48h hours and assayed for hyaluronic acid content and quality. An aliquot of the medium was loaded and ran on a 0.5% agarose gel subsequently colored with "stains all" (sigma Aldrich CAS Number 7423-31-6).

The amount of hyaluronic acid present in the medium was evaluated by a colorimetric determination after treatment with concentrated sulfuric acid and carbazole (Bitter and Muir (1962) analytical biochemistry 4, 330-334).

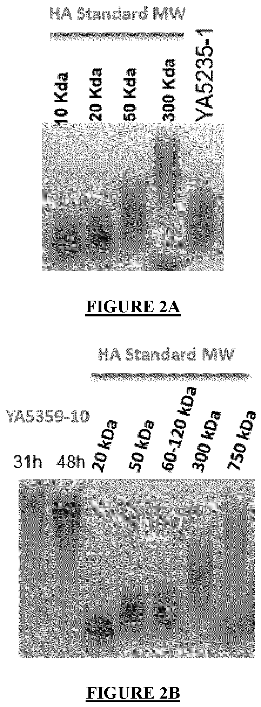

For illustrative purposes, the agarose gel obtained after running an aliquot of the supernatant of strain YA5235-1 and staining it with "stains all" (CAS Number 7423-31-6) is provided as Figure 2A. It is classically run into the gel side-by-side with HA molecular weight (MW) standards (from left to right MW of 10 kDa, 20 kDa, 50 kDa and 300 kDa).

The amounts obtained with these different strains are respectively:
- YA5234-1: 2.4 g.L⁻¹.
- YA5235-1: 4.5 g.L⁻¹.
- YA5302-2: 3.7 g.L⁻¹.

In comparision, a native yeast strain, as for example the CC788-2D strain (Chérest et al. (2000) J Biol. Chem. 275: 14056-14063), does not produce hyaluronic acid.

It results from this experiment that a recombinant strain comprising the modifications according to the invention produces a greater amount of hyarluronic acid when cultured in the same conditions as compared with other recombinant strains not comprising all the genetic modifications according to the invention.

Moreover, these three strains led to the production, after 48 hours, of hyarluronic acid having a molecular weight comprised between 20kDa and 50kDa.

**B.** Three other recombinant strains have also been obtained: YA5110-13 and YA5260-4.

These three strains are as follows:
**YA5110-13:** MAT-α, can1-100, his3::[pSAM1-UGP1-tRPL3, pMET6-QRI1-tIDP1-HIS3]x5, jlp1::[LEU2.K1, pCUP1-HASA2.Sz-tRPL41B, pCUP1-UGP1-tTPI1, pTDH3-QRI1-tMET25, pCCW12-HASB.At-tRPL15A], leu2, lyp1::[pNUP57-HYAL-3.Hn-tRPL15A, pCCW12.Sk-HASB-A.Vir-tTEF1.Sba, pCCW12-HASA-1.Vir-tDIT1, pCCW12. Sba-HASB.vir-tRPL3, pCCW12. Sm-HASA-A.Vir-tRPL15A. Sba], sam3::[LEU2.K1, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5260-4:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2, lyp1::[pTEF3-HYAL-31.Hn-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.K1, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5569** : MAT-α, his3::[pSAM1-UGP1- tRPL3, pMET6-QRI1-tIDP1, HIS3]x5, jlp1::[LEU2.K1, pCUP1-HASA-1.Sz-tRPL41B, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2, lypl::[pCWP2-HYAL-31.Csa-tR-PL15A, pCCW12.Sba-HASB.vir-tRPL3, pCCW12.Sk-HASB-A. Vir-tTEF1. Sba, pCCW12-HASA-1.Vir-tRPL41B, pCCW12. Sm-HASA-A.Vir-tRPL15A.Sba], sam3::[LEU2.K1, pPDC1-PGM1-tIDP1, pTEF1.Ago-GFA1-tRPL15A, pENO2-UGP1-tRPL3, pCWP2-GNA1-tTPI1, pTEF1-PCM1-tIDP1, pCCW12-XHASA2.X1-tRPL3, pTDH3 -QRI1-tIDP1], trp1::[tRPL3-HASB.Vir-pMET6, pMET25-HASA-1.Vir-tIDP1, TRP1]x2

HYAL-31 represents a nucleic acid sequence encoding a polypeptide having hyaluronidase activity associated with both a secretion signal and an anchoring signal.

HASA-1, HASA-A, HASA2 and XHASA2 all represent a nucleic acid sequence encoding a polypeptide having hyaluronan synthase activity. They differ from one another in that they are different reencoded versions of a nucleic acid sequence encoding the hyaluronan synthase enzyme. HASA-1 has the sequence SEQ ID NO: 1, HASA-A has the sequence SEQ ID NO: 2, HASA2 has the sequence SEQ ID NO: 7 and XHASA2 has the sequence SEQ ID NO: 6.

HASB and HASB-A represent a nucleic acid sequence encoding a polypeptide having UDP-glucose 6-dehydrogenase activity. They differ from one another in that they are different reencoded versions of a nucleic acid sequence encoding the enzyme. HASB.At has the sequence SEQ ID NO: 12, HASB.Vir has the sequence SEQ ID NO: 13 and HASB-A.Vir has the sequence SEQ ID NO: 14.

The strains were inoculated in 2 liters of the above defined SY medium at ph 6 in 2% sucrose and 500µM CuSO4 in a bioreactor. They were then grown in fed-batch adding 500 µM CuSO4 at 7h, 24h, 31h and between 130 and 175 g.L-1 of sucrose from 10 to 48 h.

For YA5110-13, pH was maintained between 5.8 and 6.2 using 15% H2SO₄ and 10% NH4OH from t=31 h.

For YA5260-4, pH maintained between 5.8 and 6.2 using 15% H2SO₄ and 10% NH4OH all along the fermentation.

For YA5569, pH maintained between 5.8 and 6.2 using 15% H2SO₄ and 10% NH4OH all along the fermentation.

Growth medium was recovered at 48h hours and assayed for hyaluronic acid content and quality as described above.

The hyaluronic acid amounts obtained with these two strains are respectively:
- YA5110-13: 23 g.L⁻¹.
- YA5260-4: 16 g.L⁻¹.
- YA5569: 32 g.L⁻¹

In comparision, a native strain does not produce hyaluronic acid.

It results from this experiment that a recombinant strain comprising the modifications according to the invention produces a greater amount of hyaluronic acid when cultured in the same conditions as compared with other recombinant strains not comprising all the genetic modifications according to the invention.

Moreover, the hyaluronic acid produced by YA5110-13 and YA5260 strains had a molecular weight of about 20 kDa while the hyaluronic acid produced by YA5569 had a molecular weight of about 20 kDa to 50 kDa.

The exact same experiment was performed using these three recombinant strains while replacing sucrose by glucose. The hyaluronic acid obtained had the same molecular weight of about 20 kDa or of about 20 kDa to about 50 kDa after 48h.

**C.** Another recombinant strain has been obtained 5672-29A, as follows:
**5672-29A:** MAT-a, his3::[ pSAM1-UGP1-tRPL3, pMET6-QRI1-tIDP1, HIS3]x5, jlp1::[LEU2.Sba, pTEF1.Sba-HASB.Vir-tRPL3.Sm, pTDH3.Sk-HASB.Vir-tTEF1.Sba, pTDH3-1.Sba-HASA-1.Vir-tRPL3.Sba, pTDH3.Sar-HASA-1.Vir-tRPL15A.Sba, pTEF1-HYAL-31.Hn-tRPL15A], leu2, sam3::[LEU2.K1, pPDC1-PGM1-tIDP1, pTEF1.Ago-GFA1-tRPL15A, pENO2-UGP1-tRPL3, pCWP2-GNA1-tTPI1, pTEF1-PCM1-tRPL41B, TRP1.Sba-loxP], trp1

HYAL-31 represents a hyaluronidase associated with an anchoring signal.

1 HASA-1 represents a nucleic acid sequence encoding a polypeptide having hyaluronan synthase activity. HASA-1 has the sequence SEQ ID NO: 1.

The strain was inoculated in 25 ml of the above defined SY medium at ph 5.5 buffered with MES 0.1 M in a baffled erlenmeyer at 28°C under vigourous agitation.

Growth medium was recovered at 48h hours and assayed for hyaluronic acid content and quality as described above.

The hyaluronic acid amount obtained with the strain was 3.7 g.L⁻¹.

### 2. Production of Hyaluronic acid having a molecular weight comprised between 50 kDa and 1000 kDa

A. Firstly, three recombinant strains are obtained: YA5233-1, YA5359-10 and YA5381-1.

Accordingly, these three strains are as follows:
**YA5233-1:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tTPI1, pTDH3-QRI1-tMET25, pCCW12-HASB.At-tRPL15A], leu2, lyp1::[pTEF1-HYAL-3.Ts-tRPL15A, pCCW12.Sk-HASB-A.Vir-tTEF1.Sba, pCCW12-HASA-1.Vir-tDIT1, pCCW12. Sba-HASB.vir-tRPL3, pCCW12. Sm-HASA-A.Vir-tRPL15A. Sba], sam3::[LEU2.K1, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5359-10:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1- tTPI1, pTDH3-QRI1-tMET25, pCCW12-HASB.At-tRPL15A], leu2, lyp1::[pCCW12.sba-HYAL-31.Ts-tRPL15A, pCCW12.Sk-HASB-A.Vir-tTEF1.Sba, pCCW12-HASA-1.Vir-tDIT1, pCCW12. Sba-HASB.vir-tRPL3, pCCW12. Sm-HASA-A.Vir-tRPL15A. Sba], sam3::[LEU2.K1, pPDC1-PGM1-tIDP1, pTEF1-GFA1-tR-PL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1-TRP1]x2
**YA5381-1:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1- tTPI1, pTDH3-QRI1-tMET25, pCCW12-HASB.At-tRPL15A], leu2, lyp1::[pCCW12.Sar-HYAL2-31.Ts-tRPL15A, pCCW12.Sk-HASB-A.Vir-tTEF1.Sba, pCCW12-HASA-1.Vir-tDIT1, pCCW12. Sba-HASB.vir-tRPL3, pCCW12.Sm-HASA-A.Vir-tRPL15A.Sba], sam3:: [ LEU2.K1, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2

HASA-1, HASA-A, HASA2 and XHASA2 are previously defined.

HASB and HASB-A are previously defined.

All these strains were incubated in 25 ml of the above-defined SY medium buffered with MES 0,1 M ph 5.5 baffled erlenmeyer for 48 hours at 28°C under vigourous agitation.

Growth medium was recovered at 48h hours and assayed for hyaluronic acid content and quality as defined above.

For illustrative purposes, the agarose gel obtained after running an aliquot of the supernatant of strain YA5359-10, after 31h and after 48h, and staining it with "stains all" (CAS Number 7423-31-6) is provided as Figure 2B. It is classically run into the gel side-by-side with HA molecular weight (MW) standards (from left to right MW of 20 kDa, 50 kDa, 60-120 kDa, 300 kDa and 750 kDa).

The amounts obtained with these different strains are respectively:
- YA5233-1: 3.1 g.L⁻¹.
- YA5359-10: 3.4 g.L⁻¹.
- YA5381-1: 3.0 g.L⁻¹.

In comparision, a native strain does not produce hyaluronic acid.

It results from this experiment that a recombinant strain comprising the modifications according to the invention produces a greater amount of hyarluronic acid when cultured in the same conditions as compared with other recombinant strains not comprising all the genetic modifications according to the invention.

Moreover, these three strains led to the production, after 48 hours, of hyaluronic acid having a molecular weight comprised:
- for YA5233-1: between 50 kDa and 500 kDa;
- for YA5359-10: between 100 kDa and 750 kDa; and
- for YA5381-1: between 50 kDa and 250 kDa.

**B.** Six other recombinant strains have also been obtained: YA5262-16, YA5326-3, YA5300-15, YA5358-4, YA5264-1 and YA5331-6.

These six strains are as follows:
**YA5262-16:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2, lyp1::[pCCW10.ago-HYAL-31.Hn-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[LEU2.K1, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.Xl-tRPL3, pTDH3-QRI1-tIDP1], trp1::[pMET6-HASB.Vir-tRPL3, pMET25-HASA-1.Vir-tIDP1- TRP1]x2
**YA5326-3:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tTPI1, pTDH3-QRI1-tMET25, pCCW12-HASB.At-tRPL15A], leu2, lyp1::[pCCW10.ago-HYAL-31.Csa-tRPL15A, pCCW12.Sk-HASB-A.Vir-tTEF1.Sba, pCCW12-HASA-1.Vir-tDIT1, pCCW12. Sba-HASB.vir-tRPL3, pCCW12. Sm-HASA-A.Vir-tRPL15A. Sba], sam3::[LEU2.K1, pPDC1-PGM1-tIDP1, pTEF1-GFA1-tR-PL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5300-15:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2, lyp1::[ pTDH3-HYAL-3.Ts-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[ LEU2.K1, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.Xl-tRPL3, pTDH3-QRI1-tIDP1], trp1::[pMET6-HASB.Vir-tRPL3, pMET25-HASA-1.Vir-tIDP1- TRP1]x2
**YA5358-4:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tRPL3, pCUP1-QRI1-tMET25, pCCW12-HASB.At-tRPL15A], leu2, lyp1::[pCCW12.Sar-HYAL-31.Li-tRPL15A, pCCW12.Sk-HASB-A.Vir-tTEF1.Sba, pCCW12-HASA-1.Vir-tDIT1, pCCW12. Sba-HASB.vir-tRPL3, pCCW12. Sm-HASA-A.Vir-tRPL15A. Sba], sam3::[LEU2.K1, pPDC1-PGM1-tIDP1, pTEF1-GFA1-tR-PL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5264-1:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tTPI1, pTDH3-QRI1-tMET25, pCCW12-HASB.At-tRPL15A], leu2, lyp1::[pTDH3-HYAL-31.Ts-tRPL15A, pCCW12.Sk-HASB-A.Vir-tTEF1.Sba, pCCW12-HASA-1.Vir-tDIT1, pCCW12. Sba-HASB.vir-tRPL3, pCCW12. Sm-HASA-A.Vir-tRPL15A. Sba], sam3::[LEU2.K1, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5331-6:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tRPL3, pCUP1-QRI1-tIDP1, pPDC1-UGP1-tTPI1, pTDH3-QRI1-tMET25, pCCW12-HASB.At-tRPL15A], leu2, lyp1::[ pTEF3-HYAL-31.Li-tRPL15A, pCCW12.Sk-HASB-A.Vir-tTEF1.Sba, pCCW12-HASA-1.Vir-tDIT1, pCCW12.Sba-HASB.vir-tRPL3, pCCW12.Sm-HASA-A.Vir-tRPL15A.Sba], sam3::[ LEU2.K1, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.Xl-tRPL3, pTDH3-QRI1-tIDP1], trp1::[pMET6-HASB.Vir-tRPL3, pMET25-HASA-1.Vir-tIDP1- TRP1]x2

HASA-1, HASA-A, HASA2 and XHASA2 are previously defined.

HASB and HASB-A are previously defined.

YA5262-16 was inoculated in 2 liters of the above defined SY medium at ph 4 in 2% glucose in a bioreactor and ph was maintained at 4 all along the fermentation using 15% H₂SO₄ and 10% NH₄OH.

YA5326-3 was:
- inoculated in 2 liters of the above defined SY medium at ph 6 in 2% glucose in a bioreactor and ph was maintained at 6 all along the fermentation using 15% H₂SO₄ and 10% NH₄OH; or
- inoculated in 2 liters of the above defined SY medium at ph 6 in 2% sucrose in a bioreactor and ph was maintained at 6 until 34h and at ph 4 from 34h to 48h with 15% H₂SO₄ and 10% NH₄OH.

YA5300-15 was inoculated in 2 liters of the above defined SY medium at ph 6 in 2% sucrose in a bioreactor and ph was maintained at 6 all along the fermentation using 15% H₂SO₄ and 10% NH₄OH.

YA5358-4 was inoculated in 2 liters of the above defined SY medium at ph 6 in 2% glucose in a bioreactor and ph was maintained at 6 all along the fermentation using 15% H₂SO₄ and 10% NH₄OH.

YA5264-1 was inoculated in 2 liters of the above defined SY medium at ph 6 in 2% sucrose in a bioreactor and ph was maintained at 6 all along the fermentation using 15% H₂SO₄ and 10% NH₄OH.

YA5331-6 was inoculated in 2 liters of the above defined SY medium at ph 6 in 2% glucose in a bioreactor and ph was maintained at 6 all along the fermentation using 15% H₂SO₄ and 10% NH₄OH.

Growth medium was recovered at 48h hours and assayed for hyaluronic acid content and quality as described above.

The hyaluronic acid amounts obtained with these two strains are respectively:
- YA5262-16: 19 g.L⁻¹;
- YA5326-3: respectively 13 g.L⁻¹ and 15 g.L⁻¹;
- YA5300-15: 9 g.L⁻¹;
- YA5358-4: 14 g.L⁻¹;
- YA5264-1: 6 g.L⁻¹; and
- YA5331-6: 9 g.L⁻¹.

In comparison, a native strain does not produce hyaluronic acid.

It results from this experiment that a recombinant strain comprising the modifications according to the invention produces a greater amount of hyaluronic acid when cultured in the same conditions as compared with other recombinant strains not comprising all the genetic modifications according to the invention.

Moreover, the hyaluronic acid produced by these strains after 48 hours had a molecular weight:
- YA5262-16: of 50 kDa;
- YA5326-3: of respectively 50 kDa or comprised between 50 kDa and 250 kDa;
- YA5300-15: comprised between 100 kDa and 1000 kDa;
- YA5358-4: comprised between 100 kDa and 1000 kDa;
- YA5264-1: comprised between 100 kDa and 1000 kDa; and
- YA5331-6: comprised between 100 kDa and 1000 kDa.

Furthermore, previously described recombinant strain YA5235-1 was inoculated in a bioreactor at ph 6 in 2% sucrose and ph was maintained at 6 untill 34h and at 4 from 34h to 48h with 15% H₂SO₄ and 10% NH₄OH.

After 48h the medium contains 18 g.L⁻¹ of 100-1000 kDa Hyaluronic acid.

The same experiment was performed using YA5235-1 while using glucose instead of sucrose. The obtained hyaluronic acid also has a molecular weight comprised between 100 and 1000 kDa.

These results show that, in particular, pH regulation along the fermentation process as well as the production of a hyaluronidase coupled to a secretion signal or a secretion signal and an anchoring signal influences, or even allows controling, the size of the hyaluronic acid polymer produced.

### 3. Production of Hyaluronic acid having a molecular weight superior to 1000 kDa

Four recombinant strains are obtained: YA5232-1, YA5303-3, YA5374-4 and YA5382-1.

Accordingly, these four strains are as follows:
**YA5232-1:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1-tTPI1, pTDH3-QRI1-tMET25, pCCW12-HASB.At-tRPL15A], leu2, lyp1::[pNUP57-HYAL-3.Ts-tRPL15A, pCCW12.Sk-HASB-A.Vir-tTEF1.Sba, pCCW12-HASA-1.Vir-tDIT1, pCCW12. Sba-HASB.vir-tRPL3, pCCW12. Sm-HASA-A.Vir-tRPL15A. Sba], sam3::[LEU2.K1, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5303-2:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1- tTPI1, pTDH3-QRI1-tMET25, pCCW12-HASB.At-tRPL15A], leu2, lyp1::[pNUP57-HYAL-3.Li-tRPL15A, pCCW12.Sk-HASB-A.Vir-tTEF1.Sba, pCCW12-HASA-1.Vir-tDIT1, pCCW12. Sba-HASB.vir-tRPL3, pCCW12. Sm-HASA-A.Vir-tRPL15A. Sba], sam3::[LEU2.K1, pPDC1-PGM1-tIDP1, pTEF1-GFA1-tRPL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5374-3:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1- tTPI1, pTDH3-QRI1-tMET25, pCCW12-HASB.At-tRPL15A], leu2, lyp1::[pCCW12.Sar-HYAL-3Ba-tRPL15A, pCCW12.Sk-HASB-A.Vir-tTEF1.Sba, pCCW12-HASA-1.Vir-tDIT1, pCCW12. Sba-HASB.vir-tRPL3, pCCW12. Sm-HASA-A.Vir-tRPL15A. Sba], sam3::[LEU2.K1, pPDC1-PGM1-tIDP1, pTEF1-GFA1-tR-PL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2
**YA5382-1:** MAT-α, can1-100, his3::[pSAM1-UGP1- tRPL3-pMET6-QRI1-tIDP1- HIS3]x5, jlp1::[LEU2.K1, pCUP1-HASA2.Sz-tRPL41B, pCUP1-UGP1- tTPI1, pTDH3-QRI1-tMET25, pCCW12-HASB.At-tRPL15A], leu2, lyp1::[pTEF1-HYAL2-31.Ba-tRPL15A, pCCW12.Sk-HASB-A.Vir-tTEF1.Sba, pCCW12-HASA-1.Vir-tDIT1, pCCW12. Sba-HASB.vir-tRPL3, pCCW12. Sm-HASA-A.Vir-tRPL15A. Sba], sam3::[LEU2.K1, pPDC1-PGM1-tIDP1, pTEF1-GFA1-tR-PL15A, pENO2-UGP1-tRPL3, pCWP2-GNA1-tTPI1, pTEF3-PCM1-tIDP1, pCCW12-XHASA2.X1-tRPL3, pTDH3-QRI1-tIDP1], trp1::[pMET6-HASB.Vir-tRPL3, pMET25-HASA-1.Vir-tIDP1- TRP1]x2

HASA-1, HASA-A, HASA2 and XHASA2 are previously defined.

HASB and HASB-A are previously defined.

All these strains were incubated in 25 ml of the above-defined SY medium buffered with MES 0,1 M ph 5.5 in a baffled erlenmeyer for 48 hours at 28°C under vigourous agitation.

Growth medium was recovered at 48h hours and assayed for hyaluronic acid content and quality as defined above.

The amounts obtained with these different strains are respectively:
- YA5232-1: 4 g.L⁻¹.
- YA5303-3: 2.7 g.L⁻¹.
- YA5374-3: 2.5 g.L⁻¹.
- YA5382-1: 3.0 g.L⁻¹.

In comparision, a native strain does not produce hyaluronic acid.

It results from this experiment that a recombinant strain comprising the modifications according to the invention produces a greater amount of hyarluronic acid when cultured in the same conditions as compared with other recombinant strains not comprising all the genetic modifications according to the invention.

Moreover, these four strains led to the production, after 48 hours, of hyaluronic acid having a molecular weight:
- for YA5232-1: greater than about 1000 kDa;
- for YA5303-3: of about 1000 kDa;
- for YA5374-3: greater than about 1000 kDa; and
- for YA5382-1: greater than about 1000 kDa.

### Example 3: Comparative examples for the production of Hyaluronic acid

A. The five following recombinant strains are obtained:
**YA5509:** MAT-α, can1-100, his3::[tRPL3-UGP1-pSAM1, pMET6-QRI1-tIDP1, HIS3]x5, jlp1::[LEU2.K1-RS, pCUP1-HASA.Sz-tRPL41B, pCUP1-UGP1-tTPI1, pTDH3-QRI1-tMET25, pCCW12-UGD1.At-tRPL15A], leu2, lyp1::[pCCW10.Ago-HYAL-31.Hn-tRPL15A, pCCW12.Sba-HASB.vir-tRPL3, pCCW12.Sk-HASB-A.Vir-tTEF1.Sba, pCCW12-HASA-1.Vir-tRPL41B, pCCW12. Sm-HASA-A.Vir-tRPL15A.Sba], sam3:: [LEU2.K1-RS, pPDC1-PGM1-tIDP1, pTEF1.Ago-GFA1-tRPL15A, pENO2-UGP1-tRPL3, pCWP2-GNA1-tTPI1, pTEF1-PCM1-tIDP1, pCCW12-XHASA2.X1-tRPL3, pTDH3-QRI1-tIDP1], trp1::[tRPL3-HASB.Vir-pMET6, pMET25-HASA.Vir-tIDP1, TRP1]x2, ura3::[tRPL3-MHPF.Ec-pPDC1, pTDH3-GDH-21.Eca-tIDP1, URA3]x2
**YA5789:** MAT-α, can1-100, his3::[tRPL3-UGP1-pSAM1, pMET6-QRI1-tIDP1, HIS3]x5, jlp1::[LEU2.K1-RS, pCUP1-HASA.Sz-tRPL41B, pCUP1-UGP1-tRPL3, pCUP1-QRI1-tIDP1, pPDC1-UGP1-tTPI1, pTDH3-QRI1-tMET25, pCCW12-UGD1.At-tRPL15A], leu2, lyp1:: [tMET3. Sba-MET3.Sba-RS-pMET3.Sba, pCCW10.Ago-HYAL-31.Hn-tRPL15A, pTEF1.Ago-GLN1-tTDH3, pCCW12.Sba-HASB.vir-tRPL3, pCCW12.Sk-HASB-A.Vir-tTEF1.Sba, pCCW12-HASA-1.Vir-tRPL41B, pCCW12.Sm-HASA-A.Vir-tRPL15A.Sba], met3::, sam3::[LEU2.K1-RS, pPDC1-PGM1-tIDP1, pTEF1.Ago-GFA1-tRPL15A, pENO2-UGP1-tRPL3, pCWP2-GNA1-tTPI1, pTEF1-PCM1-tIDP1, pCCW12-XHASA2.X1-tRPL3, pTDH3-QRI1-tIDP1], trp1::[tRPL3-HASB.Vir-pMET6, pMET25-HASA.Vir-tIDP1, TRP1]x2, ura3::[tRPL3-MHPF.Ec-pPDC1, pTDH3-GDH-21.Eca-tIDP1, URA3]x2
**YA5790:** MAT-α, can1-100, glt1::[MET3.Sba-RS, pCCW10.Ago-HYAL-31.Hn-tRPL15A, pCCW12.Sba-HASB.vir-tRPL3, pCCW12.Sk-HASB-A.Vir-tTEF1.Sba, pCCW12-HASA-1.Vir-tRPL41B, pCCW12.Sm-HASA-A.Vir-tRPL15A.Sba, pTEF1.Ago-GLN1-tTDH3], his3::[tRPL3-UGP1-pSAM1, pMET6-QRI1-tIDP1, HIS3]x5, jlp1::[LEU2.K1-RS, pCUP1-HASA.Sz-tRPL41B, pCUP1-UGP1-tRPL3, pCUP1-QRI1-tIDP1, pPDC1-UGP1-tTPI1, pTDH3-QRI1-tMET25, pCCW12-UGD1.At-tRPL15A], leu2, met3::, sam3::[LEU2.K1-RS, pPDC1-PGM1-tIDP1, pTEF1Ago-GFA1-tRPL15A, pENO2-UGP1-tRPL3, pCWP2-GNA1-tTPI1, pTEF1-PCM1-tIDP1, pCCW12-XHASA2.X1-tRPL3, pTDH3-QRI1-tIDP1], trp1::[tRPL3-HASB.Vir-pMET6, pMET25-HASA.Vir-tIDP1, TRP1]x2, ura3::[tRPL3-MHPF.Ec-pPDC1, pTDH3-GDH-21.Eca-tIDP1, URA3]x2
**YA5806:** MAT-α, can1-100, glt1::[MET3.Sba-RS, pCCW10.Ago-HYAL-31.Hn-tRPL15A, pCCW12.Sba-HASB.vir-tRPL3, pCCW12.Sk-HASB-A.Vir-tTEF1.Sba, pCCW12-HASA-1.Vir-tRPL41B, pCCW12.Sm-HASA-A.Vir-tRPL15A.Sba], his3::[tRPL3-UGP1-pSAM1, pMET6-QRI1-tIDP1, HIS3]x5, jlp1::[LEU2.K1-RS, pCUP1-HASA.Sz-tRPL41B, pCUP1-UGP1-tRPL3, pCUP1-QRI1-tIDP1, pPDC1-UGP1-tTPI1, pTDH3-QRI1-tMET25, pCCW12-UGD1.At-tRPL15A], leu2, met3::, sam3::[LEU2.K1-RS, pPDC1-PGM1-tIDP1, pTEF1.Ago-GFA1-tRPL15A, pENO2-UGP1-tRPL3, pCWP2-GNA1-tTPI1, pTEF1-PCM1-tIDP1, pCCW12-XHASA2.X1-tRPL3, pTDH3-QRI1-tIDP1], trp1::[tRPL3-HASB.Vir-pMET6, pMET25-HASA.Vir-tIDP1, TRP1]x2, ura3::[tRPL3-MHPF.Ec-pPDC1, pTDH3-GDH-21.Eca-tIDP1, URA3]x2
**YA5658:** MAT-α, can1-100, his3::[tRPL3-UGP1-pSAM1, pMET6-QRI1-tIDP1, HIS3]x5, jlp1::[LEU2.K1-RS, pCUP1-HASA.Sz-tRPL41B, pCUP1-UGP1-tRPL3, pCUP1-QRI1-tIDP1, pPDC1-UGP1-tTPI1, pTDH3-QRI1-tMET25, pCCW12-UGD1.At-tRPL15A], leu2, lyp1::[pCCW10.Ago-HYAL-31.Hn-tRPL15A, pTEF1.Ago-GLN1-tTDH3, pCCW12. Sba-HASB.vir-tRPL3, pCCW12.Sk-HASB-A.Vir-tTEF1. Sba, pCCW12-HASA-1.Vir-tRPL41B, pCCW12.Sm-HASA-A.Vir-tRPL15A.Sba], sam3::[LEU2.K1-RS, pPDC1-PGM1-tIDP1, pTEF1.Ago-GFA1-tRPL15A, pENO2-UGP1-tRPL3, pCWP2-GNA1-tTPI1, pTEF1-PCM1-tIDP1, pCCW12-XHASA2.X1-tRPL3, pTDH3-QRI1-tIDP1], trp1::[tRPL3-HASB.Vir-pMET6, pMET25-HASA.Vir-tIDP1, TRP1]x2, ura3::[tRPL3-MHPF.Ec-pPDC1, pTDH3-GDH-21.Eca-tIDP1, URA3]x2

HASA-1, HASA-A and XHASA2 are previously defined.

HASB and HASB-A are previously defined.

All these strains were incubated in 25 mL of the above-defined SY medium buffered with MES 0,1 M ph 5.5 in a baffled erlenmeyer for 48 hours at 28°C under vigorous agitation.

Growth medium was recovered at 48h hours and assayed for hyaluronic acid content and quality as defined above.

The amounts obtained with these different strains are respectively:
- YA5509: 3.5 g.L⁻¹.
- YA5789 (GLN1 overexpression): 5 g.L⁻¹.
- YA5658 (GNL1 overexpression) : 4.5 g.L⁻¹.
- YA5806 (ΔGLT1): 4.5 g.L⁻¹.
- YA5790 (GNL1 overexpression; ΔGLT1): 6.7 g.L⁻¹.

In comparision, a native strain does not produce hyaluronic acid.

It results from this experiment, under similar conditions, a recombinant strain according to the invention produces significantly more hyaluronic acid when either GLN1 is overexpressed or GLT1 is disrupted as compared with other recombinant strains according to the invention devoid of any of these two modifications (a respect increased yield of production by 27-32% and 27%).

Moreover, a recombinant strain according to the invention produces even more hyaluronic acid when both GLN1 is overexpressed and GLT1 is disrupted as compared with other recombinant strains according to the invention comprising only one of these two modifications or devoid of both (an increased yield of production by 48% compared to the one comprising none of these two modifications).

**B.** The four following recombinant strains are obtained:
**YA6178:** MAT-α, glt1::[MET3.Sba, pTDH3-HYAL-31.Csa-tRPL15A, pTEF1.Ago-GLN1-tTDH3, pCCW12.Sba-HASB.vir-tRPL3, pCCW12.Sm-HASA-A.Vir-tRPL15A.Sba], his3::[pSAM1-UGP1-tRPL3, pMET6-QRI1-tIDP1, HIS3]x3, jlp 1:: [LEU2.Sba-loxP, pTEF1.Sba-HASB.Vir-tRPL3.Sm, pTDH3.Sk-HASB-A.Vir-tTEF1.Sba, pTDH3-1.Sba-HASA-1.Vir-tRPL3.Sba, pTDH3. Sar-HASA-A.Vir-tRPL15A.Sba], leu2, met3Δ1, sam3::[LEU2.K1, pPDC1-PGM1-tIDP1, pTEF1.Ago-GFA1-tRPL15A, pENO2-UGP1-tRPL3, pCWP2-GNA1-tTPI1, pTEF1-PCM1-tRPL41B, TRP1.Sba-loxP], trp1, ura3::[ pPDC1-MHPF.Ec- tRPL3, pTDH3-GDH-21.Eca-tIDP1, URA3]x2
**YA6179:** MAT-α, glt1::[MET3.Sba, pTDH3-HYAL-31.Csa-tRPL15A, pTEF1.Ago-GLN1-tTDH3, pCCW12.Sba-HASB.vir-tRPL3, pCCW12.Sm-HASA-2.Vir-tRPL15A.Sba], his3::[pSAM1-UGP1-tRPL3, pMET6-QRI1-tIDP1, HIS3]x3, jlp 1:: [LEU2.Sba-loxP, pTEF 1.Sba-HASB.Vir-tRPL3.Sm, pTDH3.Sk-HASB-A.Vir-tTEF1.Sba, pTDH3-1.Sba-HASA-1.Vir-tRPL3.Sba, pTDH3. Sar-HASA-A.Vir-tRPL15A.Sba], leu2, met3Δ1, sam3::[LEU2.K1, pPDC1-PGM1-tIDP1, pTEF1.Ago-GFA1-tRPL15A, pENO2-UGP1-tRPL3, pCWP2-GNA1-tTPI1, pTEF1-PCM1-tRPL41B, TRP1.Sba-loxP], trp1, ura3::[pPDC1-MHPF.Ec- tRPL3, pTDH3-GDH-21.Eca-tIDP1, URA3]x2
**YA6180:** MAT-α, glt1::[MET3.Sba, pTDH3-HYAL-31.Csa-tRPL15A, pTEF1.Ago-GLN1-tTDH3,pCCW12.Sba-HASB.vir-tRPL3, pCCW12.Sm-HASA-3.Vir-tRPL15A.Sba], his3::[pSAM1-UGP1-tRPL3, pMET6-QRI1-tIDP1, HIS3]x3, jlp 1:: [LEU2.Sba-loxP, pTEF 1.Sba-HASB.Vir-tRPL3.Sm, pTDH3.Sk-HASB-A.Vir-tTEF1.Sba, pTDH3-1.Sba-HASA-1.Vir-tRPL3.Sba, pTDH3.Sar-HASA-A.Vir-tRPL15A.Sba], leu2, met3Δ1, sam3::[LEU2.K1, pPDC1-PGM1-tIDP1, pTEF1.Ago-GFA1-tRPL15A, pENO2-UGP1-tRPL3, pCWP2-GNA1-tTPI1, pTEF1-PCM1-tRPL41B, TRP1.Sba-loxP], trp1, ura3::[pPDC1-MHPF.Ec- tRPL3, pTDH3-GDH-21.Eca-tIDP1, URA3]x2
**YA6181:** MAT-α, glt1::[MET3.Sba, pTDH3-HYAL-31.Csa-tRPL15A, pTEF1.Ago-GLN1-tTDH3, pCCW12.Sba-HASB.vir-tRPL3, pCCW12.Sm-HASA-5.Vir-tRPL15A.Sba], his3::[pSAM1-UGP1-tRPL3, pMET6-QRI1-tIDP1, HIS3]x3, jlp 1:: [LEU2.Sba-loxP, pTEF 1.Sba-HASB.Vir-tRPL3.Sm, pTDH3.Sk-HASB-A.Vir-tTEF1.Sba, pTDH3-1.Sba-HASA-1.Vir-tRPL3.Sba, pTDH3.Sar-HASA-A.Vir-tRPL15A.Sba], leu2, met3Δ1, sam3::[LEU2.K1, pPDC1-PGM1-tIDP1, pTEF1.Ago-GFA1-tRPL15A, pENO2-UGP1-tRPL3, pCWP2-GNA1-tTPI1, pTEF1-PCM1-tRPL41B, TRP1.Sba-loxP], trp1, ura3::[pPDC1-MHPF.Ec- tRPL3, pTDH3-GDH-21.Eca-tIDP1, URA3]x2

HASA-1 and HASA-A are previously defined.

HASB and HASB-A are previously defined.

HASA-2, HASA-3 and HASA5 all represent a nucleic acid sequence encoding a polypeptide having hyaluronan synthase activity. They differ from one another in that they are different reencoded versions of a nucleic acid sequence encoding the hyaluronan synthase enzyme. HASA-2 has the sequence SEQ ID NO: 101, HASA-3 has the sequence SEQ ID NO: 102 and HASA-5 has the sequence SEQ ID NO: 104.

All these strains were incubated in 25 mL of the above-defined SY medium buffered with MES 0,1 M ph 5.5 in a baffled erlenmeyer for 48 hours at 28°C under vigorous agitation.

Growth medium was recovered at 48h hours and assayed for hyaluronic acid content and quality as defined above.

The amounts obtained with these different strains are respectively:
- YA6178: 6.3 g.L⁻¹.
- YA6179: 5.9 g.L⁻¹.
- YA6180: 5.2 g.L⁻¹.
- YA6181: 6.4 g.L⁻¹.

In comparision, a native strain does not produce hyaluronic acid.

Moreover, these four strains led to the production, after 48 hours, of hyaluronic acid having a molecular weight of about 20 kDa.

### SEQUENCES

**SEQ ID NO: 1** is a reencoded nucleic acid sequence of hyaluronan synthase (HASA) originating from *Chlorella virus PBCV-1* 
ATGGGTAAGAACATCATTATTATGGTTTCTTGGTACACTATTATTACATCCAATCTCATCGCAGTGGGTGGCGCC
TCACTCATACTAGCCCCAGCTATTACGGGCTATGTCCTTCACTGGAACATTGCCCTTTCAACAATTTGGGGAGTG
TCGGCCTACGGAATTTTCGTGTTTGGTTTCTTTCTTGCCCAGGTATTATTTAGTGAACTCAACCGGAAAAGGCTC
CGGAAGTGGATTTCCCTCCGACCCAAAGGGTGGAATGATGTTAGGTTGGCTGTAATTATTGCCGGCTACCGTGAG
GACCCGTATATGTTCCAAAAGTGTCTTGAAAGTGTGCGTGACTCAGACTATGGGAATGTAGCTAGACTAATATGC
GTTATTGACGGCGATGAAGACGACGACATGAGGATGGCTGCAGTGTACAAGGCTATCTATAACGACAACATCAAG
AAACCTGAGTTTGTCCTCTGTGAGAGTGACGATAAGGAGGGTGAGAGAATAGATAGCGATTTCAGCCGTGATATC
TGCGTGCTGCAACCGCATCGCGGAAAGCGTGAATGTTTGTACACAGGGTTCCAATTGGCAAAGATGGACCCCTCA
GTTAATGCCGTCGTCCTAATCGACAGTGACACTGTGTTAGAAAAGGACGCGATTCTCGAaGTAGTATACCCGCTG
GCATGCGATCCAGAAATACAGGCTGTAGCAGGCGAATGCAAAATATGGAATACTGACACACTGTTGAGTTTGCTG
GTAGCCTGGCGATATTACAGCGCATTTTGCGTAGAGCGTAGCGCCCAATCATTCTTCAGGACAGTACAATGCGTC
GGAGGACCTCTCGGCGCCTACAAGATTGATATAATTAAGGAAATCAAGGACCCATGGATCAGCCAACGTTTCCTT
GGCCAAAAGTGCACATACGGCGACGATAGACGACTCACTAATGAAATACTAATGAGGGGTAAGAAAGTAGTTTTC
ACCCCATTCGCTGTTGGCTGGTCCGACAGCCCGACGAACGTCTTCCGTTACATTGTACAGCAAACACGGTGGTCC
AAGTCGTGGTGTAGGGAGATATGGTATACACTGTTTGCAGCATGGAAGCATGGACTTTCGGGCATTTGGCTCGCA
TTCGAGTGCTTATACCAGATTACTTATTTCTTCCTGGTGATTTACCTATTCTCCCGTTTGGCTGTTGAGGCTGAC
CCACGGGCGCAAACGGCCACCGTCATTGTTTCGACCACAGTTGCGCTTATTAAGTGCGGTTACTTTAGCTTCAGA
GCTAAAGACATTAGGGCCTTCTATTTCGTCCTCTACACGTTCGTTTACTTCTTCTGCATGATTCCGGCACGAATA
ACTGCGATGATGACCCTGTGGGACATAGGTTGGGGAACCAGGGGAGGAAATGAGAAGCCTTCCGTAGGCACCAGA
GTTGCTTTGTGGGCCAAGCAATATTTGATTGCTTACATGTGGTGGGCCGCTGTCGTCGGTGCTGGCGTGTACTCC
ATCGTTCATAATTGGATGTTTGACTGGAATTCACTTTCCTACAGGTTCGCACTGGTAGGCATCTGTTCGTATATA
GTTTTCATTGTAATAGTGCTGGTAGTCTACTTTACAGGAAAGATCACGACCTGGAACTTCACGAAGCTACAGAAA
GAATTGATCGAGGACCGCGTACTGTACGACGCAACGACCAATGCCCAGTCGGTATAA
**SEQ ID NO: 2** is a reencoded nucleic acid sequence of hyaluronan synthase (HASA) originating from *Chlorella virus PBCV-1* 
ATGGGCAAGAATATTATTATCATGGTCAGTTGGTATACCATTATTACATCAAACCTAATTGCTGTCGGTGGAGCC
TCACTAATCTTAGCTCCCGCGATTACGGGTTATGTGCTTCATTGGAATATTGCTTTATCCACTATTTGGGGTGTT
AGCGCTTACGGCATCTTTGTGTTCGGTTTCTTCTTGGCTCAGGTGTTATTCTCTGAGCTAAACAGAAAGAGACTC
AGAAAGTGGATTTCGCTGCGGCCGAAGGGATGGAACGATGTTCGGTTAGCGGTGATCATTGCCGGTTATCGTGAA
GATCCTTATATGTTTCAGAAGTGTTTGGAATCTGTTCGAGACTCAGACTACGGTAACGTTGCTAGGCTGATCTGC
GTAATCGATGGAGACGAAGATGACGATATGAGAATGGCCGCGGTTTATAAAGCCATCTATAACGATAACATTAAG
AAGCCAGAGTTTGTTTTATGCGAATCGGATGACAAGGAGGGTGAGAGGATTGATTCTGACTTCTCGCGTGACATC
TGTGTCCTGCAGCCCCACAGGGGAAAGCGAGAGTGCCTCTATACAGGATTCCAATTAGCTAAAATGGACCCAAGC
 
GTTAATGCTGTCGTCCTTATCGATAGCGACACGGTGTTGGAAAAGGACGCAATATTGGAAGTAGTCTATCCACTC
GCTTGTGATCCAGAGATCCAGGCCGTGGCTGGCGAGTGCAAGATTTGGAATACAGATACGCTTCTGTCATTACTG
GTGGCGTGGCGTTATTATTCAGCATTCTGTGTGGAGAGGAGCGCACAATCGTTCTTTCGGACTGTACAATGCGTA
GGCGGTCCACTAGGAGCATATAAAATTGACATAATCAAAGAGATAAAAGATCCTTGGATTTCGCAGAGATTCCTC
GGTCAAAAGTGTACCTATGGGGACGACAGGCGGTTGACTAACGAGATTCTAATGAGAGGTAAGAAGGTGGTGTTC
ACTCCTTTTGCGGTAGGTTGGAGCGATTCGCCCACAAATGTATTCAGATACATTGTTCAACAAACAAGGTGGTCG
AAATCGTGGTGCCGAGAAATATGGTACACGTTGTTCGCCGCATGGAAACACGGCTTGTCGGGTATATGGCTCGCT
TTCGAGTGCCTTTATCAGATCACTTACTTCTTCCTGGTTATTTACTTGTTTTCAAGACTGGCAGTGGAAGCTGAT
CCAAGGGCTCAGACTGCTACTGTCATCGTTTCAACAACAGTGGCGCTAATAAAGTGTGGTTACTTCAGCTTTCGC
GCGAAGGACATAAGAGCGTTTTACTTTGTTCTTTATACCTTTGTATATTTCTTCTGTATGATCCCGGCCCGGATA
ACAGCTATGATGACATTATGGGACATTGGTTGGGGCACCAGGGGTGGGAATGAGAAGCCGTCCGTAGGAACGAGA
GTAGCGCTGTGGGCCAAGCAGTATCTTATCGCATACATGTGGTGGGCAGCTGTCGTCGGGGCAGGTGTATACAGC
ATAGTACATAACTGGATGTTTGACTGGAACTCACTCTCATATCGATTCGCATTGGTCGGGATCTGCTCTTACATC
GTGTTTATTGTTATAGTCTTAGTAGTATATTTCACTGGAAAGATAACAACATGGAATTTCACTAAGCTTCAAAAG
GAATTGATTGAAGACAGGGTGCTCTACGATGCGACAACTAATGCACAAAGCGTATAA
**SEQ ID NO: 3** is a reencoded nucleic acid sequence of hyaluronan synthase (HASA) originating from *Pasteurella multocida* 
ATGAACACCCTTTCCCAGGCAATTAAGGCTTATAATAGTAATGATTATCAACTAGCCCTCAAGCTGTTCGAAAAG
TCGGCAGAAATCTACGGCAGAAAGATAGTTGAgTTCCAAATTACGAAGTGCAAGGAGAAACTTTCTGCGCACCCA
TCCGTTAACAGTGCGCATCTCTCGGTTAACAAAGAAGAGAAGGTGAATGTTTGCGACAGTCCATTAGACATAGCC
ACTCAATTATTGTTATCGAATGTGAAAAAACTCGTCCTTAGTGATAGCGAGAAAAATACACTAAAGAATAAATGG
AAGTTGTTGACTGAAAAGAAGTCTGAGAACGCAGAGGTCCGGGCCGTAGCTTTAGTTCCCAAGGACTTTCCGAAA
GATTTAGTGCTTGCGCCGTTACCAGACCATGTGAACGATTTTACGTGGTATAAGAAGCGGAAGAAACGCCTAGGA
ATCAAACCAGAACACCAACACGTTGGCCTCTCCATTATTGTGACAACGTTCAACCGTCCTGCCATCCTCAGCATC
ACCCTGGCTTGCCTCGTCAATCAAAAGACCCATTATCCTTTTGAGGTGATCGTGACCGACGATGGTTCTCAAGAG
GATTTATCGCCTATAATCCGTCAATATGAGAACAAGCTAGACATCCGTTATGTTCGGCAAAAGGACAACGGTTTT
CAGGCATCGGCCGCACGTAATATGGGCCTACGTCTAGCCAAATATGACTTCATTGGGCTCCTTGATTGTGATATG
GCTCCCAACCCTTTGTGGGTACACTCATACGTTGCGGAACTATTAGAGGACGATGATTTGACCATAATAGGCCCC
AGGAAGTACATTGACACGCAACACATAGATCCCAAGGATTTCCTTAATAACGCCTCTCTGTTAGAGTCGTTGCCA
GAGGTTAAGACCAATAATTCCGTCGCGGCTAAGGGCGAGGGGACCGTATCTTTAGACTGGCGTTTGGAACAATTT
GAGAAGACCGAGAACTTGAGGCTATCCGATAGCCCTTTCCGATTCTTCGCAGCTGGGAATGTGGCTTTCGCCAAG
AAGTGGCTTAACAAGTCAGGATTCTTCGACGAGGAATTCAATCACTGGGGAGGTGAAGATGTAGAGTTCGGTTAT
CGTCTGTTTCGGTACGGTTCGTTCTTCAAAACTATAGACGGCATCATGGCCTATCATCAGGAACCGCCAGGTAAA
GAAAACGAAACTGACAGAGAAGCGGGCAAGAACATTACCCTCGATATAATGAGGGAGAAGGTGCCTTACATCTAC
CGTAAACTCCTGCCTATAGAAGACAGTCATATCAACCGAGTACCATTGGTATCAATTTACATCCCGGCCTACAAC
TGTGCTAACTATATTCAAAGATGTGTTGATTCAGCTTTGAATCAAACGGTAGTTGATCTCGAAGTGTGCATTTGC
AACGACGGTAGTACTGACAACACGCTGGAAGTTATTAACAAGCTGTATGGTAATAATCCGCGTGTGCGTATAATG
 
TCTAAACCCAATGGCGGCATTGCGAGTGCATCCAACGCAGCGGTCAGCTTCGCAAAGGGTTATTACATAGGACAG
TTGGACAGCGACGATTACTTAGAACCCGACGCAGTGGAGTTATGTCTCAAGGAATTTCTTAAGGATAAGACCCTT
GCGTGCGTTTACACCACTAATCGTAACGTCAACCCAGATGGCTCTTTAATAGCCAATGGCTATAACTGGCCAGAG
TTCAGTCGTGAGAAGTTGACTACGGCCATGATTGCTCATCACTTCCGGATGTTTACCATTCGTGCTTGGCATCTG
ACGGATGGGTTCAATGAGAAGATTGAGAACGCTGTTGACTACGACATGTTTCTCAAGCTCAGTGAAGTTGGTAAA
TTTAAGCATCTGAACAAAATATGTTATAATCGGGTGTTACACGGCGATAACACCTCAATCAAGAAgCTTGGCATA
CAAAAGAAGAATCATTTCGTAGTTGTCAATCAGTCTCTAAACCGCCAAGGTATAACTTATTATAACTACGATGAA
TTTGATGATCTCGATGAGAGTCGGAAATACATTTTCAACAAGACTGCAGAGTATCAAGAAGAGATAGATATTCTT
AAAGATATTAAGATCATTCAGAACAAAGACGCCAAAATAGCTGTCTCCATTTTCTATCCGAACACTCTAAACGGG
TTGGTGAAGAAGCTAAATAATATTATAGAGTATAATAAGAATATCTTCGTTATTGTACTTCATGTCGATAAGAAT
CACTTAACCCCAGACATCAAGAAGGAGATATTAGCCTTCTACCATAAGCATCAGGTGAACATCTTATTGAACAAT
GACATCTCCTACTATACATCAAATCGTCTGATTAAGACAGAAGCCCATTTGAGTAACATTAACAAGCTaAGTCAA
TTAAATCTTAACTGCGAATACATTATATTCGACAATCACGATTCCTTATTTGTGAAGAACGATTCCTATGCATAC
ATGAAGAAGTATGATGTTGGAATGAACTTCTCTGCATTGACTCATGATTGGATTGAAAAGATAAACGCTCACCCG
CCATTTAAGAAGCTgATCAAAACTTACTTCAATGACAATGATCTTAAGTCGATGAATGTAAAGGGTGCCTCCCAG
GGAATGTTTATGACATACGCATTAGCCCACGAGTTATTGACGATCATCAAGGAGGTGATAACCTCTTGTCAATCC
ATTGACTCCGTCCCCGAATACAACACAGAAGATATTTGGTTTCAGTTTGCACTTTTAATTCTGGAAAAGAAGACC
GGCCACGTATTCAACAAGACAAGCACTCTCACGTATATGCCATGGGAACGTAAACTGCAGTGGACGAATGAACAA
ATAGAGTCCGCAAAGAGGGGCGAAAACATTCCGGTAAACAAGTTCATCATTAACAGCATTACCCTTTAA
**SEQ ID NO: 4** is a reencoded nucleic acid sequence of hyaluronan synthase (HASA) originating from *Pasteurella multocida* 
ATGAACACCCTTTCCCAGGCAATTAAGGCTTATAATAGTAATGATTATCAACTAGCCCTCAAGCTGTTCGAAAAG
TCGGCAGAAATCTACGGCAGAAAGATAGTTGAgTTCCAAATTACGAAGTGCAAGGAGAAACTTTCTGCGCACCCA
TCCGTTAACAGTGCGCATCTCTCGGTTAACAAAGAAGAGAAGGTGAATGTTTGCGACAGTCCATTAGACATAGCC
ACTCAATTATTGTTATCGAATGTGAAAAAACTCGTCCTTAGTGATAGCGAGAAAAATACACTAAAGAATAAATGG
AAGTTGTTGACTGAAAAGAAGTCTGAGAACGCAGAGGTCCGGGCCGTAGCTTTAGTTCCCAAGGACTTTCCGAAA
GATTTAGTGCTTGCGCCGTTACCAGACCATGTGAACGATTTTACGTGGTATAAGAAGCGGAAGAAACGCCTAGGA
ATCAAACCAGAACACCAACACGTTGGCCTCTCCATTATTGTGACAACGTTCAACCGTCCTGCCATCCTCAGCATC
ACCCTGGCTTGCCTCGTCAATCAAAAGACCCATTATCCTTTTGAGGTGATCGTGACCGACGATGGTTCTCAAGAG
GATTTATCGCCTATAATCCGTCAATATGAGAACAAGCTAGACATCCGTTATGTTCGGCAAAAGGACAACGGTTTT
CAGGCATCGGCCGCACGTAATATGGGCCTACGTCTAGCCAAATATGACTTCATTGGGCTCCTTGATTGTGATATG
GCTCCCAACCCTTTGTGGGTACACTCATACGTTGCGGAACTATTAGAGGACGATGATTTGACCATAATAGGCCCC
AGGAAGTACATTGACACGCAACACATAGATCCCAAGGATTTCCTTAATAACGCCTCTCTGTTAGAGTCGTTGCCA
GAGGTTAAGACCAATAATTCCGTCGCGGCTAAGGGCGAGGGGACCGTATCTTTAGACTGGCGTTTGGAACAATTT
GAGAAGACCGAGAACTTGAGGCTATCCGATAGCCCTTTCCGATTCTTCGCAGCTGGGAATGTGGCTTTCGCCAAG
AAGTGGCTTAACAAGTCAGGATTCTTCGACGAGGAATTCAATCACTGGGGAGGTGAAGATGTAGAGTTCGGTTAT
CGTCTGTTTCGGTACGGTTCGTTCTTCAAAACTATAGACGGCATCATGGCCTATCATCAGGAACCGCCAGGTAAA
 
GAAAACGAAACTGACAGAGAAGCGGGCAAGAACATTACCCTCGATATAATGAGGGAGAAGGTGCCTTACATCTAC
CGTAAACTCCTGCCTATAGAAGACAGTCATATCAACCGAGTACCATTGGTATCAATTTACATCCCGGCCTACAAC
TGTGCTAACTATATTCAAAGATGTGTTGATTCAGCTTTGAATCAAACGGTAGTTGATCTCGAAGTGTGCATTTGC
AACGACGGTAGTACTGACAACACGCTGGAAGTTATTAACAAGCTGTATGGTAATAATCCGCGTGTGCGTATAATG
TCTAAACCCAATGGCGGCATTGCGAGTGCATCCAACGCAGCGGTCAGCTTCGCAAAGGGTTATTACATAGGACAG
TTGGACAGCGACGATTACTTAGAACCCGACGCAGTGGAGTTATGTCTCAAGGAATTTCTTAAGGATAAGACCCTT
GCGTGCGTTTACACCACTAATCGTAACGTCAACCCAGATGGCTCTTTAATAGCCAATGGCTATAACTGGCCAGAG
TTCAGTCGTGAGAAGTTGACTACGGCCATGATTGCTCATCACTTCCGGATGTTTACCATTCGTGCTTGGCATCTG
ACGGATGGGTTCAATGAGAAGATTGAGAACGCTGTTGACTACGACATGTTTCTCAAGCTCAGTGAAGTTGGTAAA
TTTAAGCATCTGAACAAAATATGTTATAATCGGGTGTTACACGGCGATAACACCTCAATCAAGAAgCTTGGCATA
CAAAAGAAGAATCATTTCGTAGTTGTCAATCAGTCTCTAAACCGCCAAGGTATAACTTATTATAACTACGATGAA
TTTGATGATCTCGATGAGAGTCGGAAATACATTTTCAACAAGACTGCAGAGTATCAAGAAGAGATAGATATTCTT
AAAGATATTTAA
**SEQ ID NO: 5** is a reencoded nucleic acid sequence of hyaluronan synthase (HASA) originating from *Pasteurella multocida* 
ATGAACACCCTTTCCCAGGCAATTAAGGCTTATAATAGTAATGATTATCAACTAGCCCTCAAGCTGTTCGAAAAG
TCGGCAGAAATCTACGGCAGAAAGATAGTTGAgTTCCAAATTACGAAGTGCAAGGAGAAACTTTCTGCGCACCCA
TCCGTTAACAGTGCGCATCTCTCGGTTAACAAAGAAGAGAAGGTGAATGTTTGCGACAGTCCATTAGACATAGCC
ACTCAATTATTGTTATCGAATGTGAAAAAACTCGTCCTTAGTGATAGCGAGAAAAATACACTAAAGAATAAATGG
AAGTTGTTGACTGAAAAGAAGTCTGAGAACGCAGAGGTCCGGGCCGTAGCTTTAGTTCCCAAGGACTTTCCGAAA
GATTTAGTGCTTGCGCCGTTACCAGACCATGTGAACGATTTTACGTGGTATAAGAAGCGGAAGAAACGCCTAGGA
ATCAAACCAGAACACCAACACGTTGGCCTCTCCATTATTGTGACAACGTTCAACCGTCCTGCCATCCTCAGCATC
ACCCTGGCTTGCCTCGTCAATCAAAAGACCCATTATCCTTTTGAGGTGATCGTGACCGACGATGGTTCTCAAGAG
GATTTATCGCCTATAATCCGTCAATATGAGAACAAGCTAGACATCCGTTATGTTCGGCAAAAGGACAACGGTTTT
CAGGCATCGGCCGCACGTAATATGGGCCTACGTCTAGCCAAATATGACTTCATTGGGCTCCTTGATTGTGATATG
GCTCCCAACCCTTTGTGGGTACACTCATACGTTGCGGAACTATTAGAGGACGATGATTTGACCATAATAGGCCCC
AGGAAGTACATTGACACGCAACACATAGATCCCAAGGATTTCCTTAATAACGCCTCTCTGTTAGAGTCGTTGCCA
GAGGTTAAGACCAATAATTCCGTCGCGGCTAAGGGCGAGGGGACCGTATCTTTAGACTGGCGTTTGGAACAATTT
GAGAAGACCGAGAACTTGAGGCTATCCGATAGCCCTTTCCGATTCTTCGCAGCTGGGAATGTGGCTTTCGCCAAG
AAGTGGCTTAACAAGTCAGGATTCTTCGACGAGGAATTCAATCACTGGGGAGGTGAAGATGTAGAGTTCGGTTAT
CGTCTGTTTCGGTACGGTTCGTTCTTCAAAACTATAGACGGCATCATGGCCTATCATCAGGAACCGCCAGGTAAA
GAAAACGAAACTGACAGAGAAGCGGGCAAGAACATTACCCTCGATATAATGAGGGAGAAGGTGCCTTACATCTAC
CGTAAACTCCTGCCTATAGAAGACAGTCATATCAACCGAGTACCATTGGTATCAATTTACATCCCGGCCTACAAC
TGTGCTAACTATATTCAAAGATGTGTTGATTCAGCTTTGAATCAAACGGTAGTTGATCTCGAAGTGTGCATTTGC
AACGACGGTAGTACTGACAACACGCTGGAAGTTATTAACAAGCTGTATGGTAATAATCCGCGTGTGCGTATAATG
TCTAAACCCAATGGCGGCATTGCGAGTGCATCCAACGCAGCGGTCAGCTTCGCAAAGGGTTATTACATAGGACAG
TTGGACAGCGACGATTACTTAGAACCCGACGCAGTGGAGTTATGTCTCAAGGAATTTCTTAAGGATAAGACCCTT
 
GCGTGCGTTTACACCACTAATCGTAACGTCAACCCAGATGGCTCTTTAATAGCCAATGGCTATAACTGGCCAGAG
TTCAGTCGTGAGAAGTTGACTACGGCCATGATTGCTCATCACTTCCGGATGTTTACCATTCGTGCTTGGCATCTG
ACGGATGGGTTCAATGAGAAGATTGAGAACGCTGTTGACTACGACATGTTTCTCAAGCTCAGTGAAGTTGGTAAA
TTTAAGCATCTGAACAAAATATGTTATAATCGGGTGTTACACGGCGATAACACCTCAATCAAGAAGCTTGGCATA
CAAAAGAAGAATCATTTCGTAGTTGTCAATCAGTCTCTAAACCGCCAAGGTATAACTTATTATAACTACGATGAA
TTTGATGATCTCGATGAGAGTCGGAAATACATTTTCAACAAGACTGCAGAGTATCAAGAAGAGATAGATATTCTT
AAAGATATTGGATCCGCCATTTCTCAAATCACTGACGGTCAAATCCAAGCTACTACCACTGCTACCACCGAAGCT
ACCACCACTGCTGCCCCATCTTCCACCGTTGAAACTGTTTCTCCATCCAGCACCGAAACTATCTCTCAACAAACT
GAAAATGGTGCTGCTAAGGCCGCTGTCGGTATGGGTGCCGGTGCTCTAGCTGCTGCTGCTATGTTGTTATAA
**SEQ ID NO: 6** is a reencoded nucleic acid sequence of hyaluronan synthase (HASA) originating from *Xenopus laevis* 
ATGCACTGTGAACGATTTATTTGTATATTACGAATAATTGGTACGACTTTGTTCGGAGTAAGTCTGCTACTAGGG
ATATCCGCCGCTTATATCGTGGGATACCAATTCATTCAGACGGATAATTACTACTTTTCCTTCGGTCTCTACGGT
GCTATTTTAGCTTTACATTTAATCATTCAGTCTCTCTTCGCATTCCTGGAGCATCGTAAGATGAAGCGGTCACTT
GAAACCCCTATAAAATTAAACAAGAGCGTAGCATTGTGTATCGCTGCATACCAGGAAGACGAAGATTATCTTAGA
AAATGTTTGCTTTCTGTGAAACGGCTCACATACCCCGGTATGAAGGTAATCATGGTGATAGATGGTAACTCTGAT
GACGATCTATACATGATGAACATATTCAGAGAGATCATGGGAAATGATAGCTGCGCCACCTATGTATGGAAGAAT
AATTTTCACATGAAGGGACCAAACGAGACCGACGAGACTCACCGCGAGTCCATGCAGCATGTAACACAGATGGTG
CTTTCTAACAGAAATGTCTGTATCATGCAAAAGTGGAACGGGAAACGCGAGGTGATGTATACTGCATTCAAAGCG
CTGGGTCGGTCGGTTGATTACGTCCAAGTGTGCGACTCAGATACAGTCCTTGATCCAGCAAGCTCGGTGGAGATG
GTCAAGGTGTTGGAGGAAGACATTATGGTCGGAGGTGTAGGCGGAGACGTGCAGATACTCAATAAGTACGACTCC
TGGATCAGTTTCTTGAGTTCGGTTCGTTATTGGATGGCTTTCAATATAGAACGAGCATGCCAATCATACTTTGGC
TGTGTGCAGTGTATATCGGGGCCTCTGGGCATGTACAGAAACTCACTCTTACATGAGTTCATTGAAGACTGGTAC
AATCAAGAATTCCTCGGTTCCCAATGCTCTTTCGGTGATGATCGGCATTTGACTAATCGAGTACTATCATTGGGC
TATGCTACTAAATACACTGCGCGCAGTAAGTGTCTGACAGAAACCCCCACAGAATATCTTAGATGGTTGAACCAA
CAAACCAGGTGGAGTAAGTCCTATTTCCGCGAGTGGTTGTACAACTCTTTGTGGTTCCACAAACACCATTTATGG
ATGACTTATGAAGCCGTAATTACGGGTTTCTTCCCCTTCTTCTTAATAGCGACGGTGATTCAGCTCTTCTATCGT
GGTCGAATTTGGAATATCTTACTCTTTCTACTCACAGTTCAATTAGTTGGTCTAATAAAGTCGTCGTTTGCAAGT
GCGTTGCGTGGAAACATCGTTATGGTCTTTATGAGTTTCTACAGTGTTTTATACATGAGTAGCTTGCTGCCAGCA
AAGATGTTTGCAATCGCCACGATAAACAAGGCTGGATGGGGTACTAGTGGTAGAAAGACCATAGTTGTTAATTTT
ATTGGTTTAATCCCTATTACGGTATGGTTCACAATTTTACTAGGGGGAGTATGCTACACTATATGGCGGGAAACC
AAGAAGCCGTTTAGCGAGTCTGAGAAGATCGTTCTTGCGGTCGGTGCTATATTGTATGCTTGTTATTGGGTCATG
TTGCTTACAATGTATGTCAGTCTTGTCATGAAGTGCGGGCGCCGTAGGAAGGAACCACAGCACGACCTTGTTTTA
GCATAA
**SEQ ID NO: 7** is a reencoded nucleic acid sequence of hyaluronan synthase (HASA) originating from *Streptococcus zooepidemicus* 
ATGAGGACGTTAAAGAATCTTATCACTGTTGTAGCGTTCAGTATATTCTGGGTTTTGCTGATCTATGTAAATGTC
TATCTTTTCGGTGCGAAGGGGTCTCTATCAATATATGGATTCTTGCTAATTGCGTATTTGCTTGTGAAGATGAGT
CTTTCATTCTTCTATAAACCATTCAAAGGTCGAGCTGGTCAGTATAAAGTCGCAGCGATTATCCCTTCATACAAC
GAGGATGCAGAATCGCTGTTGGAAACCTTAAAATCTGTTCAACAACAAACCTATCCCCTTGCTGAAATTTATGTA
GTTGATGATGGTTCCGCAGATGAAACCGGTATCAAGCGGATCGAGGATTATGTCAGGGATACAGGCGACTTAAGT
TCAAATGTGATTGTGCATCGATCAGAAAAGAATCAAGGCAAGAGACACGCCCAAGCATGGGCATTTGAAAGATCT
GATGCAGATGTATTTCTAACAGTCGATAGTGATACTTACATTTATCCGGACGCCCTTGAAGAGTTATTGAAAACC
TTCAATGATCCGACAGTATTTGCCGCAACTGGCCACTTGAACGTTCGTAACAGGCAGACTAATCTATTGACCAGA
CTCACTGATATAAGATACGACAATGCTTTTGGCGTCGAACGTGCTGCTCAAAGTGTAACTGGTAATATACTGGTG
TGTTCCGGGCCACTGTCCGTCTATCGCAGAGAAGTAGTAGTCCCGAACATTGACAGGTATATTAATCAGACTTTT
CTGGGTATCCCCGTGTCAATTGGGGACGACCGGTGTTTAACGAACTACGCTACTGATTTGGGCAAGACTGTATAT
CAGTCGACGGCTAAATGTATTACAGACGTCCCAGATAAGATGTCGACTTACCTTAAACAGCAGAATAGATGGAAT
AAGTCATTCTTTAGAGAATCTATCATCAGCGTGAAGAAGATCATGAATAATCCATTCGTGGCGCTTTGGACCATT
TTGGAGGTTTCCATGTTTATGATGCTTGTCTACTCTGTCGTTGATTTCTTTGTAGGTAACGTTCGAGAATTTGAT
TGGCTCAGAGTTCTTGCTTTCTTAGTTATCATCTTCATCGTTGCGCTATGCAGGAATATTCATTACATGCTAAAA
CATCCGCTTAGCTTCTTACTCAGCCCTTTCTACGGCGTTTTGCATCTCTTCGTTTTGCAGCCATTGAAGCTCTAC
TCCTTATTTACCATTCGAAACGCTGATTGGGGTACGCGCAAGAAACTATTAtaa
**SEQ ID NO: 8** is an amino acid sequence of hyaluronan synthase (HASA) originating from *Chlorella virus PBCV-1* 
MGKNIIIMVSWYTIITSNLIAVGGASLILAPAITGYVLHWNIALSTIWGVSAYGIFVFGFFLAQVLFSELNRKRL
RKWISLRPKGWNDVRLAVIIAGYREDPYMFQKCLESVRDSDYGNVARLICVIDGDEDDDMRMAAVYKAIYNDNIK
KPEFVLCESDDKEGERIDSDFSRDICVLQPHRGKRECLYTGFQLAKMDPSVNAVVLIDSDTVLEKDAILEVVYPL
ACDPEIQAVAGECKIWNTDTLLSLLVAWRYYSAFCVERSAQSFFRTVQCVGGPLGAYKIDIIKEIKDPWISQRFL
GQKCTYGDDRRLTNEILMRGKKVVFTPFAVGWSDSPTNVFRYIVQQTRWSKSWCREIWYTLFAAWKHGLSGIWLA
FECLYQITYFFLVIYLFSRLAVEADPRAQTATVIVSTTVALIKCGYFSFRAKDIRAFYFVLYTFVYFFCMIPARI
TAMMTLWDIGWGTRGGNEKPSVGTRVALWAKQYLIAYMWWAAVVGAGVYSIVHNWMFDWNSLSYRFALVGICSYI
VFIVIVLVVYFTGKITTWNFTKLQKELIEDRVLYDATTNAQSV
**SEQ ID NO: 9** is an amino acid sequence of hyaluronan synthase (HASA) originating from *Pasteurella multocida* 
MNTLSQAIKAYNSNDYQLALKLFEKSAEIYGRKIVEFQITKCKEKLSAHPSVNSAHLSVNKEEKVNVCDSPLDIA
TQLLLSNVKKLVLSDSEKNTLKNKWKLLTEKKSENAEVRAVALVPKDFPKDLVLAPLPDHVNDFTWYKKRKKRLG
IKPEHQHVGLSIIVTTFNRPAILSITLACLVNQKTHYPFEVIVTDDGSQEDLSPIIRQYENKLDIRYVRQKDNGF
QASAARNMGLRLAKYDFIGLLDCDMAPNPLWVHSYVAELLEDDDLTIIGPRKYIDTQHIDPKDFLNNASLLESLP
 
EVKTNNSVAAKGEGTVSLDWRLEQFEKTENLRLSDSPFRFFAAGNVAFAKKWLNKSGFFDEEFNHWGGEDVEFGY
RLFRYGSFFKTIDGIMAYHQEPPGKENETDREAGKNITLDIMREKVPYIYRKLLPIEDSHINRVPLVSIYIPAYN
CANYIQRCVDSALNQTVVDLEVCICNDGSTDNTLEVINKLYGNNPRVRIMSKPNGGIASASNAAVSFAKGYYIGQ
LDSDDYLEPDAVELCLKEFLKDKTLACVYTTNRNVNPDGSLIANGYNWPEFSREKLTTAMIAHHFRMFTIRAWHL
TDGFNEKIENAVDYDMFLKLSEVGKFKHLNKICYNRVLHGDNTSIKKLGIQKKNHFVVVNQSLNRQGITYYNYDE
FDDLDESRKYIFNKTAEYQEEIDILKDIKIIQNKDAKIAVSIFYPNTLNGLVKKLNNIIEYNKNIFVIVLHVDKN
HLTPDIKKEILAFYHKHQVNILLNNDISYYTSNRLIKTEAHLSNINKLSQLNLNCEYIIFDNHDSLFVKNDSYAY
MKKYDVGMNFSALTHDWIEKINAHPPFKKLIKTYFNDNDLKSMNVKGASQGMFMTYALAHELLTIIKEVITSCQS
IDSVPEYNTEDIWFQFALLILEKKTGHVFNKTSTLTYMPWERKLQWTNEQIESAKRGENIPVNKFIINSITL
**SEQ ID NO: 10** is an amino acid sequence of hyaluronan synthase (HASA) originating from *Xenopus laevis* 
MHCERFICILRIIGTTLFGVSLLLGISAAYIVGYQFIQTDNYYFSFGLYGAILALHLIIQSLFAFLEHRKMKRSL
ETPIKLNKSVALCIAAYQEDEDYLRKCLLSVKRLTYPGMKVIMVIDGNSDDDLYMMNIFREIMGNDSCATYVWKN
NFHMKGPNETDETHRESMQHVTQMVLSNRNVCIMQKWNGKREVMYTAFKALGRSVDYVQVCDSDTVLDPASSVEM
VKVLEEDIMVGGVGGDVQILNKYDSWISFLSSVRYWMAFNIERACQSYFGCVQCISGPLGMYRNSLLHEFIEDWY
NQEFLGSQCSFGDDRHLTNRVLSLGYATKYTARSKCLTETPTEYLRWLNQQTRWSKSYFREWLYNSLWFHKHHLW
MTYEAVITGFFPFFLIATVIQLFYRGRIWNILLFLLTVQLVGLIKSSFASALRGNIVMVFMSFYSVLYMSSLLPA
KMFAIATINKAGWGTSGRKTIVVNFIGLIPITVWFTILLGGVCYTIWRETKKPFSESEKIVLAVGAILYACYWVM
LLTMYVSLVMKCGRRRKEPQHDLVLA
**SEQ ID NO: 11** is an amino acid sequence of hyaluronan synthase (HASA) originating from *Streptococcus zooepidemicus* 
MRTLKNLITVVAFSIFWVLLIYVNVYLFGAKGSLSIYGFLLIAYLLVKMSLSFFYKPFKGRAGQYKVAAIIPSYN
EDAESLLETLKSVQQQTYPLAEIYVVDDGSADETGIKRIEDYVRDTGDLSSNVIVHRSEKNQGKRHAQAWAFERS
DADVFLTVDSDTYIYPDALEELLKTFNDPTVFAATGHLNVRNRQTNLLTRLTDIRYDNAFGVERAAQSVTGNILV
CSGPLSVYRREVVVPNIDRYINQTFLGIPVSIGDDRCLTNYATDLGKTVYQSTAKCITDVPDKMSTYLKQQNRWN
KSFFRESIISVKKIMNNPFVALWTILEVSMFMMLVYSVVDFFVGNVREFDWLRVLAFLVIIFIVALCRNIHYMLK
HPLSFLLSPFYGVLHLFVLQPLKLYSLFTIRNADWGTRKKLL
**SEQ ID NO: 12** is a nucleic acid sequence of UDP-Glucose dehydrogenase (HASB) originating from *Arabidopsis thaliana* 
ATGGTCAAAATATGCTGTATCGGGGCTGGATATGTCGGTGGGCCTACAATGGCCGTTATGGCCCTAAAGTGTCCA
GAGATTGAAGTCGTGGTAGTTGATATATCGGAGCCACGAATTAACGCATGGAACTCAGACCGTCTACCAATTTAT
GAGCCAGGGTTAGAGGATGTCGTCAAACAGTGTAGAGGGAAGAATTTGTTCTTCTCTACAGATGTAGAGAAGCAT
GTATTTGAGTCAGATATAGTGTTTGTTTCGGTAAACACTCCTACGAAAACGCAGGGTCTGGGTGCAGGTAAAGCA
GCAGATTTGACATATTGGGAGTCCGCTGCTCGCATGATAGCTGATGTGAGCAAATCGTCGAAAATCGTCGTTGAA
 
AAGAGTACAGTACCAGTTCGTACAGCCGAGGCTATAGAAAAGATTTTAACGCACAATTCGAAGGGTATCGAATTC
CAGATCTTATCAAATCCAGAATTCTTGGCCGAAGGGACGGCGATTAAAGACTTATATAACCCTGATAGAGTTCTA
ATCGGTGGCAGGGACACCGCTGCGGGACAAAAGGCCATTAAGGCGTTGCGTGACGTGTATGCCCACTGGGTTCCT
GTTGAGCAAATAATCTGTACTAATTTATGGAGTGCCGAGCTATCAAAGTTGGCTGCGAACGCATTTCTAGCTCAA
AGGATAAGTTCAGTAAACGCAATGTCAGCGCTTTGTGAGGCAACTGGTGCTGACGTAACCCAAGTCGCTCACGCC
GTTGGAACCGACACTAGAATTGGACCGAAGTTTCTTAACGCATCCGTAGGCTTCGGCGGATCTTGCTTTCAGAAA
GACATCCTGAATCTTATTTACATCTGCGAATGCAATGGTCTTCCAGAAGCAGCCAATTATTGGAAACAGGTAGTC
AAGGTAAATGACTACCAAAAGATTAGGTTTGCTAATCGAGTCGTATCTTCTATGTTCAACACCGTCTCCGGTAAG
AAAATTGCTATTTTGGGATTTGCGTTCAAGAAGGACACCGGCGACACGCGTGAAACTCCTGCCATAGATGTGTGT
AATCGCCTCGTGGCTGATAAAGCAAAGCTGTCGATCTATGATCCGCAAGTATTAGAAGAGCAGATCCGCCGTGAT
CTGTCCATGGCCCGATTCGATTGGGACCATCCAGTCCCACTCCAGCAGATCAAAGCTGAAGGTATCTCCGAACAG
GTTAACGTTGTGTCCGACGCCTACGAGGCTACGAAGGATGCTCATGGTTTATGTGTTTTAACCGAATGGGACGAA
TTCAAGTCACTTGATTTTAAGAAGATCTTTGATAATATGCAGAAACCCGCTTTCGTTTTCGACGGAAGAAACGTG
GTCGACGCTGTGAAATTGAGAGAAATTGGATTCATAGTATATTCCATAGGTAAACCTCTGGATAGTTGGCTCAAG
GATATGCCGGCTGTTGCATAA
**SEQ ID NO: 13** is a reencoded nucleic acid sequence of UDP-Glucose dehydrogenase (HASB) originating from *Chlorella virus PBCV-1* 
ATGAGTAGAATTGCTGTCGTTGGATGCGGTTACGTGGGTACGGCCTGCGCCGTACTTTTGGCGCAGAAGAACGAA
GTTATCGTTTTGGATATCTCGGAGGACCGGGTACAACTGATTAAGAATAAGAAGTCACCTATAGAAGATAAGGAA
ATCGAAGAATTCCTGGAGACGAAAGATTTGAATCTAACAGCGACGACGGATAAGGTGCTCGCCTATGAGAATGCT
GAATTCGTTATAATAGCTACACCGACCGATTACGATGTCGTCACTAGATATTTCAACACTAAGTCTGTTGAAAAT
GTGATAGGCGATGTCATTAAGAACACTCAGACACACCCTACTATAGTGATCAAGAGTACTATTCCAATCGGTTTC
GTGGATAAAGTTAGAGAACAGTTCGATTATCAAAATATTATCTTCTCGCCGGAATTCTTGAGAGAAGGACGAGCA
TTATATGATAATCTTTACCCCTCCCGTATCATCGTCGGTGATGACTCCCCAATTGCCTTAAAATTCGCGAATCTC
TTGGTCGAGGGTAGCAAAACTCCACTAGCTCCCGTATTAACTATGGGTACGCGAGAGGCCGAAGCTGTAAAACTA
TTTTCAAATACATATTTGGCTATGAGAGTAGCATACTTCAATGAGCTAGACACATTTGCAATGTCCCATGGTATG
AACGCAAAAGAGATTATCGACGGAGTCACACTAGAACCAAGGATAGGGCAGGGTTATTCGAATCCTTCATTTGGG
TATGGAGCCTATTGTTTCCCAAAAGATACAAAGCAATTGTTGGCTAATTTTGAAGGGGTTCCACAAGATATTATT
GGCGCAATCGTAGAGTCTAACGAAACTAGAAAGGAGGTTATTGTTTCTGAGGTCGAGAACAGATTTCCGACCACA
GTTGGCGTATATAAGTTGGCTGCTAAAGCTGGTTCGGATAATTTCAGGTCTAGTGCGATAGTCGATATCATGGAA
AGGTTGGCTAATAAGGGCTACCACATCAAAATATTTGAACCTACCGTTGAACAATTTGAGAATTTTGAGGTAGAT
AATAATCTCACAACTTTTGCAACCGAGTCTGATGTAATAATTGCGAACAGAGTTCCAGTCGAACATCGCATTCTG
TTTGGGAAGAAGTTAATAACACGGGATGTCTATGGGGATAA
**SEQ ID NO: 14** is a reencoded nucleic acid sequence of UDP-Glucose dehydrogenase (HASB) originating from *Chlorella virus PBCV-1* 
ATGTCACGAATCGCAGTAGTTGGCTGTGGGTACGTGGGAACCGCATGCGCTGTACTGCTGGCGCAAAAGAATGAG
GTAATCGTTCTTGATATTAGTGAGGATAGGGTCCAACTAATCAAGAACAAGAAATCCCCGATCGAAGATAAGGAG
ATTGAAGAATTCTTGGAAACAAAAGACCTAAACTTAACTGCAACAACTGACAAAGTTTTAGCCTACGAAAACGCT
GAGTTTGTGATTATAGCGACACCCACAGATTATGACGTAGTTACCAGATACTTTAACACGAAGTCCGTAGAGAAC
GTCATTGGAGATGTTATAAAGAATACTCAGACTCATCCTACGATAGTAATAAAGTCAACCATTCCCATAGGTTTC
GTAGATAAGGTTAGGGAGCAATTCGATTACCAGAACATTATATTTTCGCCAGAATTTCTGAGAGAGGGTCGCGCC
CTGTATGATAATCTATATCCATCACGGATTATAGTGGGCGATGACTCTCCGATTGCACTTAAGTTTGCTAATCTT
TTAGTTGAGGGCTCCAAAACTCCGCTCGCCCCAGTACTTACGATGGGTACACGTGAGGCTGAAGCTGTCAAGCTG
TTTTCAAACACATACCTTGCTATGCGAGTCGCATACTTTAACGAACTAGATACCTTTGCTATGTCGCACGGTATG
AATGCTAAAGAAATCATAGATGGCGTAACGTTGGAGCCTCGGATAGGTCAAGGATATTCCAATCCATCTTTTGGC
TACGGTGCGTATTGTTTCCCAAAGGACACGAAGCAATTATTAGCTAACTTTGAGGGTGTTCCGCAAGATATAATT
GGGGCGATAGTAGAAAGCAATGAAACACGGAAGGAGGTAATCGTGAGTGAAGTGGAAAACCGATTCCCCACTACG
GTCGGCGTTTACAAATTAGCCGCCAAGGCTGGTTCCGACAATTTCCGATCCAGCGCAATAGTAGATATTATGGAA
AGATTAGCTAATAAGGGATACCACATTAAAATCTTTGAACCTACTGTCGAACAGTTCGAGAACTTCGAGGTTGAT
AATAACTTGACGACTTTCGCAACGGAGAGCGATGTAATTATTGCAAACCGCGTACCTGTGGAACATCGAATTTTG
TTCGGAAAGAAGCTGATTACACGCGATGTATATGGCGATAACTAA
**SEQ ID NO: 15** is a reencoded nucleic acid sequence of UDP-Glucose dehydrogenase (HASB) originating from *Streptococcus zooepidemicus* 
ATGAAGATATCGGTAGCGGGTTCGGGGTACGTGGGGTTATCCTTGTCAATCTTGCTTGCCCAACATAACGATGTG
ACTGTTGTAGATATAATCGACGAAAAGGTACGGCTAATTAACCAGGGCATATCTCCGATTAAGGATGCGGACATT
GAGGAATATCTGAAGAATGCACCGTTGAATCTTACGGCTACACTAGACGGAGCTTCGGCTTATAGTAATGCTGAT
CTGATTATAATCGCAACGCCAACTAATTACGATTCAGAACGCAATTATTTCGACACCAGACACGTTGAAGAAGTA
ATTGAGCAAGTATTGGATTTAAATGCCTCCGCTACTATAATCATCAAGAGTACCATACCCTTGGGTTTTATTAAA
CACGTAAGAGAGAAATACCAAACAGACAGAATCATCTTTTCTCCAGAGTTCTTAAGAGAGTCAAAGGCATTGTAC
GATAACTTATACCCCTCTCGTATAATAGTCAGTTATGAGAAGGATGACTCTCCAAGAGTTATACAAGCAGCTAAG
GCGTTCGCGGGTTTATTAAAAGAGGGGGCAAAGAGCAAGGATACCCCAGTTCTGTTTATGGGCTCTCAAGAAGCT
GAAGCTGTCAAGCTGTTTGCTAATACTTTTCTCGCCATGAGGGTCAGTTACTTCAACGAGCTTGACACTTATAGC
GAATCAAAAGGACTAGACGCCCAAAGAGTTATAGAAGGCGTCTGCCATGATCAAAGGATAGGTAATCATTACAAT
AATCCATCCTTCGGATATGGCGGTTATTGTTTACCCAAAGACTCAAAGCAACTTTTGGCTAATTATAGAGGCATA
CCTCAGTCTCTAATGTCTGCCATCGTTGAATCGAACAAGATCCGTAAGTCGTATTTAGCTGAACAAATATTAGAT
AGGGCTTCTTCACAAAAGCAGGCTGGTGTACCTTTAACCATAGGATTTTACCGTTTGATTATGAAGTCCAACTCC
GATAACTTTAGAGAATCAGCCATTAAAGATATTATTGACATCATTAATGACTACGGTGTCAATATTGTCATTTAT
GAACCTATGTTGGGAGAAGACATTGGTTATAGAGTCGTTAAAGATTTGGAACAGTTTAAGAACGAAAGTACAATT
ATTGTTAGTAACAGGTTTGAAGATGATTTAGGTGATGTTATTGATAAAGTTTATACACGTGACGTCTTTGGTAG
**SEQ ID NO: 16** is an amino acid sequence of UDP-Glucose dehydrogenase (HASB) originating from *Arabidopsis thaliana* 
MVKICCIGAGYVGGPTMAVMALKCPEIEVVVVDISEPRINAWNSDRLPIYEPGLEDVVKQCRGKNLFFSTDVEKH
VFESDIVFVSVNTPTKTQGLGAGKAADLTYWESAARMIADVSKSSKIVVEKSTVPVRTAEAIEKILTHNSKGIEF
QILSNPEFLAEGTAIKDLYNPDRVLIGGRDTAAGQKAIKALRDVYAHWVPVEQIICTNLWSAELSKLAANAFLAQ
RISSVNAMSALCEATGADVTQVAHAVGTDTRIGPKFLNASVGFGGSCFQKDILNLIYICECNGLPEAANYWKQVV
KVNDYQKIRFANRVVSSMFNTVSGKKIAILGFAFKKDTGDTRETPAIDVCNRLVADKAKLSIYDPQVLEEQIRRD
LSMARFDWDHPVPLQQIKAEGISEQVNVVSDAYEATKDAHGLCVLTEWDEFKSLDFKKIFDNMQKPAFVFDGRNV
VDAVKLREIGFIVYSIGKPLDSWLKDMPAVA
**SEQ ID NO: 17** is an amino acid sequence of UDP-Glucose dehydrogenase (HASB) originating from *Chlorella virus PBCV1* 
MSRIAVVGCGYVGTACAVLLAQKNEVIVLDISEDRVQLIKNKKSPIEDKEIEEFLETKDLNLTATTDKVLAYENA
EFVIIATPTDYDVVTRYFNTKSVENVIGDVIKNTQTHPTIVIKSTIPIGFVDKVREQFDYQNIIFSPEFLREGRA
LYDNLYPSRIIVGDDSPIALKFANLLVEGSKTPLAPVLTMGTREAEAVKLFSNTYLAMRVAYFNELDTFAMSHGM
NAKEIIDGVTLEPRIGQGYSNPSFGYGAYCFPKDTKQLLANFEGVPQDIIGAIVESNETRKEVIVSEVENRFPTT
VGVYKLAAKAGSDNFRSSAIVDIMERLANKGYHIKIFEPTVEQFENFEVDNNLTTFATESDVIIANRVPVEHRIL
FGKKLITRDVYGDN
**SEQ ID NO: 18** is an amino acid sequence of UDP-Glucose dehydrogenase (HASB) originating from *Streptococcus zooepidemicus* 
MKISVAGSGYVGLSLSILLAQHNDVTVVDIIDEKVRLINQGISPIKDADIEEYLKNAPLNLTATLDGASAYSNAD
LIIIATPTNYDSERNYFDTRHVEEVIEQVLDLNASATIIIKSTIPLGFIKHVREKYQTDRIIFSPEFLRESKALY
DNLYPSRIIVSYEKDDSPRVIQAAKAFAGLLKEGAKSKDTPVLFMGSQEAEAVKLFANTFLAMRVSYFNELDTYS
ESKGLDAQRVIEGVCHDQRIGNHYNNPSFGYGGYCLPKDSKQLLANYRGIPQSLMSAIVESNKIRKSYLAEQILD
RASSQKQAGVPLTIGFYRLIMKSNSDNFRESAIKDIIDIINDYGVNIVIYEPMLGEDIGYRVVKDLEQFKNESTI
IVSNRFEDDLGDVIDKVYTRDVFGRD
**SEQ ID NO: 19** is a reencoded nucleic acid sequence of hyaluronidase (HYAL) originating from *Bothrops atrox* with a N-terminal secretion signal 
ATGCAATTTAGCACAGTCGCATCAGTAGCCTTCGTTGCCTTGGCCAACTTCGTGGCAGCACCGATGTATCCGAAC
GAACCGTTCTTAGTCTTTTGGAACGCGCCTACAACTCAGTGTAGACTTCGATATAAGGTTGACCTTGATCTGAAG
ACATTCCATATCGTGACAAATGCTAATGACTCGCTGTCAGGATCGGCTGTCACGATTTTCTATCCCACGCACTTA
GGGGTTTACCCACATATTGATGACAGGGGGCACTTCTTCAATGGCATCATACCCCAAAATGAATCCCTGGTAAAG
CATTTAAACAAATCTAAATCAGATATTAATCGAATGATTCCCTTAAGAACATTCCACGGGCTGGGAGTCATAGAC
TGGGAAAACTGGCGGCCACAGTGGGATAGGAATTGGGGAAGTAAGAACGTTTATAGGAATAGATCAATCCAATTC
GCGCGTGATCTCCACCCAGAGCTTAGTGAGGACAAGATTAAACGCTTGGCAAAACAGGAATTCGAGAAAGCTGCA
 
AAGAGTTTTATGAGGGATACACTATTATTAGCCGAGGAGATGCGACCAGACGGCTACTGGGGATACTATCTGTAC
CCCGATTGTCACAATTACAATTATAAGACTAAGCCAGATCAGTACACAGGAGAGTGCCCTGACATCGAGATGTCA
CGTAACAATCAACTCTTGTGGCTTTGGCGGGATAGCACTGCCCTTTTCCCCAATATCTACTTAGAGACTATACTA
AGAAGCTCTGACAATGCCCTGAAGTTTGTGCACCATAGGCTCAAGGAGGCAATGAGGATAGCCTCAATGGCTCGA
AATGACTACGCGTTGCCTTTCTTTGTTTATGCTCGACCATTCTATGCCTATACCTTCGAACCATTGACTCAGGAA
GACCTTGTGACTACGGTCGGAGAGACCGCGGACATGGGCGCCGCTGGGATCGTATTTTGGGGGAGTATGCAGTAC
GCAAGCACGGTTGAATCTTGCGGCAAAGTCAAGGACTACATGAATGGCCCACTGGGGCGTTACATTGTGAATGTT
ACAACTGCCGCCAAAATTTGCTCACGATTCTTGTGCAAACGTCATGGTAGGTGTGTAAGAAAGCACTCAGACTCC
AATGCATTCCTTCACCTATTTCCCGATTCGTTTCGCATAATGGTGCACGGGAATGCAACCGAGAAGAAAGTTATA
GTAAAGGGGAAGCTGGAATTAAAGAATCTTATATTCTTACGTAATAACTTTATGTGCCAGTGTTATCAAGGCTGG
AAAGGTCTATATTGCGAGAAGCATTCGATAAAGGAAATTCGGAAAATCTAA
**SEQ ID NO: 20** is a reencoded nucleic acid sequence of hyaluronidase (HYAL) originating from *Bothrops atrox* with a N-terminal secretion signal and a C-terminal anchoring signal 
ATGCAATTTAGCACAGTCGCATCAGTAGCCTTCGTTGCCTTGGCCAACTTCGTGGCAGCACCGATGTATCCGAAC
GAACCGTTCTTAGTCTTTTGGAACGCGCCTACAACTCAGTGTAGACTTCGATATAAGGTTGACCTTGATCTGAAG
ACATTCCATATCGTGACAAATGCTAATGACTCGCTGTCAGGATCGGCTGTCACGATTTTCTATCCCACGCACTTA
GGGGTTTACCCACATATTGATGACAGGGGGCACTTCTTCAATGGCATCATACCCCAAAATGAATCCCTGGTAAAG
CATTTAAACAAATCTAAATCAGATATTAATCGAATGATTCCCTTAAGAACATTCCACGGGCTGGGAGTCATAGAC
TGGGAAAACTGGCGGCCACAGTGGGATAGGAATTGGGGAAGTAAGAACGTTTATAGGAATAGATCAATCCAATTC
GCGCGTGATCTCCACCCAGAGCTTAGTGAGGACAAGATTAAACGCTTGGCAAAACAGGAATTCGAGAAAGCTGCA
AAGAGTTTTATGAGGGATACACTATTATTAGCCGAGGAGATGCGACCAGACGGCTACTGGGGATACTATCTGTAC
CCCGATTGTCACAATTACAATTATAAGACTAAGCCAGATCAGTACACAGGAGAGTGCCCTGACATCGAGATGTCA
CGTAACAATCAACTCTTGTGGCTTTGGCGGGATAGCACTGCCCTTTTCCCCAATATCTACTTAGAGACTATACTA
AGAAGCTCTGACAATGCCCTGAAGTTTGTGCACCATAGGCTCAAGGAGGCAATGAGGATAGCCTCAATGGCTCGA
AATGACTACGCGTTGCCTTTCTTTGTTTATGCTCGACCATTCTATGCCTATACCTTCGAACCATTGACTCAGGAA
GACCTTGTGACTACGGTCGGAGAGACCGCGGACATGGGCGCCGCTGGGATCGTATTTTGGGGGAGTATGCAGTAC
GCAAGCACGGTTGAATCTTGCGGCAAAGTCAAGGACTACATGAATGGCCCACTGGGGCGTTACATTGTGAATGTT
ACAACTGCCGCCAAAATTTGCTCACGATTCTTGTGCAAACGTCATGGTAGGTGTGTAAGAAAGCACTCAGACTCC
AATGCATTCCTTCACCTATTTCCCGATTCGTTTCGCATAATGGTGCACGGGAATGCAACCGAGAAGAAAGTTATA
GTAAAGGGGAAGCTGGAATTAAAGAATCTTATATTCTTACGTAATAACTTTATGTGCCAGTGTTATCAAGGCTGG
AAAGGTCTATATTGCGAGAAGCATTCGATAAAGGAAATTCGGAAAATCGGATCCGCCATTTCTCAAATCACTGAC
GGTCAAATCCAAGCTACTACCACTGCTACCACCGAAGCTACCACCACTGCTGCCCCATCTTCCACCGTTGAAACT
GTTTCTCCATCCAGCACCGAAACTATCTCTCAACAAACTGAAAATGGTGCTGCTAAGGCCGCTGTCGGTATGGGT
GCCGGTGCTCTAGCTGCTGCTGCTATGTTGTTATAA
**SEQ ID NO: 21** is a reencoded nucleic acid sequence of hyaluronidase (HYAL) originating from *Cupiennius salei* with a N-terminal secretion signal 
ATGCAATTCAGCACTGTTGCATCAGTTGCATTTGTCGCCCTGGCGAATTTTGTAGCCGCGTTCAAGATTTACTGG
AACGTCCCAACTTTTCAGTGCACGCATAACTACAAAATCGATTATGTCAAATTGTTGTCCACTTACGGGATACAG
GTCAATGATGGCGGTAAGTTTCAAGGAAACCAAGTGACTATCTTTTATGAAACCCAGTTGGGTTTGTATCCACGA
ATCCTAAAATCTGGTAAAATGGAAAACGGCGGAATCCCTCAACGCGGTAACTTTGAGAAACACCTAGAAAAGGCA
AGCACGGACCTCCAGAAAGTGATCCCTTGGAAAGAGTTTAGCGGATTAGGTGTGATAGATTGGGAGGCTTGGAGA
CCCACATGGGAATTTAACTGGGAACCGTTGAGGATATATCAAACCGAATCAATTAAGAGAGCTAAAGAACTACAC
CCTACCGCAAACGATTCCGCAGTAAAAGAAATTGCAGAGCGGCAATGGGAAGATTCAGCCAAGTTATACATGTTA
GAAACACTGCGGCTGGCAAAGAAACTTCGACCTCAAGCGCCTTGGTGTTACTACTTATTTCCTGATTGCTATAAT
TACGTCGGAAAGAAACCAAAAGATTTCCAATGTAGTGCCTCGATACGTAAAGGTAACGATAAGCTAAGCTGGTTG
TGGAAAGATTCTACGGCATTGTGTCCATCGATATACGTATATGAATCACAATTAGACAGGTATTCTTTTGAACAA
AGGACATGGCGCGACAATGAGAAACTTCGGGAAGCGTTGCGTGTAGCCACGAGAACCTCTAAAATATACCCATAC
GTTAACTATTTCGATAAGGAGCTTATACCGGAGCAAGAAGTATGGAGAATGCTTGCGCAGGCAGCTGCTGTCGGT
GGCAGTGGTGCGGTAATTTGGGGCTCATCTGCTGCAGTTGCATCTGAAGAGTTATGTAAATCTTTAAAACAGTAT
ATTATTGAAACGCTTGGGCCGGCGGCAGAGAAGGTGGCTTGGCGTAGTGACTTATGCAGCAAAGAAATTTGTAAT
AATCAGGGTCGCTGCACATTCCCGGACGATGATTATGCAAACGCATGGAAATTATTTACAGATGATACTGTTAAG
TTTTATGCTGGTAATATTACATGTAGGTGCTCCGAGAATTATTCTGGTCGTTTCTGCGAAAAGAAGAATTAA
**SEQ ID NO: 22** is a reencoded nucleic acid sequence of hyaluronidase (HYAL) originating from *Cupiennius salei* with a N-terminal secretion signal and a C-terminal anchoring signal 
ATGCAATTCAGCACTGTTGCATCAGTTGCATTTGTCGCCCTGGCGAATTTTGTAGCCGCGTTCAAGATTTACTGG
AACGTCCCAACTTTTCAGTGCACGCATAACTACAAAATCGATTATGTCAAATTGTTGTCCACTTACGGGATACAG
GTCAATGATGGCGGTAAGTTTCAAGGAAACCAAGTGACTATCTTTTATGAAACCCAGTTGGGTTTGTATCCACGA
ATCCTAAAATCTGGTAAAATGGAAAACGGCGGAATCCCTCAACGCGGTAACTTTGAGAAACACCTAGAAAAGGCA
AGCACGGACCTCCAGAAAGTGATCCCTTGGAAAGAGTTTAGCGGATTAGGTGTGATAGATTGGGAGGCTTGGAGA
CCCACATGGGAATTTAACTGGGAACCGTTGAGGATATATCAAACCGAATCAATTAAGAGAGCTAAAGAACTACAC
CCTACCGCAAACGATTCCGCAGTAAAAGAAATTGCAGAGCGGCAATGGGAAGATTCAGCCAAGTTATACATGTTA
GAAACACTGCGGCTGGCAAAGAAACTTCGACCTCAAGCGCCTTGGTGTTACTACTTATTTCCTGATTGCTATAAT
TACGTCGGAAAGAAACCAAAAGATTTCCAATGTAGTGCCTCGATACGTAAAGGTAACGATAAGCTAAGCTGGTTG
TGGAAAGATTCTACGGCATTGTGTCCATCGATATACGTATATGAATCACAATTAGACAGGTATTCTTTTGAACAA
AGGACATGGCGCGACAATGAGAAACTTCGGGAAGCGTTGCGTGTAGCCACGAGAACCTCTAAAATATACCCATAC
GTTAACTATTTCGATAAGGAGCTTATACCGGAGCAAGAAGTATGGAGAATGCTTGCGCAGGCAGCTGCTGTCGGT
GGCAGTGGTGCGGTAATTTGGGGCTCATCTGCTGCAGTTGCATCTGAAGAGTTATGTAAATCTTTAAAACAGTAT
ATTATTGAAACGCTTGGGCCGGCGGCAGAGAAGGTGGCTTGGCGTAGTGACTTATGCAGCAAAGAAATTTGTAAT
AATCAGGGTCGCTGCACATTCCCGGACGATGATTATGCAAACGCATGGAAATTATTTACAGATGATACTGTTAAG
TTTTATGCTGGTAATATTACATGTAGGTGCTCCGAGAATTATTCTGGTCGTTTCTGCGAAAAGAAGAATGGATCC
GCCATTTCTCAAATCACTGACGGTCAAATCCAAGCTACTACCACTGCTACCACCGAAGCTACCACCACTGCTGCC
 
CCATCTTCCACCGTTGAAACTGTTTCTCCATCCAGCACCGAAACTATCTCTCAACAAACTGAAAATGGTGCTGCT

AAGGCCGCTGTCGGTATGGGTGCCGGTGCTCTAGCTGCTGCTGCTATGTTGTTATAA
**SEQ ID NO: 23** is a reencoded nucleic acid sequence of hyaluronidase (HYAL) originating from *Hirudo Nipponia* with a N-terminal secretion signal 
ATGCAATTCTCTACTGTCGCTTCCGTTGCTTTCGTCGCTTTGGCTAACTTTGTTGCCGCTATGAAGGAGATAGCA
GTTACCATCGATGATAAGAATGTTATAGCGTCTGTCAGTGAAAGCTTCCATGGTGTGGCTTTCGACGCAAGCCTA
TTCTCACCTAAAGGGCTATGGTCGTTTGTTGACATTACTTCACCCAAGTTATTCAAGCTTCTGGAGGGACTCTCC
CCTGGTTATTTCCGTGTCGGCGGCACATTTGCGAACTGGTTATTCTTCGACCTCGATGAAAACAACAAGTGGAAG
GATTACTGGGCTTTTAAGGACAAGACTCCAGAGACCGCCACGATTACCCGACGGTGGCTCTTCAGGAAGCAAAAC
AACCTCAAGAAGGAAACCTTTGATGATTTGGTAAAGCTTACAAAGGGGTCCAAGATGCGGCTGCTATTTGATCTA
AACGCAGAGGTCCGTACAGGATATGAGATAGGCAAGAAGATGACATCAACTTGGGACTCAAGCGAGGCAGAAAAG
TTGTTCAAATATTGCGTTAGCAAGGGATATGGAGATAATATCGATTGGGAGTTAGGCAACGAACCTGATCACACG
TCAGCACACAATTTGACAGAGAAGCAAGTAGGTGAAGATTTTAAGGCCCTACACAAGGTGTTGGAAAAGTATCCA
ACACTTAATAAAGGTAGCTTGGTTGGTCCAGACGTTGGGTGGATGGGAGTGTCGTACGTCAAGGGTCTGGCTGAC
GGGGCTGGAGATCATGTGACCGCTTTTACTCTACATCAGTATTATTTCGATGGAAATACGAGTGATGTTAGTACC
TACTTGGATGCGACGTACTTTAAGAAGTTGCAACAGTTATTCGATAAGGTAAAAGACGTACTCAAGAATTCTCCA
CATAAGGACAAACCCTTGTGGCTAGGGGAAACCTCTTCCGGCTACAACAGTGGGACTAAAGATGTATCCGATAGA
TACGTGTCGGGGTTCTTGACGCTGGACAAGTTGGGGCTTTCGGCGGCAAATAACGTCAAGGTGGTCATCAGACAA
ACGATTTATAATGGTTACTATGGTTTGCTTGACAAGAATACTTTGGAGCCCAATCCGGACTACTGGCTGATGCAC
GTCCACAATTCCTTAGTTGGTAATACCGTTTTCAAGGTCGATGTCAGCGATCCCACAAACAAAGCCCGTGTTTAC
GCTCAGTGTACGAAGACCAATAGTAAACATACCCAGTCACGTTATTATAAGGGGTCCCTAACCATTTTCGCGTTG
AATGTAGGTGATGAAGATGTTACACTCAAGATTGATCAATACAGTGGTAAGAAAATATACTCATATATTCTGACG
CCTGAAGGCGGCCAGTTAACCTCTCAAAAGGTACTGCTTAACGGTAAAGAATTGAAGTTAGTGTCAGATCAACTT
CCAGAACTTAACGCGGACGAAAGTAAAACATCCTTCACATTGTCTCCCAAAACTTTTGGATTCTTTGTGGTCTCG
GATGCCAACGTTGAAGCTTGCAAGAAATAA
**SEQ ID NO: 24** is a reencoded nucleic acid sequence of hyaluronidase (HYAL) originating from *Hirudo Nipponia* with a N-terminal secretion signal and a C-terminal anchoring signal 
ATGCAATTCTCTACTGTCGCTTCCGTTGCTTTCGTCGCTTTGGCTAACTTTGTTGCCGCTATGAAGGAGATAGCA
GTTACCATCGATGATAAGAATGTTATAGCGTCTGTCAGTGAAAGCTTCCATGGTGTGGCTTTCGACGCAAGCCTA
TTCTCACCTAAAGGGCTATGGTCGTTTGTTGACATTACTTCACCCAAGTTATTCAAGCTTCTGGAGGGACTCTCC
CCTGGTTATTTCCGTGTCGGCGGCACATTTGCGAACTGGTTATTCTTCGACCTCGATGAAAACAACAAGTGGAAG
GATTACTGGGCTTTTAAGGACAAGACTCCAGAGACCGCCACGATTACCCGACGGTGGCTCTTCAGGAAGCAAAAC
AACCTCAAGAAGGAAACCTTTGATGATTTGGTAAAGCTTACAAAGGGGTCCAAGATGCGGCTGCTATTTGATCTA
AACGCAGAGGTCCGTACAGGATATGAGATAGGCAAGAAGATGACATCAACTTGGGACTCAAGCGAGGCAGAAAAG
TTGTTCAAATATTGCGTTAGCAAGGGATATGGAGATAATATCGATTGGGAGTTAGGCAACGAACCTGATCACACG
TCAGCACACAATTTGACAGAGAAGCAAGTAGGTGAAGATTTTAAGGCCCTACACAAGGTGTTGGAAAAGTATCCA
 
ACACTTAATAAAGGTAGCTTGGTTGGTCCAGACGTTGGGTGGATGGGAGTGTCGTACGTCAAGGGTCTGGCTGAC
GGGGCTGGAGATCATGTGACCGCTTTTACTCTACATCAGTATTATTTCGATGGAAATACGAGTGATGTTAGTACC
TACTTGGATGCGACGTACTTTAAGAAGTTGCAACAGTTATTCGATAAGGTAAAAGACGTACTCAAGAATTCTCCA
CATAAGGACAAACCCTTGTGGCTAGGGGAAACCTCTTCCGGCTACAACAGTGGGACTAAAGATGTATCCGATAGA
TACGTGTCGGGGTTCTTGACGCTGGACAAGTTGGGGCTTTCGGCGGCAAATAACGTCAAGGTGGTCATCAGACAA
ACGATTTATAATGGTTACTATGGTTTGCTTGACAAGAATACTTTGGAGCCCAATCCGGACTACTGGCTGATGCAC
GTCCACAATTCCTTAGTTGGTAATACCGTTTTCAAGGTCGATGTCAGCGATCCCACAAACAAAGCCCGTGTTTAC
GCTCAGTGTACGAAGACCAATAGTAAACATACCCAGTCACGTTATTATAAGGGGTCCCTAACCATTTTCGCGTTG
AATGTAGGTGATGAAGATGTTACACTCAAGATTGATCAATACAGTGGTAAGAAAATATACTCATATATTCTGACG
CCTGAAGGCGGCCAGTTAACCTCTCAAAAGGTACTGCTTAACGGTAAAGAATTGAAGTTAGTGTCAGATCAACTT
CCAGAACTTAACGCGGACGAAAGTAAAACATCCTTCACATTGTCTCCCAAAACTTTTGGATTCTTTGTGGTCTCG
GATGCCAACGTTGAAGCTTGCAAGAAAGGATCCGCCATTTCTCAAATCACTGACGGTCAAATCCAAGCTACTACC
ACTGCTACCACCGAAGCTACCACCACTGCTGCCCCATCTTCCACCGTTGAAACTGTTTCTCCATCCAGCACCGAA
ACTATCTCTCAACAAACTGAAAATGGTGCTGCTAAGGCCGCTGTCGGTATGGGTGCCGGTGCTCTAGCTGCTGCT
GCTATGTTGTTATAA
**SEQ ID NO: 25** is a reencoded nucleic acid sequence of hyaluronidase (HYAL) originating from *Loxosceles intermedia* with a N-terminal secretion signal 
ATGCAGTTTTCAACCGTTGCGAGCGTCGCTTTTGTGGCTCTTGCTAATTTCGTTGCCGCCTTCGATGTCTTCTGG
AATGTTCCGTCGCAGCAATGCAAGAAATATGGTATGAAATTCGTTCCGCTCTTAGAGCAATACTCTATCCTAGTC
AACAAAGAAGACAATTTCAAGGGCGACAAAATAACGATCTTTTATGAGTCACAGCTCGGGCTTTACCCACATATT
GGTGCAAACGACGAGTCGTTTAATGGCGGGATACCACAATTAGGTGACCTGAAAGCACACTTAGAAAAGTCAGCG
GTTGATATACGACGTGATATTTTGGATAAGTCGGCGACTGGTCTAAGAATTATAGACTGGGAAGCATGGAGACCA
ATATGGGAATTCAACTGGTCTAGCCTACGAAAGTACCAAGATAAAATGAAGAAAGTCGTCCGCCAGTTTAACCCG
ACTGCTCATGAATCCACAGTGGCCAAACTAGCACATAATGAGTGGGAAAATAGTAGTAAGTCTTGGATGCTTTCT
ACATTGCAGTTGGGTAAGCAACTTCGTCCAAACTCTGTATGGTGCTATTATTTATTTCCCGACTGTTATAACTAT
GATGGCAACTCAGTCCAAGAATTTCAGTGTTCTGAAGCTATCCGTAAGGGGAACGATAGGTTGAAATGGTTGTGG
GAAGAATCGACAGCTGTATGCCCATCTATCTACATAAAAGAAGGCCAACTGACCAATTATACCTTGCAAAAGAGA
ATCTGGTTTACCAATGGGAGATTACAGGAAGCCTTGAGAGTAGCTCAACCTAAAGCGCGTATTTATCCATACATA
AATTACTCCATCAAACCCGGAATGATGGTGCCTGAAGTTGAGTTTTGGCGGTTAATCGCTCAGATAGCCTCGCTG
GGTATGGATGGAGCAGTGATTTGGGGATCAAGTGCGAGTGTAGGCAGTAAGAATCATTGTGCGCAATTAATGAAG
TACATTGCAGACGTATTGGGTCCTGCAACTTTGCGCATAAAAGAAAATGTAGCACGGTGCAGTAAACAGGCGTGC
TCTGGTAGGGGTAGATGTACCTGGCCTAAAGATACCTCTGTTATTGCTTGGAAGTTCCTCGTTGAAAAGGAAGAC
TATGACTTTTATCTGGGTGATATAGAGTGTAAATGTGTTGAAGGCTACGAAGGTAGGTACTGTGAACAAAAGACT
AAGTAA
**SEQ ID NO: 26** is a reencoded nucleic acid sequence of hyaluronidase (HYAL) originating from *Loxosceles intermedia* with a N-terminal secretion signal and a C-terminal anchoring signal 
ATGCAGTTTTCAACCGTTGCGAGCGTCGCTTTTGTGGCTCTTGCTAATTTCGTTGCCGCCTTCGATGTCTTCTGG
AATGTTCCGTCGCAGCAATGCAAGAAATATGGTATGAAATTCGTTCCGCTCTTAGAGCAATACTCTATCCTAGTC
AACAAAGAAGACAATTTCAAGGGCGACAAAATAACGATCTTTTATGAGTCACAGCTCGGGCTTTACCCACATATT
GGTGCAAACGACGAGTCGTTTAATGGCGGGATACCACAATTAGGTGACCTGAAAGCACACTTAGAAAAGTCAGCG
GTTGATATACGACGTGATATTTTGGATAAGTCGGCGACTGGTCTAAGAATTATAGACTGGGAAGCATGGAGACCA
ATATGGGAATTCAACTGGTCTAGCCTACGAAAGTACCAAGATAAAATGAAGAAAGTCGTCCGCCAGTTTAACCCG
ACTGCTCATGAATCCACAGTGGCCAAACTAGCACATAATGAGTGGGAAAATAGTAGTAAGTCTTGGATGCTTTCT
ACATTGCAGTTGGGTAAGCAACTTCGTCCAAACTCTGTATGGTGCTATTATTTATTTCCCGACTGTTATAACTAT
GATGGCAACTCAGTCCAAGAATTTCAGTGTTCTGAAGCTATCCGTAAGGGGAACGATAGGTTGAAATGGTTGTGG
GAAGAATCGACAGCTGTATGCCCATCTATCTACATAAAAGAAGGCCAACTGACCAATTATACCTTGCAAAAGAGA
ATCTGGTTTACCAATGGGAGATTACAGGAAGCCTTGAGAGTAGCTCAACCTAAAGCGCGTATTTATCCATACATA
AATTACTCCATCAAACCCGGAATGATGGTGCCTGAAGTTGAGTTTTGGCGGTTAATCGCTCAGATAGCCTCGCTG
GGTATGGATGGAGCAGTGATTTGGGGATCAAGTGCGAGTGTAGGCAGTAAGAATCATTGTGCGCAATTAATGAAG
TACATTGCAGACGTATTGGGTCCTGCAACTTTGCGCATAAAAGAAAATGTAGCACGGTGCAGTAAACAGGCGTGC
TCTGGTAGGGGTAGATGTACCTGGCCTAAAGATACCTCTGTTATTGCTTGGAAGTTCCTCGTTGAAAAGGAAGAC
TATGACTTTTATCTGGGTGATATAGAGTGTAAATGTGTTGAAGGCTACGAAGGTAGGTACTGTGAACAAAAGACT
AAGGGATCCGCCATTTCTCAAATCACTGACGGTCAAATCCAAGCTACTACCACTGCTACCACCGAAGCTACCACC
ACTGCTGCCCCATCTTCCACCGTTGAAACTGTTTCTCCATCCAGCACCGAAACTATCTCTCAACAAACTGAAAAT
GGTGCTGCTAAGGCCGCTGTCGGTATGGGTGCCGGTGCTCTAGCTGCTGCTGCTATGTTGTTATAA
**SEQ ID NO: 27** is a reencoded nucleic acid sequence of hyaluronidase (HYAL) originating from *Tityus serrulatus* with a N-terminal secretion signal 
ATGCAATTCTCTACTGTCGCTTCCGTTGCTTTCGTCGCTTTGGCTAACTTTGTTGCCGCTGCTGATTTTAAAGTT
TACTGGGAAGTGCCTTCCTTCCTTTGTTCTAAACGTTTTAAAATTAATGTAACGGAAGTTTTAACAAGTCACGAG
ATTCTTGTCAATCAGGGTGAGAGTTTCAACGGTGACAAGATAGTAATCTTTTACGAAAACCAATTGGGGAAGTAC
CCGCATATTGACTCAAACAATGTGGAGATCAATGGAGGAATACTTCAAGTAGCCGATTTGGCGAAGCATTTGAAA
GTAGCCAAGGATAATATCACTAAATTCGTCCCGAATCCTAATTTCAACGGTGTCGGAGTGATCGACTGGGAAGCT
TGGCGGCCATCATGGGAATTTAACTGGGGTAAGTTAAAAGTATATAAAGAAAAGAGCATTGACTTGGTCAAGTCG
AAACATCCGGAGTGGCCCTCCGACAGGGTTGAAAAGGTTGCTAAAGAGGAGTGGGAGGAGAGTGCCAAAGAATGG
ATGGTGAAGACCCTGAAGTTAGCACAGGAAATGCGACCGAACGCAGTTTGGTGCTATTATCTATTCCCTGACTGC
TACAATTATTTCGGTAAGGATCAACCCTCTCAATTCAGCTGCTCGTCTCGAATTCAGAAGGAAAATTCTCGTCTT
TCTTGGCTCTGGAATCAATCAACAGCCATTTGCCTAAGCATTTATATCCAGGAATCCCATGTTACCAAATATAAT
ATGTCCCAGCGGACATGGTGGATCGATGCGAGATTAAGAGAAGCAATTCGAGTCAGCGAACACAGACCAAACATA
CCCATCTACCCTTACATTAATTATATTCTACCTGGAACTAATCAAACTGTACCAGCAATGGACTTTAAAAGGACA
CTGGGTCAAATAGCTAGCCTCGGCCTAGATGGTGCTTTGTTATGGGGATCTAGCTATCATGTTTTAACAGAATCT
 
CAATGCAAAATCACTTCTGATTATGTGAAATCAGTGATTGCTCCTACCGTGGCTACTGTCGTTCTCAATACAAAC
AGATGCTCACAGATAATTTGTAAGGGTCGCGGCAACTGTGTTTGGCCTGAAGAACCATTTAGTTCTTGGAAATAC
TTAGTTGACCCCAAAATGCCAGTGTTCAAGCCAACCAACATCCACTGTAAATGTAAAGGTTACCTAGGTAGATAC
TGTGAGATCCCAAAGTAA
**SEQ ID NO: 28** is a reencoded nucleic acid sequence of hyaluronidase (HYAL) originating from *Tityus serrulatus* with a N-terminal secretion signal and a C-terminal anchoring signal 
ATGCAATTCTCTACTGTCGCTTCCGTTGCTTTCGTCGCTTTGGCTAACTTTGTTGCCGCTGCTGATTTTAAAGTT
TACTGGGAAGTGCCTTCCTTCCTTTGTTCTAAACGTTTTAAAATTAATGTAACGGAAGTTTTAACAAGTCACGAG
ATTCTTGTCAATCAGGGTGAGAGTTTCAACGGTGACAAGATAGTAATCTTTTACGAAAACCAATTGGGGAAGTAC
CCGCATATTGACTCAAACAATGTGGAGATCAATGGAGGAATACTTCAAGTAGCCGATTTGGCGAAGCATTTGAAA
GTAGCCAAGGATAATATCACTAAATTCGTCCCGAATCCTAATTTCAACGGTGTCGGAGTGATCGACTGGGAAGCT
TGGCGGCCATCATGGGAATTTAACTGGGGTAAGTTAAAAGTATATAAAGAAAAGAGCATTGACTTGGTCAAGTCG
AAACATCCGGAGTGGCCCTCCGACAGGGTTGAAAAGGTTGCTAAAGAGGAGTGGGAGGAGAGTGCCAAAGAATGG
ATGGTGAAGACCCTGAAGTTAGCACAGGAAATGCGACCGAACGCAGTTTGGTGCTATTATCTATTCCCTGACTGC
TACAATTATTTCGGTAAGGATCAACCCTCTCAATTCAGCTGCTCGTCTCGAATTCAGAAGGAAAATTCTCGTCTT
TCTTGGCTCTGGAATCAATCAACAGCCATTTGCCTAAGCATTTATATCCAGGAATCCCATGTTACCAAATATAAT
ATGTCCCAGCGGACATGGTGGATCGATGCGAGATTAAGAGAAGCAATTCGAGTCAGCGAACACAGACCAAACATA
CCCATCTACCCTTACATTAATTATATTCTACCTGGAACTAATCAAACTGTACCAGCAATGGACTTTAAAAGGACA
CTGGGTCAAATAGCTAGCCTCGGCCTAGATGGTGCTTTGTTATGGGGATCTAGCTATCATGTTTTAACAGAATCT
CAATGCAAAATCACTTCTGATTATGTGAAATCAGTGATTGCTCCTACCGTGGCTACTGTCGTTCTCAATACAAAC
AGATGCTCACAGATAATTTGTAAGGGTCGCGGCAACTGTGTTTGGCCTGAAGAACCATTTAGTTCTTGGAAATAC
TTAGTTGACCCCAAAATGCCAGTGTTCAAGCCAACCAACATCCACTGTAAATGTAAAGGTTACCTAGGTAGATAC
TGTGAGATCCCAAAGGGATCCGCCATTTCTCAAATCACTGACGGTCAAATCCAAGCTACTACCACTGCTACCACC
GAAGCTACCACCACTGCTGCCCCATCTTCCACCGTTGAAACTGTTTCTCCATCCAGCACCGAAACTATCTCTCAA
CAAACTGAAAATGGTGCTGCTAAGGCCGCTGTCGGTATGGGTGCCGGTGCTCTAGCTGCTGCTGCTATGTTGTTA
TAA
**SEQ ID NO: 29** is a reencoded nucleic acid sequence of hyaluronidase (HYAL) originating from *Vespa magnifica* with a N-terminal secretion signal 
ATGCAATTTTCTACAGTGGCAAGTGTTGCATTCGTTGCACTAGCCAACTTTGTGGCGGCAGATAGCTGTGGGTCA
AACTGCGAAAAGAGTGAGAGACCGAAAAGGGTCTTCAACATTTACTGGAACGTACCTACATTCATGTGTCACCAG
TACGGACTATACTTTGACGAGGTCACGAATTTTAATATAAAGCACAACAGCAAAGACAATTTTCAAGGGGACAAG
ATCGCGATCTTTTATGACCCCGGGGAGTTTCCCGCTCTGCTGCCACTAAACTATGGTAAGTACAAGATCAGGAAT
GGTGGTGTTCCACAAGAGGGTAACATCACCATCCATCTGCAGAGATTCATAGAGTACCTAGATAAGACCTATCCG
AACCGTAACTTTTCAGGCATCGGTGTGATCGATTTCGAGAGGTGGAGACCAATTTTCAGACAGAATTGGGGTAAT
ATGAAGATTTACAAGAACTTCTCCATCGATCTTGTGCGTAAAGAGCATCCTTTCTGGAATAAGAAAATGATCGAG
TTGGAAGCTTCTAAAAGATTCGAGAAATACGCCCGTCTGTTCATGGAAGAAACATTAAAGTTGGCTAAGAAAACT
 
AGAAAACAGGCCGATTGGGGCTACTACGGTTACCCCTATTGCTTCAACATGTCTCCTACTAATTTCGTTCCTGAC
TGCGATGTCACAGCTAGGGATGAGAACAACGAGATGTCTTGGTTGTTTAACAACCAGAATGTCCTATTACCAAGT
GTATACATTAGGAGAGAGCTAACTCCTGACCAGAGGATTGGGCTTGTACAGGGGAGAGTGAAGGAAGCTGTGAGA
ATTTCAAATAAACTGAAGCACTCACCTAAAGTCTTCAGCTATTGGTGGTATGTTTACCAAGACGAGACCAACACC
TTCTTAACGGAGACCGACGTCAAGAAGACGTTTCAGGAGATTGTGATCAACGGTGGAGATGGGATTATAATCTGG
GGTTCGTCCTCTGATGTAAACAGCTTGTCCAAGTGTACGAGGTTAAGGGAGTACCTATTGACAGTCTTGGGACCA
ATTGCTGTTAACGTGACTGAAGCAGTAAACTAA
**SEQ ID NO: 30** is a reencoded nucleic acid sequence of hyaluronidase (HYAL) originating from *Vespa magnifica* with a N-terminal secretion signal and a C-terminal anchoring signal 
ATGCAATTTTCTACAGTGGCAAGTGTTGCATTCGTTGCACTAGCCAACTTTGTGGCGGCAGATAGCTGTGGGTCA
AACTGCGAAAAGAGTGAGAGACCGAAAAGGGTCTTCAACATTTACTGGAACGTACCTACATTCATGTGTCACCAG
TACGGACTATACTTTGACGAGGTCACGAATTTTAATATAAAGCACAACAGCAAAGACAATTTTCAAGGGGACAAG
ATCGCGATCTTTTATGACCCCGGGGAGTTTCCCGCTCTGCTGCCACTAAACTATGGTAAGTACAAGATCAGGAAT
GGTGGTGTTCCACAAGAGGGTAACATCACCATCCATCTGCAGAGATTCATAGAGTACCTAGATAAGACCTATCCG
AACCGTAACTTTTCAGGCATCGGTGTGATCGATTTCGAGAGGTGGAGACCAATTTTCAGACAGAATTGGGGTAAT
ATGAAGATTTACAAGAACTTCTCCATCGATCTTGTGCGTAAAGAGCATCCTTTCTGGAATAAGAAAATGATCGAG
TTGGAAGCTTCTAAAAGATTCGAGAAATACGCCCGTCTGTTCATGGAAGAAACATTAAAGTTGGCTAAGAAAACT
AGAAAACAGGCCGATTGGGGCTACTACGGTTACCCCTATTGCTTCAACATGTCTCCTACTAATTTCGTTCCTGAC
TGCGATGTCACAGCTAGGGATGAGAACAACGAGATGTCTTGGTTGTTTAACAACCAGAATGTCCTATTACCAAGT
GTATACATTAGGAGAGAGCTAACTCCTGACCAGAGGATTGGGCTTGTACAGGGGAGAGTGAAGGAAGCTGTGAGA
ATTTCAAATAAACTGAAGCACTCACCTAAAGTCTTCAGCTATTGGTGGTATGTTTACCAAGACGAGACCAACACC
TTCTTAACGGAGACCGACGTCAAGAAGACGTTTCAGGAGATTGTGATCAACGGTGGAGATGGGATTATAATCTGG
GGTTCGTCCTCTGATGTAAACAGCTTGTCCAAGTGTACGAGGTTAAGGGAGTACCTATTGACAGTCTTGGGACCA
ATTGCTGTTAACGTGACTGAAGCAGTAAACGGATCCGCCATTTCTCAAATCACTGACGGTCAAATCCAAGCTACT
ACCACTGCTACCACCGAAGCTACCACCACTGCTGCCCCATCTTCCACCGTTGAAACTGTTTCTCCATCCAGCACC
GAAACTATCTCTCAACAAACTGAAAATGGTGCTGCTAAGGCCGCTGTCGGTATGGGTGCCGGTGCTCTAGCTGCT
GCTGCTATGTTGTTATAA
**SEQ ID NO: 31** is an amino acid sequence of hyaluronidase (HYAL) originating from *Bothrops atrox* with a N-terminal secretion signal 
MQFSTVASVAFVALANFVAAPMYPNEPFLVFWNAPTTQCRLRYKVDLDLKTFHIVTNANDSLSGSAVTIFYPTHL
GVYPHIDDRGHFFNGIIPQNESLVKHLNKSKSDINRMIPLRTFHGLGVIDWENWRPQWDRNWGSKNVYRNRSIQF
ARDLHPELSEDKIKRLAKQEFEKAAKSFMRDTLLLAEEMRPDGYWGYYLYPDCHNYNYKTKPDQYTGECPDIEMS
RNNQLLWLWRDSTALFPNIYLETILRSSDNALKFVHHRLKEAMRIASMARNDYALPFFVYARPFYAYTFEPLTQE
DLVTTVGETADMGAAGIVFWGSMQYASTVESCGKVKDYMNGPLGRYIVNVTTAAKICSRFLCKRHGRCVRKHSDS
NAFLHLFPDSFRIMVHGNATEKKVIVKGKLELKNLIFLRNNFMCQCYQGWKGLYCEKHSIKEIRKI
**SEQ ID NO: 32** is an amino acid sequence of hyaluronidase (HYAL) originating from *Bothrops atrox* with a N-terminal secretion signal and a C-terminal anchoring signal 
MQFSTVASVAFVALANFVAAPMYPNEPFLVFWNAPTTQCRLRYKVDLDLKTFHIVTNANDSLSGSAVTIFYPTHL
GVYPHIDDRGHFFNGIIPQNESLVKHLNKSKSDINRMIPLRTFHGLGVIDWENWRPQWDRNWGSKNVYRNRSIQF
ARDLHPELSEDKIKRLAKQEFEKAAKSFMRDTLLLAEEMRPDGYWGYYLYPDCHNYNYKTKPDQYTGECPDIEMS
RNNQLLWLWRDSTALFPNIYLETILRSSDNALKFVHHRLKEAMRIASMARNDYALPFFVYARPFYAYTFEPLTQE
DLVTTVGETADMGAAGIVFWGSMQYASTVESCGKVKDYMNGPLGRYIVNVTTAAKICSRFLCKRHGRCVRKHSDS
NAFLHLFPDSFRIMVHGNATEKKVIVKGKLELKNLIFLRNNFMCQCYQGWKGLYCEKHSIKEIRKIGSAISQITD
GQIQATTTATTEATTTAAPSSTVETVSPSSTETISQQTENGAAKAAVGMGAGALAAAAMLL
**SEQ ID NO: 33** is an amino acid sequence of hyaluronidase (HYAL) originating from *Cupiennius salei* with a N-terminal secretion signal 
MQFSTVASVAFVALANFVAAFKIYWNVPTFQCTHNYKIDYVKLLSTYGIQVNDGGKFQGNQVTIFYETQLGLYPR
ILKSGKMENGGIPQRGNFEKHLEKASTDLQKVIPWKEFSGLGVIDWEAWRPTWEFNWEPLRIYQTESIKRAKELH
PTANDSAVKEIAERQWEDSAKLYMLETLRLAKKLRPQAPWCYYLFPDCYNYVGKKPKDFQCSASIRKGNDKLSWL
WKDSTALCPSIYVYESQLDRYSFEQRTWRDNEKLREALRVATRTSKIYPYVNYFDKELIPEQEVWRMLAQAAAVG
GSGAVIWGSSAAVASEELCKSLKQYIIETLGPAAEKVAWRSDLCSKEICNNQGRCTFPDDDYANAWKLFTDDTVK
FYAGNITCRCSENYSGRFCEKKN
**SEQ ID NO: 34** is an amino acid sequence of hyaluronidase (HYAL) originating from *Cupiennius salei* with a N-terminal secretion signal and a C-terminal anchoring signal 
MQFSTVASVAFVALANFVAAFKIYWNVPTFQCTHNYKIDYVKLLSTYGIQVNDGGKFQGNQVTIFYETQLGLYPR
ILKSGKMENGGIPQRGNFEKHLEKASTDLQKVIPWKEFSGLGVIDWEAWRPTWEFNWEPLRIYQTESIKRAKELH
PTANDSAVKEIAERQWEDSAKLYMLETLRLAKKLRPQAPWCYYLFPDCYNYVGKKPKDFQCSASIRKGNDKLSWL
WKDSTALCPSIYVYESQLDRYSFEQRTWRDNEKLREALRVATRTSKIYPYVNYFDKELIPEQEVWRMLAQAAAVG
GSGAVIWGSSAAVASEELCKSLKQYIIETLGPAAEKVAWRSDLCSKEICNNQGRCTFPDDDYANAWKLFTDDTVK
FYAGNITCRCSENYSGRFCEKKNGSAISQITDGQIQATTTATTEATTTAAPSSTVETVSPSSTETISQQTENGAA
KAAVGMGAGALAAAAMLL
**SEQ ID NO: 35** is an amino acid sequence of hyaluronidase (HYAL) originating from *Hirudo Nipponia* with a N-terminal secretion signal 
MQFSTVASVAFVALANFVAA~MKEIAVTIDDKNVIASVSESFHGVAFDASLFSPKGLWSFVDITSPKLFKLLEGL
SPGYFRVGGTFANWLFFDLDENNKWKDYWAFKDKTPETATITRRWLFRKQNNLKKETFDDLVKLTKGSKMRLLFD
LNAEVRTGYEIGKKMTSTWDSSEAEKLFKYCVSKGYGDNIDWELGNEPDHTSAHNLTEKQVGEDFKALHKVLEKY
PTLNKGSLVGPDVGWMGVSYVKGLADGAGDHVTAFTLHQYYFDGNTSDVSTYLDATYFKKLQQLFDKVKDVLKNS
PHKDKPLWLGETSSGYNSGTKDVSDRYVSGFLTLDKLGLSAANNVKVVIRQTIYNGYYGLLDKNTLEPNPDYWLM
 
HVHNSLVGNTVFKVDVSDPTNKARVYAQCTKTNSKHTQSRYYKGSLTIFALNVGDEDVTLKIDQYSGKKIYSYIL
TPEGGQLTSQKVLLNGKELKLVSDQLPELNADESKTSFTLSPKTFGFFVVSDANVEACKKGSAISQITDGQIQAT
TTATTEATTTAAPSSTVETVSPSSTETISQQTENGAAKAAVGMGAGALAAAAMLL
**SEQ ID NO: 36** is an amino acid sequence of hyaluronidase (HYAL) originating from *Hirudo Nipponia* with a N-terminal secretion signal and a C-terminal anchoring signal 
MQFSTVASVAFVALANFVAA~MKEIAVTIDDKNVIASVSESFHGVAFDASLFSPKGLWSFVDITSPKLFKLLEGL
SPGYFRVGGTFANWLFFDLDENNKWKDYWAFKDKTPETATITRRWLFRKQNNLKKETFDDLVKLTKGSKMRLLFD
LNAEVRTGYEIGKKMTSTWDSSEAEKLFKYCVSKGYGDNIDWELGNEPDHTSAHNLTEKQVGEDFKALHKVLEKY
PTLNKGSLVGPDVGWMGVSYVKGLADGAGDHVTAFTLHQYYFDGNTSDVSTYLDATYFKKLQQLFDKVKDVLKNS
PHKDKPLWLGETSSGYNSGTKDVSDRYVSGFLTLDKLGLSAANNVKVVIRQTIYNGYYGLLDKNTLEPNPDYWLM
HVHNSLVGNTVFKVDVSDPTNKARVYAQCTKTNSKHTQSRYYKGSLTIFALNVGDEDVTLKIDQYSGKKIYSYIL
TPEGGQLTSQKVLLNGKELKLVSDQLPELNADESKTSFTLSPKTFGFFVVSDANVEACKK
**SEQ ID NO: 37** is an amino acid sequence of hyaluronidase (HYAL) originating from *Loxosceles intermedia* with a N-terminal secretion signal 
MQFSTVASVAFVALANFVAAFDVFWNVPSQQCKKYGMKFVPLLEQYSILVNKEDNFKGDKITIFYESQLGLYPHI
GANDESFNGGIPQLGDLKAHLEKSAVDIRRDILDKSATGLRIIDWEAWRPIWEFNWSSLRKYQDKMKKVVRQFNP
TAHESTVAKLAHNEWENSSKSWMLSTLQLGKQLRPNSVWCYYLFPDCYNYDGNSVQEFQCSEAIRKGNDRLKWLW
EESTAVCPSIYIKEGQLTNYTLQKRIWFTNGRLQEALRVAQPKARIYPYINYSIKPGMMVPEVEFWRLIAQIASL
GMDGAVIWGSSASVGSKNHCAQLMKYIADVLGPATLRIKENVARCSKQACSGRGRCTWPKDTSVIAWKFLVEKED
YDFYLGDIECKCVEGYEGRYCEQKTK
**SEQ ID NO: 38** is an amino acid sequence of hyaluronidase (HYAL) originating from *Loxosceles intermedia* with a N-terminal secretion signal and a C-terminal anchoring signal 
MQFSTVASVAFVALANFVAAFDVFWNVPSQQCKKYGMKFVPLLEQYSILVNKEDNFKGDKITIFYESQLGLYPHI
GANDESFNGGIPQLGDLKAHLEKSAVDIRRDILDKSATGLRIIDWEAWRPIWEFNWSSLRKYQDKMKKVVRQFNP
TAHESTVAKLAHNEWENSSKSWMLSTLQLGKQLRPNSVWCYYLFPDCYNYDGNSVQEFQCSEAIRKGNDRLKWLW
EESTAVCPSIYIKEGQLTNYTLQKRIWFTNGRLQEALRVAQPKARIYPYINYSIKPGMMVPEVEFWRLIAQIASL
GMDGAVIWGSSASVGSKNHCAQLMKYIADVLGPATLRIKENVARCSKQACSGRGRCTWPKDTSVIAWKFLVEKED
YDFYLGDIECKCVEGYEGRYCEQKTKGSAISQITDGQIQATTTATTEATTTAAPSSTVETVSPSSTETISQQTEN
GAAKAAVGMGAGALAAAAMLL
**SEQ ID NO: 39** is an amino acid sequence of hyaluronidase (HYAL) originating from *Tityus serrulatus* with a N-terminal secretion signal 
MQFSTVASVAFVALANFVAAADFKVYWEVPSFLCSKRFKINVTEVLTSHEILVNQGESFNGDKIVIFYENQLGKY
PHIDSNNVEINGGILQVADLAKHLKVAKDNITKFVPNPNFNGVGVIDWEAWRPSWEFNWGKLKVYKEKSIDLVKS
KHPEWPSDRVEKVAKEEWEESAKEWMVKTLKLAQEMRPNAVWCYYLFPDCYNYFGKDQPSQFSCSSRIQKENSRL
SWLWNQSTAICLSIYIQESHVTKYNMSQRTWWIDARLREAIRVSEHRPNIPIYPYINYILPGTNQTVPAMDFKRT
LGQIASLGLDGALLWGSSYHVLTESQCKITSDYVKSVIAPTVATVVLNTNRCSQIICKGRGNCVWPEEPFSSWKY
LVDPKMPVFKPTNIHCKCKGYLGRYCEIPK
**SEQ ID NO: 40** is an amino acid sequence of hyaluronidase (HYAL) originating from *Tityus serrulatus* with a N-terminal secretion signal and a C-terminal anchoring signal 
MQFSTVASVAFVALANFVAAADFKVYWEVPSFLCSKRFKINVTEVLTSHEILVNQGESFNGDKIVIFYENQLGKY
PHIDSNNVEINGGILQVADLAKHLKVAKDNITKFVPNPNFNGVGVIDWEAWRPSWEFNWGKLKVYKEKSIDLVKS
KHPEWPSDRVEKVAKEEWEESAKEWMVKTLKLAQEMRPNAVWCYYLFPDCYNYFGKDQPSQFSCSSRIQKENSRL
SWLWNQSTAICLSIYIQESHVTKYNMSQRTWWIDARLREAIRVSEHRPNIPIYPYINYILPGTNQTVPAMDFKRT
LGQIASLGLDGALLWGSSYHVLTESQCKITSDYVKSVIAPTVATVVLNTNRCSQIICKGRGNCVWPEEPFSSWKY
LVDPKMPVFKPTNIHCKCKGYLGRYCEIPKGSAISQITDGQIQATTTATTEATTTAAPSSTVETVSPSSTETISQ
QTENGAAKAAVGMGAGALAAAAMLL
**SEQ ID NO: 41** is an amino acid sequence of hyaluronidase (HYAL) originating from *Vespa magnifica* with a N-terminal secretion signal 
MQFSTVASVAFVALANFVAADSCGSNCEKSERPKRVFNIYWNVPTFMCHQYGLYFDEVTNFNIKHNSKDNFQGDK
IAIFYDPGEFPALLPLNYGKYKIRNGGVPQEGNITIHLQRFIEYLDKTYPNRNFSGIGVIDFERWRPIFRQNWGN
MKIYKNFSIDLVRKEHPFWNKKMIELEASKRFEKYARLFMEETLKLAKKTRKQADWGYYGYPYCFNMSPTNFVPD
CDVTARDENNEMSWLFNNQNVLLPSVYIRRELTPDQRIGLVQGRVKEAVRISNKLKHSPKVFSYWWYVYQDETNT
FLTETDVKKTFQEIVINGGDGIIIWGSSSDVNSLSKCTRLREYLLTVLGPIAVNVTEAVN
**SEQ ID NO: 42** is an amino acid sequence of hyaluronidase (HYAL) originating from *Vespa magnifica* with a N-terminal secretion signal and a C-terminal anchoring signal 
MQFSTVASVAFVALANFVAADSCGSNCEKSERPKRVFNIYWNVPTFMCHQYGLYFDEVTNFNIKHNSKDNFQGDK
IAIFYDPGEFPALLPLNYGKYKIRNGGVPQEGNITIHLQRFIEYLDKTYPNRNFSGIGVIDFERWRPIFRQNWGN
MKIYKNFSIDLVRKEHPFWNKKMIELEASKRFEKYARLFMEETLKLAKKTRKQADWGYYGYPYCFNMSPTNFVPD
CDVTARDENNEMSWLFNNQNVLLPSVYIRRELTPDQRIGLVQGRVKEAVRISNKLKHSPKVFSYWWYVYQDETNT
FLTETDVKKTFQEIVINGGDGIIIWGSSSDVNSLSKCTRLREYLLTVLGPIAVNVTEAVNAISQITDGQIQATTT
ATTEATTTAAPSSTVETVSPSSTETISQQTENGAAKAAVGMGAGALAAAAMLL
**SEQ ID NO: 43** is a nucleic acid sequence of the secretion sequence added in 5' ATGCAATTTAGCACAGTCGCATCAGTAGCCTTCGTTGCCTTGGCCAACTTCGTGGCAGCA
**SEQ ID NO: 44** is an amino acid sequence of the secretion sequence added in N-ter MQFSTVASVAFVALANFVAA
**SEQ ID NO: 45** is a nucleic acid sequence of the anchoring sequence added in 3' 
GGATCCGCCATTTCTCAAATCACTGACGGTCAAATCCAAGCTACTACCACTGCTACCACCGAAGCTACCACCACT
GCTGCCCCATCTTCCACCGTTGAAACTGTTTCTCCATCCAGCACCGAAACTATCTCTCAACAAACTGAAAATGGT
GCTGCTAAGGCCGCTGTCGGTATGGGTGCCGGTGCTCTAGCTGCTGCTGCTATGTTGTTATAA
**SEQ ID NO: 46** is an amino acid sequence of the anchoring sequence added in C-ter AISQITDGQIQATTTATTEATTTAAPSSTVETVSPSSTETISQQTENGAAKAAVGMGAGALAAAAMLL
**SEQ ID NO: 47** is a nucleic acid sequence of glutamine-fructose-6-phosphate amidotransferase (GFA1) originating from *Saccharomyces cerevisiae* 
ATGTGTGGTATCTTTGGTTACTGCAATTATCTAGTGGAAAGATCCAGAGGAGAAATTATCGACACCTTAGTGGAT
GGTTTACAAAGATTAGAATATAGAGGCTATGATTCCACCGGTATTGCTATCGATGGTGACGAAGCTGATTCTACT
TTCATCTATAAGCAAATCGGTAAAGTGAGTGCTTTGAAAGAGGAGATTACTAAGCAAAATCCGAACAGAGACGTT
ACTTTTGTCTCTCATTGTGGTATTGCGCATACTAGATGGGCTACTCACGGTCGACCAGAACAAGTTAACTGTCAC
CCTCAAAGATCTGACCCAGAAGACCAATTTGTGGTCGTTCATAATGGTATCATCACAAATTTTAGAGAACTGAAG
ACTCTTTTAATTAACAAAGGTTATAAATTCGAAAGTGATACCGATACCGAGTGTATTGCTAAACTATATTTGCAT
TTATACAATACAAATTTACAAAATGGGCATGACTTAGATTTCCACGAATTAACCAAGCTAGTTCTTTTAGAACTA
GAAGGTTCATACGGGTTATTATGTAAATCTTGTCACTATCCTAATGAGGTTATCGCCACTAGAAAAGGGTCCCCT
TTACTGATTGGTGTCAAATCTGAAAAAAAACTAAAAGTCGACTTCGTGGATGTGGAATTTCCCGAAGAAAACGCT
GGTCAACCGGAAATTCCATTGAAATCTAACAACAAATCATTTGGCTTGGGCCCAAAGAAAGCTCGTGAATTTGAA
GCTGGTTCCCAAAATGCCAATTTACTACCAATTGCCGCCAATGAATTTAACTTGAGACATTCTCAATCCAGGGCT
TTCCTATCAGAAGATGGATCTCCAACACCGGTGGAATTTTTTGTTTCTTCGGATGCGGCATCTGTTGTTAAACAT
ACCAAGAAGGTGCTATTTTTAGAAGATGACGATTTGGCTCATATTTACGATGGTGAGTTACATATTCATAGATCT
AGAAGAGAAGTAGGCGCATCAATGACAAGGTCCATTCAAACTTTAGAGATGGAGTTAGCTCAGATCATGAAGGGC
CCTTACGACCATTTTATGCAAAAGGAAATCTATGAGCAACCAGAATCTACTTTCAATACTATGAGAGGTAGAATC
GACTATGAAAATAATAAAGTGATATTGGGTGGTTTAAAGGCATGGTTACCAGTTGTCAGAAGAGCACGGAGACTG
ATCATGATCGCATGCGGTACTTCTTATCATTCATGTTTGGCTACTCGTGCTATCTTCGAAGAATTATCAGATATC
CCAGTTAGTGTGGAATTAGCGTCTGACTTTCTGGACAGAAAATGCCCTGTCTTCAGAGACGATGTATGCGTGTTT
GTTTCACAAAGTGGTGAAACTGCGGATACCATGCTGGCTCTAAATTATTGTTTAGAAAGAGGAGCCTTAACTGTC
GGAATTGTTAACAGTGTTGGTTCTTCTATCTCTCGTGTCACCCACTGTGGTGTTCATATTAACGCTGGTCCTGAA
ATTGGTGTTGCCTCTACAAAAGCTTATACTTCCCAGTATATTGCCTTAGTGATGTTTGCTCTATCGCTGTCAGAT
 
GACCGTGTATCGAAAATAGACAGAAGAATTGAAATCATTCAAGGCTTGAAGTTAATCCCGGGCCAAATTAAGCAG
GTATTAAAGCTGGAACCAAGAATAAAAAAGCTCTGTGCGACTGAATTAAAGGATCAAAAATCTCTATTGTTATTG
GGTAGAGGTTACCAATTTGCTGCTGCTCTGGAAGGTGCTTTGAAGATCAAAGAAATTTCTTATATGCATTCTGAA
GGTGTTTTGGCAGGTGAGTTGAAGCACGGTGTCTTGGCCTTGGTGGACGAAAACTTGCCAATCATTGCTTTTGGT
ACCAGAGACTCTCTATTCCCTAAAGTAGTTTCCTCTATTGAGCAAGTTACTGTAAGAAAGGGCCATCCAATTATT
ATTTGTAACGAAAATGATGAAGTGTGGGCGCAAAAATCTAAATCAATCGACCTGCAAACCTTAGAAGTTCCACAA
ACTGTTGATTGTTTACAAGGTCTAATTAATATTATTCCATTACAACTAATGTCATATTGGTTGGCTGTTAATAAA
GGGATTGATGTTGATTTTCCAAGAAACTTGGCTAAATCTGTTACCGTCGAATAA
**SEQ ID NO: 48** is a reencoded nucleic acid sequence of glutamine-fructose-6-phosphate amidotransferase (GFA1) originating from *Chlorella virus 1* (PBCV-1) 
ATGTGCGGGATCTTCGGTGCTGTGTCGAACAATAATAGCATAGAAGTTTCCATCAAGGGTATACAGAAGCTAGAG
TACCGCGGGTACGATTCGTGTGGAATAGCCTATACAGACGGAGGCGCCATTGAACGGATCAGGTCAATAGATGGG
ATCGACGACTTAAGGAAGAAAACAATAACAGAGTCTTCCCCGGTAGCTATCGCTCATTCAAGGTGGAGTACCACT
GGGATTCCAAGTGTTGTGAACGCCCACCCGCACATCTCTCGGGGCACGTCTGGATGCGAGTCGCGCATTGCAGTC
GTACACAATGGCATTATTGAAAATTACCAGCAGATCCGCAAGTACTTGATAAATCTTGGGTATACTTTCGATTCA
CAGACCGACACGGAAGTCATTGCTCATCTAATAGACTCGCAGTATAACGGGAACATCCTGCACACCGTCCAAATG
GCAGTTAAGCACCTCAAAGGAAGCTACGCCATAGCAGTCATGTGCCATAAGGAGTCCGGGAAGATCGTGGTGGCA
AAACAGAAGAGTCCGTTGGTATTGGGTATAGGAAGCGACGGAGCTTATTATATCGCATCGGACGTATTGGCTCTT
CCCACTAACAAAGTGGTCTACATAAGTGACGGATTCTCTGCTGAGTTAAGTCCTGGATCGATGACTATCTATGAT
CTGGATGGCAATAAGGTAGAATACGAGGTGGAAGACGTTGAAATGGAGCAGACGTCGATGTCTTTAGATAACTTT
GATCACTACATGATCAAAGAGATCAATGAACAGCCGATATCAATTCTCAATACTATTAAGAACAAAGGATTCTAT
GCAGAGATATTTGGTGATCTAGCACATGAAATATTTCAGAAGATCGACAATATATTGATTCTAGCCTGCGGAACT
TCGTATCATGCAGGTCTCGTCGGGAAGCAATGGATAGAGACCATCTCACGCATCCCGGTGGATGTTCATATTGCA
AGCGAGTATGAGCCGACAATTCCACGAGCCAATACACTTGTGATAACGATTAGTCAAAGCGGCGAGACTGCGGAT
ACAATCGCAGCCCTTCAACGAGCGCAAAACGCAGGAATGATATACACTTTGTGTATCTGCAACAGCCCCAAGTCA
ACTCTAGTGCGCGAGTCGATAATGAAATACATAACCAAATGCGGTTCAGAAGTTTCTGTTGCCAGTACTAAAGCA
TTTACGTCGCAACTTGTGGTGTTGTATATGCTTGCGAACGTTCTTGCGAACAAAACTGATGATCTGCTAGGGGAT
CTTCCTCAAGCTATCGAGAGAGTAATCTGTCTTACTAATGACGAAATGAAGCGTTGGGCGGATGAAATTTGTACT
GCCAAAAGTGCCATCTTCTTAGGGAGAGGACTGAACGCACCTGTAGCATTTGAGGGCGCGCTAAAGTTGAAGGAA
ATCTCATATATCCATGCCGAGGGTTTCCTCGGTGGAGAGTTGAAGCATGGCCCGCTGGCTTTGTTGGACGATAAG
ATCCCTGTAATCGTGACTGTTGCTGACCACGCTTATTTGGATCACATTAAGGCTAACATCGACGAGGTATTGGCA
CGAAACGTTACCGTATACGCGATCGTCGATCAGTATGTTAATATTGAACCACAGGAGCGACTGCATGTAGTAAAA
GTGCCGTTTGTGAGTAAAGAGTTTTCTCCCATCATCCACACAATTCCGATGCAATTGTTATCGTATTATGTCGCG
ATCAAGCTGGGCAAGAACGTTGACAAGCCACGTAACCTGGCGAAAAGTGTTACAACATTCtaa
**SEQ ID NO: 49** is a reencoded nucleic acid sequence of glutamine-fructose-6-phosphate amidotransferase (GFA1) originating from *Chlorella virus 1* (PBCV-1) 
ATGTGTGGCATCTTTGGAGCAGTGTCAAACAACAACTCTATCGAGGTGTCAATCAAGGGTATTCAGAAGCTAGAA
TATCGTGGGTATGATTCGTGCGGTATTGCGTATACAGATGGGGGTGCGATTGAGCGTATACGTTCTATTGACGGC
ATTGACGATCTGCGTAAGAAAACAATCACAGAATCATCACCAGTGGCCATTGCTCACTCGCGGTGGAGCACCACT
GGAATTCCATCAGTGGTGAACGCACATCCTCATATTTCTCGCGGAACCAGTGGGTGTGAGTCTCGTATCGCGGTA
GTCCACAACGGTATCATTGAAAACTATCAGCAGATCCGAAAATATCTCATCAATCTTGGTTATACGTTTGATAGT
CAAACGGACACAGAGGTCATTGCGCATTTGATTGATTCTCAGTACAATGGGAATATCTTGCACACCGTCCAAATG
GCTGTCAAGCACCTGAAGGGCTCTTATGCCATTGCAGTTATGTGTCATAAAGAGTCTGGTAAAATAGTCGTGGCG
AAACAGAAGTCACCCCTCGTACTTGGAATCGGCTCAGATGGTGCTTACTACATCGCTTCGGACGTGCTGGCGCTG
CCGACAAATAAAGTTGTTTATATTTCAGACGGTTTCTCCGCAGAACTATCTCCAGGGAGTATGACCATTTACGAT
CTTGATGGAAATAAAGTAGAATATGAAGTAGAGGACGTTGAAATGGAACAAACTAGTATGTCTCTCGATAACTTT
GATCATTACATGATTAAGGAAATTAATGAGCAACCAATCAGTATTCTAAACACTATAAAAAATAAAGGGTTCTAT
GCAGAAATATTCGGTGATTTGGCTCATGAAATCTTCCAAAAAATAGACAACATCCTGATACTGGCTTGTGGTACA
AGTTATCACGCCGGTCTTGTAGGAAAACAGTGGATAGAGACCATCTCtAGAATCCCCGTGGATGTTCACATCGCG
AGTGAATACGAACCTACTATTCCGAGAGCGAACACATTGGTAATCACTATTTCACAGTCGGGTGAAACTGCGGAC
ACGATAGCGGCTTTGCAACGGGCCCAAAACGCCGGGATGATTTATACATTGTGTATTTGCAATTCACCAAAGAGC
ACTCTTGTTCGTGAGAGCATTATGAAGTACATCACGAAATGTGGTTCTGAGGTGTCAGTGGCATCAACGAAGGCG
TTTACTTCTCAGCTCGTAGTACTGTACATGCTGGCAAACGTATTGGCAAATAAAACCGATGATTTGCTGGGAGAC
CTCCCACAGGCAATAGAACGGGTAATTTGTTTGACAAATGACGAAATGAAACGATGGGCCGACGAAATTTGCACT
GCGAAATCCGCGATCTTCCTGGGAAGAGGACTAAACGCACCAGTTGCCTTTGAGGGAGCGTTGAAGCTCAAAGAA
ATCTCTTACATTCATGCAGAGGGCTTCCTGGGAGGTGAGTTGAAACACGGCCCCCTCGCACTCCTTGATGACAAG
ATTCCTGTTATCGTAACCGTAGCAGATCATGCTTATTTGGACCATATCAAAGCAAATATCGACGAAGTGCTTGCG
AGGAACGTTACGGTATACGCCATAGTAGACCAGTATGTGAACATCGAGCCCCAGGAACGCCTTCACGTCGTCAAG
GTTCCGTTTGTATCCAAAGAATTTTCTCCGATAATTCACACTATCCCGATGCAACTGCTTTCGTATTACGTGGCA
ATTAAGCTTGGAAAGAACGTTGACAAACCAAGGAATCTTGCAAAATCCGTGACTACCTTTTAA
**SEQ ID NO: 50** is an amino acid sequence of glutamine-fructose-6-phosphate amidotransferase (GFA1) originating from *Saccharomyces cerevisiae* 
MCGIFGYCNYLVERSRGEIIDTLVDGLQRLEYRGYDSTGIAIDGDEADSTFIYKQIGKVSALKEEITKQNPNRDV
TFVSHCGIAHTRWATHGRPEQVNCHPQRSDPEDQFVVVHNGIITNFRELKTLLINKGYKFESDTDTECIAKLYLH
LYNTNLQNGHDLDFHELTKLVLLELEGSYGLLCKSCHYPNEVIATRKGSPLLIGVKSEKKLKVDFVDVEFPEENA
GQPEIPLKSNNKSFGLGPKKAREFEAGSQNANLLPIAANEFNLRHSQSRAFLSEDGSPTPVEFFVSSDAASVVKH
TKKVLFLEDDDLAHIYDGELHIHRSRREVGASMTRSIQTLEMELAQIMKGPYDHFMQKEIYEQPESTFNTMRGRI
DYENNKVILGGLKAWLPVVRRARRLIMIACGTSYHSCLATRAIFEELSDIPVSVELASDFLDRKCPVFRDDVCVF
VSQSGETADTMLALNYCLERGALTVGIVNSVGSSISRVTHCGVHINAGPEIGVASTKAYTSQYIALVMFALSLSD
DRVSKIDRRIEIIQGLKLIPGQIKQVLKLEPRIKKLCATELKDQKSLLLLGRGYQFAAALEGALKIKEISYMHSE
 
GVLAGELKHGVLALVDENLPIIAFGTRDSLFPKVVSSIEQVTARKGHPIIICNENDEVWAQKSKSIDLQTLEVPQ

TVDCLQGLINIIPLQLMSYWLAVNKGIDVDFPRNLAKSVTVE
**SEQ ID NO: 51** is an amino acid sequence of glutamine-fructose-6-phosphate amidotransferase (GFA1) originating from *Chlorella virus 1* (PBCV-1) 
MCGIFGAVSNNNSIEVSIKGIQKLEYRGYDSCGIAYTDGGAIERIRSIDGIDDLRKKTITESSPVAIAHSRWSTT
GIPSVVNAHPHISRGTSGCESRIAVVHNGIIENYQQIRKYLINLGYTFDSQTDTEVIAHLIDSQYNGNILHTVQM
AVKHLKGSYAIAVMCHKESGKIVVAKQKSPLVLGIGSDGAYYIASDVLALPTNKVVYISDGFSAELSPGSMTIYD
LDGNKVEYEVEDVEMEQTSMSLDNFDHYMIKEINEQPISILNTIKNKGFYAEIFGDLAHEIFQKIDNILILACGT
SYHAGLVGKQWIETISRIPVDVHIASEYEPTIPRANTLVITISQSGETADTIAALQRAQNAGMIYTLCICNSPKS
TLVRESIMKYITKCGSEVSVASTKAFTSQLVVLYMLANVLANKTDDLLGDLPQAIERVICLTNDEMKRWADEICT
AKSAIFLGRGLNAPVAFEGALKLKEISYIHAEGFLGGELKHGPLALLDDKIPVIVTVADHAYLDHIKANIDEVLA
RNVTVYAIVDQYVNIEPQERLHVVKVPFVSKEFSPIIHTIPMQLLSYYVAIKLGKNVDKPRNLAKSVTTF
**SEQ ID NO: 52** is a nucleic acid sequence of UDP-N-acetylglucosamine pyrophosphorylase (QRI1) originating from *Saccharomyces cerevisiae* 
ATGACTGACACAAAACAGCTATTCATTGAAGCCGGACAAAGTCAACTTTTCCACAATTGGGAAAGCTTGTCTCGC
AAAGACCAAGAAGAATTGCTTTCAAACCTGGAGCAAATATCTTCCAAGAGGTCCCCTGCAAAACTACTGGAAGAC
TGTCAAAATGCTATTAAATTCTCACTAGCTAACTCTTCTAAGGATACTGGCGTCGAAATTTCACCATTGCCCCCT
ACTTCGTACGAGTCGCTTATTGGCAACAGTAAGAAAGAAAATGAATACTGGCGTTTAGGCCTTGAAGCTATTGGC
AAGGGTGAAGTCGCAGTGATTTTAATGGCTGGCGGACAAGGTACGCGGTTAGGATCCTCTCAACCAAAGGGCTGT
TACGACATTGGATTGCCTTCTAAGAAATCTCTTTTTCAAATTCAAGCTGAAAAGTTGATCAGGTTGCAAGATATG
GTAAAGGACAAAAAGGTAGAAATTCCTTGGTATATTATGACATCAGGCCCCACTAGAGCGGCTACTGAGGCATAC
TTTCAAGAACACAATTATTTTGGCTTGAATAAAGAACAAATTACGTTCTTCAACCAGGGAACCCTGCCTGCCTTT
GATTTAACCGGGAAGCATTTCCTAATGAAAGACCCAGTAAACCTATCTCAATCACCAGATGGAAATGGTGGACTC
TACCGTGCCATCAAGGAAAACAAGTTGAACGAAGACTTTGATAGGAGAGGAATCAAGCATGTTTACATGTACTGT
GTCGATAATGTCCTATCTAAAATCGCAGACCCTGTATTTATTGGTTTTGCCATCAAGCATGGCTTCGAACTGGCC
ACCAAAGCCGTTAGAAAGAGAGATGCGCATGAATCAGTTGGGTTAATTGCTACTAAAAACGAGAAACCATGTGTC
ATAGAATATTCTGAAATTTCCAATGAATTGGCTGAAGCAAAGGATAAAGATGGCTTATTAAAACTACGCGCAGGC
AACATTGTAAATCATTATTACCTAGTGGATTTACTAAAACGTGATTTGGATCAGTGGTGTGAGAATATGCCATAT
CACATTGCGAAGAAGAAAATTCCAGCTTATGATAGTGTTACCGGCAAGTACACTAAGCCTACCGAACCAAACGGT
ATAAAATTAGAGCAATTCATATTTGATGTCTTTGACACTGTACCACTGAACAAGTTTGGGTGCTTAGAAGTAGAT
AGATGCAAAGAATTTTCACCTTTAAAAAACGGTCCTGGTTCTAAGAACGATAATCCTGAGACCAGCAGACTAGCA
TATTTGAAACTAGGAACCTCGTGGTTGGAAGATGCAGGCGCTATTGTAAAAGATGGGGTACTAGTCGAAGTTTCC
AGCAAATTGAGTTATGCAGGTGAAAATCTATCCCAGTTCAAAGGTAAAGTCTTTGACAGAAGTGGTATAGTATTA
GAAAAATAA
**SEQ ID NO: 53** is an amino acid sequence of UDP-N-acetylglucosamine pyrophosphorylase (QRI1) originating from *Saccharomyces cerevisiae* 
MTDTKQLFIEAGQSQLFHNWESLSRKDQEELLSNLEQISSKRSPAKLLEDCQNAIKFSLANSSKDTGVEISPLPP
TSYESLIGNSKKENEYWRLGLEAIGKGEVAVILMAGGQGTRLGSSQPKGCYDIGLPSKKSLFQIQAEKLIRLQDM
VKDKKVEIPWYIMTSGPTRAATEAYFQEHNYFGLNKEQITFFNQGTLPAFDLTGKHFLMKDPVNLSQSPDGNGGL
YRAIKENKLNEDFDRRGIKHVYMYCVDNVLSKIADPVFIGFAIKHGFELATKAVRKRDAHESVGLIATKNEKPCV
IEYSEISNELAEAKDKDGLLKLRAGNIVNHYYLVDLLKRDLDQWCENMPYHIAKKKIPAYDSVTGKYTKPTEPNG
IKLEQFIFDVFDTVPLNKFGCLEVDRCKEFSPLKNGPGSKNDNPETSRLAYLKLGTSWLEDAGAIVKDGVLVEVS
SKLSYAGENLSQFKGKVFDRSGIVLEK
**SEQ ID NO: 54** is a nucleic acid sequence of Phosphoglucomutase-1 (PGM1) originating from *Saccharomyces cerevisiae* 
ATGTCACTTCTAATAGATTCTGTACCAACAGTTGCTTATAAGGACCAAAAACCGGGTACTTCAGGTTTACGTAAG
AAGACCAAGGTTTTCATGGATGAGCCTCATTATACTGAGAACTTCATTCAAGCAACAATGCAATCTATCCCTAAT
GGCTCAGAGGGAACCACTTTAGTTGTTGGAGGAGATGGTCGTTTCTACAACGATGTTATCATGAACAAGATTGCC
GCAGTAGGTGCTGCAAACGGTGTCAGAAAGTTAGTCATTGGTCAAGGCGGTTTACTTTCAACACCAGCTGCTTCT
CATATAATTAGAACATACGAGGAAAAGTGTACCGGTGGTGGTATCATATTAACTGCCTCACACAACCCAGGCGGT
CCAGAGAATGATTTAGGTATCAAGTATAATTTACCTAATGGTGGGCCAGCTCCAGAGAGTGTCACTAACGCTATC
TGGGAAGCGTCTAAAAAATTAACTCACTATAAAATTATAAAGAACTTCCCCAAGTTGAATTTGAACAAGCTTGGT
AAAAACCAAAAATATGGCCCATTGTTAGTGGACATAATTGATCCTGCCAAAGCATACGTTCAATTTCTGAAGGAA
ATTTTTGATTTTGACTTAATTAAAAGCTTCTTAGCGAAACAGCGCAAAGACAAAGGGTGGAAGTTGTTGTTTGAC
TCCTTAAATGGTATTACAGGACCATATGGTAAGGCTATATTTGTTGATGAATTTGGTTTACCGGCAGAGGAAGTT
CTTCAAAATTGGCACCCTTTACCTGATTTCGGCGGTTTACATCCCGATCCGAATCTAACCTATGCACGAACTCTT
GTTGACAGGGTTGACCGCGAAAAAATTGCCTTTGGAGCAGCCTCCGATGGTGATGGTGATAGGAATATGATTTAC
GGTTATGGCCCTGCTTTCGTTTCGCCAGGTGATTCTGTTGCCATTATTGCCGAATATGCACCCGAAATTCCATAC
TTCGCCAAACAAGGTATTTATGGCTTGGCACGTTCATTTCCTACATCCTCAGCCATTGATCGTGTTGCAGCAAAA
AAGGGATTAAGATGTTACGAAGTTCCAACCGGCTGGAAATTCTTCTGTGCCTTATTTGATGCTAAAAAGCTATCA
ATCTGTGGTGAAGAATCCTTCGGTACAGGTTCCAATCATATCAGAGAAAAGGACGGTCTATGGGCCATTATTGCT
TGGTTAAATATCTTGGCTATCTACCATAGGCGTAACCCTGAAAAGGAAGCTTCGATCAAAACTATTCAGGACGAA
TTTTGGAACGAGTATGGCCGTACTTTCTTCACAAGATACGATTACGAACATATCGAATGCGAGCAGGCCGAAAAA
GTTGTAGCTCTTTTGAGTGAATTTGTATCAAGGCCAAACGTTTGTGGCTCCCACTTCCCAGCTGATGAGTCTTTA
ACCGTTATCGATTGTGGTGATTTTTCGTATAGAGATCTAGATGGCTCCATCTCTGAAAATCAAGGCCTTTTCGTA
AAGTTTTCGAATGGGACTAAATTTGTTTTGAGGTTATCCGGCACAGGCAGTTCTGGTGCAACAATAAGATTATAC
GTAGAAAAGTATACTGATAAAAAGGAGAACTATGGCCAAACAGCTGACGTCTTCTTGAAACCCGTCATCAACTCC
ATTGTAAAATTCTTAAGATTTAAAGAAATTTTAGGAACAGACGAACCAACAGTCCGCACATAG
**SEQ ID NO: 55** is an amino acid sequence Phosphoglucomutase-1 (PGM1) originating from *Saccharomyces cerevisiae* 
MSLLIDSVPTVAYKDQKPGTSGLRKKTKVFMDEPHYTENFIQATMQSIPNGSEGTTLVVGGDGRFYNDVIMNKIA
AVGAANGVRKLVIGQGGLLSTPAASHIIRTYEEKCTGGGIILTASHNPGGPENDLGIKYNLPNGGPAPESVTNAI
WEASKKLTHYKIIKNFPKLNLNKLGKNQKYGPLLVDIIDPAKAYVQFLKEIFDFDLIKSFLAKQRKDKGWKLLFD
SLNGITGPYGKAIFVDEFGLPAEEVLQNWHPLPDFGGLHPDPNLTYARTLVDRVDREKIAFGAASDGDGDRNMIY
GYGPAFVSPGDSVAIIAEYAPEIPYFAKQGIYGLARSFPTSSAIDRVAAKKGLRCYEVPTGWKFFCALFDAKKLS
ICGEESFGTGSNHIREKDGLWAIIAWLNILAIYHRRNPEKEASIKTIQDEFWNEYGRTFFTRYDYEHIECEQAEK
VVALLSEFVSRPNVCGSHFPADESLTVIDCGDFSYRDLDGSISENQGLFVKFSNGTKFVLRLSGTGSSGATIRLY
VEKYTDKKENYGQTADVFLKPVINSIVKFLRFKEILGTDEPTVRT
**SEQ ID NO: 56** is a nucleic acid sequence of UTP--glucose-1-phosphate uridylyltransferase (UGP1) originating from *Saccharomyces cerevisiae* 
ATGTCCACTAAGAAGCACACCAAAACACATTCCACTTATGCATTCGAGAGCAACACAAACAGCGTTGCTGCCTCA
CAAATGAGAAACGCCTTAAACAAGTTGGCGGACTCTAGTAAACTTGACGATGCTGCTCGCGCTAAGTTTGAGAAC
GAACTGGATTCGTTTTTCACGCTTTTCAGGAGATATTTGGTAGAGAAGTCTTCTAGAACCACCTTGGAATGGGAC
AAGATCAAGTCTCCCAACCCGGATGAAGTGGTTAAGTATGAAATTATTTCTCAGCAGCCCGAGAATGTCTCAAAC
CTTTCCAAATTGGCTGTTTTGAAGTTGAACGGTGGGCTGGGTACCTCCATGGGCTGCGTTGGCCCTAAATCTGTT
ATTGAAGTGAGAGAGGGAAACACCTTTTTGGATTTGTCTGTTCGTCAAATTGAATACTTGAACAGACAGTACGAT
AGCGACGTGCCATTGTTATTGATGAATTCTTTCAACACTGACAAGGATACGGAACACTTGATTAAGAAGTATTCC
GCTAACAGAATCAGAATCAGATCTTTCAATCAATCCAGGTTCCCAAGAGTCTACAAGGATTCTTTATTGCCTGTC
CCCACCGAATACGATTCTCCACTGGATGCTTGGTATCCACCAGGTCACGGTGATTTGTTTGAATCTTTACACGTA
TCTGGTGAACTGGATGCCTTAATTGCCCAAGGAAGAGAAATATTATTTGTTTCTAACGGTGACAACTTGGGTGCT
ACCGTCGACTTAAAAATTTTAAACCACATGATCGAGACTGGTGCCGAATATATAATGGAATTGACTGATAAGACC
AGAGCCGATGTTAAAGGTGGTACTTTGATTTCTTACGATGGTCAAGTCCGTTTATTGGAAGTCGCCCAAGTTCCA
AAAGAACACATTGACGAATTCAAAAATATCAGAAAGTTTACCAACTTCAACACGAATAACTTATGGATCAATCTG
AAAGCAGTAAAGAGGTTGATCGAATCGAGCAATTTGGAGATGGAAATCATTCCAAACCAAAAAACTATAACAAGA
GACGGTCATGAAATTAATGTCTTACAATTAGAAACCGCTTGTGGTGCTGCTATCAGGCATTTTGATGGTGCTCAC
GGTGTTGTCGTTCCAAGATCAAGATTCTTGCCTGTCAAGACCTGTTCCGATTTGTTGCTGGTTAAATCAGATCTA
TTCCGTCTGGAACACGGTTCTTTGAAGTTAGACCCATCCCGTTTTGGTCCAAACCCATTAATCAAGTTGGGCTCG
CATTTCAAAAAGGTTTCTGGTTTTAACGCAAGAATCCCTCACATCCCAAAAATCGTCGAGCTAGATCATTTGACC
ATCACTGGTAACGTCTTTTTAGGTAAAGATGTCACTTTGAGGGGTACTGTCATCATCGTTTGCTCCGACGGTCAT
AAAATCGATATTCCAAACGGCTCCATATTGGAAAATGTTGTCGTTACTGGTAATTTGCAAATCTTGGAACATTGA
**SEQ ID NO: 57** is an amino acid sequence of UTP--glucose-1-phosphate uridylyltransferase (UGP1) originating from *Saccharomyces cerevisiae* 
MSTKKHTKTHSTYAFESNTNSVAASQMRNALNKLADSSKLDDAARAKFENELDSFFTLFRRYLVEKSSRTTLEWD
KIKSPNPDEVVKYEIISQQPENVSNLSKLAVLKLNGGLGTSMGCVGPKSVIEVREGNTFLDLSVRQIEYLNRQYD
SDVPLLLMNSFNTDKDTEHLIKKYSANRIRIRSFNQSRFPRVYKDSLLPVPTEYDSPLDAWYPPGHGDLFESLHV
SGELDALIAQGREILFVSNGDNLGATVDLKILNHMIETGAEYIMELTDKTRADVKGGTLISYDGQVRLLEVAQVP
KEHIDEFKNIRKFTNFNTNNLWINLKAVKRLIESSNLEMEIIPNQKTITRDGHEINVLQLETACGAAIRHFDGAH
GVVVPRSRFLPVKTCSDLLLVKSDLFRLEHGSLKLDPSRFGPNPLIKLGSHFKKVSGFNARIPHIPKIVELDHLT
ITGNVFLGKDVTLRGTVIIVCSDGHKIDIPNGSILENVVVTGNLQILEH
**SEQ ID NO: 58** is a nucleic acid sequence of Glucosamine 6-phosphate N-acetyltransferase (GNA1) originating from *Saccharomyces cerevisiae* 
ATGAGCTTACCCGATGGATTTTATATAAGGCGAATGGAAGAGGGGGATTTGGAACAGGTCACTGAGACGCTAAAG
GTTTTGACCACCGTGGGCACTATTACCCCCGAATCCTTCAGCAAACTCATAAAATACTGGAATGAAGCCACAGTA
TGGAATGATAACGAAGATAAAAAAATAATGCAATATAACCCCATGGTGATTGTGGACAAGCGCACCGAGACGGTT
GCCGCTACGGGGAATATCATCATCGAAAGAAAGATCATTCATGAACTGGGGCTATGTGGCCACATCGAGGACATT
GCAGTAAACTCCAAGTATCAGGGCCAAGGTTTGGGCAAGCTCTTGATTGATCAATTGGTAACTATCGGCTTTGAC
TACGGTTGTTATAAGATTATTTTAGATTGCGATGAGAAAAATGTCAAATTCTATGAAAAATGTGGGTTTAGCAAC
GCAGGCGTGGAAATGCAAATTAGAAAATAG
**SEQ ID NO: 59** is an amino acid sequence of Glucosamine 6-phosphate N-acetyltransferase (GNA1) originating from *Saccharomyces cerevisiae* 
MSLPDGFYIRRMEEGDLEQVTETLKVLTTVGTITPESFSKLIKYWNEATVWNDNEDKKIMQYNPMVIVDKRTETV
AATGNIIIERKIIHELGLCGHIEDIAVNSKYQGQGLGKLLIDQLVTIGFDYGCYKIILDCDEKNVKFYEKCGFSN
AGVEMQIRK
**SEQ ID NO: 60** is a nucleic acid sequence of phosphoacetylglucosamine mutase (PCM1) originating from *Saccharomyces cerevisiae* 
ATGAAGGTTGATTACGAGCAATTGTGCAAACTCTACGATGACACGTGCCGCACAAAGAATGTGCAGTTCAGTTAC
GGTACGGCCGGATTCAGAACGCTGGCCAAGAATTTGGATACGGTGATGTTCAGTACTGGTATACTGGCGGTTCTC
AGGTCGCTGAAGCTTCAGGGTCAGTATGTGGGGGTGATGATCACGGCGTCGCACAACCCATACCAGGACAACGGG
GTCAAGATCGTGGAACCAGACGGATCGATGCTTTTGGCCACATGGGAGCCATATGCCATGCAGTTGGCCAATGCG
GCCTCTTTTGCCACTAATTTTGAAGAATTTCGTGTTGAGTTGGCCAAGCTGATTGAACACGAAAAGATTGATTTG
AATACAACCGTCGTGCCTCACATCGTGGTTGGGAGAGACTCTAGGGAAAGTAGTCCATACTTGCTGCGCTGCTTG
ACTTCCTCCATGGCCAGCGTCTTCCACGCGCAAGTTTTGGACCTAGGCTGTGTCACTACGCCTCAATTGCATTAC
ATTACTGATTTGTCCAACAGGCGGAAACTGGAAGGAGACACAGCGCCAGTTGCCACAGAACAGGACTACTATTCG
TTCTTTATAGGAGCCTTCAACGAGCTCTTCGCCACGTATCAGCTGGAGAAGAGGCTGTCTGTCCCAAAATTGTTC
 
ATAGACACAGCCAATGGTATCGGTGGTCCACAGTTGAAAAAACTACTGGCCTCCGAAGATTGGGACGTGCCAGCG
GAGCAAGTTGAGGTAATCAACGACAGGTCCGATGTTCCAGAACTGTTGAATTTTGAATGCGGTGCGGATTATGTG
AAGACTAACCAGAGATTACCCAAGGGTCTTTCTCCATCCTCGTTTGATTCGCTATATTGCTCCTTTGATGGTGAC
GCAGACAGGGTTGTGTTCTACTATGTCGACTCAGGATCAAAATTTCATTTGTTGGATGGTGACAAAATTTCCACT
TTGTTTGCAAAGTTCTTGTCTAAACAACTAGAATTGGCACACCTAGAACATTCTTTGAAGATTGGTGTTGTGCAA
ACTGCCTATGCAAACGGCAGTTCCACCGCTTACATAAAAAATACGTTGCACTGTCCCGTGTCTTGCACTAAGACA
GGTGTTAAACACTTGCATCATGAAGCTGCCACTCAGTACGATATTGGCATTTATTTCGAAGCAAATGGACATGGT
ACGATTATATTCAGCGAAAAATTTCATCGAACTATCAAATCTGAATTATCCAAGTCCAAGTTAAATGGTGATACG
TTAGCTTTGAGAACTTTGAAGTGTTTCTCTGAATTGATTAATCAGACCGTGGGAGATGCTATTTCAGACATGCTT
GCTGTCCTTGCTACTTTGGCGATTTTGAAAATGTCGCCAATGGATTGGGATGAAGAGTATACTGATTTGCCCAAC
AAGCTGGTTAAGTGCATCGTTCCTGATAGGTCAATTTTCCAAACCACGGACCAGGAAAGAAAATTGCTCAATCCA
GTGGGGTTGCAAGACAAGATAGATCTTGTGGTAGCCAAGTATCCCATGGGAAGAAGCTTTGTCAGAGCCAGTGGT
ACGGAGGATGCGGTGAGGGTTTATGCGGAATGTAAGGACTCCTCTAAGTTAGGTCAATTTTGTGACGAAGTGGTG
GAGCACGTTAAGGCATCTGCTTGA
**SEQ ID NO: 61** is an amino acid sequence of phosphoacetylglucosamine mutase (PCM1) originating from *Saccharomyces cerevisiae* 
MKVDYEQLCKLYDDTCRTKNVQFSYGTAGFRTLAKNLDTVMFSTGILAVLRSLKLQGQYVGVMITASHNPYQDNG
VKIVEPDGSMLLATWEPYAMQLANAASFATNFEEFRVELAKLIEHEKIDLNTTVVPHIVVGRDSRESSPYLLRCL
TSSMASVFHAQVLDLGCVTTPQLHYITDLSNRRKLEGDTAPVATEQDYYSFFIGAFNELFATYQLEKRLSVPKLF
IDTANGIGGPQLKKLLASEDWDVPAEQVEVINDRSDVPELLNFECGADYVKTNQRLPKGLSPSSFDSLYCSFDGD
ADRVVFYYVDSGSKFHLLDGDKISTLFAKFLSKQLELAHLEHSLKIGVVQTAYANGSSTAYIKNTLHCPVSCTKT
GVKHLHHEAATQYDIGIYFEANGHGTIIFSEKFHRTIKSELSKSKLNGDTLALRTLKCFSELINQTVGDAISDML
AVLATLAILKMSPMDWDEEYTDLPNKLVKCIVPDRSIFQTTDQERKLLNPVGLQDKIDLVVAKYPMGRSFVRASG
TEDAVRVYAECKDSSKLGQFCDEVVEHVKASA
**SEQ ID NO: 62** is the nucleic acid sequence of promotor pTDH3 
CTGCTGTAACCCGTACATGCCCAAAATAGGGGGCGGGTTACACAGAATATATAACATCGTAGGTGTCTGGGTGAA
CAGTTTATTCCTGGCATCCACTAAATATAATGGAGCCCGCTTTTTAAGCTGGCATCCAGAAAAAAAAAGAATCCC
AGCACCAAAATATTGTTTTCTTCACCAACCATCAGTTCATAGGTCCATTCTCTTAGCGCAACTACAGAGAACAGG
GGCACAAACAGGCAAAAAACGGGCACAACCTCAATGGAGTGATGCAACCTGCCTGGAGTAAATGATGACACAAGG
CAATTGACCCACGCATGTATCTATCTCATTTTCTTACACCTTCTATTACCTTCTGCTCTCTCTGATTTGGAAAAA
GCTGAAAAAAAAGGTTGAAACCAGTTCCCTGAAATTATTCCCCTACTTGACTAATAAGTATATAAAGACGGTAGG
TATTGATTGTAATTCTGTAAATCTATTTCTTAAACTTCTTAAATTCTACTTTTATAGTTAGTCTTTTTTTTAGTT
TTAAAACACCAAGAACTTAGTTTCGAATAAACACACATAAACAAACAAA
**SEQ ID NO:63** is the nucleic acid sequence of promotor pTDH3.Sk 
CAATTCATCGGCCCTTTTAGCGGCTACCCGCGCCATCTAAATGATAGGGCGGGTGACACTATGGTAAATCCCATA
ATTAGGTGTCTGGGTGAGTGGTTCTGATGCCGGCATCCACTAAATATATTGGAGCCCATTTTTTACGCGGGCTTC
CAGAAAAAAAGAGAATCCCAGCACCAAAAGGTGGTTCTCTTCACCAACCATCAGATCATAGGTCCACAACCACAC
ATAACAGGGGCACAAAAAGGCAAAAAACGGACATAACCTCAATGGAGTGATGCAAATTGACTGGAGCAAAAGCTG
ACACAAGGCATTGATTGACCTACGCATGTATCTGTATTCTTTTCTTACACCTTCTATTACCTTCTAACTCTTTGG
GTTGGAAAAAACTGAAAAAAAAGGTTGGGACCTGGTTCCCCCAAGTTGTCCCCCTACTTGGTTATTAAATATATA
AAGACAGCAAGTGTTGATTATAATCTTGTAAATCTATAGTTCTTAATCTATACTTCTATTTATATTTTAAATTAG
TCTTTTTATTTCCAAGTCCCCAAGAACTTAGTTTCGAATAAACACACACAAATAAACACA
**SEQ ID NO: 64** is the nucleic acid sequence of promotor pTDH3-1.sba 
GCAGCGCTTCTTCCGCTCTAGTTTTTATAGTTATTATTACTACCACCTTAAAAATACGTAAATACTCAAAATAGT
AGTGATATTCCCAACCTTATTCATCCAAGGCACATCATCATCATCAGCCATTCATCTTTCACCTGCCATTAGTAA
CCCGTCTTCTCATTGAGCGGGTTACGGCAGCCACAGGCCACATTCCGAATGTCTGGGTGAGCGGTCCCTTTTCCA
GCATCCACTAAATATCTCCGATCCCGCTTTTTAATCTGGCTTCCTGAAAAAAAGAGAATCCCAGCACCAAAAAAT
GGCTCTCTTCACCAACCATCAGATCATAGGTCCCATTCTCTTACCGCAACCGTACAGAACAGGGGAAAACGGGTA
CAACCTCAATGGAGTGATGCAAACTGACTGGAGCAAAAAGCTGACACAAGGCAATCGACCTACGTGTCTGTCTAT
TTTCTCACACCTTCTATTACCTTCTAACTCTCTGGGTTGGAAAAAACTGAAAAAAAGGTTGAGACCAGTTTCCAC
AAATCATCCCCCTGTTTGATTAATAAATATATAAAGACGACAACTATCGATCATAAACTCATAAAACTATAACTC
CTTTACACTTCTTATTTTATAGTTATTCTATTTTAATTCTTATTGATTTTAAAACCCCAAGAACTTAGTTTCGAA
AACACACACACACAAACAATTAAAA
**SEQ ID NO: 65** is the nucleic acid sequence of promotor pTDH3.Sar 
GAGCTAAATATCAGCCCTTCGGGTCCTGCCTGCTACCCGGTCCTGTTCGAATAAAAACGCGGGTAACACGACCCA
GTAACACCTGTCGTTGGGTGTCTGGGTCAGAAGTTCTGATACCGGCTTCCACTAAATAGATTGGGTTCCGCTCTT
TACGCTGGCTTCCTGAAAAAAGAGATTCCGGGCACCAAAAAATTGGTCTCTTTGCCAACCATCAGATCATAGGTC
CATTCTCTTACCATAACCACACAGGATAGGGGCACCACAGGCGAAAATGGGCACAAAATCTCAATGGAGTGATGC
AAATTAGCTGGAACAAAAGCTGACACAAGGCAATTAACCTGCGCATGTATCCATCTCCTTTTCTTACACCTTCTC
TTACGTTCTAACTGTTTGGGTTGGAAAAATTAAAAAAAAAAGGTTGAGACCAGTTTCCCCAAATCGTCCCCCTAC
TTGATTCATAAATATATAAAGACGACAACTATTGATTATAATCTTGTAAATCTATAACTCTTTACTTTCTCCTAT
TTATAATTTAACTTAATCTTTTTAGATTTAAAACCCCAAGAACTTAGTTTCGAACAAACACACACAAATAAACAA
AA
**SEQ ID NO: 66** is the nucleic acid sequence of promotor pENO2 
CGCTCAGCATCTGCTTCTTCCCAAAGATGAACGCGGCGTTATGTCACTAACGACGTGCACCAACTTGCGGAAAGT
GGAATCCCGTTCCAAAACTGGCATCCACTAATTGATACATCTACACACCGCACGCCTTTTTTCTGAAGCCCACTT
TCGTGGACTTTGCCATATGCAAAATTCATGAAGTGTGATACCAAGTCAGCATACACCTCACTAGGGTAGTTTCTT
TGGTTGTATTGATCATTTGGTTCATCGTGGTTCATTAATTTTTTTTCTCCATTGCTTTCTGGCTTTGATCTTACT
 
ATCATTTGGATTTTTGTCGAAGGTTGTAGAATTGTATGTGACAAGTGGCACCAAGCATATATAAAAAAAAAAAGC
ATTATCTTCCTACCAGAGTTGATTGTTAAAAACGTATTTATAGCAAACGCAATTGTAATTAATTCTTATTTTGTA
TCTTTTCTTCCCTTGTCTCAATCTTTTATTTTTATTTTATTTTTCTTTTCTTAGTTTCTTTCATAACACCAAGCA
ACTAATACTATAACATACAATAATA
**SEQ ID NO: 67** is the nucleic acid sequence of promotor pTEF3 
GGCTGATAATAGCGTATAAACAATGCATACTTTGTACGTTCAAAATACAATGCAGTAGATATATTTATGCATATT
ACATATAATACATATCACATAGGAAGCAACAGGCGCGTTGGACTTTTAATTTTCGAGGACCGCGAATCCTTACAT
CACACCCAATCCCCCACAAGTGATCCCCCACACACCATAGCTTCAAAATGTTTCTACTCCTTTTTTACTCTTCCA
GATTTTCTCGGACTCCGCGCATCGCCGTACCACTTCAAAACACCCAAGCACAGCATACTAAATTTCCCCTCTTTC
TTCCTCTAGGGTGTCGTTAATTACCCGTACTAAAGGTTTGGAAAAGAAAAAAGAGACCGCCTCGTTTCTTTTTCT
TCGTCGAAAAAGGCAATAAAAATTTTTATCACGTTTCTTTTTCTTGAAAATTTTTTTTTTTGATTTTTTTCTCTT
TCGATGACCTCCCATTGATATTTAAGTTAATAAACGGTCTTCAATTTCTCAAGTTTCAGTTTCATTTTTCTTGTT
CTATTACAACTTTTTTTACTTCTTGCTCATTAGAAAGAAAGCATAGCAATCTAATCTAAGTTTTAATTACAAA
**SEQ ID NO: 68** is the nucleic acid sequence of promotor pTEF1 
GTTTAGCTTGCCTCGTCCCCGCCGGGTCACCCGGCCAGCGACATGGAGGCCCAGAATACCCTCCTTGACAGTCTT
GACGTGCGCAGCTCAGGGGCATGATGTGACTGTCGCCCGTACATTTAGCCCATACATCCCCATGTATAATCATTT
GCATCCATACATTTTGATGGCCGCACGGCGCGAAGCAAAAATTACGGCTCCTCGCTGCAGACCTGCGAGCAGGGA
AACGCTCCCCTCACAGACGCGTTGAATTGTCCCCACGCCGCGCCCCTGTAGAGAAATATAAAAGGTTAGGATTTG
CCACTGAGGTTCTTCTTTCATATACTTCCTTTTAAAATCTTGCTACGATACAGTTCTCACATCACATCCGAACAT
AAACAACC
**SEQ ID NO: 69** is the nucleic acid sequence of promotor pTEF1.ago 
GTTTAGCTTGCCTCGTCCCCGCCGGGTCACCCGGCCAGCGACATGGAGGCCCAGAATACCCTCCTTGACAGTCTT

GACGTGCGCAGCTCAGGGGCATGATGTGACTGTCGCCCGTACATTTAGCCCATACATCCCCATGTATAATCATTT

GCATCCATACATTTTGATGGCCGCACGGCGCGAAGCAAAAATTACGGCTCCTCGCTGCAGACCTGCGAGCAGGGA

AACGCTCCCCTCACAGACGCGTTGAATTGTCCCCACGCCGCGCCCCTGTAGAGAAATATAAAAGGTTAGGATTTG

CCACTGAGGTTCTTCTTTCATATACTTCCTTTTAAAATCTTGCTACGATACAGTTCTCACATCACATCCGAACAT

AAACAACC
**SEQ ID NO: 70** is the nucleic acid sequence of promotor pTEF1.Sba 
CGCCAACAAACCTTCGAACACTTTAATTTTCGAGGACCGCAGATCCTCACATCACACCCACACCCAAGACTGCTT
CCCCCACACACCCTGCATCTGTACACTTTCTTCTGCTCTGTTTTTCTCTCCGGCGTTCTCTCGGGTCGCCCGCAT
CGCCGCGCCGGCTGGAACCGCCCACGCACCGCATATTGCAAATCGCCTGCCCCCTCTTGCTCCTTTTTGAGGGCG
CGCCGTTACCCGCGCCCAGGGTCCGGAAAAAGAAACAAGGCTCTACCGCGTTTCTTTTTCCTTGTCGAAAAAGGC
AAAAATGAAAATTTTTATCACGTTTCTTTTTTTTTGAAAAATTTTTTTTTTGGTTTTTTTTCTTTCGATGGCCTC
 
CCATTGATATTTAAGTTAATAAATGGTTTTCAGTTTTCAAGTTTCAGTTTGTGTTCTTCTTTGCTAACTTTCACT

TACACCTCGAAAGAAAGTATAGCAATCTAATCTTAGTTTTAATTACAA
**SEQ ID NO: 71** is the nucleic acid sequence of promotor pPDC1 
TTATTTACCTATCTCTAAACTTCAACACCTTATATCATAACTAATATTTCTTGAGATAAGCACACTGCACCCATA
CCTTCCTTAAAAACGTAGCTTCCAGTTTTTGGTGGTTCCGGCTTCCTTCCCGATTCCGCCCGCTAAACGCATATT
TTTGTTGCCTGGTGGCATTTGCAAAATGCATAACCTATGCATTTAAAAGATTATGTATGCTCTTCTGACTTTTCG
TGTGATGAGGCTCGTGGAAAAAATGAATAATTTATGAATTTGAGAACAATTTTGTGTTGTTACGGTATTTTACTA
TGGAATAATCAATCAATTGAGGATTTTATGCAAATATCGTTTGAATATTTTTCCGACCCTTTGAGTACTTTTCTT
CATAATTGCATAATATTGTCCGCTGCCCCTTTTTCTGTTAGACGGTGTCTTGATCTACTTGCTATCGTTCAACAC
CACCTTATTTTCTAACTATTTTTTTTTTAGCTCATTTGAATCAGCTTATGGTGATGGCACATTTTTGCATAAACC
TAGCTGTCCTCGTTGAACATAGGAAAAAAAAATATATAAACAAGGCTCTTTCACTCTCCTTGCAATCAGATTTGG
GTTTGTTCCCTTTATTTTCATATTTCTTGTCATATTCCTTTCTCAATTATTATTTCTACTCATAACCTCACGCA
AAATAACACAGTCAAATCAATCAAA
**SEQ ID NO: 72** is the nucleic acid sequence of promotor pCCW12 
aaccagggcaaagcaaaataaaagAAACTTAATACGTTATGCCGTAATGAAGGGCTACCAAAAACGATAATCTCA
ACTGTAAACAGGTACAATGCGGACCCTTTTGCCACAAAACATACATCATTCATTGCCGGAAAAAGAAAGAAGTGA
AGACAGCAGTGCAGCCAGCCATGTTGCGCCAATCTAATTATAGATGCTGGTGCCCTGAGGATGTATCTGGAGCCA
GCCATGGCATCATGCGCTACCGCCGGATGTAAAATCCGACACGCAAAAGAAAACCTTCGAGGTTGCGCACTTCGC
CCACCCATGAACCACACGGTTAGTCCAAAAGGGGCAGTTCAGATTCCAGATGCGGGAATTAGCTTGCTGCCACCC
TCACCTCACTAACGCTGCGGTGTGCGGATACTTCATGCTATTTATAGACGCGCGTGTCGGAATCAGCACGCGCAA
GAACCAAATGGGAAAATCGGAATGGGTCCAGAACTGCTTTGAGTGCTGGCTATTGGCGTCTGATTTCCGTTTTGG
GAATCCTTTGCCGCGCGCCCCTCTCAAAACTCCGCACAAGTCCCAGAAAGCGGGAAAGAAATAAAACGCCACCAA
AAAAAAAAATAAAAGCCAATCCTCGAAGCGTGGGTGGTAGGCCCTGGATTATCCCGTACAAGTATTTCTCAGGAG
TAAAAAAACCGTTTGTTTTGGAATTCCCCATTTCGCGGCCACCTACGCCGCTATCTTTGCAACAACTATCTGCGA
TAACTCAGCAAATTTTGCATATTCGTGTTGCAGTATTGCGATAATGGGAGTCTTACTTCCAACATAACGGCAGAA
AGAAATGTGAGAAAATTTTGCATCCTTTGCCTCCGTTCAAGTATATAAAGTCGGCATGCTTGATAATCTTTCTTT
CCATCCTACATTGTTCTAATTATTCTTATTCTCCTTTATTCTTTCCTAACATACCAAGAAATTAATCTTctgtca
ttcgcttaaacactatatcaata
**SEQ ID NO: 73** is the nucleic acid sequence of promotor pCCW12.Sm 
CGTTGTGCTGTAGTGAAGGAAGACTAAAAAGGATAATCACAGTTGTAAAGAGGTATAATGCGGACCCTTTTGCCA

CAAAACACACATCTTTCGTTTCCAAAATAAGAAAGAAGAAAGCAAAAAGATTAGCAGCCACGTTGCTGCGATCTA

ATTATAGACGGTGGCGTCATCATTCTCACCCAAGATTGTGTCTTGAACCTGCCACGGGTCCTGCGTTATCGCCGG

ATGTAAAACTAGACATGCAAAAAAAGGACCTTCCAGGTAGCGTGCTCCACACCACCCATGACCACCACAGTTAGT

CCAAAAGAGGCAGCACCACTTCCCGATGGGGGAATTAGATTGCTGCCACCCTCACCTCACTAATGCTGCGGTGTG

CGGATATGCCCTGCTATATATAGCTCCGCGTTTTTGAACCAGCACAGCGCGAGCACCAAAAAGGAAAATCGCATA
 
GGCCCAGAACTGATTTCAGCACGGGCTATTGGCGTTGGGTTTCCGTTCTGGGAAACCTTCGCCGCGTCCCCCTCA
CAAACCTCCGCACAAGTTCGAGCAAGCGGGAAAAAACGAAAAACGCCATTAATACTAAATAAAGCAAATCCTCGA
AGCGTGGGTGGCAAGCCCCTGGATTTTTCCGCACAAGTACTCTTCTCAGGAGTAAAAAAACCCGTTTGTTTTGGA
ATTCCCCATTTCGCGGCCATCTACGCCGGTATCTTCGCAATATCTATCAGCGATAACTCAGCAATTTTAATATTC
GTGTTGCAGTGCTGCGATAGCGGGAGTCTTGTTTGTAACATAACGGCAGAAAGGAATGAGAGAAAATTTTCCATT
CTTTGGCCTCCGTTCAAGTATATAAAGCCGGCATGCTTGGTAATCTTTCTCTCTCTTCTGTATTGTTTCTATAAT
ACTTTTATCTTCTAATTATTTTCTGAAAAAACCAAGAAATTAATCTTCTGTCATTCGCTTAAACACTATATCAAT
A
**SEQ ID NO: 74** is the nucleic acid sequence of promotor pCCW12.Sk 
TGTAAAATCCTACACGCAAAAAACCTCTTGGGTTGCGCGTTTTCCACCACCCACGACCCGCACAATCAATCCAAA
AGGGGCAACGCCAGTTCCCAATGCGGGAATTAGCTCCTCACCCCCCTCACCCGCTAACGCTGCGGTGTGCGGACA
CGCAGCAGTATTTATAGATCCTCGTGTTGGAACCAGCCCGCGTGAGCACCAAATTGGAAAGTCGCAATGGGCCCA
GAACCGCTTTCAGTACTGGGCCATTGGCGTCTAGTTTCCGTTTTGAGCGTCCTTCGCCGCGTCTCTCTGTGAAAT
CCCCGCACAAGTCTGAGCAGGCAAAAAAAAAAAACGCCACCAAAAATAAATAAAGCCAATCCTCGAAGGATGAGT
AGGAAAGGAAGCCCCTGGTTTTTTCCCGCACGAATATTTTTCAGGAGTGAAAAAATCCGTTTGTTTTGGAATTCC
CCATTTCGCGCTCACCTACGCCGGTATCTTTGGAACAACTATCAGCGATAACTCAGCAAAATTTGCATATCCGTG
CTGCAATAGTGCGATAGTGGGATTGGGAGTCTTGTTGCATCATAACGGCAGAAAGGAATGAATAAAAATTTTCCG
TTCTTTGTCTCCGTTCAAGTATATAAAGTCGGCATGCTTGATTATCTTTTCTTCTCTTCTACTACATTTCTATTT
CACTTTCTACTCTATTCTTCCCTGAAAAACCCAAGAAAATAATCTTCTGTCATTCGCTTAAACAATATATCAAAA
**SEQ ID NO: 75** is the nucleic acid sequence of promotor pCCW12.Sba 
ACCATGCCACGGTGCTGGCCCCACTTCCACCCACGACCCGCACGGTTGGCCCGAAAGGGGCAACACCAGTTCCCA
ATACGGAAATTAGTCCGCCACCACCCTCACCTCGCTAGAGCTGCGGTGTGCGGGCGTGCATCGCTATTTATAGAC
ATGCCTGCTGGCGTCACCGCGCGCGAGCACCAAACAGGAAAATCGCACTGGGCCCAGAACCACGCTATGCGCTGG
GCCGATGGCGTCCGGTTTCCCTTTGGGAGCCCCCTGCCACGTTCGCCTAACAAATCCCCGCACCGGCTTGAGAAA
AAAGCGAAAAGCGAAAAAAAAAAATCAACGCCACCAAAATTAAAAAAAAAGAGCCATCCTCGAAGGGTGAATAGT
AGCCCCTGACTTTTCCCGCACAGACAGACACCTTTCAGGAGTGAACAAAAAAGCAGTTTGTTTTGGAATTCCCCC
ATTTCGCGGTGGCCTGCGCAGGTATCTCTGCGTCAACTATCAGCGATAACTCAGCAAATTTTGCATATTCGTGTT
GCGATACTACGATAATGGGAGTCTGTCGCCTAATAACGGCAACAAGGAATGAGAGAGAAAAATTTTCTTCATTCT
CCAGCTCCCGTTCAAGTATATAAGGTCGGCATGGTCGATTGTCTTTCCTTCTCTTCAGTTACGTCTCTCTATTTA
CATTATTCTTATTTTTATTTAATAAAAACCCCAAGAAATTAATCTTCTGTCATTCGCTTAAACACTATATCAAAA
**SEQ ID NO: 76** is the nucleic acid sequence of promotor pCCW12.Sar 
AAAAAACAACCTTCTGCCAACCTGCGTGCTTCTCACCACCCATGACCCACACAATTGACCCGAATGGGGCAACTC
CAGTTCCCAATACGGGAATTAACTCGCCACCATATTTACCGCGTTGAAGCTGTGGTGTGCGGACACTCCGTACTA
TTTATAGACCCACGCGGTGGAACCAGCACGCGCGCGCACTAAACAGGAAAATCGCATTGAGTCCAGAACCGCCAC
CAGCACTTGGCCATTGGCGTCTAATTTCCGTTTTCGGCGCCCCTCACCGCGTCCTTCTAACAAAGCGCGCACAAG
 
CTTGAGCAAGTGAAAAGAAAATTAAAAATAAAAAACCGCCACCAAAACAAATAAAGCAATCTTCGAAGTGTGGGT
TGGTGGGAAGCCCCTGGCTTTTCCCGCACCAGTCGTTTTCAGGAGTAAAAAAATACCCGTTTGTTTTGGAATTCC
CCATTTCGCGGCGACCTGCGCCGGTATCTTTGCAACAACTATTTGCGATAACTCAGCAAAATTTGCATATTCGTG
TTGGGATATTGCGATAGTGGGAGTCTTGTTGCATAATAACGGTAAAAAGAAGTGAAGGAAAAAAATTTGCATCCT
TTAGTCTCAGTTCAAGTATATAAAGTCGGGATATTCAATTATCTTTCTTTCTCTTGCTCAAAGGTTTCTATATTT
TTTTTATAGTATTTCTTTTGTTATAAAATACCAAGAAATTAATCTTCTGTCATTCGCTTAAATACTACATCAATA
**SEQ ID NO: 77** is the nucleic acid sequence of promotor pNUP57 
TCATCTGCGCAATGACTATCAAGACCTTCTGCAAGAATTTCAAATCTCACTGAAAATCTTGACCGAAAAGTGTCT
TGAAAACCCATCAAGCCTGCAAAACCTATCTTTGACATTAGTCTCCATTATAAAAACGGCATAGTTGGGAGAAAA
CTTTCCATACTTCAATTGTGGACTGATATAAGTATTTTAGTTTTGCCCGCATGATCATCCCACATGGCTACAGCA
GTTCTCTCATAGGAAAATAGTACAATAGCTACGTGATATAATCTAAATAATTGTTGCCAATGTGTAATTATATCA
TTTTGAACGTACGCGAAATGGATTATTTTCAAAAATTTTGTTTCTTGAAATGAGTAAAAGCAAAAGTCCAACTCT
CCAAGTCGATGTAAACAACTTTTTGCCAAAGGGACTGAAAGACTAAATCGAGGATTATCCCGTTCAAACTATTCC
AGAAACGCTCGTTAGTAACAAAAGACATACCTTGTTGACCAATTGATCAC
**SEQ ID NO: 78** is the nucleic acid sequence of promotor pCCW10.ago 
GGTACCACGGCAACCTCGTTCGCTGTTCATCCCCTTCGTCACACAGGACGTTGGATGCCGTAAGCAGCGTTGCTT
TTGATCCTCagGATCGGCCGGGTAACCCGCGGCtGCTTCTATTTTAGTATTCATATCTCAAGCACATCCATTCCG
GCCGTTTGGGGGCGCCGCCGCACTCGTGTCCATTCCTACCGTGGCACTTAGGGCTATCCTGTCGGAGCGCCCCGC
CGACCGCCTTATCGGCACCAAAAGTAGAAGCCCCGGCCCCGCGTGGCTCAGACTCACCATCGGTGCTATTTACTT
TTCGATCAGATCGCGGCGCGCGGTGGCCGGCATTTCCGGAAGCGGCCACGGAGCAGAGGTGGCGCATTCGAATCG
CATACGTCTTCGCCACGCCGGAAAAAAAATTTTCGGCTATATAAGGAGAGGCGGCCGTCTTGCTGCAGGCAGTTT
CACTTTCTCTAAAACCAAAGAACATCGATTTCTTTAGTCACTCGCTTCCTTACACCGAACTCGAGGCGGCCGC
**SEQ ID NO: 79** is the nucleic acid sequence of promotor pCWP2 
CTAGCCTCCCCTTTTTATTTTGTGCGGTCACCGCAAGGGACAAAGCTTTTCTTAGAAAACCGTCTGAGAAGCATA
ACGTACGCCATCCCCTAGACATATTAATAATGCTACAGATACTATGCTGCTCGTCTTTTTTTGACGACCCTTTTA
TTGCAATGTGCAACTAATGGCAAACAACCACATAGTATCACAGTATTACATTGCCTCCACCGATGCGGATGTTAG
GGCGCCAAGTCTGTCATGAAGCATGTTCCTGTCATAATCTTGTATGCAAAATACCGCGTTCTGCGCCACTGATAT
GCTAGGCAGCAGCAACCTATGCAGAAGATTGCTTTTCCCACGCCTGTTTTACGTCTCCAGGGCACTTGAAACAAT
GCAGCGATCGCCGCCACAACACGCCAAAGAGAAGCGAAAGTGGGCCTGGGCGGCCTCAGTTTCGGCAGAGGTAAA
CAACACGAACTGAACTGCCTTAGCTCCGAAGGGCAATTCCACAGGCACTCCGCGGGGCCCGGCCAAGGCCCAAAA
GGCGTGGAATATGCGCGTTTTGGGGCCATAACACCCAGTACCACGGCCGGAACGGGCCATATAATAAGTTTTTCA
CTCTCAAGAATGGTAAACGTAAATAGGAACATCCCACTACCCTAGAAATTGCGGAAATTTCGCGCTTATCATTAG
AAAATCTGGAACCGTCCTTTTTCCTCTTTCTTGCATTTCCCTTTCCGTATTATTGCCATTCTTTAACTGCATTTG
GGGAACCGTAGACCAAAAGCCAAACAGAGAAATGTAACGTTCTAAAAAAAAAACAACGAAAAAATTGAAAAATAA
 
GATACAATAATCGTATATAAATCAGGCTTCTTGTTCATCATTTTCAATTCTCTTCTTGCCATCCCTTTTCCTATC
TTTGTTCTTTTCTTCTCATAATCAAGAATAAATAACTTCATCA
**SEQ ID NO: 80** is the nucleic acid sequence of promotor pRPLA1 
TCAAGTTGGATACTGATCTGATCTCTCCGCCCTACTACCAGGGACCCTCATGATTACCGCTCGAATGCGACGTTT
CCTGCCTCATAAAACTGGCTTGAAAATATTTATTCGCTGAACAGTAGCCTAGCTTATAAAAATTTCATTTAATTA
ATGTAATATGAAAACTCACATGCCTTCTGTTTCTAAAATTGTCACAGCAAGAAATAACATTACCATACGTGATCT
TATTAAACTCTAGTATCTTGTCTAATACTTCATTTAAAAGAAGCCTTAACCCTGTAGCCTCATCTATGTCTGCTA
CATATCGTGAGGTACGAATATCGTAAGATGATACCACGCAACTTTGTAATGATTTTTTTTTTTTCATTTTTTAAA
GAATGCCTTTACATGGTATTTGAAAAAAATATCTTTATAAAGTTTGCGATCTCTTCTGTTCTGAATAATTTTTAG
TAAAAGAAATCAAAAGAATAAAGAAATAGTCCGCTTTGTCCAATACAACAGCTTAAACCGATTATCTCTAAAATA
ACAAGAAGAA
**SEQ ID NO: 81** is the nucleic acid sequence of promotor pCUP1 
CGGCAAACTTCAACGATTTCTATGATGCATTTTATAATTAGTAAGCCGATCCCATTACCGACATTTGGGCGCTAT
ACGTGCATATGTTCATGTATGTATCTGTATTTAAAACACTTTTGTATTATTTTTCCTCATATATGTGTATAGGTT
TATACGGATGATTTAATTATTACTTCACCACCCTTTATTTCAGGCTGATATCTTAGCCTTGTTACTAGTTAGAAA
AAGACATTTTTGCTGTCAGTCACTGTCAAGAGATTCTTTTGCTGGCATTTCTTCTAGAAGCAAAAAGAGCGATGC
GTCTTTTCCGCTGAACCGTTCCAGCAAAAAAGACTACCAACGCAATATGGATTGTCAGAATCATATAAAAGAGAA
GCAAATAACTCCTTGTCTTGTATCAATTGCATTATAATATCTTCTTGTTAGTGCAATATCATATAGAAGTCATCG
AAATAGATATTAAGAAAAACAAACTGTACAATCAATCAATCAATCATCACATAAA
**SEQ ID NO: 82** is the nucleic acid sequence of promotor pMET6 
CCACAGGAAATATTTCACGTGACTTACAAACAGAGTCGTACGTCAGGACCGGAGTCAGGTGAAAAAATGTGGGCC
GGTAAAGGGAAAAAACCAGAAACGGGACTACTATCGAACTCGTTTAGTCGCGAACGTGCAAAAGGCCAATATTTT
TCGCTAGAGTCATCGCAGTCATGGCAGCTCTTTCGCTCTATCTCCCGGTCGCAAAACTGTGGTAGTCATAGCTCG
TTCTGCTCAATTGAGAACTGTGAATGTGAATATGGAACAAATGCGATAGATGCACTAATTTAAGGGAAGCTAGCT
AGTTTTCCCAACTGCGAAAGAAAAAAAGGAAAGAAAAAAAAATTCTATATAAGTGATAGATATTTCCATCTTTAC
TAGCATTAGTTTCTCTTTTACGTATTCAATATTTTTGTTAAACTCTTCCTTTATCATAAAAAAGCAAGCATCTAA
GAGCATTGACAACACTCTAAGAAACAAAATACCAATATAATTTCAAAGTACATATCAAAA
**SEQ ID NO: 83** is the nucleic acid sequence of promotor pMET25 
TTACATTATCAATCCTTGCGTTTCAGCTTCCACTAATTTAGATGACTATTTCTCATCATTTGCGTCATCTTCTAA
CACCGTATATGATAATATACTAGTAACGTAAATACTAGTTAGTAGATGATAGTTGATTTTTATTCCAACACTAAG
AAATAATTTCGCCATTTCTTGAATGTATTTAAAGATATTTAATGCTATAATAGACATTTAAATCCAATTCTTCCA
ACATACAATGGGAGTTTGGCCGAGTGGTTTAAGGCGTCAGATTTAGGTGGATTTAACCTCTAAAATCTCTGATAT
CTTCGGATGCAAGGGTTCGAATCCCTTAGCTCTCATTATTTTTTGCTTTTTCTCTTGAGGTCACATGATCGCAAA
 
ATGGCAAATGGCACGTGAAGCTGTCGATATTGGGGAACTGTGGTGGTTGGCAAATGACTAATTAAGTTAGTCAAG

GCGCCATCCTCATGAAAACTGTGTAACATAATAACCGAAGTGTCGAAAAGGTGGCACCTTGTCCAATTGAACACG

CTCGATGAAAAAAATAAGATATATATAAGGTTAAGTAAAGCGTCTGTTAGAAAGGAAGTTTTTCCTTTTTCTTGC

TCTCTTGTCTTTTCATCTACTATTTCCTTCGTGTAATACAGGGTCGTCAGATACATAGATACAATTCTATTACCC

CCATCCATACA
**SEQ ID NO: 84** is the nucleic acid sequence of promotor pSAM1 
GAAACGGACGTAAGACGGAAATAGAATTTGAAGATAAAGTTATATATCACTACACACGAATACTTTCTTTTTTTT
TTTTCACAGGAAAACTGTGGTGGCGCCCTTGCCTACTAGTGCATTTCTTTTTTCGGGTTCTTGTCTCGAcGAAAT
TTTAGCCTCATCGTAGTTTTTCACTCTGGTATCGATGAAAAAGGGAAGAGTAAAAAGTTTTCCGTTTAGTACTTA
ATGGGATTGGTTTGGGACGTATATATCGACTGGTGTTGTCTGTTATTCATCGTTGTTTTTCGGTTAGCTTCGAAA
AAAAAATAGAGTAAAAACCAGGAATTTACCCTAAAAACAAGAAAAAATAAGATAAACGAAAAT
**SEQ ID NO: 85** is the nucleic acid sequence of terminator tTPI1 
GATTAATATAATTATATAAAAATATTATCTTCTTTTCTTTATATCTAGTGTTATGTAAAATAAATTGATGACTAC
GGAAAGCTTTTTTATATTGTTTCTTTTTCATTCTGAGCCACTTAAATTTCGTGAATGTTCTTGTAAGGGACGGTA
GATTTACAAGTGATACAACAAAAAGCAAGGCGCTTTTTCTAATAAAAAGAAGAAAAGCATTTAACAATTGAACAC
CTCTATATCAACGAAGAATATTACTTTGTCTCTAAATCCTTGTAAAATGTGTACGATCTCTATATGGGTTACTCA
**SEQ ID NO: 86** is the nucleic acid sequence of terminator tMET25 
GTGTGCGTAATGAGTTGTAAAATTATGTATAAACCTACTTTCTCTCACAAGTACTATACTTTTATAAAACGAACT
TTATTGAAATGAATATCCTTTTTTTCCCTTGTTACATGTCGTGACTCGTACTTTGAACCTAAATTGTTCTAACAT
CAAAGAACAGTGTTAATTCGCAGTCGAGAAGAAAAATATGGTGAACAAGACTCATCTACTTCATGAGACTACTTT
ACGCCTCCTATAAAGCTGTCACACTGGATAAATTTATTGTAGGACCAAGTTACAAAAGAGGATGATGGAGGTTT
**SEQ ID NO: 87** is the nucleic acid sequence of terminator tDIT1 
TAAAGTAAGAGCGCTACATTGGTCTACCTTTTTGTTCTTTTACTTAAACATTAGTTAGTTCGTTTTCTTTTTCTC
ATTTTTTTATGTTTCCCCCCCAAAGTTCTGATTTTATAATATTTTATTTCACACAATTCCATTTAACAGAGGGGG
AATAGATTCTTTAGCTTAGAAAATTAGTGATCAATATATATTTGCCTTTCTTTTCATCTTTTCAGTGATATTAAT
GGTTTCGAGACACTGCAATGGCCCTAGTTGTCTAAGAGGATAGATGTTACTGTCAAAGATGATATTTTGAATTTC
**SEQ ID NO: 88** is the nucleic acid sequence of terminator tRPL3 
GAAGTTTTGTTAGAAAATAAATCATTTTTTAATTGAGCATTCTTATTCCTATTTTATTTAAATAGTTTTATGTAT

TGTTAGCTACATACAACAGTTTAAATCAAATTTTCTTTTTCCCAAGTCCAAAATGGAGGTTTATTTTGATGACCC

GCATGCGATTATGTTTTGAAAGTATAAGACTACATACATGTACATATATTTAAACATGTAAACCCGTCCATTATA

TTGCTTACTTTCTTCTTTTTTGCCGTTTTGACTTGGACCTCTGGTTTGCTATTTCCTTACAATCTTTGCTACAAT
**SEQ ID NO: 89** is the nucleic acid sequence of terminator tRPL3.sm 
GAAGTTTTTAAAGCATTTTTTAGACACTTCTCATTTTTCTAAGTTTTTTTAAAATAGTTTTATGTATTTACTACG
TATCACAATTTGAAATAATTCATCTTCCCAAAAAACTAAGATTTTTATCCTTGTCACGATCCGTAACCAGTTTAT
AATATTTTAGAGCTTATACACGTACGTATACACACGTGTCGGTACATGAGAATTACGTTCAAAATTATTCACTTT
TTTTTTCTCTGCCGTTTTACTTTTGAACTCTGTCTCGCTATTTCCTTACAATCTTCGCTACAATACCACTTGCCC
TTGG
**SEQ ID NO: 90** is the nucleic acid sequence of terminator tRPL3.sba 
GAAGTTTTCTGACAAAAACATAACGTTTTTTCCAATCATTTCTTATTTTTCCGGTTTATTTAAATAGTTTTTATG
TACTATTATACGTATGACTATTTAACTTAAATTCTTCCTCCCAAGAAATCTCCCAAGTTTTTCATTATCATGGCA
TACACCACTATCAGTTACAAAATGGTAGCTCAACCATATATATATCTCTATATACACATATAAATGCAAACAGGT
CCAAGTCACCGCTCACTGCAGTTTCTTTTGCCGTTTTGACTTCGATCTCTGCTTGGCTATTTTCTCACAATCCT
**SEQ ID NO: 91** is the nucleic acid sequence of terminator tRPL41B 
GCGGATTGAGAGCAAATCGTTAAGTTCAGGTCAAGTAAAAATTGATTTCGAAAACTAATTTCTCTTATACAATCC
TTTGATTGGACCGTCATCCTTTCGAATATAAGATTTTGTTAAGAATATTTTAGACAGAGATCTACTTTATATTTA
ATATCTAGATATTACATAATTTCCTCTCTAATAAAATATCATTAATAAAATAAAAATGAAGCGATTTGATTTTGT
GTTGTCAACTTAGTTTGCCGCTATGCCTCTTGGGTAATGCTATTATTGAATCGAAGGGCTTTATTATATTACCCT
**SEQ ID NO: 92** is the nucleic acid sequence of terminator tRPL15A 
GCTGGTTGATGGAAAATATAATTTTATTGGGCAAACTTTTGTTTATCTGATGTGTTTTATACTATTATCTTTTTA
ATTAATGATTCTATATACAAACCTGTATATTTTTTCTTTAACCAATTTTTTTTTTTATAGACCTAGAGCTGTACT
TTTATTCTGCTATCAAGCAAACCCCTACCCCCTCTTCTCAATCCTCCCCTCAGGCAGAACTTATCTACCTGTATC
AAGGAGCGGACGAGGGAGTCCTAATTGTTCTACGTATACCAATGCTAGCAGCTTACATAGGTGGTGGCACTACCA
**SEQ ID NO: 93** is the nucleic acid sequence of terminator tRPL15A.sba 
GCTGGCTGATGAAAAATATAGTTCTGTTGGGCAAGCATTTGTTTACCTAGCATCTCTTTTATACTATTATTATCT
TTATATTTGATGATTTTATATACAAGTTGTATACCTTTTCTTTAACCAATTTTTTTTTTTCTAATGGTGCACCTA
GAAGTACATTTTTTCTCACCAATAGATAGTCAAGATACTCCCAGCCTCTATGGCGTTACCACGGAGCCGACAAGG
GAAAGCTGCTTATCTTACATATGCAGATGCCAAGGCCCGTTACAGGCCGCCTTATTGATGTTTAGAAATAGCTTC
**SEQ ID NO: 94** is the nucleic acid sequence of terminator tIDP1 
TCGAATTTACGTAGCCCAATCTACCACTTTTTTTTTTCATTTTTTAAAGTGTTATACTTAGTTATGCTCTAGGAT

AATGAACTACTTTTTTTTTTTTTTTTTTACTGTTATCATAAATATATATACCTTATTGTTGTTTGCAACCGTCGG

TTAATTCCTTATCAAGGTTCCCCAAGTTCGGATCATTACCATCAATTTCCAACATTTTCATGAGTTCTTCTTCTT

CATTACCGTGTTTTAGGGGGCTGTTCGCACTTCTAATAGGGCTATCACCAAGCTGTTCTAATTCGTCCAAAAGTT
**SEQ ID NO: 95** is the nucleic acid sequence of terminator tTEF1.sba 
GGAGATTGATAGGACTTTTCTAGTTGCATATCTTTTATTTTTAAATCTTATCTATTAGTTAATTTTTTGTAATTT
ATCCTTATATATATAGTTTGGTTATTCTAAAACATCATTTCAGTATCTAAAACCTCTCTTATTCATTACCTTTTT
ATTTAATGGTTTTTGCTACAGGCAAAAATTTAATGGTTTTTGCTACAGGCAAAAATCCCGCCGTGGACTTATTCC
ACGTTAACTCGGTTACAGGGTCATGAACCATTTTGTCAATTATCGAAATAACTTCTTCAAAAGTCCCTCTTACTT
**SEQ ID NO: 96** is a nucleic acid sequence of Glutamine synthetase (GLN1) originating from *Saccharomyces cerevisiae* 
ATGGCTGAAGCAAGCATCGAAAAGACTCAAATTTTACAAAAATATCTAGAACTGGACCAAAGAGGTAGAATAATT
GCCGAATACGTTTGGATCGATGGTACTGGTAACTTACGTTCCAAAGGTAGAACTTTGAAGAAGAGAATCACATCC
ATTGACCAATTGCCAGAATGGAACTTCGACGGTTCTTCTACCAACCAAGCGCCAGGCCACGACTCTGACATCTAT
TTGAAACCCGTTGCTTACTACCCAGATCCCTTCAGGAGAGGTGACAACATTGTTGTCTTGGCCGCATGTTACAAC
AATGACGGTACTCCAAACAAGTTCAACCACAGACACGAAGCTGCCAAGCTATTTGCTGCTCATAAGGATGAAGAA
ATCTGGTTTGGTCTAGAACAAGAATACACTCTATTTGACATGTATGACGATGTTTACGGATGGCCAAAGGGTGGG
TACCCAGCTCCACAAGGTCCTTACTACTGTGGTGTTGGTGCCGGTAAGGTTTATGCCAGAGACATGATCGAAGCT
CACTACAGAGCTTGTTTGTATGCCGGATTAGAAATTTCTGGTATTAACGCTGAAGTCATGCCATCTCAATGGGAA
TTCCAAGTCGGTCCATGTACCGGTATTGACATGGGTGACCAATTATGGATGGCCAGATACTTTTTGCACAGAGTG
GCAGAAGAGTTTGGTATCAAGATCTCATTCCATCCAAAGCCATTGAAGGGTGACTGGAACGGTGCCGGTTGTCAC
ACTAACGTTTCCACCAAGGAAATGAGACAACCAGGTGGTATGAAATACATCGAACAAGCCATCGAGAAGTTATCC
AAGAGACACGCTGAACACATTAAGTTGTACGGTAGCGATAACGACATGAGATTAACTGGTAGACATGAAACCGCT
TCCATGACTGCCTTTTCTTCTGGTGTCGCCAACAGAGGTAGCTCAATTAGAATCCCAAGATCCGTCGCCAAGGAA
GGTTACGGTTACTTTGAAGACCGTAGACCAGCTTCCAACATCGACCCATACTTGGTTACAGGTATCATGTGTGAA
ACTGTTTGCGGTGCTATTGACAATGCTGACATGACGAAGGAATTTGAAAGAGAATCTTCATAA
**SEQ ID NO: 97** is an amino acid sequence of Glutamine synthetase (GLN1) originating from *Saccharomyces cerevisiae* 
MAEASIEKTQILQKYLELDQRGRIIAEYVWIDGTGNLRSKGRTLKKRITSIDQLPEWNFDGSSTNQAPGHDSDIY
LKPVAYYPDPFRRGDNIVVLAACYNNDGTPNKFNHRHEAAKLFAAHKDEEIWFGLEQEYTLFDMYDDVYGWPKGG
YPAPQGPYYCGVGAGKVYARDMIEAHYRACLYAGLEISGINAEVMPSQWEFQVGPCTGIDMGDQLWMARYFLHRV
AEEFGIKISFHPKPLKGDWNGAGCHTNVSTKEMRQPGGMKYIEQAIEKLSKRHAEHIKLYGSDNDMRLTGRHETA
SMTAFSSGVANRGSSIRIPRSVAKEGYGYFEDRRPASNIDPYLVTGIMCETVCGAIDNADMTKEFERESS
**SEQ ID NO: 98** is a nucleic acid sequence of Glutamate synthase (GLT1) originating from *Saccharomyces cerevisiae* 
ATGCCAGTGTTGAAATCAGACAATTTCGATCCATTGGAAGAAGCTTACGAAGGTGGGACAATTCAAAACTATAAC
GATGAACACCATCTTCATAAATCTTGGGCAAATGTGATTCCGGACAAACGAGGACTTTACGACCCTGATTATGAA
CATGACGCTTGTGGTGTCGGTTTCGTAGCAAATAAGCATGGTGAACAGTCTCACAAGATTGTTACTGACGCTAGA
 
TATCTTTTAGTGAATATGACACATCGTGGTGCCGTCTCATCTGATGGGAACGGTGACGGTGCCGGTATTCTGCTA
GGTATTCCTCACGAATTTATGAAAAGAGAATTCAAGTTAGATCTTGATCTAGACATACCTGAGATGGGCAAATAC
GCCGTAGGTAACGTCTTCTTCAAGAAGAACGAAAAAAATAACAAGAAAAATTTAATTAAGTGTCAGAAGATTTTC
GAGGATTTAGCTGCATCCTTCAACTTATCCGTATTAGGTTGGAGAAACGTCCCCGTAGATTCTACTATTTTAGGA
GACGTTGCATTATCTCGTGAACCTACTATTCTACAGCCATTATTGGTTCCATTGTATGATGAAAAACAACCGGAG
TTTAATGAAACTAAATTTAGAACTCAATTGTATCTTTTAAGGAAGGAGGCCTCTCTTCAAATAGGACTGGAAAAC
TGGTTCTATGTTTGTTCCCTAAACAATACCACCATTGTTTACAAGGGTCAATTGACGCCAGCTCAAGTGTATAAC
TACTATCCCGACTTGACTAATGCGCATTTCAAATCCCACATGGCGTTGGTCCATTCAAGATTTTCCACTAATACT
TTCCCCTCTTGGGATAGAGCTCAACCTTTACGTTGGCTAGCTCATAATGGTGAAATTAACACCTTAAGAGGTAAC
AAGAATTGGATGCGCTCCAGAGAAGGTGTGATGAATTCAGCAACTTTCAAAGATGAGTTAGACAAACTATACCCA
ATTATCGAAGAAGGTGGTTCTGATTCAGCTGCATTGGATAACGTTTTAGAACTATTGACTATTAATGGCACATTA
TCTCTACCTGAAGCTGTTATGATGATGGTTCCTGAAGCGTATCATAAGGATATGGATTCTGACCTAAAAGCATGG
TACGACTGGGCTGCATGTCTGATGGAACCTTGGGATGGTCCAGCTTTGTTAACTTTCACTGATGGACGTTACTGT
GGTGCTATATTGGATAGAAATGGTTTAAGACCTTGTCGTTATTACATCACTAGTGATGACAGAGTTATCTGTGCT
TCAGAGGTAGGTGTCATTCCTATCGAAAATTCATTGGTTGTTCAAAAAGGTAAACTGAAGCCAGGTGATTTATTC
CTAGTGGATACTCAATTGGGTGAAATGGTCGATACTAAAAAGTTAAAATCTCAAATCTCAAAAAGACAAGATTTT
AAGTCTTGGTTATCCAAAGTCATCAAGTTAGACGACTTGTTATCAAAAACCGCTAATTTGGTTCCTAAAGAATTT
ATATCACAGGATTCATTGTCTTTGAAAGTTCAAAGTGACCCACGTCTATTGGCCAATGGTTATACCTTCGAACAA
GTCACATTTCTGTTAACTCCAATGGCTTTAACAGGTAAAGAAGCTTTAGGTTCGATGGGTAACGATGCGCCACTG
GCTTGTTTAAATGAAAATCCTGTCTTACTTTATGATTATTTCAGACAATTGTTTGCTCAAGTGACCAATCCTCCA
ATTGACCCAATTCGTGAAGCAAATGTTATGTCGTTAGAATGTTATGTCGGACCTCAAGGCAACCTTTTGGAAATG
CATTCATCTCAATGTGATCGTTTATTATTGAAATCTCCTATTTTGCATTGGAATGAGTTCCAAGCTTTGAAAAAC
ATTGAAGCTGCTTACCCATCATGGTCTGTAGCAGAAATTGATATCACATTCGACAAGAGTGAGGGTCTATTGGGC
TATACCGACACAATTGATAAAATCACTAAGTTAGCGAGCGAAGCAATTGATGATGGTAAAAAGATCTTAATAATT
ACTGACAGGAAAATGGGTGCCAACCGTGTTTCCATCTCCTCTTTGATTGCAATTTCATGTATTCATCATCACCTA
ATCAGAAACAAGCAGCGTTCCCAAGTTGCTTTGATTTTGGAAACAGGTGAAGCCAGAGAAATTCACCATTTCTGT
GTCCTACTAGGTTATGGTTGTGATGGTGTTTATCCATACTTAGCCATGGAAACTTTGGTCAGAATGAATAGAGAA
GGTCTACTTCGTAATGTCAACAATGACAATGATACACTTGAGGAAGGGCAAATACTAGAAAATTACAAGCACGCT
ATTGATGCAGGTATCTTGAAGGTTATGTCTAAAATGGGTATCTCCACTCTAGCATCCTACAAAGGTGCTCAAATT
TTTGAAGCCCTAGGTTTAGATAACTCTATTGTTGATTTGTGTTTCACAGGTACTTCTTCCAGAATTAGAGGTGTA
ACTTTCGAGTATTTGGCTCAAGATGCCTTTTCTTTACATGAGCGTGGTTATCCATCCAGACAAACCATTAGTAAA
TCTGTTAACTTACCAGAAAGTGGTGAATACCACTTTAGGGATGGTGGTTACAAACACGTCAACGAACCAACCGCA
ATTGCTTCGTTACAAGATACTGTCAGAAACAAAAATGATGTCTCTTGGCAATTATATGTAAAGAAGGAAATGGAA
GCAATTAGAGACTGTACACTAAGAGGACTGTTAGAATTAGATTTTGAAAATTCTGTCAGTATCCCTCTAGAACAA
GTTGAACCATGGACTGAAATTGCCAGAAGATTTGCGTCAGGTGCAATGTCTTATGGTTCTATTTCTATGGAAGCT
CACTCTACATTGGCTATTGCCATGAATCGTTTAGGGGCCAAATCCAATTGTGGTGAAGGTGGTGAAGACGCAGAA
CGTTCTGCTGTTCAAGAAAACGGTGATACTATGAGATCTGCTATCAAACAAGTTGCTTCCGCTAGATTCGGTGTA
ACTTCATACTACTTGTCAGATGCTGATGAAATCCAAATTAAGATTGCTCAGGGTGCTAAGCCGGGTGAAGGTGGT
GAACTACCAGCCCACAAAGTGTCTAAGGATATCGCAAAAACCAGGCACTCCACCCCTAATGTTGGGTTAATCTCT
 
CCTCCTCCTCATCACGATATTTATTCCATTGAAGATTTGAAACAACTGATTTATGATTTGAAATGTGCTAATCCA
AGAGCGGGAATTTCTGTAAAGTTGGTTTCCGAAGTTGGTGTTGGTATTGTTGCCTCTGGTGTAGCTAAGGCTAAA
GCCGATCATATCTTAGTTTCTGGTCATGATGGTGGTACAGGTGCTGCAAGATGGACGAGTGTCAAATATGCGGGT
TTGCCATGGGAATTAGGTCTAGCTGAAACTCACCAGACTTTAGTCTTGAATGATTTAAGACGTAATGTTGTTGTC
CAAACCGATGGTCAATTGAGAACTGGGTTTGATATTGCTGTTGCAGTTTTATTAGGGGCAGAATCTTTTACCTTG
GCAACAGTTCCATTAATTGCTATGGGTTGTGTTATGTTAAGAAGATGTCACTTGAACTCTTGTGCTGTTGGTATT
GCCACACAAGATCCATATTTGAGAAGTAAGTTTAAGGGTCAGCCCGAACATGTTATCAACTTCTTCTATTACTTG
ATCCAAGATTTAAGACAAATCATGGCCAAGTTAGGATTCCGTACCATTGACGAAATGGTGGGTCATTCTGAAAAA
TTAAAGAAAAGGGACGACGTAAATGCCAAAGCCATAAATATCGATTTATCTCCTATTTTGACCCCAGCACATGTT
ATTCGTCCAGGTGTTCCAACCAAGTTCACTAAGAAACAAGACCACAAACTCCACACCCGTCTAGATAATAAGTTA
ATCGATGAGGCTGAAGTTACTTTGGATCGTGGCTTACCAGTGAATATTGACGCCTCTATAATCAATACTGATCGT
GCACTCGGTTCTACTTTATCTTACAGAGTCTCGAAGAAATTTGGTGAAGATGGTTTGCCAAAGGACACCGTTGTC
GTTAACATAGAAGGTTCAGCGGGTCAATCTTTTGGTGCTTTCCTAGCTTCTGGTATCACTTTTATCTTGAATGGT
GATGCTAATGATTATGTTGGTAAAGGTTTATCCGGTGGTATTATTGTCATTAAACCACCAAAGGATTCTAAATTC
AAGAGTGATGAAAATGTAATTGTTGGTAACACTTGTTTCTATGGTGCTACTTCTGGTACTGCATTCATTTCAGGT
AGTGCCGGTGAGCGTTTCGGTGTCAGAAACTCTGGTGCCACCATCGTTGTTGAGAGAATTAAGGGTAACAATGCC
TTTGAGTATATGACTGGTGGTCGTGCCATTGTCTTATCACAAATGGAATCCCTAAACGCCTTCTCTGGTGCTACT
GGTGGTATTGCATACTGTTTAACTTCCGATTACGACGATTTTGTTGGAAAGATTAACAAAGATACTGTTGAGTTA
GAATCATTATGTGACCCGGTCGAGATTGCGTTTGTTAAGAATTTGATCCAGGAGCATTGGAACTACACACAATCT
GATCTAGCAGCCAGGATTCTCGGTAATTTCAACCATTATTTGAAAGATTTCGTTAAAGTCATTCCAACTGATTAT
AAGAAAGTTTTGTTGAAGGAGAAAGCAGAAGCTGCCAAGGCAAAGGCTAAGGCAACTTCAGAATACTTAAAGAAG
TTTAGATCGAACCAAGAAGTTGATGACGAAGTCAATACTCTATTGATTGCTAATCAAAAAGCTAAAGAGCAAGAA
AAAAAGAAGAGTATTACTATTTCAAATAAGGCCACTTTGAAGGAGCCTAAGGTTGTTGATTTAGAAGATGCAGTT
CCAGATTCCAAACAGCTAGAGAAGAATAGCGAAAGGATTGAAAAAACACGTGGTTTTATGATCCACAAACGTCGT
CATGAGACACACAGAGATCCAAGAACCAGAGTTAATGACTGGAAAGAATTTACTAACCCTATTACCAAGAAGGAT
GCCAAATATCAAACTGCGAGATGTATGGATTGTGGTACACCATTCTGTTTATCTGATACCGGTTGTCCCCTATCT
AACATTATCCCCAAGTTTAATGAATTGTTATTCAAGAACCAATGGAAGTTGGCACTGGACAAATTGCTAGAGACA
AACAATTTCCCAGAATTCACTGGAAGAGTATGTCCAGCACCCTGTGAGGGAGCTTGTACACTAGGTATTATTGAA
GACCCAGTCGGCATAAAATCGGTTGAAAGAATTATCATTGACAATGCTTTCAAGGAAGGATGGATTAAGCCTTGT
CCACCAAGTACACGCACTGGCTTTACAGTGGGTGTCATTGGTTCTGGTCCAGCAGGTTTAGCGTGTGCTGATATG
TTGAACCGTGCCGGACATACGGTCACTGTTTATGAAAGATCCGACCGTTGTGGTGGGTTATTGATGTATGGTATT
CCAAACATGAAGTTGGATAAGGCTATAGTGCAACGTCGTATTGATCTATTGAGTGCCGAAGGTATTGACTTTGTT
ACCAACACCGAAATTGGTAAAACCATAAGCATGGATGAGCTAAAGAACAAGCACAATGCAGTAGTGTATGCTATC
GGTTCTACCATTCCACGTGACTTACCTATTAAGGGTCGTGAATTGAAGAATATTGATTTTGCCATGCAGTTGTTG
GAATCTAACACAAAAGCTTTATTGAACAAAGATCTGGAAATCATTCGTGAAAAGATCCAAGGTAAGAAAGTAATT
GTTGTCGGTGGTGGTGACACAGGTAACGATTGTTTAGGTACATCTGTAAGACACGGTGCAGCATCAGTTTTGAAT
TTCGAATTGTTGCCTGAGCCACCAGTGGAACGTGCCAAAGACAATCCATGGCCTCAATGGCCGCGTGTCATGAGA
GTGGACTACGGTCATGCTGAAGTGAAAGAGCATTATGGTAGAGACCCTCGTGAATACTGCATCTTGTCCAAGGAA
TTTATCGGTAACGATGAGGGTGAAGTCACTGCCATCAGAACTGTGCGCGTAGAATGGAAGAAGTCACAAAGTGGC
 
GTATGGCAAATGGTAGAAATTCCCAACAGTGAAGAGATCTTTGAAGCCGATATCATTTTGTTGTCTATGGGTTTC
GTGGGTCCTGAATTGATCAATGGCAACGATAACGAAGTTAAGAAGACAAGACGTGGTACGATTGCCACACTCGAC
GACTCCTCATACTCTATTGATGGAGGAAAGACTTTTGCATGTGGTGACTGTAGAAGAGGGCAATCTTTGATTGTC
TGGGCCATCCAAGAAGGTAGAAAATGTGCTGCCTCTGTCGATAAGTTCCTAATGGACGGCACTACGTATCTACCA
AGTAATGGTGGTATCGTTCAACGTGATTACAAACTATTGAAAGAATTAGCTAGTCAAGTCTAA
**SEQ ID NO: 99** is an amino acid sequence of Glutamate synthase (GLT1) originating from *Saccharomyces cerevisiae* 
MPVLKSDNFDPLEEAYEGGTIQNYNDEHHLHKSWANVIPDKRGLYDPDYEHDACGVGFVANKHGEQSHKIVTDAR
YLLVNMTHRGAVSSDGNGDGAGILLGIPHEFMKREFKLDLDLDIPEMGKYAVGNVFFKKNEKNNKKNLIKCQKIF
EDLAASFNLSVLGWRNVPVDSTILGDVALSREPTILQPLLVPLYDEKQPEFNETKFRTQLYLLRKEASLQIGLEN
WFYVCSLNNTTIVYKGQLTPAQVYNYYPDLTNAHFKSHMALVHSRFSTNTFPSWDRAQPLRWLAHNGEINTLRGN
KNWMRSREGVMNSATFKDELDKLYPIIEEGGSDSAALDNVLELLTINGTLSLPEAVMMMVPEAYHKDMDSDLKAW
YDWAACLMEPWDGPALLTFTDGRYCGAILDRNGLRPCRYYITSDDRVICASEVGVIPIENSLVVQKGKLKPGDLF
LVDTQLGEMVDTKKLKSQISKRQDFKSWLSKVIKLDDLLSKTANLVPKEFISQDSLSLKVQSDPRLLANGYTFEQ
VTFLLTPMALTGKEALGSMGNDAPLACLNENPVLLYDYFRQLFAQVTNPPIDPIREANVMSLECYVGPQGNLLEM
HSSQCDRLLLKSPILHWNEFQALKNIEAAYPSWSVAEIDITFDKSEGLLGYTDTIDKITKLASEAIDDGKKILII
TDRKMGANRVSISSLIAISCIHHHLIRNKQRSQVALILETGEAREIHHFCVLLGYGCDGVYPYLAMETLVRMNRE
GLLRNVNNDNDTLEEGQILENYKHAIDAGILKVMSKMGISTLASYKGAQIFEALGLDNSIVDLCFTGTSSRIRGV
TFEYLAQDAFSLHERGYPSRQTISKSVNLPESGEYHFRDGGYKHVNEPTAIASLQDTVRNKNDVSWQLYVKKEME
AIRDCTLRGLLELDFENSVSIPLEQVEPWTEIARRFASGAMSYGSISMEAHSTLAIAMNRLGAKSNCGEGGEDAE
RSAVQENGDTMRSAIKQVASARFGVTSYYLSDADEIQIKIAQGAKPGEGGELPAHKVSKDIAKTRHSTPNVGLIS
PPPHHDIYSIEDLKQLIYDLKCANPRAGISVKLVSEVGVGIVASGVAKAKADHILVSGHDGGTGAARWTSVKYAG
LPWELGLAETHQTLVLNDLRRNVVVQTDGQLRTGFDIAVAVLLGAESFTLATVPLIAMGCVMLRRCHLNSCAVGI
ATQDPYLRSKFKGQPEHVINFFYYLIQDLRQIMAKLGFRTIDEMVGHSEKLKKRDDVNAKAINIDLSPILTPAHV
IRPGVPTKFTKKQDHKLHTRLDNKLIDEAEVTLDRGLPVNIDASIINTDRALGSTLSYRVSKKFGEDGLPKDTVV
VNIEGSAGQSFGAFLASGITFILNGDANDYVGKGLSGGIIVIKPPKDSKFKSDENVIVGNTCFYGATSGTAFISG
SAGERFGVRNSGATIVVERIKGNNAFEYMTGGRAIVLSQMESLNAFSGATGGIAYCLTSDYDDFVGKINKDTVEL
ESLCDPVEIAFVKNLIQEHWNYTQSDLAARILGNFNHYLKDFVKVIPTDYKKVLLKEKAEAAKAKAKATSEYLKK
FRSNQEVDDEVNTLLIANQKAKEQEKKKSITISNKATLKEPKVVDLEDAVPDSKQLEKNSERIEKTRGFMIHKRR
HETHRDPRTRVNDWKEFTNPITKKDAKYQTARCMDCGTPFCLSDTGCPLSNIIPKFNELLFKNQWKLALDKLLET
NNFPEFTGRVCPAPCEGACTLGIIEDPVGIKSVERIIIDNAFKEGWIKPCPPSTRTGFTVGVIGSGPAGLACADM
LNRAGHTVTVYERSDRCGGLLMYGIPNMKLDKAIVQRRIDLLSAEGIDFVTNTEIGKTISMDELKNKHNAVVYAI
GSTIPRDLPIKGRELKNIDFAMQLLESNTKALLNKDLEIIREKIQGKKVIVVGGGDTGNDCLGTSVRHGAASVLN
FELLPEPPVERAKDNPWPQWPRVMRVDYGHAEVKEHYGRDPREYCILSKEFIGNDEGEVTAIRTVRVEWKKSQSG
VWQMVEIPNSEEIFEADIILLSMGFVGPELINGNDNEVKKTRRGTIATLDDSSYSIDGGKTFACGDCRRGQSLIV
WAIQEGRKCAASVDKFLMDGTTYLPSNGGIVQRDYKLLKELASQV
**SEQ ID NO: 100** is the nucleic acid sequence of terminator tTDH3 
GTGAATTTACTTTAAATCTTGCATTTAAATAAATTTTCTTTTTATAGCTTTATGACTTAGTTTCAATTTATATAC
TATTTTAATGACATTTTCGATTCATTGATTGAAAGCTTTGTGTTTTTTCTTGATGCGCTATTGCATTGTTCTTGT
CTTTTTCGCCACATGTAATATCTGTAGTAGATACCTGATACATTGTGGATGCTGAGTGAAATTTTAGTTAATAAT
GGAGGCGCTCTTAATAATTTTGGGGATATTGGCTTTTTTTTTTAAAGTTTACAAATGAATTTTTTCCGCCAGGAT
**SEQ ID NO: 101** is a reencoded nucleic acid sequence of hyaluronan synthase (HASA) originating from *Chlorella virus CviKI* 
ATGCTTTTCAGTTGTTTGTCCTTCGTGATCTGCCATCTTAGTTTTATTTTGCGTTATTACAAGAGGCTTCTAAAA
GTGACGATGGGAAAGAACATTATAATAATGGTGAGTTGGTACACAATAATAACTTCCAACATCATCGCCGTCGGT
GGGGCGTCTCTGATTTTGGCCCCAGCCATCACTGGATACATCTTGCACTGGAACATCGCCTTGTCAACCATCTGG
GGAGTTTCCGCTTATGGGATATTCGTGTTCGGATTCTTCCTGGCTCAGGTTTTATTTAGTGAGCTAAATCGTAAA
AGACTAAGAAAGTGGATTTCTCTAAGACCAAAGGGTTGGAACGACGTACGTCTGGCTGTCATCATAGCTGGTTAC
AGGGAAGACCCGTACATGTTTCAGAAATGCCTTGAGTCAGTAAGGGACTCTGACTATGGTAATGTCGCTCGTCTT
ATCTGTGTAATTGACGGAGATGAGGACGACGACATGAAGATGGCAGCCGTTTATAAGGCGATATATAATGACAAT
ATCAAGAAGCCAGAGTTTGTGTTATGTGAGAGCGATGATAAGGAAGGCGAGAGAATTGACTCAGATTTTAGCAGG
GACATCTGCGTCTTACAACCACACCGTGGCAAAAGGGAGTGCTTGTATACCGGCTTTCAGCTGGCCAAAATGGAC
CCGTCGGTAAACGCCGTTGTTCTTATTGACTCTGACACGGTCTTGGAAAAGGACGCAATCTTGGAAGTCGTGTAT
CCATTGGCCTGTGACCCGGAAATTCAAGCTGTCGCAGGAGAGTGCAAAATATGGAACACAGATACACTTTTGTCG
TTGTTGGTGGCTTGGAGATATTATTCAGCCTTTTGCGTGGAGAGATCCGCCCAATCATTCTTTAGGACAGTCCAG
TGTGTAGGTGGACCCTTGGGCGCCTACAAGATAGATATCATCAAAGAGATCAAAGATCCCTGGATCTCCCAACGT
TTCTTAGGTCAGAAGTGCACCTACGGTGACGACCGTAGGTTAACAAATGAAATCTTAATGAGGGGTAAGAAGGTC
GTCTTCACTCCTTTCGCAGTTGGCTGGAGTGACTCACCAACCAACGTGTTCAGGTACATCGTACAACAGACTAGG
TGGAGTAAATCATGGTGCCGTGAGATATGGTACACTTTGTTTGCTGCGTGGAAGCACGGACTAAGCGGAATTTGG
TTGGCATTTGAGTGCCTTTATCAGATTACGTACTTCTTCCTAGTCATATATTTATTTTCGAGACTAGCCGTTGAA
GCTGACCCAAGAGCACAGACCGCAACAGTAATCGTGTCTACCACGGTAGCCCTGATTAAGTGTGGTTACTTCTCT
TTTCGTGCGAAAGATATTCGTGCTTTCTACTTCGTCCTGTACACATTTGTCTACTTCTTCTGTATGATCCCAGCA
AGGGTGACAGCCATGATGACACTTTGGGATATCGGCTGGGGCACGCGTGGCGGAAACGAGAAACCAAGTGTCGGC
ACTAGGGTCGCTCTGTGGGCTAAACAGTATTTGATAGCATACATGTGGTGGGCAGCAGTTGTTGGCGCTGGGGTA
TACTCCATTGTTCATAATTGGATGTTTGACTGGAACTCACTATCCTATAGGTTCGCACTTGTAGGTATATGCTCT
TACATCGTATTCATCACAATCGTTCTAGTCATCTATTTCACAGGAAAGATTACCACATGGAATTTCACCAAATTA
CAGAAGGAGTTAATCAAGGATAGGGTCTTATACGATGCTTCCACTAACGCCCAGACTGTATAA
**SEQ ID NO: 102** is a reencoded nucleic acid sequence of hyaluronan synthase (HASA) originating from *Chlorella virus IL-5-2s1* 
ATGGCTGCTTCATTCTATGTTATCTTCTTGTTCGTAATATGCCACCTGTCCTTCGTCTTATATCGTCAAGTTCTA

TACATAATTTTGGGTAGATACTGGAGTGTTAACAAGCTGACAATGATCTCTTGGTATACCATTATATCATCTAAT

CTAATCGCAATCGGTGGGGCTTCCCTTATTTTGGCACCGGCCATAACCGGCTACATTTTGCATTGGAACATAGCT
 
CTAAGCACAATTTGGGGTGTGTCAGCCTATGGCATTTTCGTGTTCGGGTTCTTTCTTGCTCAGGTTTTGTTCAGT
GAGCTTAACAGGAAGAGGTTGAGAAAATGGATCTCTTTGAGACCAGATAACTGGAACGCGGTGAGAGTTGCCGTG
ATAATCGCTGGTTACCGTGAAGACCCATACATGTTTCAAAAGTGTTTGGAATCGGTGAGAGACTCCGATTATGGT
AACATTGCGAGGCTTATATGCGTTATAGATGGGGACGAAGATGACGACATGAAGATGGCAGATGTATACAAGGCT
ATTTACAATGACAATATAAAGAAGCCGGAAATTATCCTATGTGAGTCCGACGACAAAGAGGGAGAAAGGATAGAC
AGTGATTTTAGCCGTGATATTTGCGTGCTTCAGCCTCACAGAGGTAAGCGTGAATGTTTATATACTGGCTTTCAG
TTAGCTAAAATGGACCCCTCTGTACACGCGGTTGTGCTTATTGACTCGGATACAGTTTTGGAGAAGGATGCCATC
CTGGAAGTGGTCTACCCACTTGCTTGCGACCATGAGATTCAAGCAGTCGCAGGTGAATGCAAGATATGGAATACG
GACACCTTATTATCCATATTAGTCGCCTGGCGTTACTACTCAGCTTTCTGCGTTGAGCGTAGCGCTCAGTCCTTC
TTCCGTACTGTGCAATGCGTCGGCGGTCCGTTAGGGGCATACAAAATAGACATAATTAAAGAGATAAAGGAGCCG
TGGATTTCACAAAGATTTCTGGGTCAGAAGTGTACGTACGGAGATGACAGAAGATTAACTAACGAAGTGTTGATG
CGTGGAAAGAAAGTAGTCTTCACACCTTTTGCGGTAGGATGGAGCGACTCGCCTACTAATGTCTTCCGTTATATT
GTGCAGCAGACGAGGTGGAGTAAGAGCTGGTGCAGGGAGATTTGGTATACACTGTTCGCGGCGTGGAAGCATGGG
TTATCAGGCATTTGGTTGGCATTTGAGTGCTTGTATCAGATCACATACTTCTTTCTAGTGATATATCTGTTCAGT
AGACTAGCAGTCGAGGCTGACCCGAGAGCGCAAACTGCAACGGTTATAGTCAGCACAATGGTTTCACTAATCAAG
TGTGGATATTTCTCATTCCGTGCTAAGGACATTAGAGCGTTTTATTTCGTACTATATACTTTTGTGTACTTCTTT
TGCATGATCCCAGCAAGAGTCACTGCTATGATGACCTTGTGGGATATTGGGTGGGGTACTAGGGGTGGAAACGTG
AAACCATCGATCGGAACTAGGATCTCTTTGTGGGCTAAGCAGTACTTAATCGCGTACATGTGGTGGGCAGCTGTG
ATCGCCGCAGGAGTCTATAGTATAATACATAATTGGATGTTTGATTGGAACTCCTTGTCCTATCGTTTCGCGTTA
ATAGGCATCTGCTCGTATATCGTATTCATTGCCATTGTTATAGTGATATACTTCACAGGTAAGATCACCACTTGG
AATTTTACCAAGCTACAGAAAGAGTTAATTGAAGATAGAGTCTTGCATGATGCCGATGTGGACATACAGAACGTA
TAA
**SEQ ID NO: 103** is a reencoded nucleic acid sequence of hyaluronan synthase (HASA) originating from *Chlorella virus CZ-2* 
ATGACATCTTGGAGAACGATTGTGTCAGCTAACCTTTTCGCAGTTGGTGGGGCTCTTCTAATGTTAGCCCCAGCT
ATCGTGGGCTATGTATTTCAGTGGAATATAGGCGTGTCCGCAGTCTGGGGCATCTCCGTATACGGCGTCTTTGTG
TTGGGATTTTATATCGCTCAAATCGTCTTCTCTGAGTTCAACAGGATGAGATTATCCGATTGGATAAGTCTTAGA
CCAGACAACTGGAACGCCACAAGGGTTGCGGTCATTATAGCTGGATACAGAGAAGATCCTTTTATGTTCAAGAAG
TGCTTAGAGAGTGTGAGGGATTCGGAATACGGCAACGTAGCAAGGCTTATATGCGTAATTGACGGAGACGAAGAA
GAGGACCTAAAGATGGCTGAGATTTACAAGCAGGTTTACAATGACAACGTGAAGAAACCAGGTGTAGTGTTATGC
GAATCGGAGAACAAGAATGGATCGACTATAGATTCGGACGTATCTAAGAATATATGCATACTGCAACCCCACCGT
GGGAAACGTGAGAGCCTATATACCGGTTTCCAATTGGCAAGCATGGACCCTTCCGTACATGCGGTTGTTCTTATA
GATTCCGACACTGTATTAGAGAAGAATGCTATTCTTGAGGTCGTATACCCTCTATCTTGTGACCCCAACATTAAG
GCCGTCGCCGGGGAGTGCAAGATATGGAATACAGACACCATCTTGTCCATGCTTGTCTCTTGGAGATACTTCTCT
GCCTTCAATGTCGAGCGTGGAGCCCAGTCATTATGGAAGACTGTGCAATGCGTAGGCGGGCCACTTGGCGCATAC
ACTATCGACATCATAAATGAAATTAAAGACCCGTGGATTACCCAGACATTCCTGGGCAACAAGTGCACTTATGGC
GACGATAGACGTCTTACCAATGAGGTTTTAATGAGGGGCAAGAAGATTGTATACACTCCGTTTGCCGTCGGTTGG
 
AGCGACAGTCCGACGAATGTCATGAGGTACATTGTGCAGCAAACTAGATGGAGTAAGTCATGGTGCAGAGAGATA
TGGTACACGTTAGGCAGTGCCTGGAAACATGGATTCTCCGGCATATACCTTGCGTTTGAGTGTATGTACCAGATA
ATGTACTTCTTCTTAGTCATGTACCTGTTTTCGTATATCGCAATAAAGGCAGACATCCGTGCTCAGACTGCAACT
GTGTTGGTTTCTACTCTAGTCACCATAATAAAGAGCAGTTACCTAGCGCTGAGGGCCAAGAACCTGAAGGCTTTC
TATTTCGTTCTGTATACCTATGTCTATTTCTTTTGCATGATACCTGCTAGGATTACCGCTATGTTTACAATGTTT
GATATAGCTTGGGGTACACGTGGTGGTAATGCGAAAATGACAATCGGGGCTAGAGTTTGGCTGTGGGCGAAGCAG
TTCTTAATAACTTATATGTGGTGGGCAGGAGTACTAGCGGCGGGAGTTTACAGTATAGTTGACAATTGGTACTTC
GATTGGGCTGATATACAGTACAGGTTTGCATTGGTAGGTATTTGCTCGTATTTGGTCTTTGTCAGTATTGTACTG
GTAATCTACTTGATCGGTAAGATAACAACGTGGAACTACACACCGCTACAGAAGGAACTTATTGAGGAGAGATAC
TTGCATAACGCCTCTGAGAATGCCCCGGAAGTTTAA
**SEQ ID NO: 104** is a reencoded nucleic acid sequence of hyaluronan synthase (HASA) originating from *Chlorella virus CVG-1* 
ATGACTTCTTGGCGTACAATTGTTTCTGCTAACTTGTTCGCAGTTGGTGGCGCCTTGTTGATGTTAGCTCCCGCG
ATAGCCGGTTATGTATTTAAATGGAACATAGGCGTTTCTGCCGTTTGGGGTATTTCAGTGTACGGGGTCTTCGTG
CTTGGTTTCTATATTGCACAGGTAGTCTTCAGCGAGTTTAATAGGATGCAGCTGTCAGACTGGATCTCATTGAGA
CCCGACAACTGGAACGCCACCAGAGTAGCGGTAATTATTGCTGGTTACAGGGAAGACCCCTTTATGTTCAAGAAG
TGCCTAGAATCTGTGAGAGACTCAGAATACGGGAACATAGCGAGGCTAATCTGTGTTATCGACGGCGATGAAGAG
GAAGACCTTAAGATGGCTGAGATATACAAGCAAGTATACAACGACAACGTCAAGACCCCCGGTGTTGTGCTATGC
GAGAATGAAAATAAGAATGGTAGCACCATTGACCCAGATTTTAGTAAGAACATCTGCATTCTGCAACCACACAGG
GGCAAACGTGAATCTTTGTACACCGGATTCCAGATGGCTGGCATGGACCCATCAGTGCACGCAGTTGTACTTATA
GACAGTGATACTGTTCTTGAAAAGAACGCTATCCTAGAGGTTGTGTACCCTCTATCTTGTGATCCAAATATCAAA
GCTGTCGCGGGAGAGTGTAAAATCTGGAACACAGATACCATTTTGTCTATGCTAGTTTCATGGCGTTATTTCTCG
GCTTTCAACGTGGAGAGAGGCGCACAGTCCCTTTGGAAAACAGTCCAGTGTGTCGGCGGGCCTTTGGGAGCATAC
ACTATAGACATCATAAACGAGATAAAAGACCCCTGGATTACTCAGACATTCTTAGGAAACAAATGTACGTATGGT
GACGACAGAAGGTTAACCAATGAAGTGCTTATGAGGGGAAAGAAGATTGTCTATACTCCCTTTGCTGTCGGTTGG
TCCGATTCGCCCACAAACGTTATGAGGTACATCGTCCAGCAGACAAGGTGGTCTAAGAGCTGGTGCCGTGAGATT
TGGTACACCTTGGGATCAGCATGGAAACATGGTTTCTCCGGAATCTACTTAGCGTTCGAGTGCATGTACCAGATT
ATGTATTTCTTTATTGTGATGTATTTGTTCAGTTACATTGCCATTAAAGCCAACATCAGGGCACAAGCAGCCACG
GTATTGGTCAGTACGCTTGTCGCAGTAATCAAATCTAGTTATTTGGCATTAAGAGCGAAGAACCTAAAAGCTTTG
TATTTCGTCCTGTATACCTACGTCTACTTCTTCTGCATGATTCCAGCTAGAATAACAGCGATGTTCACGATGTTC
GATATTGCATGGGGTACTAGAGGCGGTAACGCAAAGATGACGATCGGTGCTAGAGTCTGGTTGTGGGCTAAACAA
TTCTTAATTACATACATGTGGTGGGTGGGAGTTTTGGCCGCTGGGGTCTACAGTATAGTCGATAACTGGTACTTC
GACTGGGCGGACATCCAGTACAGATTCGCGCTAGTGGGGATTTGCAGTTACTTAGGTTTCGTTTCCATAATGCTG
GTATTCTATCTGATTGGTAAAATAACTACGTGGAATTACACTCCTCTACAAAAGGAGCTGATTAAAGAGAGGTTA
CACGCCGCTAATGCAACAGAGGTCTAA
**SEQ ID NO: 105** is an amino acid sequence of hyaluronan synthase (HASA) originating from *Chlorella virus CviKI* 
MLFSCLSFVICHLSFILRYYKRLLKVTMGKNIIIMVSWYTIITSNIIAVGGASLILAPAITGYILHWNIALSTIW
GVSAYGIFVFGFFLAQVLFSELNRKRLRKWISLRPKGWNDVRLAVIIAGYREDPYMFQKCLESVRDSDYGNVARL
ICVIDGDEDDDMKMAAVYKAIYNDNIKKPEFVLCESDDKEGERIDSDFSRDICVLQPHRGKRECLYTGFQLAKMD
PSVNAVVLIDSDTVLEKDAILEVVYPLACDPEIQAVAGECKIWNTDTLLSLLVAWRYYSAFCVERSAQSFFRTVQ
CVGGPLGAYKIDIIKEIKDPWISQRFLGQKCTYGDDRRLTNEILMRGKKVVFTPFAVGWSDSPTNVFRYIVQQTR
WSKSWCREIWYTLFAAWKHGLSGIWLAFECLYQITYFFLVIYLFSRLAVEADPRAQTATVIVSTTVALIKCGYFS
FRAKDIRAFYFVLYTFVYFFCMIPARVTAMMTLWDIGWGTRGGNEKPSVGTRVALWAKQYLIAYMWWAAVVGAGV
YSIVHNWMFDWNSLSYRFALVGICSYIVFITIVLVIYFTGKITTWNFTKLQKELIKDRVLYDASTNAQTV
**SEQ ID NO: 106** is an amino acid sequence of hyaluronan synthase (HASA) originating from *Chlorella virus IL-5-2s1* 
MAASFYVIFLFVICHLSFVLYRQVLYIILGRYWSVNKLTMISWYTIISSNLIAIGGASLILAPAITGYILHWNIA
LSTIWGVSAYGIFVFGFFLAQVLFSELNRKRLRKWISLRPDNWNAVRVAVIIAGYREDPYMFQKCLESVRDSDYG
NIARLICVIDGDEDDDMKMADVYKAIYNDNIKKPEIILCESDDKEGERIDSDFSRDICVLQPHRGKRECLYTGFQ
LAKMDPSVHAVVLIDSDTVLEKDAILEVVYPLACDHEIQAVAGECKIWNTDTLLSILVAWRYYSAFCVERSAQSF
FRTVQCVGGPLGAYKIDIIKEIKEPWISQRFLGQKCTYGDDRRLTNEVLMRGKKVVFTPFAVGWSDSPTNVFRYI
VQQTRWSKSWCREIWYTLFAAWKHGLSGIWLAFECLYQITYFFLVIYLFSRLAVEADPRAQTATVIVSTMVSLIK
CGYFSFRAKDIRAFYFVLYTFVYFFCMIPARVTAMMTLWDIGWGTRGGNVKPSIGTRISLWAKQYLIAYMWWAAV
IAAGVYSIIHNWMFDWNSLSYRFALIGICSYIVFIAIVIVIYFTGKITTWNFTKLQKELIEDRVLHDADVDIQNV
**SEQ ID NO: 107** is an amino acid sequence of hyaluronan synthase (HASA) originating from *Chlorella virus CZ-2* 
MTSWRTIVSANLFAVGGALLMLAPAIVGYVFQWNIGVSAVWGISVYGVFVLGFYIAQIVFSEFNRMRLSDWISLR
PDNWNATRVAVIIAGYREDPFMFKKCLESVRDSEYGNVARLICVIDGDEEEDLKMAEIYKQVYNDNVKKPGVVLC
ESENKNGSTIDSDVSKNICILQPHRGKRESLYTGFQLASMDPSVHAVVLIDSDTVLEKNAILEVVYPLSCDPNIK
AVAGECKIWNTDTILSMLVSWRYFSAFNVERGAQSLWKTVQCVGGPLGAYTIDIINEIKDPWITQTFLGNKCTYG
DDRRLTNEVLMRGKKIVYTPFAVGWSDSPTNVMRYIVQQTRWSKSWCREIWYTLGSAWKHGFSGIYLAFECMYQI
MYFFLVMYLFSYIAIKADIRAQTATVLVSTLVTIIKSSYLALRAKNLKAFYFVLYTYVYFFCMIPARITAMFTMF
DIAWGTRGGNAKMTIGARVWLWAKQFLITYMWWAGVLAAGVYSIVDNWYFDWADIQYRFALVGICSYLVFVSIVL
VIYLIGKITTWNYTPLQKELIEERYLHNASENAPEV
**SEQ ID NO: 108** is an amino acid sequence of hyaluronan synthase (HASA) originating from *Chlorella virus CVG-1*

MTSWRTIVSANLFAVGGALLMLAPAIAGYVFKWNIGVSAVWGISVYGVFLGFYIAQVVFSEFNRMQLSDWISLR

PDNWNATRVAVIIAGYREDPFMFKKCLESVRDSEYGNIARLICVIDGDEEEDLKMAEIYKQVYNDNVKTPGVVLC

ENENKNGSTIDPDFSKNICILQPHRGKRESLYTGFQMAGMDPSVHAVVLIDSDTVLEKNAILEVVYPLSCDPNIK
AVAGECKIWNTDTILSMLVSWRYFSAFNVERGAQSLWKTVQCVGGPLGAYTIDIINEIKDPWITQTFLGNKCTYG
DDRRLTNEVLMRGKKIVYTPFAVGWSDSPTNVMRYIVQQTRWSKSWCREIWYTLGSAWKHGFSGIYLAFECMYQI
MYFFIVMYLFSYIAIKANIRAQAATVLVSTLVAVIKSSYLALRAKNLKALYFVLYTYVVYFFCMIPARITAMFTMF
DIAWGTRGGNAKMTIGARVWLWAKQFLITYMWWVGVLAAGVYSIVDNWYFDWADIQYRFALVGICSYLGFVSIML
VFYLIGKITTWNYTPLQKELIKERLHAANATEV

## Claims

1. A recombinant yeast cell producing hyaluronic acid (HA) wherein the recombinant cell comprises:
(a) one or more recombinant nucleic acids encoding a polypeptide having hyaluronan synthase activity;
(b) one or more recombinant nucleic acids encoding a polypeptide having UDP-Glucose dehydrogenase (UDP-GlcDH or HASB) activity;
(c) one or more recombinant nucleic acids encoding a polypeptide having hyaluronidase activity wherein the polypeptide having hyaluronidase activity comprises a secretion signal so that hyaluronic acid, in particular of a desired molecular weight (HAMW) is produced by the recombinant yeast cell, and
(d)
(i) one or more recombinant nucleic acids encoding a polypeptide having a glutamine synthetase (GLN1) activity; and/or
(ii) one or more disrupted endogeneous nucleic acids encoding a glutamate synthase (GLT1);
wherein said recombinant yeast cell belongs to the *Saccharomyces* genus, or to the *Candida* genus, or to the *Kluyveromyces* genus, or to the *Ogataea* genus, or to the *Yarrowia* genus, or to the *Debaryomyces* genus, or to the *Ashbya* genus.

2. A recombinant host cell producing hyaluronic acid (HA) wherein the recombinant host cell comprises:
(a) one or more recombinant nucleic acids encoding a polypeptide having hyaluronan synthase activity;
(b) one or more recombinant nucleic acids encoding a polypeptide having UDP-Glucose dehydrogenase (UDP-GlcDH or HASB) activity;
(c) one or more recombinant nucleic acids encoding a polypeptide having hyaluronidase activity wherein the polypeptide having hyaluronidase activity comprises a secretion signal and an anchoring signal so that hyaluronic acid, in particular of a desired molecular weight (HAMW) is produced by the host cell; and
(d)
(i) one or more recombinant nucleic acids encoding a polypeptide having a glutamine synthetase (GLN1) activity; and/or
(ii) one or more disrupted endogeneous nucleic acids encoding a glutamate synthase (GLT1).

3. The recombinant cell according to claim 1 or 2, wherein the molecular weight of the HA is in the range of less than 50kDa, preferably in the range of about 20kDa to about 50kDa.

4. The recombinant cell according to claim 1 or 2, wherein the molecular weight of the HA is in the range of greater than 50kDa, preferably in the range of about 50kDa to about 250kDa.

5. The recombinant cell according to claim 1 or 2, wherein the molecular weight of the HA is in the range of greater than 100kDa, preferably in the range of about 100kDa to about 1500kDa.

6. The recombinant cell according to any one of claims 1 to 5, wherein the nucleic acid encoding a polypeptide having a glutamine synthetase activity is obtained or derived from *Saccharomyces cerevisiae.*

7. The recombinant cell according to any one of claims 1 to 6, wherein the nucleic acid encoding a polypeptide having hyaluronidase activity is obtained or derived from at least one of *Cupiennius salei, Loxosceles intermedia, Hirudo nipponia, Bothrops atrox* or *Tityus serrulatus.*

8. The recombinant cell according to any one of claims 1 to 7, wherein the nucleic acid encoding a polypeptide having hyaluronan synthase activity is obtained or derived from *Streptococcus zooepidemicus, Chlorella virus PBCV1, Chlorella virus CviKl, Chlorella virus IL-5-2s1, Chlorella virus CZ-2, Chlorella virus CVG-1, Xenopus laevis* or *Pasteurella multocida,* and is in particular obtained or derived from at least one of *Streptococcus zooepidemicus, Chlorella virus PBCV1, Chlorella virus CviKl, Chlorella virus IL-5-2s1, Chlorella virus CZ-2, Chlorella virus CVG-1* or *Xenopus laevis.*

9. The recombinant cell according to any one of claims 1 to 8, wherein the nucleic acid encoding a polypeptide having UDP-Glucose dehydrogenase activity is obtained or derived from at least one of *Arabidopsis thaliana, Chlorella virus PBCV1* or *Streptococcus zooepidemicus,* and in particular from *Arabidopsis thaliana* or *Chlorella virus PBCV1.*

10. The recombinant cell according to any one of claims 1 to 9, wherein the recombinant cell comprises a recombinant nucleic acid encoding one or more of:
(i) a polypeptide having glutamine-fructose-6-phosphate amidotransferase (GFA1) activity; and/or
(ii) a polypeptide having UDP-N-acetylglucosamine pyrophosphorylase (QRI1) activity.

11. The recombinant cell according to any one of claims 1 to 10, wherein the recombinant cell comprises at least one recombinant nucleic acid encoding one or more of:
(i) a polypeptide having Phosphoglucomutase-1 (PGM1) activity; and/or
(ii) a polypeptide having UTP-glucose-1-phosphate uridylyltransferase (UGP1) activity; and/or
(iii) a polypeptide having Glucosamine-6-phosphate N-acetyltransferase (GNA1) activity; and/or
(iv) a polypeptide having phosphoacetylglucosamine mutase (PCM1) activity.

12. The recombinant yeast cell according to any one of claims 1 and 3 to 11, wherein the recombinant yeast cell is selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens, Ogataea polymorpha, Yarrowia lypolytica, Debaryomyces hansenii,* and *Ashbya gossypii,* and is preferably *Saccharomyces cerevisiae.*

13. The recombinant host cell according to any one of claims 2 to 11 wherein the recombinant host cell belongs to the *Saccharomycetales* order, and is in particular selected from the group consisting of *Saccharomyces cerevisiae, Saccharomyces boulardii, Saccharomyces bayanus, Saccharomyces paradoxus, Saccharomyces mikatae, Saccharomyces castelli, Candida albicans, Candida glabrata, Candida tropicalis, Kluyveromyces lactis, Kluyveromyces marxianus, Kluyveromyces polysporus, Kluyveromyces thermotolerens, Ogataea polymorpha, Yarrowia lypolytica, Debaryomyces hansenii,* and *Ashbya gossypii,* and is preferably *Saccharomyces cerevisiae.*

14. A method of producing hyaluronic acid (HA) of a desired molecular weight (HAMW) comprising:
(a) cultivating a recombinant cell as defined in any one of claims 1 to 13 in a cultivation medium for a time sufficient to produce hyaluronic acid (HA) of the desired molecular weight; and
(b) optionally isolating or recovering the hyaluronic acid (HA) from the recombinant cell and/or from the cultivation medium.

15. The method according to claim 14, wherein the HA has a molecular weight of from about 20kDa to about 50kDA, and preferably from about 20 kDa to about 30 kDa.

16. The method according to claim 15, wherein the HA has a molecular weight of from about 30 kDa to about 50 kDa.

17. The method according to claim 14, wherein the molecular weight of the HA is of from about 50 kDa to about 150 kDa.

18. The method according to claim 14, wherein the molecular weight of the HA is of from about 150 kDa to about 1500 kDa.

19. The method according to any one of claims 14 to 18, wherein the recombinant cell comprises at least one recombinant nucleic acid encoding one or more of:
(i) a polypeptide having glutamine-fructose-6-phosphate amidotransferase (GFA1) activity; and/or
(ii) a polypeptide having UDP-N-acetylglucosamine pyrophosphorylase (QRI1) activity.

20. The method according to any one of claims 14 to 19, wherein the recombinant cell comprises at least one recombinant nucleic acid encoding one or more of:
(i) a polypeptide having Phosphoglucomutase-1 (PGM1) activity;
(ii) a polypeptide having UTP-glucose-1-phosphate uridylyltransferase (UGP1) activity;
(iii) a polypeptide having Glucosamine-6-phosphate N-acetyltransferase (GNA1) activity; and/or
(iv) a polypeptide having phosphoacetylglucosamine mutase (PCM1) activity.

21. The method according to any one of claims 14 to 20, wherein the recombinant cell is a member of the genus *Saccharomyces,* and in particular is *Saccharomyces cerevisiae.*

22. The method according according to any one of claims 14 to 21, wherein the time sufficient to produce hyaluronic acid (HA) of the desired molecular weight is a period of from about 35 hours to about 50 hours, preferably from about 40 hours to about 50 hours, preferably about 48 hours.

23. The method according according to any one of claims 14 to 22, wherein the molecular weight of the hyaluronic acid is controlled by regulating the pH of the cultivation medium.

24. The method according to any one of claims 14 to 23, wherein the method is carried out on an industrial scale, preferably where the cultivation medium is at least about 100L, more preferably in the range of about 1000L to about 3000L, even more preferably about 10,000L, even more preferably about 100,000L, or even about 250,000L.

25. Hyaluronic acid (HA) obtainable from a recombinant cell of any one of claims 1 to 11 or from the method according to any one of claims 14 to 24.

26. A cultivation medium comprising the HA according to claim 25.

27. A composition comprising the Hyaluronic acid (HA) according to claim 25.

28. An industrial product or a consumer product or a consumable comprising (i) the HA according to claim 25, (ii) the cultivation medium of claim 26 or (iii) the composition of claim 27.

29. The industrial product or the consumer product or the consumable of claim 28, wherein the industrial product or the consumer product or the consumable is a cosmetic product, a flavour product, a fragrance product, a food product, a food, a beverage, a texturant, a pharmaceutical composition, a dietary supplement, a nutraceutical, a cleaning product and/or a dental and/or an oral hygiene composition.

30. Use of a recombinant cell as defined in any one of claims 1 to 13 for the production of hyaluronic acid (HA) having a molecular weight in the range of from about 20kDa to about 50kDa or from about 50kDa to about 1000 kDa.

31. A method for producing hyaluronic acid comprising the steps of:
(a) culturing a recombinant yeast as defined in any one of claims 1 and 3 to 12 in a culture medium; and
(b) recovering the hyaluronic acid from said culture medium,
wherein the hyaluronic acid recovered in step (b) has a molecular weight controlled through the selection of:
- the nature and origin of the nucleic acid encoding the hyaluronidase of the recombinant yeast,
- the nature and origin of the promoter controlling the expression of the nucleic acid encoding the hyaluronidase(s) of the recombinant yeast,
- the presence of an anchoring and/or of a secretion signal associated to the encoded hyaluronidase(s) of the recombinant yeast,
- the pH of the culture medium during the step of culturing the recombinant yeast, and/or
- the duration of the culturing of the recombinant yeast.
